# EUROPEAN PATENT APPLICATION

(11) **EP 2 484 668 A1**
(43) Date of publication of application: **08.08.2012**
(21) Application number: 10820625.1
(22) Date of filing: 30.09.2010
(51) Int. Cl.: C07D 231/56, A61K 31/416, A61K 31/422, A61K 31/427, A61P 13/10, A61P 43/00, C07D 401/12, C07D 409/12, C07D 413/12, C07D 417/12

(54) **INDAZOLE ANALOGUE**

(30) Priority: 30.09.2009 US 247083 P
(71) Applicant: Asahi Kasei Pharma Corporation, Tokyo 101-8101 (JP)
(72) Inventor: WADA Yasuhiro, Tokyo, 1018101 (JP); MORIMOTO Akifumi, Tokyo, 1018101 (JP)
(74) Representative: Forstmeyer, Dietmar
(86) International application number: PCT/JP2010/067048
(87) International publication number: WO 2011/040510

(57) **Abstract**

[Objective] To provide a drug that selectively stimulates the β3-adrenergic receptors, particularly a drug capable of preferentially stimulating the β3-adrenergic receptors over the α1-adrenergic receptors. This drug can be used in the treatment and prevention of diabetes, obesity, hyperlipidemia, depression, cholelithiasis, diseases caused by biliary hyperkinesia, diseases caused by hyperfunction of the gastrointestinal tract, interstitial cystitis, overactive bladder or urinary incontinence, diseases associated with decreased lacrimation, and the like.

[Solution] Indazole analogs represented by the general formula (I) or a salt thereof. Drugs that contains these indazole analogs or a salt thereof as the active ingredient.

## Description

### TECHNICAL FIELD

The present invention relates to an indazole analog that acts to stimulate the β3-adrenergic receptors, a drug composition containing it, and uses thereof.

### BACKGROUND ART

Norepinephrine and epinephrine are known to exert a variety of effects on nerves, smooth muscles, and the like as neurotransmitters and hormones in the body. The adrenergic receptors that respond by binding to these neurotransmitters/hormones are known to be important target molecules of drugs in various types of treatment.

The adrenergic receptors belong to the G protein-coupled receptor family and are classified into three subfamilies: α1-, α2-, and β-adrenergic receptors. In short, the adrenergic receptor subfamilies act by binding with both norepinephrine and epinephrine, but are known to utilize different intracellular signaling pathways thereafter. The main triggers have been suggested to be an increase in calcium ion in the α1-adrenergic receptors, inhibition of adenylyl cyclase in the α2-adrenergic receptors, and stimulation of adenylyl cyclase in the β-adrenergic receptors to (for example, see Non-patent Reference 1).

Therefore, the physiological effects related to the activation of the above subfamilies also differ. For example, the β-adrenergic receptor subfamily is further classified into three subtypes: β1, β2, and β3. A β1-adrenergic receptor stimulating effect causes an increase in heart rate, and a β2-adrenergic receptor stimulating effect induces relaxation of smooth muscle tissues, and triggers a decline in blood pressure, especially when the vascular smooth muscles are relaxed.

β3-Adrenergic receptors are reported to be present in the adipose tissue, brain, gall bladder, prostate gland, intestine, and the like. A β3-adrenergic receptor stimulating effect is therefore known to be useful in the prevention and treatment of diabetes, obesity, hyperlipidemia, depression, cholelithiasis, diseases caused by biliary hyperkinesia, diseases caused by hyperfunction of the gastrointestinal tract, diseases associated with decreased lacrimation, and the like (for example, see Non-patent References 2-9 and Patent References 1 and 2).

β3-Adrenergic receptors are also expressed in bladder smooth muscle, and stimulation of the β3-adrenergic receptors has also been shown to relax bladder smooth muscle (for example, see Non-patent References 10 and 11). A drug that acts on the β3-adrenergic receptors can therefore be expected to be useful in the prevention and treatment of frequent urinary in overactive bladder and in urinary incontinence.

On the other hand, α1-adrenergic receptors, which are another adrenergic receptor subfamily, are reported to be expressed in the vas deferens, submandibular glands, kidneys, spleen, liver, and aorta, as well as the prostate gland, urethra, and the like. Certain selective antagonists of these receptors are used in the treatment of benign prostatic hyperplasia (for example, see Non-patent References 1 and 13).

In contrast, drugs that act on the α1-adrenergic receptors, such as phenylephrine, methoxamine, metaraminol, midodrine, and the like, are known to elevate the blood pressure through vasoconstriction of the peripheral tissues and are used as vasopressors (for example, see Non-patent Reference 12). Non-patent Reference 12 also describes the relationship between the subtype-selective effects of α1-adrenergic receptors and urinary incontinence. In short, α1-adrenergic receptors are further classified as α1A, α1B, α1D, and so on. Drugs that act selectively on the α1A subtype among them are expected to be useful in the treatment and prevention of stress urinary incontinence through a constrictive effect on the bladder neck and the urethral smooth muscles.

As is evident from the above, it usually is preferable to take into consideration the selectivity of drugs for receptor subfamilies and the subtypes among them when adrenergic receptor agonists or antagonists are used to treat specific diseases in accordance with the goal. When planning to treat diabetes, obesity, hyperlipidemia, depression, cholelithiasis, diseases caused by biliary hyperkinesia, diseases caused by hyperfunction of the gastrointestinal tract, frequent urination and urinary incontinence in overactive bladder, and diseases associated with decreased lacrimation using β-adrenergic receptor agonists in particular, an agonist having high selectivity for the β3-adrenergic receptor subtype among them is usually selected. In other words, as was mentioned above, stimulation of the β1- and β2-adrenergic receptor subtypes is also judged to be a concern as it may trigger an undesirable increase in heart rate or drop in blood pressure, depending on the patient.

Similarly, stimulation of the α1-adrenergic receptors, which are another subfamily, is also preferably kept in mind as a cause of unintended secondary physiological effects on the blood vessels of the peripheral tissues and the like, depending on the patient.

Patent References 3-6 and Non-patent Reference 14 describe specific compounds having a stimulating effect on the β3-adrenergic receptors (General formulae (4)-(8) below). Nonetheless, none of the prior art discloses the compounds of the present invention.

General Formula (4) described in Patent Reference 3:

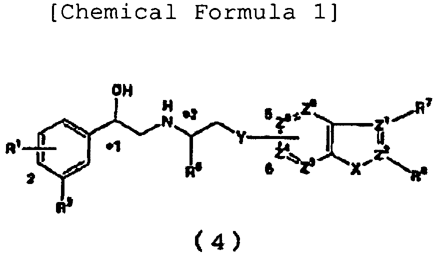

General Formula (5) described in Patent Reference 4:

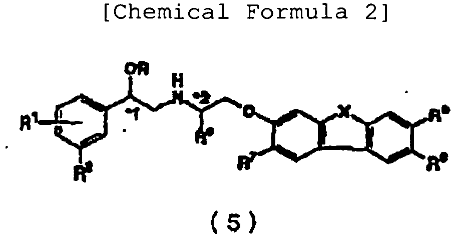

General Formula (6) described in Patent Reference 5:

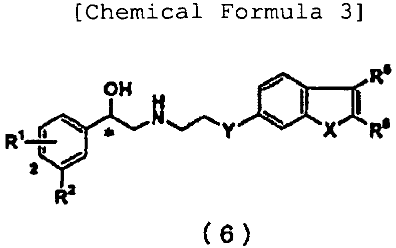

General Formula (7) described in Patent Reference 6:

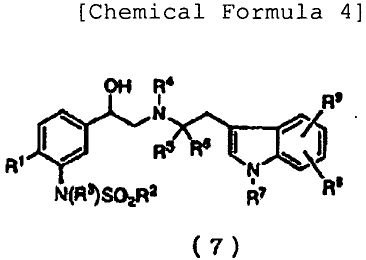

General Formula (8) described in Non-patent Reference 14:

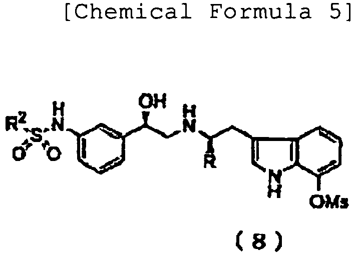

Patent References 3-6 and Non-patent Reference 14 also disclose selective stimulation of the β3-adrenergic receptors compared to stimulation of the α1-adrenergic receptors.

### PRIOR ART REFERENCES

### PATENT REFERENCES

Patent Reference 1: International Publication No. WO99/31045 pamphlet
Patent Reference 2: International Publication No. WO2007/026630 pamphlet
Patent Reference 3: International Publication No. WO03/035620 pamphlet
Patent Reference 4: International Publication No. WO97/25311 pamphlet
Patent Reference 5: International Publication No. WO01/83451 pamphlet
Patent Reference 6: Japan Patent Office Kokai Patent 2004-323519

### NON-PATENT REFERENCES

Non-Patent Reference 1: Eur. J. Pharmacol., Vol. 375, pp. 261-276, 1999
Non-Patent Reference 2: Nature, Vol. 309, pp. 163-165, 1984
Non-Patent Reference 3: Int. J. Obes. Relat. Metab. Disord., Vol. 20, pp. 191-199, 1996
Non-Patent Reference 4: Drug Development Research, Vol. 32, pp. 69-76, 1994
Non-Patent Reference 5: J. Clin. Invest., Vol. 101, pp. 2387-2393, 1998
Non-Patent Reference 6: Eur. J. Pharmacol., Vol. 289, pp. 223-228, 1995
Non-Patent Reference 7: Drugs of the Future, Vol. 18, No. 6, pp. 529-549, 1993
Non-Patent Reference 8: Pharmacology, Vol. 51, pp. 288-297, 1995
Non-Patent Reference 9: Brain Res. Mol. Brain Res., Vol. 29, No. 2, pp. 369-375, 1995
Non-Patent Reference 10: J. Urology, Vol. 161, pp. 680-685, 1999
Non-Patent Reference 11: J. Pharmacol. Exp. Ther., Vol. 288, pp. 1367-1373, 1999
Non-Patent Reference 12: Current Topics in Medicinal Chemistry, Vol. 7, pp. 135-145, 2007
Non-Patent Reference 13: Br. J. Pharmacol., Vol. 147, pp. S88-S119, 2006
Non-Patent Reference 14: Bioorg. Med. Chem. Lett., Vol. 14, pp. 5963-5966, 2004

### SUMMARY OF THE INVENTION

### PROBLEMS TO BE SOLVED BY THE INVENTION

An object of the present invention is to provide a drug that selectively stimulates the β3-adrenergic receptors, in particular a drug capable of stimulating the β3-adrenergic receptors preferentially over the α1-adrenergic receptors (also referred to as a "selective β3/α1-adrenergic receptor agonist" in this specification). This drug can be used in the treatment and prevention of diabetes, obesity, hyperlipidemia, depression, cholelithiasis, diseases caused by biliary hyperkinesia, diseases caused by hyperfunction of the gastrointestinal tract, interstitial cystitis, overactive bladder, urinary incontinence, diseases associated with decreased lacrimation, and the like while minimizing the appearance of undesirable physiological effects that accompany stimulation of the α1-adrenergic receptors.

### MEANS USED TO SOLVE THE ABOVE-MENTIONED PROBLEMS

Certain types of compounds having a specific structure were discovered to be able to stimulate the β3-adrenergic receptors preferentially over the α1-adrenergic receptors. These compounds can therefore be utilized as the selective β3/α1-adrenergic receptor agonist of the present invention.

Specifically, the present invention relates to the following.

[1] A compound shown by the following general formula (I)

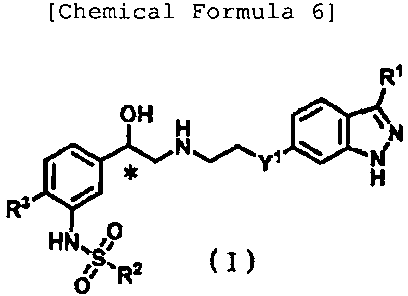

(in general formula (I), R¹ is a lower alkyl group, optionally substituted cyclic lower alkyl group, halogen atom, perfluoroalkyl group, -OR⁴-, -CH(R⁵)OR⁶, or -(CH₂)ₙCONR⁷⁻¹R⁷⁻²; R² is an optionally substituted cyclic lower alkyl group, lower alkyl group substituted by one, two, or more halogen atoms, optionally substituted heterocycle, or substituted phenyl group; R³ is a hydrogen atom or halogen atom; Y¹ is an oxygen atom, -NR⁸-, or methylene group; R⁴ is a lower alkyl group, optionally substituted cyclic lower alkyl group, difluoromethyl group, or trifluoromethyl group; R⁵ is a hydrogen atom, lower alkyl group, or optionally substituted cyclic lower alkyl group; R⁶ is a hydrogen atom, lower alkyl group, optionally substituted cyclic lower alkyl group, difluoromethyl group, or trifluoromethyl group; R⁷⁻¹ and R⁷⁻² may be the same or different and are each independently a hydrogen atom, lower alkyl group, or optionally substituted cyclic lower alkyl group; R⁸ is a hydrogen atom, lower alkyl group, or optionally substituted cyclic lower alkyl group, n is 0, 1, or 2, and the "*" symbol denotes an asymmetric carbon); or a salt thereof.
[1-1] A compound shown by the following general formula (I) 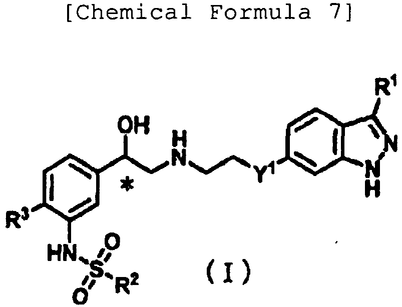 (in general formula (I), R¹ is a lower alkyl group, optionally substituted cyclic lower alkyl group, halogen atom, perfluoroalkyl group, -OR⁴-, -CH(R⁵)OR⁶, or -(CH₂)ₙCONR⁷⁻¹R⁷⁻²; R² is an optionally substituted cyclic lower alkyl group, lower alkyl group substituted by one, two, or more halogen atoms, optionally substituted heterocycle, or substituted phenyl group; R³ is a hydrogen atom or halogen atom; Y¹ is an oxygen atom, -NR⁸-, or methylene group; R⁴ is a lower alkyl group, optionally substituted cyclic lower alkyl group, difluoromethyl group, or trifluoromethyl group; R⁵ is a hydrogen atom, lower alkyl group, or optionally substituted cyclic lower alkyl group; R⁶ is a hydrogen atom, lower alkyl group, optionally substituted cyclic lower alkyl group, difluoromethyl group, or trifluoromethyl group; R⁷⁻¹ and R⁷⁻² may be the same or different and are each independently a hydrogen atom, lower alkyl group, or optionally substituted cyclic lower alkyl group; R⁸ is a hydrogen atom, lower alkyl group, or optionally substituted cyclic lower alkyl group, n is 0, 1, or 2; however, excluding compounds in which R¹ is an ethyl group, trifluoromethyl group, -OMe, or -CH₂OH, R³ is a hydrogen atom; and Y¹ is an oxygen atom when R² is a cyclobutyl group; and the "*" symbol denotes an asymmetric carbon); or a salt thereof.
[1-2] Compounds shown by the following general formula (I) 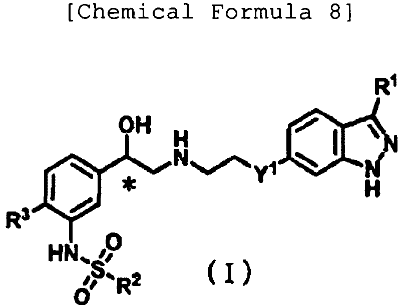 (in general formula (I), R¹ is a lower alkyl group, optionally substituted cyclic lower alkyl group, halogen atom, perfluoroalkyl group, -OR⁴-, -CH(R⁵)OR⁶, or -(CH₂)ₙCONR⁷⁻¹R⁷⁻²; R² is an optionally substituted cyclic lower alkyl group, lower alkyl group substituted by one, two, or more halogen atoms, optionally substituted heterocycle, or substituted phenyl group; R³ is a hydrogen atom or halogen atom; Y¹ is an oxygen atom, -NR⁸-, or methylene group; R⁴ is a lower alkyl group, optionally substituted cyclic lower alkyl group, difluoromethyl group, or trifluoromethyl group; R⁵ is a hydrogen atom, lower alkyl group, or optionally substituted cyclic lower alkyl group; R⁶ is a hydrogen atom, lower alkyl group, optionally substituted cyclic lower alkyl group, difluoromethyl group, or trifluoromethyl group; R⁷⁻¹ and R⁷⁻² may be the same or different and are each independently a hydrogen atom, lower alkyl group, or optionally substituted cyclic lower alkyl group; R⁸ is a hydrogen atom, lower alkyl group, or optionally substituted cyclic lower alkyl group; n is 0, 1, or 2; with a proviso that when R² is a cyclobutyl group, then R¹ is a methyl group, isopropyl group, cyclopropyl group, cyclobutyl group, chlorine atom, or -OCHF₂, R³ is a hydrogen atom and Y¹ is an oxygen atom; and the "*" symbol denotes an asymmetric carbon); or a salt thereof.
[1-3] A compound shown by the following general formula (I) 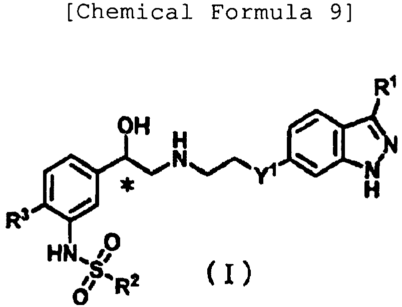 (in general formula (I), R¹ is a lower alkyl group, optionally substituted cyclic lower alkyl group, halogen atom, perfluoroalkyl group, -OR⁴-, -CH(R⁵)OR⁶, or -(CH₂)ₙCONR⁷⁻¹R⁷⁻²; R² is an optionally substituted cyclic lower alkyl group, lower alkyl group substituted by one, two, or more halogen atoms, optionally substituted heterocycle, or substituted phenyl group; R³ is a hydrogen atom or halogen atom; Y¹ is an oxygen atom, -NR⁸-, or methylene group; R⁴ is a lower alkyl group, optionally substituted cyclic lower alkyl group, difluoromethyl group, or trifluoromethyl group; R⁵ is a hydrogen atom, lower alkyl group, or optionally substituted cyclic lower alkyl group; R⁶ is a hydrogen atom, lower alkyl group, optionally substituted cyclic lower alkyl group, difluoromethyl group, or trifluoromethyl group; R⁷⁻¹ and R⁷⁻² may be the same or different and are each independently a hydrogen atom, lower alkyl group, or optionally substituted cyclic lower alkyl group; R⁸ is a hydrogen atom, lower alkyl group, or optionally substituted cyclic lower alkyl group, n is 0, 1, or 2; with a proviso that when R² is a cyclobutyl group, then R¹ is a methyl group or cyclobutyl group, R³ is a hydrogen atom and Y¹ is an oxygen atom; and the "*" symbol denotes an asymmetric carbon); or a salt thereof.

[2] The compound or salt thereof described in [1] above in which R¹ is a lower alkyl group, optionally substituted cyclic lower alkyl group, halogen atom, trifluoromethyl group, -OR⁴-, -CH₂(R⁶)OR⁶, or -(CH₂)ₙCONR⁷⁻¹R⁷⁻²; R² is an optionally substituted cyclic lower alkyl group, optionally substituted heterocycle, or substituted phenyl group; R³ is a hydrogen atom, fluorine atom, or chlorine atom; Y¹ is an oxygen atom, - NH-, or methylene group; R⁴ is a lower alkyl group, optionally substituted cyclic lower alkyl group, difluoromethyl group, or trifluoromethyl group; R⁶ is a hydrogen atom, lower alkyl group, or cyclic lower alkyl group; and R⁷⁻¹ and R⁷⁻² may be the same or different and are each independently a hydrogen atom, lower alkyl group, or optionally substituted cyclic lower alkyl group.

[3] The compound or salt thereof described in [1] above in which R¹ is a lower alkyl group, optionally substituted cyclic lower alkyl group, halogen atom, trifluoromethyl group, -OR⁴-, -CH₂OH, or -(CH₂)ₙCONR⁷⁻¹R⁷⁻²; R² is an optionally substituted cyclic lower alkyl group, optionally substituted heterocycle, or substituted phenyl group; R³ is a hydrogen atom, fluorine atom, or chlorine atom; Y¹ is an oxygen atom; R⁴ is a lower alkyl group, optionally substituted cyclic lower alkyl group, difluoromethyl group, or trifluoromethyl group; and R⁷⁻¹ and R⁷⁻² may be the same or different and are each independently a hydrogen atom or optionally substituted cyclic lower alkyl group; or a salt thereof.
[4] The compound or salt thereof described in any of [1]-[3] above in which R¹ is a methyl group, ethyl group, isopropyl group, cyclopropyl group, cyclobutyl group, chlorine atom, trifluoromethyl group, -OMe, or -OCHF₂.
[5] The compound or salt thereof described in any of [1]-[4] above in which R² is an optionally substituted cyclic lower alkyl group, a group shown by any of the following formulas (V-I)-(V-V) 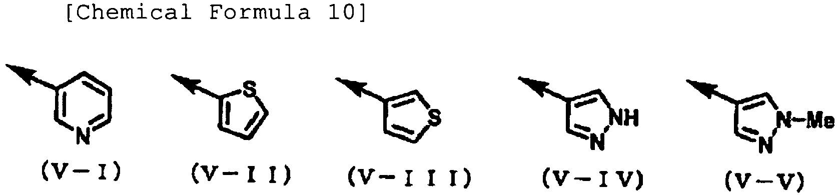 or a group shown by any of the following formulas (VII-I)-(VII-XI) 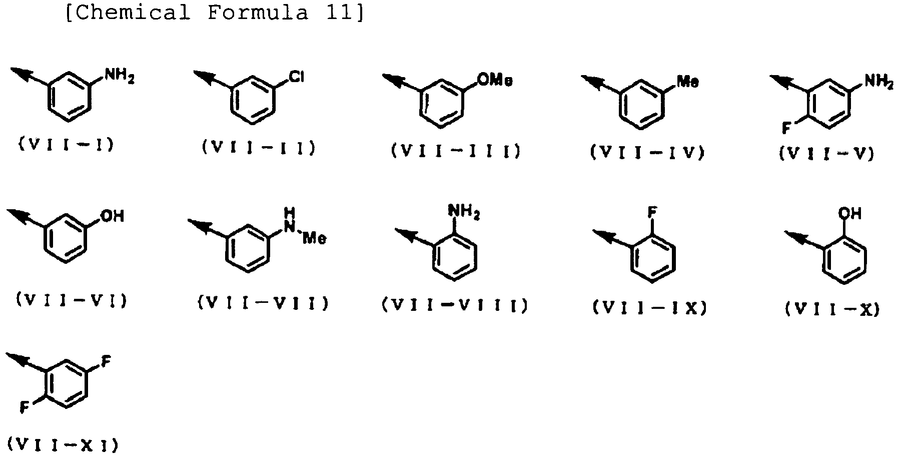
[6] The compound or salt thereof described in any of [1]-[5] above in which R² is an optionally substituted cyclic lower alkyl group.
[7] The compound or salt thereof described in any of [1]-[5] above in which R² is a group shown by any of the following formulas (V-I)-(V-V) 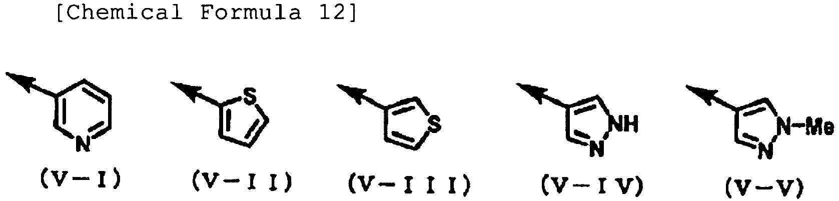 or a group shown by any of the following formulas (VII-I)-(VII-V) and (VII-IX) 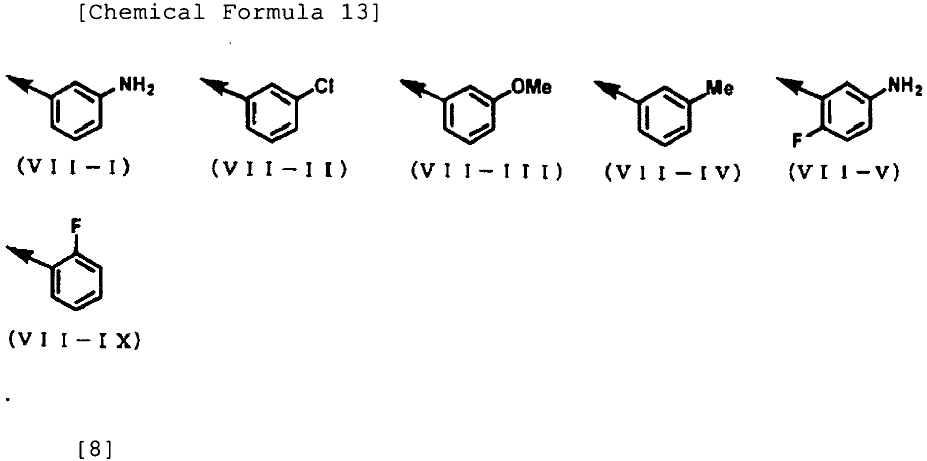 The compound or salt thereof described in any of [1]-[6] above in which, when R² is a cyclobutyl group or cyclopentyl group, R¹ is a methyl group, isopropyl group, cyclopropyl group, cyclobutyl group, chlorine atom, or -OCHF₂.
[9] The compound or salt thereof described in any of [1]-[6] above in which, when R² is a cyclopropyl group, R¹ is a methyl group, ethyl group, isopropyl group, cyclopropyl group, cyclobutyl group, chlorine atom, trifluoromethyl group, -OMe, or -OCHF₂.
[10] The compound or salt thereof described in any of [1]-[9] above in which Y¹ is an oxygen atom.
[11] The compound or salt thereof described in any of [1]-[10] above in which R³ is a hydrogen atom.
[12] The compound or salt thereof described in any of [1]-[11] above in which R¹ is a methyl group.
[13] The compound or salt thereof described in any of [1]-[11] above in which R¹ is -OCHF₂.
[14] The compound or salt thereof described in [1] above in which R¹ is a lower alkyl group, optionally substituted cyclic lower alkyl group, chlorine atom, trifluoromethyl group, -OR⁴, -CH₂OH, or -(CH₂)₂CONMe₂; R² is an optionally substituted cyclic lower alkyl group, optionally substituted heterocycle, or substituted phenyl group; R³ is a hydrogen atom, fluorine atom, or chlorine atom; Y¹ is an oxygen atom; and R⁴ is a lower alkyl group, optionally substituted cyclic lower alkyl group, difluoromethyl group, or trifluoromethyl group.

[15] The compound or salt thereof described in [1] above in which R¹ is a lower alkyl group, optionally substituted cyclic lower alkyl group, chlorine atom, trifluoromethyl group, -OR⁴, or -(CH₂)₂CONMe₂; R² is a cyclopropyl group, cyclobutyl group, optionally substituted heterocycle, or substituted phenyl group; R³ is a hydrogen atom, fluorine atom, or chlorine atom; Y¹ is an oxygen atom; and R⁴ is a lower alkyl group, cyclic lower alkyl group, difluoromethyl group, or difluoromethyl group.

[16] The compound or salt thereof described in [1] above in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclic lower alkyl group, optionally substituted heterocycle, or substituted phenyl group; R³ is a hydrogen atom, fluorine atom, or chlorine atom; and Y¹ is an oxygen atom.

[17] The compound or salt thereof described in [1] above in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group, cyclobutyl group, optionally substituted heterocycle, or substituted phenyl group; R³ is a hydrogen atom, fluorine atom, or chlorine atom; and Y¹ is an oxygen atom.

[18] The compound or salt thereof described in [1] above in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group, cyclobutyl group, optionally substituted heterocycle, or substituted phenyl group; R³ is a hydrogen atom; and Y¹ is an oxygen atom.

[19] The compound or salt thereof described in [1] above in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group, cyclobutyl group, group shown by any of the following formulas (V-I)-(V-V)

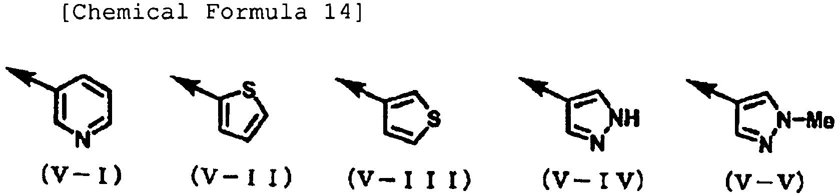

or group shown by any of the following formulas (VII-I)-(VII-X)

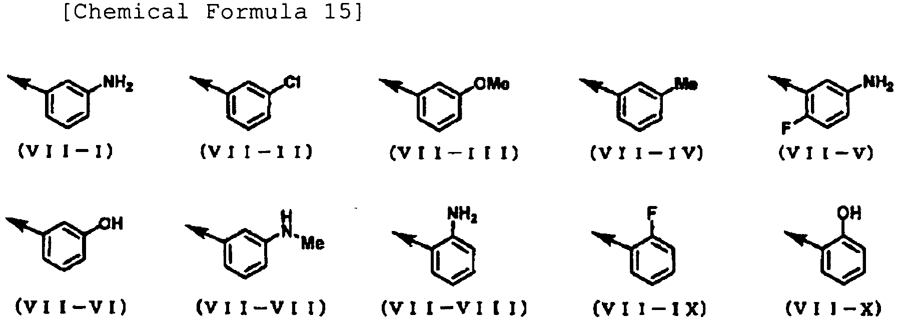

; R³ is a hydrogen atom; and Y¹ is an oxygen atom.

[20] The compound or salt thereof described in [1] above in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group or cyclobutyl group; R³ is a hydrogen atom; and Y¹ is an oxygen atom.

[21] The compound or salt thereof described in [1] above in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a group shown by any of the following formulas (V-I)-(V-V)

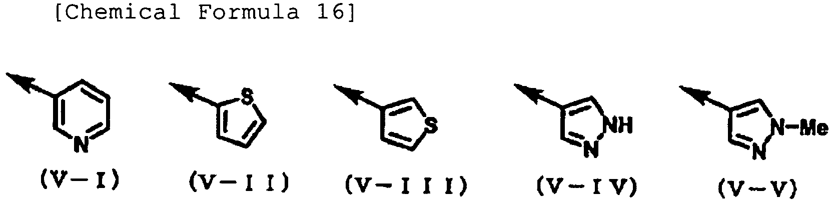

; R³ is a hydrogen atom; and Y¹ is an oxygen atom.

[22] The compound or salt thereof described in [1] above in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a group shown by any of the following formulas (VII-I)-(VII-X)

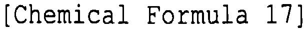

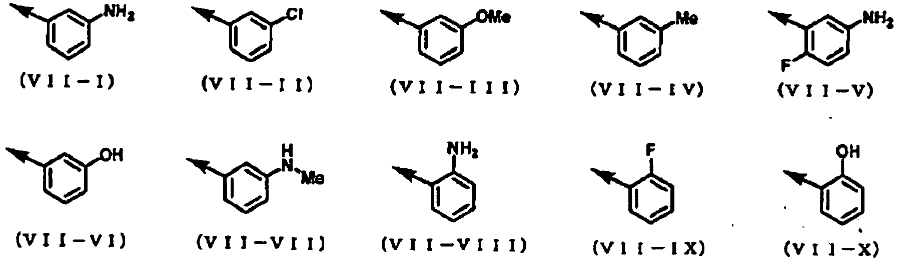

; R³ is a hydrogen atom; and Y¹ is an oxygen atom.

[23] The compound or salt thereof described in [1] above in which R¹ is a methyl group or -OCHF₂ group; R² is a cyclopropyl group, cyclobutyl group, or 2-fluorophenyl group; R³ is a hydrogen atom; and Y¹ is an oxygen atom.

[24] The compound or salt thereof described in [1] above in which R¹ is a methyl group or -OCHF₂ group; R² is a cyclopropyl group or cyclobutyl group; R³ is a hydrogen atom; and Y¹ is an oxygen atom.

[25] The compound or salt thereof described in Claim 1 in which R¹ is a methyl group, chlorine atom, trifluoromethyl group, or -OMe; R² is a group shown by the following formulas (V-II), (V-III), (VII-I), or (VII-IX) 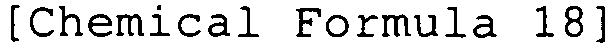 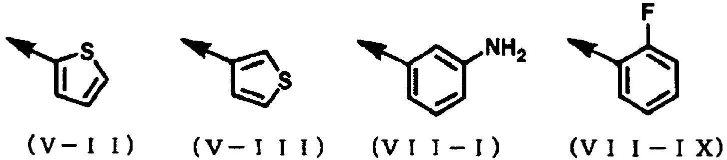 ; R³ is a hydrogen atom, fluorine atom, or chlorine atom; and Y¹ is an oxygen atom.

[26] The compound or salt thereof described in [1]-[25] above in which the configuration of the asymmetric carbon shown by the "*" symbol is (R).

[27] A compound selected from the group consisting of
(R)-3-amino-N-(3-(1-hydroxy-2-(2-(3-methoxyindazol-6-yloxy)ethylamino)ethyl)phenyl)benzene sulfonamide;
(R)-N-(3-(1-hydroxy-2-(2-(3-methoxyindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide;
(R)-2-fluoro-N-(3-(1-hydroxy-2-(2-(3-methoxyindazol-6-yloxy)ethylamino)ethyl)phenyl)benzene sulfonamide;
(R)-5-amino-2-fluoro-N-(3-(1-hydroxy-2-(2-(3-methoxyindazol-6-yloxy)ethylamino)ethyl)phenyl)benzene sulfonamide;
(R)-N-(3-(1-hydroxy-2-(2-(3-methoxyindazol-6-yloxy)ethylamino)ethyl)phenyl)thiophene-3-sulfonamide;
(R)-N-(3-(1-hydroxy-2-(2-(3-methoxyindazol-6-yloxy)ethylamino)ethyl)phenyl)pyridine-3-sulfonamide;
(R)-3-chloro-N-(3-(1-hydroxy-2-(2-(3-methoxyindazol-6-yloxy)ethylamino)ethyl)phenyl)benzene sulfonamide;
(R)-N-(3-(1-hydroxy-2-(2-(3-methoxyindazol-6-yloxy)ethylamino)ethyl)phenyl)cyclopropane sulfonamide;
(R)-3-amino-N-(3-(1-hydroxy-2-(2-(3-methylindazol-6-yloxy)ethylamino)ethyl)phenyl)benzene sulfonamide;
(R)-3-chloro-N-(3-(1-hydroxy-2-(2-(3-methylindazol-6-yloxy)ethylamino)ethyl)phenyl)benzene sulfonamide;
(R)-N-(3-(1-hydroxy-2-(2-(3-methylindazol-6-yloxy)ethylamino)ethyl)phenyl)-3-methoxybenzene sulfonamide;
(R)-N-(3-(1-hydroxy-2-(2-(3-methylindazol-6-yloxy)ethylamino)ethyl)phenyl)pyridine-3-sulfonamide;
(R)-N-(3-(1-hydroxy-2-(2-(3-methylindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)thiophene-3-sulfonamide;
(R)-N-(3-(1-hydroxy-2-(2-(3-methylindazol-6-yloxy)ethylamino)ethyl)phenyl)cyclopropane sulfonamide;
(R)-N-(3-(1-hydroxy-2-(2-(3-methylindazol-6-yloxy)ethylamino)ethyl)phenyl)-3-methylbenzene sulfonamide;
(R)-2-amino-N-(3-(1-hydroxy-2-(2-(3-methylindazol-6-yloxy)ethylamino)ethyl)phenyl)benzene sulfonamide;
(R)-5-amino-2-fluoro-N-(3-(1-hydroxy-2-(2-(3-methylindazol-6-yloxy)ethylamino)ethyl)phenyl)benzene sulfonamide;
(R)-N-(3-(1-hydroxy-2-(2-(3-methylindazol-6-yloxy)ethylamino)ethyl)phenyl)-1-methylpyrazole-4-sulfonamide;
(R)-N-(3-(1-hydroxy-2-(2-(3-methylindazol-6-yloxy)ethylamino)ethyl)phenyl)pyrazole-4-sulfonamide;
(R)-3-amino-N-(3-(2-(2-(3-(difluoromethoxy)-indazol-6-yloxy)ethylamino)-1-hydroxyethyl)phenyl)benzene sulfonamide;
(R)-N-(3-(2-(2-(3-(difluoromethoxy)-indazol-6-yloxy)ethylamino)-1-hydroxyethyl)phenyl)pyrazole-4-sulfonamide;
(R)-3-amino-N-(3-(2-(2-(3-ethylindazol-6-yloxy)ethylamino)-1-hydroxyethyl)phenyl)benzene sulfonamide;
(R)-N-(3-(2-(2-(3-ethylindazol-6-yloxy)ethylamino)-1-hydroxyethyl)phenyl)cyclopropane sulfonamide;
(R)-N-(3-(2-(2-(3-ethylindazol-6-yloxy)ethylamino)-1-hydroxyethyl)phenyl)-3-methylbenzene sulfonamide;
(R)-N-(3-(2-(2-(3-ethylindazol-6-yloxy)ethylamino)-1-hydroxyethyl)phenyl)-1-methylpyrazole-4-sulfonamide;
(R)-N-(3-(2-(2-(3-ethylindazol-6-yloxy)ethylamino)-1-hydroxyethyl)phenyl)pyrazole-4-sulfonamide;
(R)-N-(3-(2-(2-(3-chloroindazol-6-yloxy)ethylamino)-1-hydroxyethyl)phenyl)cyclopropane sulfonamide;
(R)-3-amino-N-(3-(2-(2-(3-chloroindazol-6-yloxy)ethylamino)-1-hydroxyethyl)phenyl)benzene sulfonamide;
(R)-N-(3-(1-hydroxy-2-(2-(3-(trifluoromethyl)-indazol-6-yloxy)ethylamino)ethyl)phenyl)cyclopropane sulfonamide;
(R)-N-(3-(2-(2-(3-cyclopropylindazol-6-yloxy)ethylamino)-1-hydroxyethyl)phenyl)cyclopropane sulfonamide;
(R)-3-amino-N-(3-(1-hydroxy-2-(2-(3-isopropylindazol-6-yloxy)ethylamino)ethyl)phenyl)benzene sulfonamide;
(R)-3-(6-(2-(2-(3-(3-amino-phenylsulfonamide)phenyl)-2-hydroxyethylamino)ethoxy)indazol-3-yl)-N,N-dimethyl propanamide;
(R)-2-fluoro-N-(3-(1-hydroxy-2-(2-(3-methylindazol-6-yloxy)ethylamino)ethyl)phenyl)benzene sulfonamide;
(R)-3-hydroxy-N-(3-(1-hydroxy-2-(2-(3-methylindazol-6-yloxy)ethylamino)ethyl)phenyl)benzene sulfonamide;
(R)-N-(3-(1-hydroxy-2-(2-(3-methylindazol-6-yloxy)ethylamino)ethyl)phenyl)-3-(methylamino)-benzene sulfonamide;
(R)-2-hydroxy-N-(3-(1-hydroxy-2-(2-(3-methylindazol-6-yloxy)ethylamino)ethyl)phenyl)benzene sulfonamide;
(R)-N-(3-(2-(2-(3-cyclobutylindazol-6-yloxy)ethylamino)-1-hydroxyethyl)phenyl)cyclobutane sulfonamide;
(R)-N-(3-(2-(2-(3-cyclobutylindazol-6-yloxy)ethylamino)-1-hydroxyethyl)phenyl)cyclopropane sulfonamide;
(R)-N-(3-(2-(2-(3-chloroindazol-6-yloxy)ethylamino)-1-hydroxyethyl)phenyl)-2-fluorobenzene sulfonamide;
(R)-2-fluoro-N-(3-(1-hydroxy-2-(2-(3-(trifluoromethyl)-indazol-6-yloxy)ethylamino)ethyl)phenyl)benzene sulfonamide; and
(R)-2,5-difluoro-N-(3-(1-hydroxy-2-(2-(3-methylindazol-6-yloxy)ethylamino)ethyl)phenyl)benzene sulfonamide; or a salt thereof.

[27-1] (R)-N-(3-(2-(2-(3-cyclobutylindazol-6-yloxy)ethylamino)-1-hydroxyethyl)phenyl)cyclobutane sulfonamide or a salt thereof.

[27-2] (R)-N-(3-(2-(2-(3-cyclobutylindazol-6-yloxy)ethylamino)-1-hydroxyethyl)phenyl)cyclopropane sulfonamide or a salt thereof.

[27-3] (R)-N-(3-(2-(2-(3-chloroindazol-6-yloxy)ethylamino)-1-hydroxyethyl)phenyl)-2-fluorobenzene sulfonamide or a salt thereof.

[27-4] (R)-2-fluoro-N-(3-(1-hydroxy-2-(2-(3-(trifluoromethyl)-indazol-6-yloxy)ethylamino)ethyl)phenyl)benzene sulfonamide or a salt thereof.
[27-5] (R)-2,5-difluoro-N-(3-(1-hydroxy-2-(2-(3-methylindazol-6-yloxy)ethylamino)ethyl)phenyl)benzene sulfonamide or a salt thereof.

[28] A compound selected from the group consisting of
(R)-3-amino-N-(3-(1-hydroxy-2-(2-(3-methoxyindazol-6-yloxy)ethylamino)ethyl)phenyl)benzene sulfonamide;
(R)-N-(3-(1-hydroxy-2-(2-(3-methoxyindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide;
(R)-2-fluoro-N-(3-(1-hydroxy-2-(2-(3-methoxyindazol-6-yloxy)ethylamino)ethyl)phenyl)benzene sulfonamide;
(R)-5-amino-2-fluoro-N-(3-(1-hydroxy-2-(2-(3-methoxyindazol-6-yloxy)ethylamino)ethyl)phenyl)benzene sulfonamide;
(R)-N-(3-(1-hydroxy-2-(2-(3-methoxyindazol-6-yloxy)ethylamino)ethyl)phenyl)thiophene-3-sulfonamide;
(R)-N-(3-(1-hydroxy-2-(2-(3-methoxyindazol-6-yloxy)ethylamino)ethyl)phenyl)pyridine-3-sulfonamide;
(R)-3-chloro-N-(3-(1-hydroxy-2-(2-(3-methoxyindazol-6-yloxy)ethylamino)ethyl)phenyl)benzene sulfonamide;
(R)-N-(3-(1-hydroxy-2-(2-(3-methoxyindazol-6-yloxy)ethylamino)ethyl)phenyl)cyclopropane sulfonamide;
(R)-3-amino-N-(3-(1-hydroxy-2-(2-(3-methylindazol-6-yloxy)ethylamino)ethyl)phenyl)benzene sulfonamide;
(R)-3-chloro-N-(3-(1-hydroxy-2-(2-(3-methylindazol-6-yloxy)ethylamino)ethyl)phenyl)benzene sulfonamide;
(R)-N-(3-(1-hydroxy-2-(2-(3-methylindazol-6-yloxy)ethylamino)ethyl)phenyl)-3-methoxybenzene sulfonamide;
(R)-N-(3-(1-hydroxy-2-(2-(3-methylindazol-6-yloxy)ethylamino)ethyl)phenyl)pyridine-3-sulfonamide;
(R)-N-(3-(1-hydroxy-2-(2-(3-methylindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)thiophene-3-sulfonamide;
(R)-N-(3-(1-hydroxy-2-(2-(3-methylindazol-6-yloxy)ethylamino)ethyl)phenyl)cyclopropane sulfonamide;
(R)-N-(3-(1-hydroxy-2-(2-(3-methylindazol-6-yloxy)ethylamino)ethyl)phenyl)-3-methylbenzene sulfonamide;
(R)-2-amino-N-(3-(1-hydroxy-2-(2-(3-methylindazol-6-yloxy)ethylamino)ethyl)phenyl)benzene sulfonamide;
(R)-5-amino-2-fluoro-N-(3-(1-hydroxy-2-(2-(3-methylindazol-6-yloxy)ethylamino)ethyl)phenyl)benzene sulfonamide;
(R)-N-(3-(1-hydroxy-2-(2-(3-methylindazol-6-yloxy)ethylamino)ethyl)phenyl)-1-methylpyrazole-4-sulfonamide;
(R)-N-(3-(1-hydroxy-2-(2-(3-methylindazol-6-yloxy)ethylamino)ethyl)phenyl)pyrazole-4-sulfonamide;
(R)-3-amino-N-(3-(2-(2-(3-(difluoromethoxy)-indazol-6-yloxy)ethylamino)-1-hydroxyethyl)phenyl)benzene sulfonamide;
(R)-N-(3-(2-(2-(3-(difluoromethoxy)-indazol-6-yloxy)ethylamino)-1-hydroxyethyl)phenyl)pyrazole-4-sulfonamide;
(R)-3-amino-N-(3-(2-(2-(3-ethylindazol-6-yloxy)ethylamino)-1-hydroxyethyl)phenyl)benzene sulfonamide;
(R)-N-(3-(2-(2-(3-ethylindazol-6-yloxy)ethylamino)-1-hydroxyethyl)phenyl)cyclopropane sulfonamide;
(R)-N-(3-(2-(2-(3-ethylindazol-6-yloxy)ethylamino)-1-hydroxyethyl)phenyl)-3-methylbenzene sulfonamide;
(R)-N-(3-(2-(2-(3-ethylindazol-6-yloxy)ethylamino)-1-hydroxyethyl)phenyl)-1-methylpyrazole-4-sulfonamide;
(R)-N-(3-(2-(2-(3-ethylindazol-6-yloxy)ethylamino)-1-hydroxyethyl)phenyl)pyrazole-4-sulfonamide;
(R)-N-(3-(2-(2-(3-chloroindazol-6-yloxy)ethylamino)-1-hydroxyethyl)phenyl)cyclopropane sulfonamide;
(R)-3-amino-N-(3-(2-(2-(3-chloroindazol-6-yloxy)ethylamino)-1-hydroxyethyl)phenyl)benzene sulfonamide;
(R)-N-(3-(1-hydroxy-2-(2-(3-(trifluoromethyl)-indazol-6-yloxy)ethylamino)ethyl)phenyl)cyclopropane sulfonamide;
(R)-N-(3-(2-(2-(3-cyclopropylindazol-6-yloxy)ethylamino)-1-hydroxyethyl)phenyl)cyclopropane sulfonamide;
(R)-3-amino-N-(3-(1-hydroxy-2-(2-(3-isopropylindazol-6-yloxy)ethylamino)ethyl)phenyl)benzene sulfonamide;
(R)-3-(6-(2-(2-(3-(3-amino-phenylsulfonamide)phenyl)-2-hydroxyethylamino)ethoxy)indazol-3-yl)-N,N-dimethyl propanamide;
(R)-2-fluoro-N-(3-(1-hydroxy-2-(2-(3-methylindazol-6-yloxy)ethylamino)ethyl)phenyl)benzene sulfonamide;
(R)-3-hydroxy-N-(3-(1-hydroxy-2-(2-(3-methylindazol-6-yloxy)ethylamino)ethyl)phenyl)benzene sulfonamide;
(R)-N-(3-(1-hydroxy-2-(2-(3-methylindazol-6-yloxy)ethylamino)ethyl)phenyl)-3-(methylamino)-benzene sulfonamide; and
(R)-2-hydroxy-N-(3-(1-hydroxy-2-(2-(3-methylindazol-6-yloxy)ethylamino)ethyl)phenyl)benzene sulfonamide; or a salt thereof.

[28-1] (R)-3-amino-N-(3-(1-hydroxy-2-(2-(3-methoxyindazol-6-yloxy)ethylamino)ethyl)phenyl)benzene sulfonamide; or a salt thereof.

[28-2] (R)-N-(3-(1-hydroxy-2-(2-(3-methoxyindazol-6-yloxy)ethylamino)ethyl)phenyl)thiophene-2-sulfonamide; or a salt thereof.

[28-3] (R)-N-(3-(1-hydroxy-2-(2-(3-methylindazol-6-yloxy)ethylamino)ethyl)phenyl)cyclobutane sulfonamide; or a salt thereof.

[28-4] (R)-N-(3-(2-(2-(3-(difluoromethoxy)-indazol-6-yloxy)ethylamino)-1-hydroxyethyl)phenyl)cyclopropane sulfonamide; or a salt thereof.

[28-5] (R)-2-fluoro-N-(3-(1-hydroxy-2-(2-(3-methoxyindazol-6-yloxy)ethylamino)ethyl)phenyl)benzene sulfonamide; or a salt thereof.

[28-6] (R)-5-amino-2-fluoro-N-(3-(1-hydroxy-2-(2-(3-methoxyindazol-6-yloxy)ethylamino)ethyl)phenyl)benzene sulfonamide; or a salt thereof.

[28-7] (R)-N-(3-(1-hydroxy-2-(2-(3-methoxyindazol-6-yloxy)ethylamino)ethyl)phenyl)thiophene-3-sulfonamide; or a salt thereof.

[28-8] (R)-N-(3-(1-hydroxy-2-(2-(3-methoxyindazol-6-yloxy)ethylamino)ethyl)phenyl)pyridine-3-sulfonamide; or a salt thereof.

[28-9] (R)-3-chloro-N-(3-(1-hydroxy-2-(2-(3-methoxyindazol-6-yloxy)ethylamino)ethyl)phenyl)benzene sulfonamide; or a salt thereof.

[28-10] (R)-N-(3-(1-hydroxy-2-(2-(3-methoxyindazol-6-yloxy)ethylamino)ethyl)phenyl)cyclopropane sulfonamide; or a salt thereof.

[28-11] (R)-3-amino-N-(3-(1-hydroxy-2-(2-(3-methylindazol-6-yloxy)ethylamino)ethyl)phenyl)benzene sulfonamide;; or a salt thereof.

[28-12] (R)-3-chloro-N-(3-(1-hydroxy-2-(2-(3-methylindazol-6-yloxy)ethylamino)ethyl)phenyl)benzene sulfonamide; or a salt thereof.

[28-13] (R)-N-(3-(1-hydroxy-2-(2-(3-methylindazol-6-yloxy)ethylamino)ethyl)phenyl)-3-methoxybenzene sulfonamide; or a salt thereof.

[24-14] (R)-N-(3-(1-hydroxy-2-(2-(3-methylindazol-6-yloxy)ethylamino)ethyl)phenyl)pyridine-3-sulfonamide; or a salt thereof.

[28-15] (R)-N-(3-(1-hydroxy-2-(2-(3-methylindazol-6-yloxy)ethylamino)ethyl)phenyl)thiophene-2-sulfonamide; or a salt thereof.

[28-16] (R)-N-(3-(1-hydroxy-2-(2-(3-methylindazol-6-yloxy)ethylamino)ethyl)phenyl)thiophene-3-sulfonamide; or a salt thereof.

[28-17] (R)-N-(3-(1-hydroxy-2-(2-(3-methylindazol-6-yloxy)ethylamino)ethyl)phenyl)cyclopropane sulfonamide; or a salt thereof.

[28-18] (R)-N-(3-(1-hydroxy-2-(2-(3-methylindazol-6-yloxy)ethylamino)ethyl)phenyl)-3-methylbenzene sulfonamide; or a salt thereof.

[28-19] (R)-2-amino-N-(3-(1-hydroxy-2-(2-(3-methylindazol-6-yloxy)ethylamino)ethyl)phenyl)benzene sulfonamide; or a salt thereof.

[28-20] (R)-5-amino-2-fluoro-N-(3-(1-hydroxy-2-(2-(3-methylindazol-6-yloxy)ethylamino)ethyl)phenyl)benzene sulfonamide; or a salt thereof.

[28-21] (R)-N-(3-(1-hydroxy-2-(2-(3-methylindazol-6-yloxy)ethylamino)ethyl)phenyl)-1-methylpyrazole-4-sulfonamide; or a salt thereof.

[28-22] (R)-N-(3-(1-hydroxy-2-(2-(3-methylindazol-6-yloxy)ethylamino)ethyl)phenyl)pyrazole-4-sulfonamide; or a salt thereof.

[28-23] (R)-3-amino-N-(3-(2-(2-(3-(difluoromethoxy)-indazol-6-yloxy)ethylamino)-1-hydroxyethyl)phenyl)benzen sulfonamide; or a salt thereof.

[28-24] (R)-N-(3-(2-(2-(3-(difluoromethoxy)-indazol-6-yloxy)ethylamino)-1-hydroxyethyl)phenyl)pyrazole-4-sulfonamide; or a salt thereof.

[28-25] (R)-3-amino-N-(3-(2-(2-(3-ethylindazol-6-yloxy)ethylamino)-1-hydroxyethyl)phenyl)benzene sulfonamide; or a salt thereof.

[28-26] (R)-N-(3-(2-(2-(3-ethylindazol-6-yloxy)ethylamino)-1-hydroxyethyl)phenyl)cyclopropane sulfonamide; or a salt thereof.

[28-27] (R)-N-(3-(2-(2-(3-ethylindazol-6-yloxy)ethylamino)-1-hydroxyethyl)phenyl)-3-methylbenzene sulfonamide; or a salt thereof.

[28-28] (R)-N-(3-(2-(2-(3-ethylindazol-6-yloxy)ethylamino)-1-hydroxyethyl)phenyl)-1-methylpyrazole-4-sulfonamide; or a salt thereof.

[28-29] (R)-N-(3-(2-(2-(3-ethylindazol-6-yloxy)ethylamino)-1-hydroxyethyl)phenyl)pyrazole-4-sulfonamide; or a salt thereof.

[28-30] (R)-N-(3-(2-(2-(3-chloroindazol-6-yloxy)ethylamino)-1-hydroxyethyl)phenyl)cyclopropane sulfonamide; or a salt thereof.

[28-31] (R)-3-amino-N-(3-(2-(2-(3-chloroindazol-6-yloxy)ethylamino)-1-hydroxyethyl)phenyl)benzene sulfonamide; or a salt thereof.

[28-32] (R)-N-(3-(1-hydroxy-2-(2-(3-(trifluoromethyl)-indazol-6-yloxy)ethylamino)ethyl)phenyl)cyclopropane sulfonamide; or a salt thereof.

[28-33] (R)-N-(3-(2-(2-(3-cyclopropylindazol-6-yloxy)ethylamino)-1-hydroxyethyl)phenyl)cyclopropane sulfonamide; or a salt thereof.

[28-34] (R)-3-amino-N-(3-(1-hydroxy-2-(2-(3-isopropylindazol-6-yloxy)ethylamino)ethyl)phenyl)benzene sulfonamide; or a salt thereof.

[28-35] (R)-3-(6-(2-(2-(3-(3-amino-phenylsulfonamide)phenyl)-2-hydroxyethylamino)ethoxy)indazol-3-yl)-N,N-dimethyl propanamide; or a salt thereof.

[28-36] (R)-2-fluoro-N-(3-(1-hydroxy-2-(2-(3-methylindazol-6-yloxy)ethylamino)ethyl)phenyl)benzene sulfonamide; or a salt thereof.

[28-38] (R)-3-hydroxy-N-(3-(1-hydroxy-2-(2-(3-methylindazol-6-yloxy)ethylamino)ethyl)phenyl)benzene sulfonamide; or a salt thereof.

[28-39] (R)-N-(3-(1-hydroxy-2-(2-(3-methylindazol-6-yloxy)ethylamino)ethyl)phenyl)-3-(methylamino)-benzene sulfonamide; or a salt thereof.

[28-40] (R)-2-hydroxy-N-(3-(1-hydroxy-2-(2-(3-methylindazol-6-yloxy)ethylamino)ethyl)phenyl)benzene sulfonamide; or a salt thereof.

[29] A compound selected from the group consisting of
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide; and
(R)-2-fluoro-N-(3-(1-hydroxy-2-(2-(3-methylindazol-6-yloxy)ethylamino)ethyl)phenyl)benzene sulfonamide; or a salt thereof.

[30] A compound selected from the group consisting of
(R)-N-(3-(1-hydroxy-2-(2-(3-methylindazol-6-yloxy)ethylamino)ethyl)phenyl)cyclobutane sulfonamide and
(R)-N-(3-(2-(2-(3-(difluoromethoxy)-indazol-6-yloxy)ethylamino)-1-hydroxyethyl)phenyl)cyclopropane sulfonamide; or a salt thereof.

[31] A compound selected from the group consisting of
(R)-N-(3-(2-(2-(3-(difluoromethoxy)-indazol-6-yloxy)ethylamino)-1-hydroxyethyl)phenyl)cyclopropane sulfonamide and
(R)-2-fluoro-N-(3-(1-hydroxy-2-(2-(3-methylindazol-6-yloxy)ethylamino)ethyl)phenyl)benzene sulfonamide; or a salt thereof.

[32] A compound selected from the group consisting of
   (R)-N-(3-(1-hydroxy-2-(2-(3-methylindazol-6-yloxy)ethylamino)ethyl)phenyl)cyclobutane sulfonamide and
   (R)-2-fluoro-N-(3-(1-hydroxy-2-(2-(3-methylindazol-6-yloxy)ethylamino)ethyl)phenyl)benzene sulfonamide; or a salt thereof.
[33] The compound described in [1] above and any one of [27]-[32] above; or a salt thereof.

[34] A β3-adrenergic receptor agonist containing the compound described in any one of [1]-[33] above or a salt thereof as the active ingredient.

[35] A drug containing the compound described in any one of [1]-[33] above or a salt thereof as the active ingredient.

[36] The drug described in [35] above that is a drug to prevent and/or treat overactive bladder and urinary incontinence.

[37] A method for acting on a β3-adrenergic receptor in the body of a patient characterized in that the compound described in any one of [1]-[33] above or a salt thereof is administered to a patient who requires prevention and/or treatment of overactive bladder and urinary incontinence.

[37-1] The method described in [37] above in which the above administration substantially does not act on the α1-adrenergic receptor in the body of the above patient.

[37-2] The method described in [37] above in which the above patient is a patient who should avoid substantial action on the α1-adrenergic receptor by drug administration.

[38] A method for preventing and/or treating overactive bladder and urinary incontinence characterized in that an effective dose of the compound described in any one of [1]-[33] above is administered to a patient.

[38-1] The method described in [38] above in which the above patient is a patient who should avoid substantial action on the α1-adrenergic receptors by drug administration.
[39] The compound described in any one of [1]-[33] above or a salt thereof to be used as a preventative drug or a therapeutic drug.
[40] A drug composition that contains the compound described in any one of [1]-[33] above or a salt thereof and a pharmaceutically acceptable salt.
[41] The compound described in any one of [1]-[33] above or a salt thereof to be used in the prevention and/or treatment of overactive bladder and urinary incontinence.
[42] Use of the compound described in any one of [1]-[33] above or a salt thereof to manufacture a drug composition for the prevention and/or treatment of overactive bladder and urinary incontinence.

When administered to humans and animals, the "compounds shown by the general formula (I) and salts thereof" (occasionally referred to below simply as "compounds of the present invention") have the effect of relaxing the smooth muscles of the bladder by strong β3-adrenergic receptor agonist activity and have the excellent characteristic of high β3/α1-adrenergic receptor selectivity, making it possible to provide drug compositions that are excellent for the treatment of overactive bladder and urinary incontinence.

### BEST MODE FOR CARRYING OUT THE INVENTION

The compounds of the present invention are explained in detail below.

The terminology used in this specification is explained below.

"Lower alkyl group" means an optionally branched C₁-C₆ alkyl group, preferably a methyl group, ethyl group, n-propyl group, n-butyl group, isopropyl group, isobutyl group, or sec-butyl group, more preferably a method group, ethyl group, or isopropyl group, and especially preferably a methyl group. There are also other embodiments in which an ethyl group and isopropyl group are preferred.

"Cyclic lower alkyl group" means a C₃-C₆ cyclic alkyl group. When this "cyclic lower alkyl group" is optionally substituted, the substituent is selected as is appropriate from lower alkyl groups. Therefore, groups shown by the following general formulae (II)-(III)

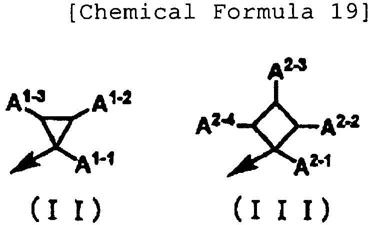

[in general formulae (II)-(III), A¹⁻¹, A¹⁻², A¹⁻³, A²⁻¹, A²⁻², A²⁻³, and A²⁻⁴ may be the same and are each independently a hydrogen atom or methyl group] are preferred as examples of "optionally substituted cyclic lower alkyl groups." A 1-methylcyclopropyl group, cyclopropyl group, 1-methylcyclobutyl group, or cyclobutyl group is more preferred, and a cyclopropyl group or cyclobutyl group is especially preferred.

"Halogen atom" means a fluorine atom, chlorine atom, bromine atom, or iodine atom. A fluorine atom or chlorine atom is preferred, and a chlorine atom is especially preferred. There are also other embodiments in which a fluorine atom is preferred.

"Perfluoroalkyl group" means a perfluoroalkyl group of from C₁ to C₃. Examples include a trifluoromethyl group, pentafluoroethyl group, and 1,1,1,2,2,3,3-heptafluoropropyl group, and a trifluoromethyl group is more preferred.

"Lower alkyl group substituted by one, two, or more halogen atoms" means an optionally branched C₁-C₆ alkyl group having one, two, or more of the above halogen atoms. The above halogen atoms are a fluorine atom, chlorine atom, bromine atom, and iodine atom. A fluorine atom or chlorine atom is preferred, and a fluorine atom is especially preferred. There are also other embodiments in which a chlorine atom is preferred. Examples of this optionally branched C₁-C₆ lower group having one, two, or more halogen atoms include a methyl group, ethyl group, n-propyl group, n-butyl group, isopropyl group, isobutyl group, and sec-butyl group.

The heterocycle in the "optionally substituted heterocycle" is usually a 5- to 6-membered monocyclic ring, and at least one or more of the atoms of this ring is a nitrogen, sulfur, or oxygen atom. This heterocycle may be aromatic or non-aromatic, or it may be partially unsaturated. An aromatic heterocycle is preferred.

When this heterocycle has substituents, these substituents may be lower alkyl groups, cyclic lower alkyl group, halogen atoms, -N(P¹⁻¹) (P¹⁻²) (where, P¹⁻¹ and P¹⁻² may be the same or different and are each independently a hydrogen atom, lower alkyl group, cyclic lower alkyl group, or acetyl group}, or -OP² {where, P² is a hydrogen atom, lower alkyl group, or cyclic lower alkyl group}. Therefore, rings shown by the following general formulae (IV-I)-(IV-XI)

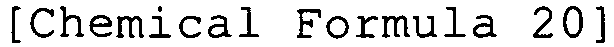

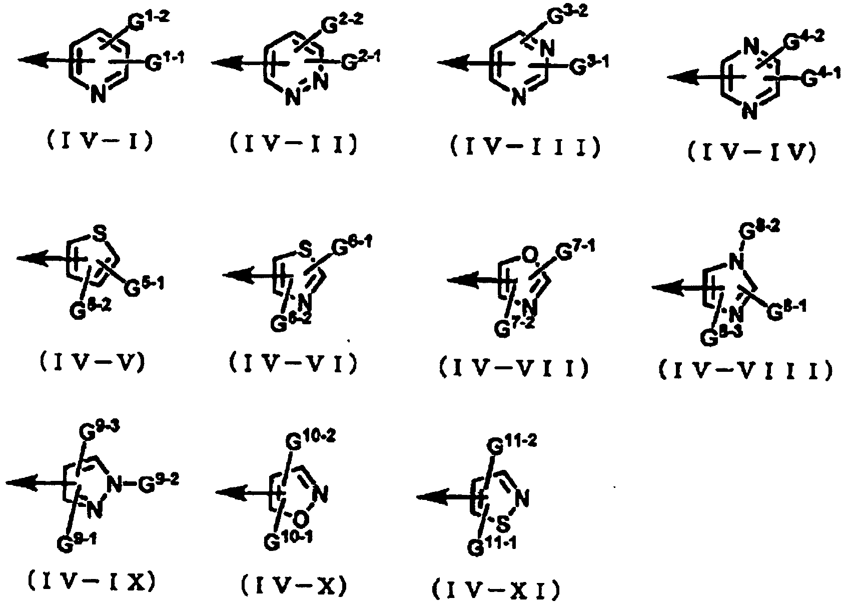

(in general formulae (IV-I)-(IV-XI), G¹⁻¹, G¹⁻², G²⁻¹, G²⁻², G³⁻¹, G³⁻², G⁴⁻¹, G⁴⁻², G⁵⁻¹, G⁵⁻², G⁶⁻¹, G⁶⁻², G⁷⁻¹, G⁷⁻², G⁸⁻¹, G⁸⁻², G⁸⁻³, G⁹⁻¹, G⁹⁻², G⁹⁻³, G¹⁰⁻¹, G¹⁰⁻², G¹¹⁻¹, and G¹¹⁻² are a hydrogen atom, lower alkyl group, cyclic lower alkyl group, halogen atom, - N(P¹⁻¹) (P¹⁻²) , or -OP², P¹⁻¹ and P¹⁻² may be the same or different and are each independently a hydrogen atom, lower alkyl group, cyclic lower alkyl group, or acetyl group, P² is a hydrogen atom, lower alkyl group, or cyclic lower alkyl group; however, G⁸⁻² and G⁹⁻² are not halogen atoms, -N(P¹⁻¹) (P¹⁻²), or -OP²) can be given as examples of a suitable "optionally substituted heterocycle." Rings shown by the following general formulae (IV-I)-(IV-XI)

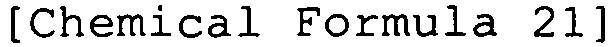

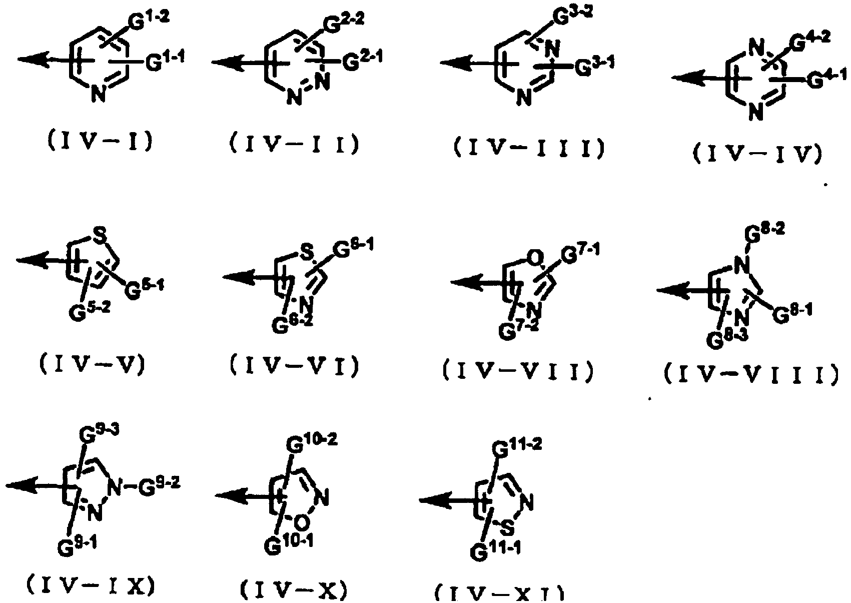

(in general formulae (IV-I)-(IV-XI), G¹⁻¹, G¹⁻², G²⁻¹, G²⁻² G³⁻¹, G³⁻², G⁹⁻¹, G⁴⁻², G⁵⁻¹, G⁵⁻² G⁶⁻¹, G⁶⁻², G⁷⁻¹,G⁷⁻², G⁸⁻¹, G⁸⁻², G⁸⁻³, G⁹⁻¹, G⁹⁻², G⁹⁻³, G¹⁰⁻¹, G¹⁰⁻², G¹¹⁻¹, and G¹¹⁻² are a hydrogen atom, lower alkyl group, cyclic lower alkyl group, halogen atom, - N(P¹⁻¹) (P¹⁻²), or -OP², P¹⁻¹ and P¹⁻² may be the same or different and are each independently a hydrogen atom, lower alkyl group, cyclic lower alkyl group, or acetyl group, P² is a hydrogen atom, lower alkyl group, or cyclic lower alkyl group; however, excluding combinations in which G⁸⁻¹ is a hydrogen atom and G⁸⁻³ is a hydrogen atom when G⁸⁻² is a methyl group; and G⁸⁻² and G⁹⁻² are not halogen atoms, -N(P¹⁻¹) (P¹⁻²), or -OP²) are preferred, and rings shown by the following formulas (V-I)-(V-V)

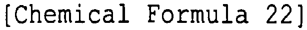

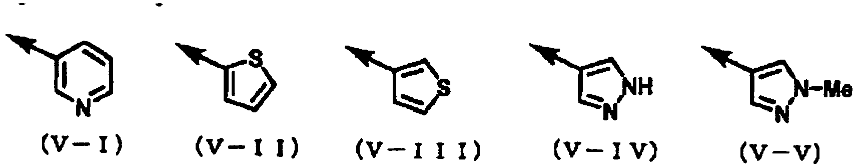

are more preferred.

In the present invention, examples of a "substituted phenyl group" include groups shown by the following general formula (VI)

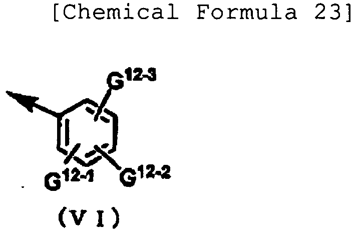

(in general formula (VI), G¹²⁻¹, G¹²⁻², and G¹²⁻³ are a hydrogen atom, lower alkyl group, cyclic lower alkyl group, halogen atom, trifluoromethyl group -COOH, nitro group, -N(Q¹⁻¹) (Q¹⁻²), or -OQ², Q¹⁻¹ and Q¹⁻² may be the same or different and are each independently a hydrogen atom, lower alkyl group, cyclic lower alkyl group, or acetyl group, Q² is a hydrogen atom, lower alkyl group, cyclic lower alkyl group, difluoromethoxy group, or trifluoromethyl group; however, excluding combinations in which G¹²⁻¹, G¹²⁻², and G¹²⁻³ are all hydrogen atoms). Groups shown by the following formulas (VII-I)-(VII-XI) 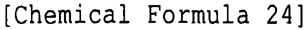 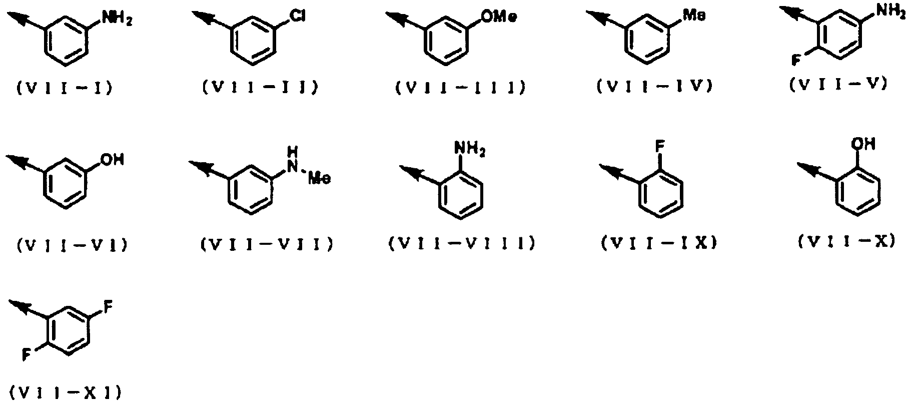 are preferred; groups shown by the following formulas (VII-I)-(VII-X) 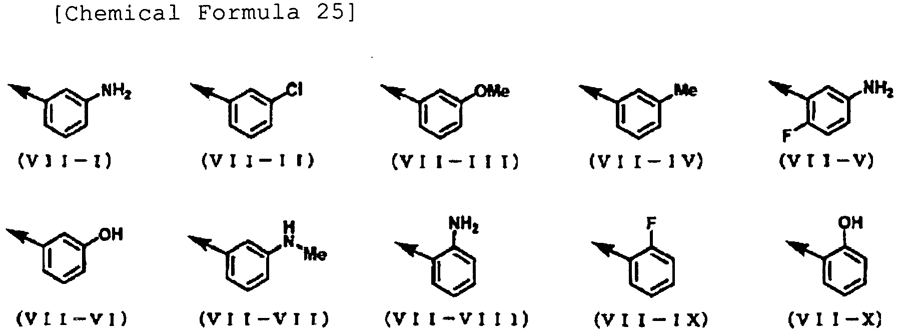 are more preferred; and groups shown by the following formulas (VII-I)-(VII-V) and (VII-IX) 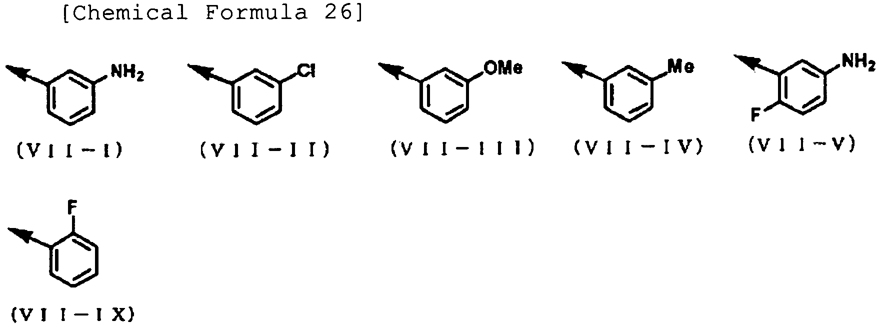 are especially preferred.

In this specification, the symbol

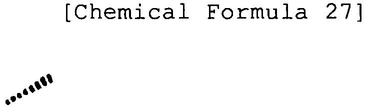

means bonding in the direction heading away from the reader (i.e., an α-arrangement); the symbol

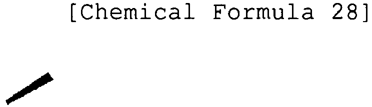

means bonding in the direction heading towards the reader (i.e., a β-arrangement); and the symbol

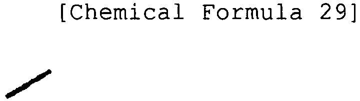

means either an α- arrangement or a β- arrangement, or a mixture of the two, as will be apparent to those skilled in the art.

The compounds of the present invention are explained concretely below.

General formula (I) below:

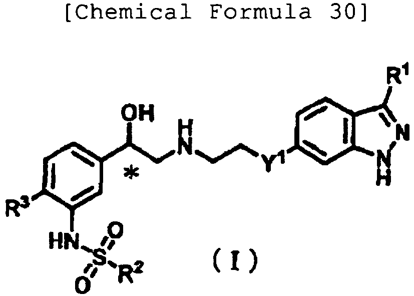

In general formula (I), R¹ is a lower alkyl group, optionally substituted cyclic lower alkyl group, halogen atom, perfluoroalkyl group, -OR⁴-, CH(R⁵)OR⁶, or -(CH₂)ₙCONR⁷⁻¹R⁷⁻²; R² is an optionally substituted cyclic lower alkyl group, lower alkyl group substituted by one, two, or more halogen atoms, optionally substituted heterocycle, or substituted phenyl group; R³ is a hydrogen atom or halogen atom, Y¹ is an oxygen atom, -NR⁸-, or methylene group; R⁴ is a lower alkyl group, optionally substituted cyclic lower alkyl group, difluoromethyl group, or trifluoromethyl group; R⁵ is a hydrogen atom, lower alkyl group, or optionally substituted cyclic lower alkyl group; R⁶ is a hydrogen atom, lower alkyl group, optionally substituted cyclic lower alkyl group, difluoromethyl group, or trifluoromethyl group; R⁷⁻¹ and R⁷⁻² may be the same or different and are each independently a hydrogen atom, lower alkyl group, or optionally substituted cyclic lower alkyl group; R⁸ is a hydrogen atom, lower alkyl group, or optionally substituted cyclic lower alkyl group, n is 0, 1, or 2; and the "*" symbol denotes an asymmetric carbon.

Examples of R¹ include a lower alkyl group, optionally substituted cyclic lower alkyl group, halogen atom, perfluoroalkyl group, OR⁴-, -CH(R⁵)OR⁶, or -(CH₂)ₙCONR⁷⁻¹R⁷⁻². A lower alkyl group, optionally substituted cyclic lower alkyl group, halogen atom, perfluoroalkyl group, OR⁴- or -(CH₂)ₙCONR⁷⁻¹R⁷⁻² are preferred; a methyl group, ethyl group, isopropyl group, cyclopropyl group, cyclobutyl group, chlorine atom, trifluoromethyl group, -OMe, -OCHF₂, and -(CH₂)₂CONMe₂ are more preferred; and a methyl group, cyclopropyl group, chlorine atom, trifluoromethyl group, -OMe, and -OCHF₂ are especially preferred. A methyl group is ideal. There are also other embodiments in which an ethyl group is preferred, other embodiments in which a cyclopropyl group is preferred, other embodiments in which a chlorine atom is preferred, other embodiments in which a trifluoromethyl group is preferred, other embodiments in which -OMe is preferred, and other embodiments in which -OCHF₂ is preferred.
Examples of R² include an optionally substituted cyclic lower alkyl group, optionally substituted heterocycle, and substituted phenyl group. Groups shown by the following general formulae (II)-(III)

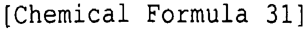

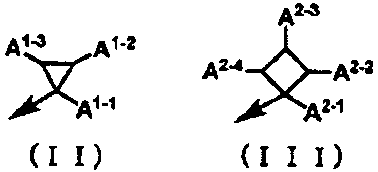

(in general formulae (II)-(III), A¹⁻¹, A¹⁻², A¹⁻³, A²⁻¹, A²⁻², A²⁻³, and A²⁻⁴ may be the same and are each independently a hydrogen atom or methyl group), rings shown by the following general formulae (IV-I)-(IV-XI)

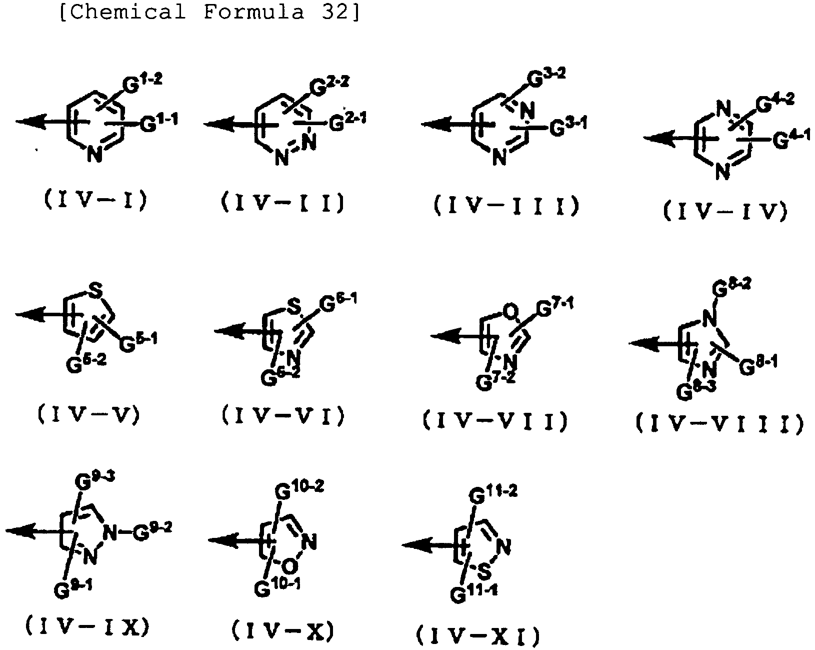

(in general formulae (IV-I)-(IV-XI), G¹⁻¹, G¹⁻², G²⁻¹, G²⁻², G³⁻¹, G³⁻², G⁴⁻¹, G⁴⁻², G⁵⁻¹, G⁵⁻², G⁶⁻¹, G⁶⁻², G⁷⁻¹, G⁷⁻², G⁸⁻¹, G⁸⁻², G⁸⁻³, G⁹⁻¹ G³⁻², G⁴⁻¹, G⁴⁻², G⁵⁻¹, G⁵⁻², G⁶⁻², G⁶⁻², G⁷⁻¹, G⁷⁻²,G⁸⁻¹, G⁸⁻², G⁸⁻³, G⁹⁻¹, G⁹⁻², G⁹⁻³, G¹⁰⁻¹, G¹⁰⁻², G¹¹⁻¹, and G¹¹⁻² are a hydrogen atom, lower alkyl group, cyclic lower alkyl group, halogen atom, - N (P¹⁻¹) (P¹⁻²), or -OP², P¹⁻¹ and P¹⁻² may be the same or different and are each independently a hydrogen atom, lower alkyl group, cyclic lower alkyl group, or acetyl group, P² is a hydrogen atom, lower alkyl group, or cyclic lower alkyl group; however, excluding combinations in which G⁸⁻¹ is a hydrogen atom and G⁸⁻³ is a hydrogen atom when G⁸⁻² is a methyl group; and G⁸⁻² and G⁹⁻² are not halogen atoms, -N(P¹⁻¹) (P¹⁻²), or -OP²), and groups shown by the following general formula (VI)

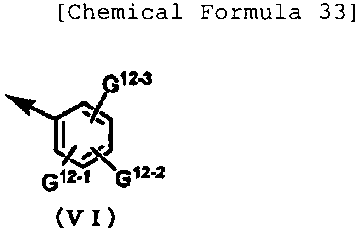

(in general formula (VI), G¹²⁻¹, G¹²⁻² , and G¹²⁻³ are a hydrogen atom, lower alkyl group, cyclic lower alkyl group, halogen atom, trifluoromethyl group -COOH, nitro group, -N(Q¹⁻¹)(Q¹⁻²), or -OQ², Q¹⁻¹ and Q¹⁻² may be the same or different and are each independently a hydrogen atom, lower alkyl group, cyclic lower alkyl group, or acetyl group, Q² is a hydrogen atom, lower alkyl group, cyclic lower alkyl group, difluoromethoxy group, or trifluoromethyl group; however, excluding combinations in which G¹²⁻¹, G¹²⁻², and G¹²⁻³ are all hydrogen atoms) are preferred; a 1-methylcyclopropyl group, cyclopropyl group, 1-methylcyclobutyl group, cyclobutyl group, formulas (V-I)-(V-V)

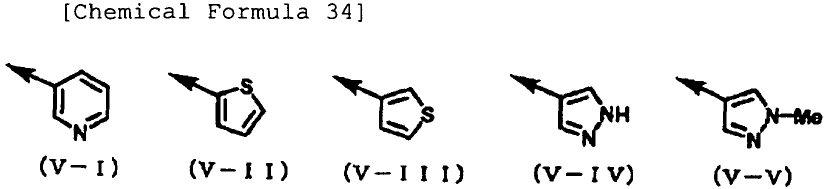

formulas (VII-I)-(VII-XI)

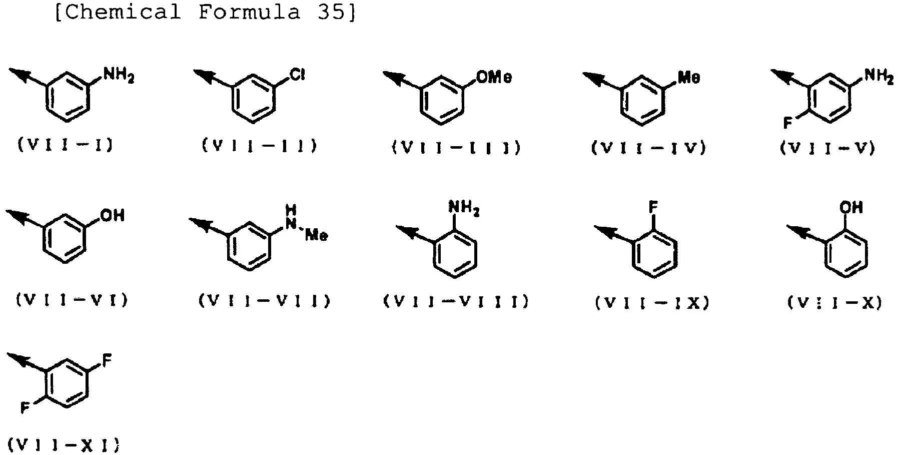

are more preferred; a 2-fluorophenyl group, cyclopropyl group, cyclobutyl group are especially preferred; and a cyclobutyl group is ideal. There are also other embodiments in which a 2-fluorophenyl group is preferred, other embodiments in which a cyclopropyl group is preferred, other embodiments in which formulas (V-I)-(V-V) are preferred, and other embodiments in which formulas (VII-I)-(VII-XI) are more preferred.

R³ is a hydrogen atom or halogen atom. A hydrogen atom, fluorine atom, and chlorine atom are preferred, and a hydrogen atom is especially preferred. There are also other embodiments in which a fluorine atom is preferred, and other embodiments in which a chlorine atom is more preferred.

Y¹ is an oxygen atom, -NR⁸-, or a methylene group. An oxygen atom is preferred.

R⁴ is a lower alkyl group, optionally substituted cyclic lower alkyl group, difluoromethyl group, or trifluoromethyl group. A methyl group or difluoromethyl group is preferred.

R⁵ is a hydrogen atom, lower alkyl group, or cyclic lower alkyl group. A hydrogen atom or methyl group is preferred, and a hydrogen atom is more preferred. There are also other embodiments in which a methyl group is preferred.

R⁶ is a hydrogen atom, lower alkyl group, cyclic lower alkyl group, difluoromethyl group, or trifluoromethyl group. A hydrogen atom or methyl group is preferred, and a hydrogen atom is more preferred. There are also other embodiments in which a methyl group is preferred.

R⁷⁻¹ and R⁷⁻² may be the same or different and are each independently a hydrogen atom, lower alkyl group, or optionally substituted cyclic lower alkyl group. A lower alkyl group or optionally substituted cyclic lower alkyl group is preferred, and a methyl group is more preferred.

R⁸ is a hydrogen atom, lower alkyl group, or optionally substituted cyclic lower alkyl group. A hydrogen atom is preferred.

n is 0, 1, or 2, and is preferably 2.

The "*" symbol denotes an asymmetric carbon. The configuration of this asymmetric carbon may be S or R. An R configuration is preferred.

The compounds of the present invention include any optically pure optical isomers based on this asymmetric carbon, any mixtures of optical isomers, and racemic mixtures thereof. For example, isomers based on the presence of an asymmetric carbon (R- or S-isomers, isomers based on α- or β-configuration, enantiomers, diastereomers, and the like), optically active substances having optical rotation (D- or L-compounds or d- or 1-compounds), isomers based on differences in polarity by chiral chromatography (high-polarity compounds and low-polarity compounds), equilibrium compounds, rotamers, tautomers, and mixtures of any proportions thereof, and racemic mixtures are all included among compounds of the present invention.

For example, a compound in which R¹ is -CH(R⁵)OR⁶ and the configuration of the asymmetric carbon shown by the "*" symbol is (R) is a mixture of diastereomers, but each of the optically active compounds are also included among compounds of the present invention.

"Compounds shown by the general formula (I)" in this specification is generally interpreted as free compounds shown by the general formula (I). The following salts can be given as examples of salts thereof.

Specifically, the type of salt is not particularly restricted in compounds of the present invention. It may be an acid addition salt or may take the form of an intramolecular counterion. Pharmaceutically acceptable salts are especially preferred as the salt in the case of an active ingredient of a drug in particular. The salt in the compounds of the present invention is usually interpreted to be a pharmaceutically acceptable salt when disclosed in relationship to use as a drug. The types of acids that form pharmaceutically acceptable salts are known to those skilled in the art, including, for example, those listed by Berge et al. in J. Pharm. Sci., 1-19 (1977). For example, acid addition salts include hydrochlorides, hydrobromides, hydroiodides, nitrates, sulfates, hydrogensulfates, phosphates, hydrogenphosphates, and other such mineral acid salts, acetates, trifluoroacetates, gluconates, lactates, salicylates, citrates, tartrates, ascorbates, succinates, maleates, fumarates, formates, benzoates, methanesulfonates, ethanesulfonates, p-toluenesulfonates, and other such organic acid salts.

For example, when obtaining a salt of an inorganic acid, it is preferable to dissolve a compound shown by the general formula (I) in an aqueous solution that contains at least one equivalent of the desired inorganic acid. Methanol, ethanol, acetone, dioxane, or another such water-miscible, inert organic solvent may be added in this reaction. For example, a solution of a hydrochloride is obtained by using hydrochloric acid.

A compound of the present invention may be an anhydride. It may also be preferable that a compound of the present invention is a hydrate.

It may also be preferable that a compound of the present invention is a solvate, but there are also preferred examples in which it is a non-solvate.

A compound of the present invention may be crystalline or amorphous. The above crystals may be monocrystalline, a mixture of multiple crystal forms, or any mixture of crystalline and amorphous.

More concretely, a "compound shown by the general formula (I)" may be an anhydride and non-solvate or a hydrate and/or solvate, and there are also examples in which crystals of these are preferred.

Furthermore, a "salt of a compound shown by the general formula (I)" may be an anhydride and non-solvate, a hydrate and/or solvate of this salt, an anhydride and non-solvate of this salt, or a hydrate and/or solvate of this salt.

The following combinations can be given as examples of preferred combinations of substituents in the compounds of the present invention shown by the general formula (I); or a salt thereof.

(1) Compounds of the present invention in which the configuration of the asymmetric carbon shown by the "*" symbol is (R); or a salt thereof.
(2) Compounds of the present invention in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R² is a cyclopropyl group, cyclobutyl group, group shown by any of formulas (V-I)-(V-V), or group shown by any of formulas (VII-I)-(VII-X)

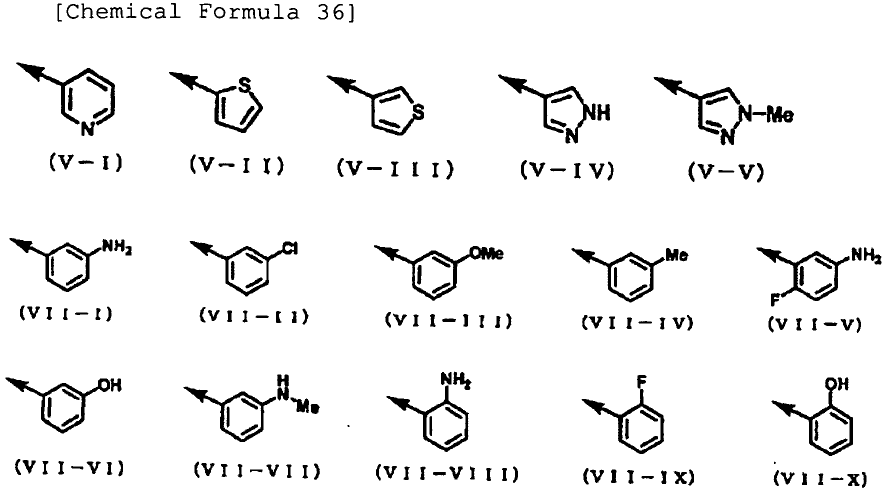

R³ is a hydrogen atom, fluorine atom, or chlorine atom, Y¹ is an oxygen atom, -NH-, or a methylene group, and the configuration of the asymmetric carbon shown by the "*" symbol is (R); or a salt thereof.

(3) Compounds of the present invention in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R² is a cyclopropyl group, cyclobutyl group, group shown by any of the formulas (V-I)-(V-V), or group shown by any of the formulas (VII-I)-(VII-X)

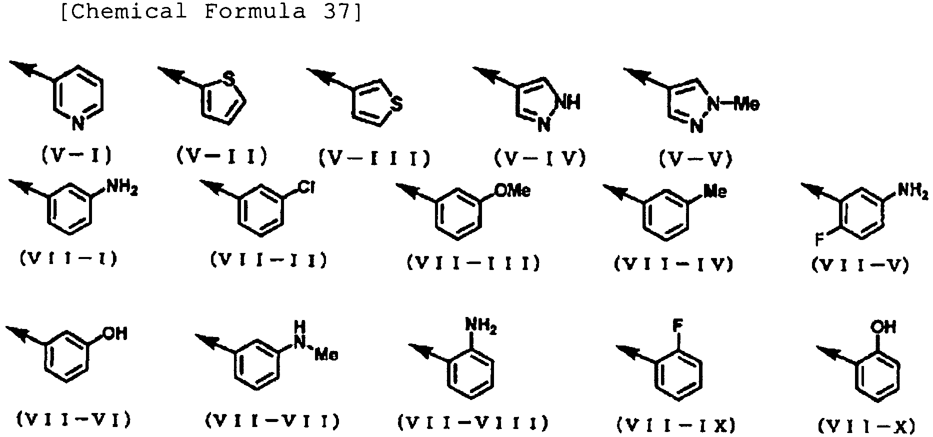

R³ is a hydrogen atom, fluorine atom, or chlorine atom, Y¹ is an oxygen atom, and the configuration of the asymmetric carbon shown by the "*" symbol is (R); or a salt thereof.
(4) Compounds of the present invention in which R¹ is a methyl group, R² is a cyclopropyl group, cyclobutyl group, group shown by any of the formulas (V-I)-(V-V), or group shown by any of the formulas (VII-I)-(VII-XI) 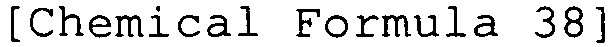 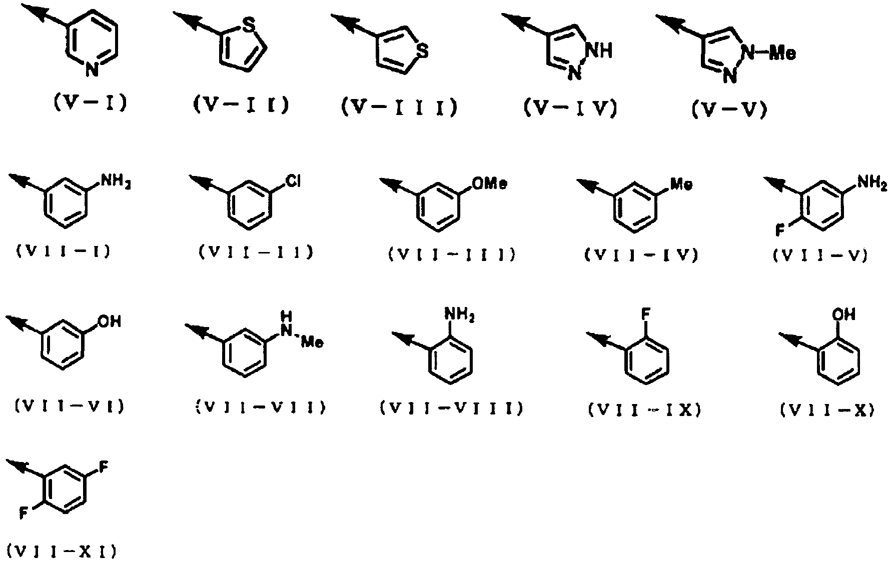
R³ is a hydrogen atom, Y¹ is an oxygen atom, and the configuration of the asymmetric carbon shown by the "*" symbol is (R); or a salt thereof.

(5) Compounds of the present invention in which R¹ is a methyl group, R² is a cyclopropyl group, cyclobutyl group, group shown by any of the formulas (V-I)-(V-V), or group shown by any of the formulas (VII-I)-(VII-XI) 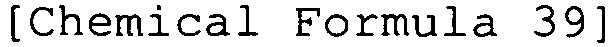 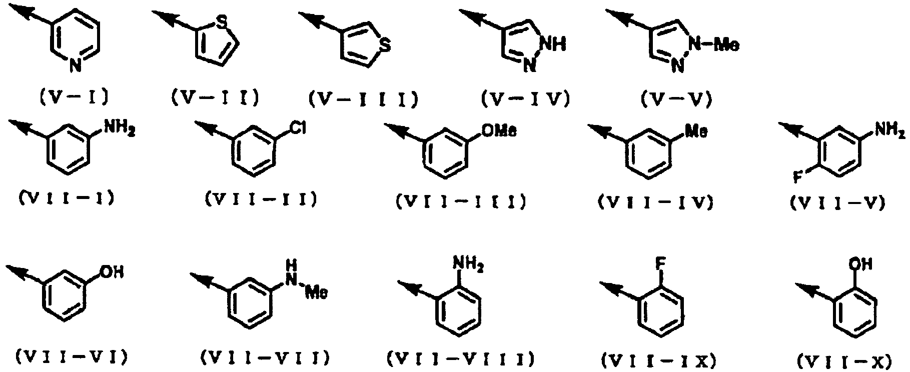 R³ is a hydrogen atom, fluorine atom, or chlorine atom, Y¹ is an oxygen atom, and the configuration of the asymmetric carbon shown by the "*" symbol is (R); or a salt thereof.
(6) Compounds of the present invention in which R¹ is a methyl group, R² is a cyclopropyl group, cyclobutyl group, group shown by any of the formulas (V-I)-(V-V), or group shown by any of the formulas (VII-I)-(VII-XI) 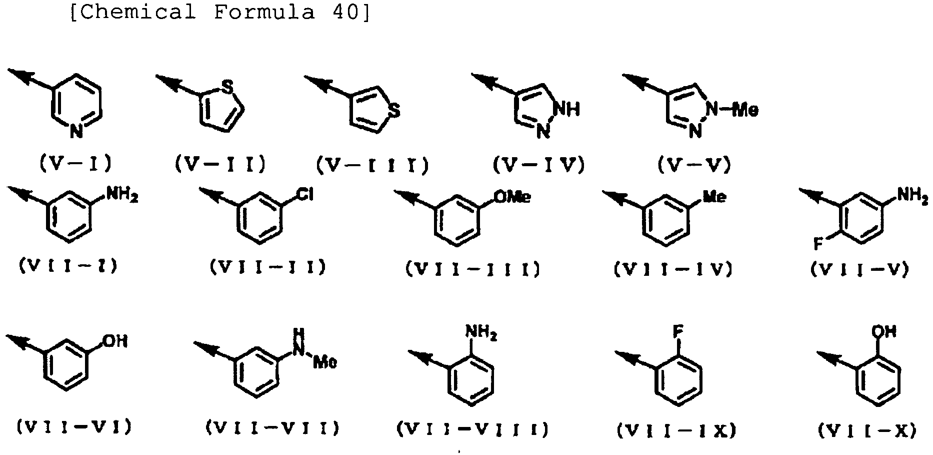 R³ is a hydrogen atom, Y¹ is an oxygen atom, and the configuration of the asymmetric carbon shown by the "*" symbol is (R); or a salt thereof.

(7) Compounds of the present invention in which R¹ is a methyl group, R² is a cyclopropyl group, cyclobutyl group, group shown by any of the following formulas (V-I)-(V-V) 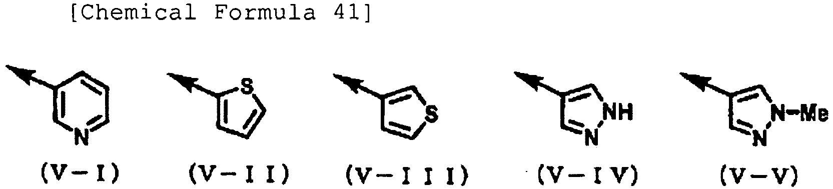 or group shown by any of the following formulas (VII-I)-(VII-V) and (VII-IX) 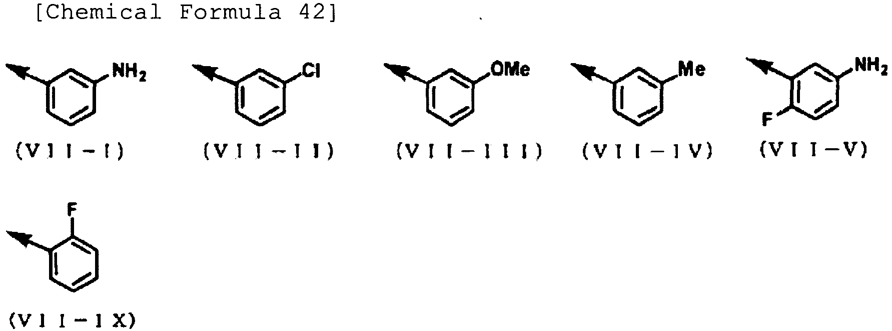 R³ is a hydrogen atom, Y¹ is an oxygen atom, and the configuration of the asymmetric carbon shown by the "*" symbol is (R); or a salt thereof.

(8) Compounds of the present invention in which R¹ is an ethyl group, R² is a cyclopropyl group, group shown by any of the following formulas (V-I)-(V-V) 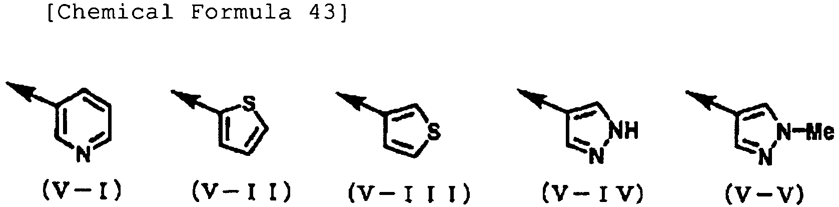 or group shown by any of the following formulas (VII-I)-(VII-V) and (VII-IX) 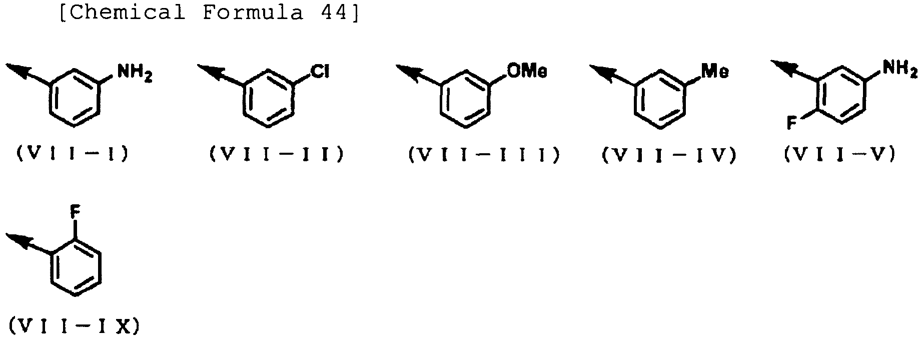 R³ is a hydrogen atom, Y¹ is an oxygen atom, and the configuration of the asymmetric carbon shown by the "*" symbol is (R); or a salt thereof.

(9) Compounds of the present invention in which R¹ is a cyclopropyl group, R² is a cyclopropyl group, cyclobutyl group, group shown by any of the following formulas (V-I)-(V-V) 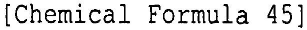 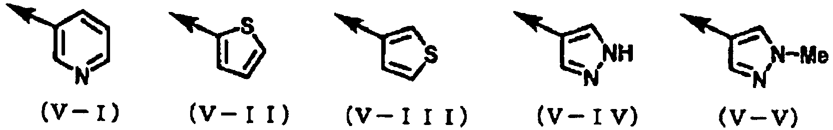 or group shown by any of the following formulas (VII-I)-(VII-V) and (VII-IX) 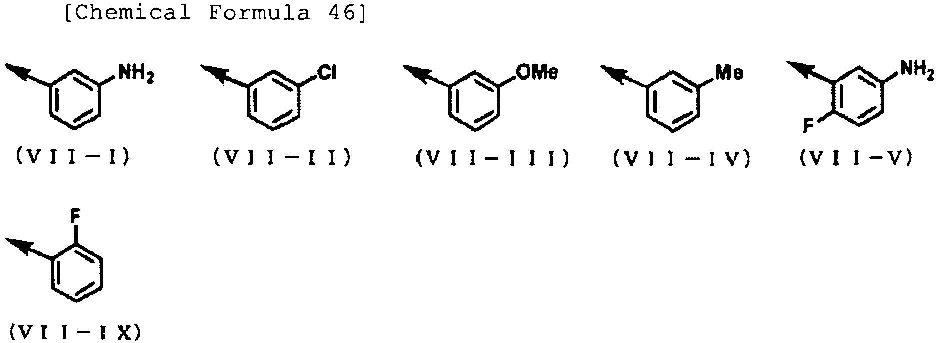 R³ is a hydrogen atom, Y¹ is an oxygen atom, and the configuration of the asymmetric carbon shown by the "*" symbol is (R); or a salt thereof.
(10) Compounds of the present invention in which R¹ is a cyclopropyl group, R² is a cyclopropyl group, group shown by any of the following formulas (V-I)-(V-V) 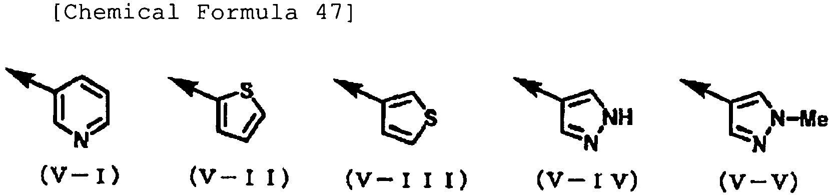 or group shown by any of the following formulas (VII-I)-(VII-V) and (VII-IX) 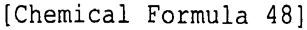 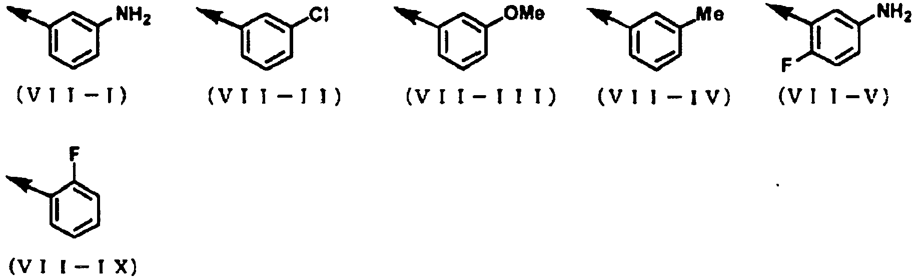 R³ is a hydrogen atom, Y¹ is an oxygen atom, and the configuration of the asymmetric carbon shown by the "*" symbol is (R); or a salt thereof.

(11) Compounds of the present invention in which R¹ is a chlorine atom, R² is a cyclopropyl group, cyclobutyl group, group shown by any of the following formulas (V-I)-(V-V) 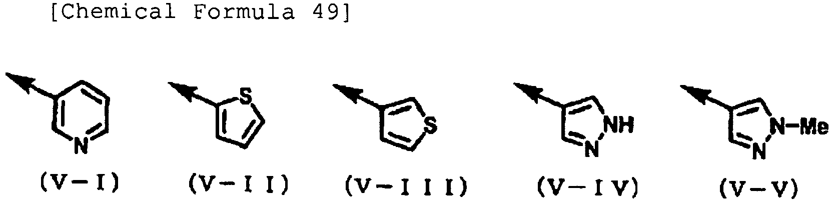 or group shown by any of the following formulas (VII-I)-(VII-V) and (VII-IX) 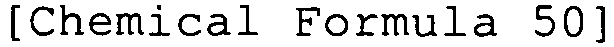 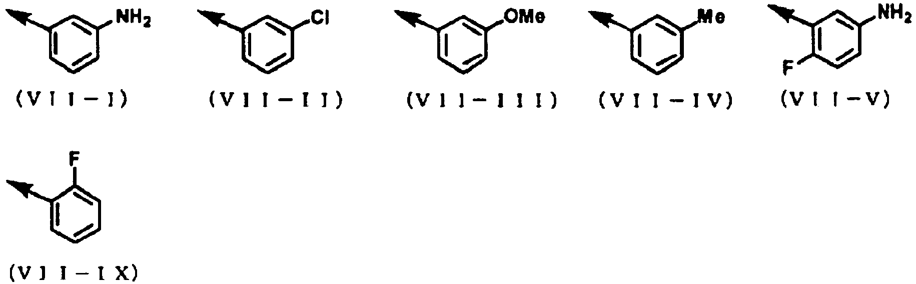 R³ is a hydrogen atom, Y¹ is an oxygen atom, and the configuration of the asymmetric carbon shown by the "*" symbol is (R); or a salt thereof.
(12) Compounds of the present invention in which R¹ is a chlorine atom, R² is a cyclopropyl group, group shown by any of the following formulas (V-I)-(V-V) 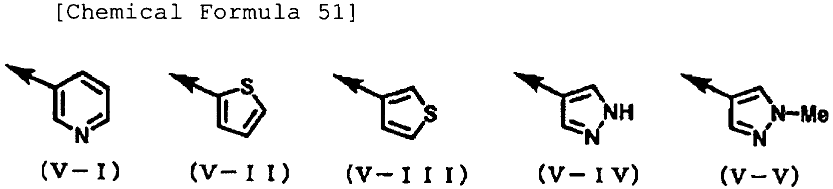 or group shown by any of the following formulas (VII-I)-(VII-V) and (VII-IX) 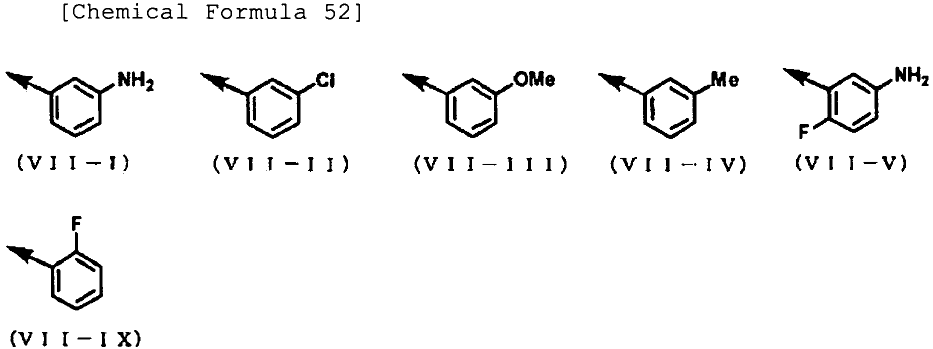 R³ is a hydrogen atom, Y¹ is an oxygen atom, and the configuration of the asymmetric carbon shown by the "*" symbol is (R); or a salt thereof.

(13) Compounds of the present invention in which R¹ is a trifluoromethyl group, R² is a cyclopropyl group, group shown by any of the following formulas (V-I)-(V-V) 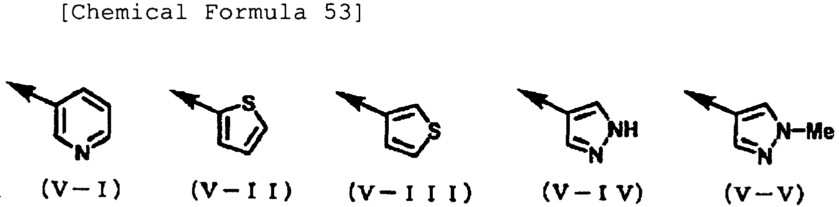 or group shown by any of the following formulas (VII-I)-(VII-V) and (VII-IX) 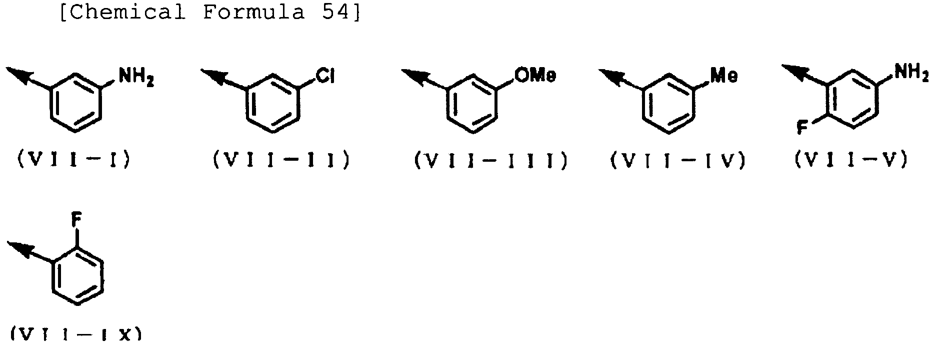 R³ is a hydrogen atom, Y¹ is an oxygen atom, and the configuration of the asymmetric carbon shown by the "*" symbol is (R); or a salt thereof.

(14) Compounds of the present invention in which R¹ is - OMe, R² is a cyclopropyl group, group shown by any of the following formulas (V-I)-(V-V) 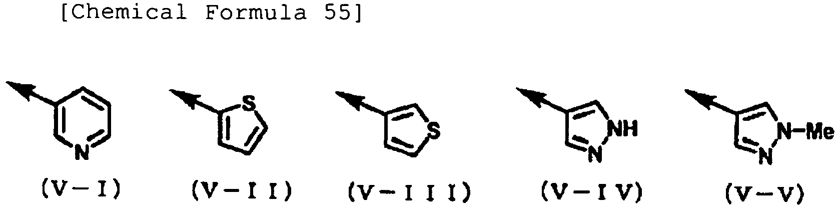 or group shown by any of the following formulas (VII-I)-(VII-V) and (VII-IX) 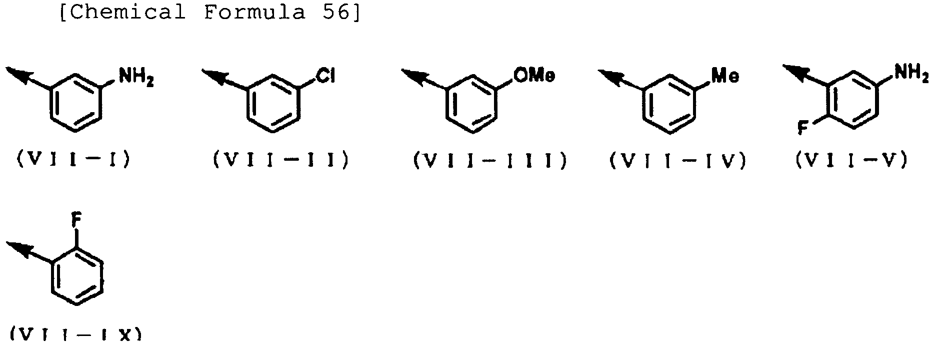 R³ is a hydrogen atom, Y¹ is an oxygen atom, and the configuration of the asymmetric carbon shown by the "*" symbol is (R); or a salt thereof.

(15) Compounds of the present invention in which R¹ is -OCHF₂, R² is a cyclopropyl group, cyclobutyl group, group shown by any of the following formulas (V-I)-(V-V) 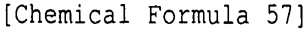 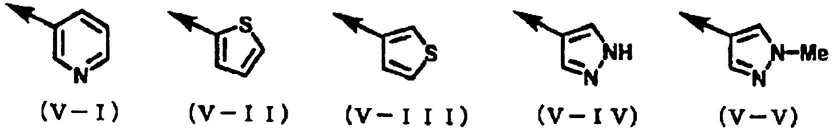 or group shown by any of the following formulas (VII-I)-(VII-V) and (VII-IX) 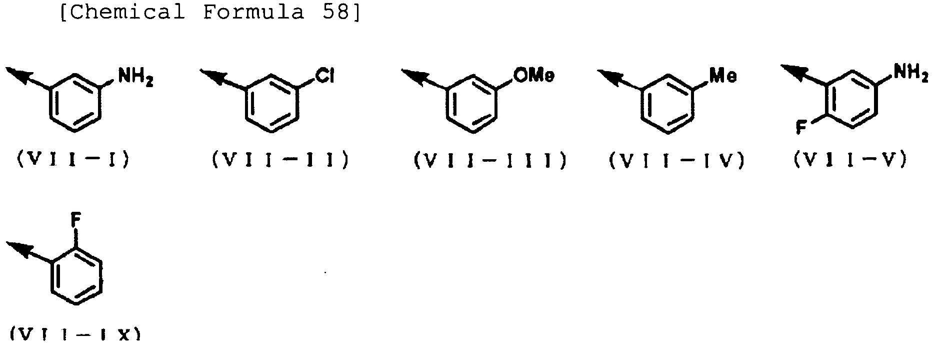 R³ is a hydrogen atom, Y¹ is an oxygen atom, and the configuration of the asymmetric carbon shown by the "*" symbol is (R); or a salt thereof.

(16) Compounds of the present invention in which R¹ is a methyl group, isopropyl group, cyclopropyl group, cyclobutyl group, chlorine atom, or -OCHF₂, R² is a cyclopropyl group or cyclobutyl group, R³ is a hydrogen atom, Y¹ is an oxygen atom, and the configuration of the asymmetric carbon shown by the "*" symbol is (R); or a salt thereof.

(17) Compounds of the present invention in which R¹ is a methyl group, ethyl group, isopropyl group, cyclopropyl group, cyclobutyl group, chlorine atom, trifluoromethyl group, -OMe, or -OCHF₂, R² is a cyclopropyl group, R³ is a hydrogen atom, Y¹ is an oxygen atom, and the configuration of the asymmetric carbon shown by the "*" symbol is (R); or a salt thereof.

(18) Compounds of the present invention in which R¹ is a methyl group, isopropyl group, cyclopropyl group, cyclobutyl group, chlorine atom, or -OCHF₂, R² is a cyclobutyl group, R³ is a hydrogen atom, Y¹ is an oxygen atom, and the configuration of the asymmetric carbon shown by the "*" symbol is (R); or a salt thereof.
(19) Compounds of the present invention in which R¹ is a methyl group or cyclobutyl group, R² is a cyclobutyl group, R³ is a hydrogen atom, Y¹ is an oxygen atom, and the configuration of the asymmetric carbon shown by the "*" symbol is (R); or a salt thereof.

(20) Compounds of the present invention in which R² is a cyclopropyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, fluorine atom, or chlorine atom, Y¹ is an oxygen atom, -NH-, or a methylene group, and the configuration of the asymmetric carbon shown by the "*" symbol is (R); or a salt thereof.

(21) Compounds of the present invention in which R² is a cyclopropyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, fluorine atom, or chlorine atom, Y¹ is an oxygen atom, and the configuration of the asymmetric carbon shown by the "*" symbol is (R); or a salt thereof.

(22) Compounds of the present invention in which R² is a cyclopropyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, Y¹ is an oxygen atom, -NH-, or a methylene group, and the configuration of the asymmetric carbon shown by the "*" symbol is (R); or a salt thereof.

(23) Compounds of the present invention in which R² is a cyclopropyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, Y¹ is an oxygen atom, and the configuration of the asymmetric carbon shown by the "*" symbol is (R); or a salt thereof.

(24) Compounds of the present invention in which R² is a cyclobutyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, fluorine atom, or chlorine atom, Y¹ is an oxygen atom, -NH-, or a methylene group, and the configuration of the asymmetric carbon shown by the "*" symbol is (R); or a salt thereof.

(25) Compounds of the present invention in which R² is a cyclobutyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, fluorine atom, or chlorine atom, Y¹ is an oxygen atom, and the configuration of the asymmetric carbon shown by the "*" symbol is (R); or a salt thereof.

(26) Compounds of the present invention in which R² is a cyclobutyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, Y¹ is an oxygen atom, -NH-, or a methylene group, and the configuration of the asymmetric carbon shown by the "*" symbol is (R); or a salt thereof.

(27) Compounds of the present invention in which R² is a cyclobutyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, Y¹ is an oxygen atom, and the configuration of the asymmetric carbon shown by the "*" symbol is (R); or a salt thereof.

(28) Compounds of the present invention in which R² is a 2-fluorophenyl group, R¹ is a methyl group, ethyl group, cyclopropyl group, chlorine atom, trifluoromethyl group, -OMe, or -OCHF₂, R³ is a hydrogen atom, fluorine atom, or chlorine atom, Y¹ is an oxygen atom, -NH-, or a methylene group, and the configuration of the asymmetric carbon shown by the "*" symbol is (R); or a salt thereof.

(29) Compounds of the present invention in which R² is a 2-fluorophenyl group, R¹ is a methyl group, ethyl group, cyclopropyl group, chlorine atom, trifluoromethyl group, -OMe, or -OCHF₂, R³ is a hydrogen atom, fluorine atom, or chlorine atom, Y¹ is an oxygen atom, and the configuration of the asymmetric carbon shown by the "*" symbol is (R); or a salt thereof.

(30) Compounds of the present invention in which R² is a 2-fluorophenyl group, R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(31) Compounds of the present invention in which R² is a 2-fluorophenyl group, R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(32) Compounds of the present invention in which R² is a 3-aminophenyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, fluorine atom, or chlorine atom, Y¹ is an oxygen atom, -NH-, or a methylene group, and the configuration of the asymmetric carbon shown by the "*" symbol is (R); or a salt thereof.

(33) Compounds of the present invention in which R² is a 3-aminophenyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, fluorine atom, or chlorine atom, Y¹ is an oxygen atom, and the configuration of the asymmetric carbon shown by the "*" symbol is (R); or a salt thereof.

(34) Compounds of the present invention in which R² is a 3-aminophenyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, Y¹ is an oxygen atom, -NH-, or a methylene group, and the configuration of the asymmetric carbon shown by the "*" symbol is (R); or a salt thereof.

(35) Compounds of the present invention in which R² is a 3-aminophenyl group, R¹ is a methyl group, ethyl group, isopropyl group, cyclopropyl group, cyclobutyl group, chlorine atom, trifluoromethyl group, -OMe, -OCHF₂, or -(CH₂)₂CONMe₂, R³ is a hydrogen atom, Y¹ is an oxygen atom, and the configuration of the asymmetric carbon shown by the "*" symbol is (R); or a salt thereof.

(36) Compounds of the present invention in which R² is a 2-thienyl group, R¹ is a methyl group, ethyl group, cyclopropyl group, chlorine atom, trifluoromethyl group, -OMe, or -OCHF₂, R³ is a hydrogen atom, fluorine atom, or chlorine atom, Y¹ is an oxygen atom, -NH-, or a methylene group, and the configuration of the asymmetric carbon shown by the "*" symbol is (R); or a salt thereof.

(37) Compounds of the present invention in which R² is a 2-thienyl group, R¹ is a methyl group, ethyl group, cyclopropyl group, chlorine atom, trifluoromethyl group, -OMe, or -OCHF₂, R³ is a hydrogen atom, fluorine atom, or chlorine atom, Y¹ is an oxygen atom, and the configuration of the asymmetric carbon shown by the "*" symbol is (R); or a salt thereof.

(38) Compounds of the present invention in which R² is a 2-thienyl group, R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(39) Compounds of the present invention in which R² is a 2-thienyl group, R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(40) Compounds of the present invention in which R² is a 3-thienyl group, R¹ is a methyl group, ethyl group, cyclopropyl group, chlorine atom, trifluoromethyl group, -OMe, or -OCHF₂, R³ is a hydrogen atom, fluorine atom, or chlorine atom, Y¹ is an oxygen atom, -NH-, or a methylene group, and the configuration of the asymmetric carbon shown by the "*" symbol is (R); or a salt thereof.

(41) Compounds of the present invention in which R² is a 3-thienyl group, R¹ is a methyl group, ethyl group, cyclopropyl group, chlorine atom, trifluoromethyl group, -OMe, or -OCHF₂, R³ is a hydrogen atom, fluorine atom, or chlorine atom, Y¹ is an oxygen atom, and the configuration of the asymmetric carbon shown by the "*" symbol is (R); or a salt thereof.

(42) Compounds of the present invention in which R² is a 3-thienyl group, R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(43) Compounds of the present invention in which R² is a 3-thienyl group, R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(44) Compounds of the present invention in which R² is the formula (V-IV)

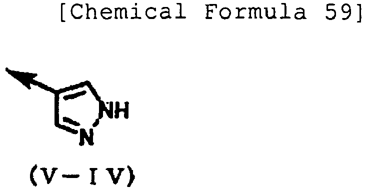

R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(45) Compounds of the present invention in which R² is the formula (V-IV)

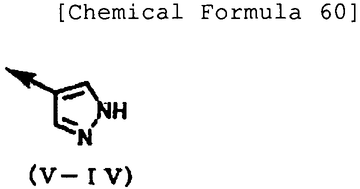

R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(46) Compounds of the present invention in which R² is the formula (V-V)

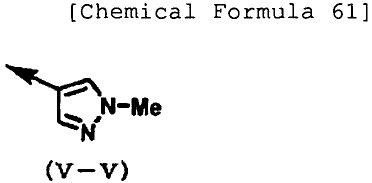

R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(47) Compounds of the present invention in which R² is the formula (V-V)

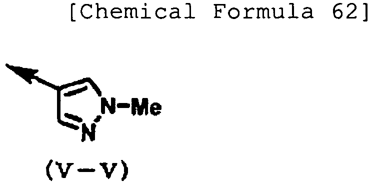

R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(48) Compounds of the present invention in which R² is a 3-pyridyl group, R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(49) Compounds of the present invention in which R² is a 3-pyridyl group, R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(50) Compounds of the present invention in which R² is a 3-chlorophenyl group, R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(51) Compounds of the present invention in which R² is a 3-chlorophenyl group, R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(52) Compounds of the present invention in which R² is a 3-methylphenyl group, R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(53) Compounds of the present invention in which R² is a 3-methylphenyl group, R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(54) Compounds of the present invention in which R² is a 3-methoxyphenyl group, R¹ is a methyl group, ethyl group, cyclopropyl group, chlorine atom, trifluoromethyl group, -OMe, or -OCHF₂, R³ is a hydrogen atom, Y¹ is an oxygen atom, -NH-, or a methylene group, and the configuration of the asymmetric carbon shown by the "*" symbol is (R); or a salt thereof.

(55) Compounds of the present invention in which R² is a 3-methoxyphenyl group, R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.
(56) Compounds of the present invention in which R² is the formula (VII-XI) 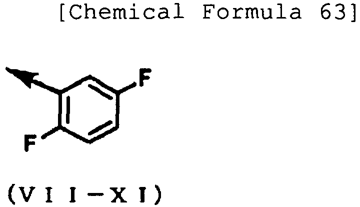 R¹ is a methyl group, ethyl group, cyclopropyl group, chlorine atom, trifluoromethyl group, -OMe, or -OCHF₂, R³ is a hydrogen atom, Y¹ is an oxygen atom, and the configuration of the asymmetric carbon shown by the "*" symbol is (R); or a salt thereof.

(57) Compounds of the present invention described in any of (1)-(56) above in the form of free compounds can be given as one preferred embodiment of the present invention. Salts of these are also a preferred embodiment, and hydrochlorides as salts of these can be given as especially preferred embodiments.

The following compounds and salts thereof can be given as concrete examples of preferred compounds of the present invention.
(R)-3-amino-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide;
(R)-2-fluoro-N-(3-(1-hydroxy-2-(2-(3-methoxyindazol-6-yloxy)ethylamino)ethyl)phenyl)benzenesulfonamide;
(R)-5-amino-2-fluoro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-3-sulfonamide;
(R)-N-(3-(1-hydroxy-2-(2-(3-methoxyindazol-6-yloxy)ethylamino)ethyl)phenyl)pyridine-3-sulfonamide;
(R)-3-chloro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)cyclopropane sulfonamide;
(R)-3-amino-N-(3-(1-hydroxy-2-(2-(3-methylindazol-6-yloxy)ethylamino)ethyl)phenyl)benzenesulfonamide;
(R)-3-chloro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-methoxybenzenesulfonamide;
(R)-N-(3-(1-hydroxy-2-(2-(3-methylindazol-6-yloxy)ethylamino)ethyl)phenyl)pyridine-3-sulfonamide;
(R)-N-(3-(1-hydroxy-2-(2-(3-methylindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)thiophene-3-sulfonamide;
(R)-N-(3-(1-hydroxy-2-(2-(3-methylindazol-6-yloxy)ethylamino)ethyl)phenyl)cyclopropane sulfonamide;
(R)-N-(3-(1-hydroxy-2-(2-(3-methylindazol-6-yloxy)ethylamino)ethyl)phenyl)-3-methylbenzenesulfonamide;
(R)-2-amino-N-(3-(1-hydroxy-2-(2-(3-methylindazol-6-yloxy)ethylamino)ethyl)phenyl)benzenesulfonamide;
(R)-5-amino-2-fluoro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-1-methylpyrazole-4-sulfonamide;
(R)-N-(3-(1-hydroxy-2-(2-(3-methylindazol-6-yloxy)ethylamino)ethyl)phenyl)pyrazole-4-sulfonamide;
(R)-3-amino-N-(3-(2-(2-(3-(difluoromethoxy)-indazol-6-yloxy)ethylamino)-1-hydroxyethyl)phenyl)benzenesulfonamide;
(R)-N-(3-(2-(2-(3-(difluoromethoxy)-indazol-6-yloxy)ethylamino)-1-hydroxyethyl)phenyl)pyrazole-4-sulfonamide;
(R)-3-amino-N-(3-(2-(2-(3-ethylindazol-6-yloxy)ethylamino)-1-hydroxyethyl)phenyl)benzenesulfonamide;
(R)-N-(3-(2-(2-(3-ethylindazol-6-yloxy)ethylamino)-1-hydroxyethyl)phenyl)cyclopropane sulfonamide;
(R)-N-(3-(2-(2-(3-ethylindazol-6-yloxy)ethylamino)-1-hydroxyethyl)phenyl)-3-methylbenzenesulfonamide;
(R)-N-(3-(2-(2-(3-ethylindazol-6-yloxy)ethylamino)-1-hydroxyethyl)phenyl)-1-methylpyrazole-4-sulfonamide;
(R)-N-(3-(2-(2-(3-ethylindazol-6-yloxy)ethylamino)-1-hydroxyethyl)phenyl)pyrazole-4-sulfonamide;
(R)-N-(3-(2-(2-(3-chloroindazol-6-yloxy)ethylamino)-1-hydroxyethyl)phenyl)cyclopropane sulfonamide;
(R)-3-amino-N-(3-(2-(2-(3-chloroindazol-6-yloxy)ethylamino)-1-hydroxyethyl)phenyl)benzenesulfonamide;
(R)-N-(3-(1-hydroxy-2-(2-(3-(trifluoromethyl)-indazol-6-yloxy)ethylamino)ethyl)phenyl)cyclopropane sulfonamide;
(R)-N-(3-(2-(2-(3-cyclopropylindazol-6-yloxy)ethylamino)-1-hydroxyethyl)phenyl)cyclopropane sulfonamide;
(R)-3-amino-N-(3-(1-hydroxy-2-(2-(3-isopropylindazol-6-yloxy)ethylamino)ethyl)phenyl)benzenesulfonamide;
(R)-3-(6-(2-(2-(3-(3-amino-phenylsulfonamide)phenyl)-2-hydroxyethylamino)ethoxy)indazol-3-yl)-N,N-dimethyl propanamide;
(R)-2-fluoro-N-(3-(1-hydroxy-2-(2-(3-methylindazol-6-yloxy)ethylamino)ethyl)phenyl)benzenesulfonamide;
(R)-3-hydroxy-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-(methylamino)-benzenesulfonamide;
(R)-2-hydroxy-N-(3-(1-hydroxy-2-(2-(3-methylindazol-6-yloxy)ethylamino)ethyl)phenyl)benzenesulfonamide;
(R)-N-(3-(2-(2-(3-cyclobutylindazol-6-yloxy)ethylamino)-1-hydroxyethyl)phenyl)cyclobutane sulfonamide;
(R)-N-(3-(2-(2-(3-cyclobutylindazol-6-yloxy)ethylamino)-1-hydroxyethyl)phenyl)cyclopropane sulfonamide;
(R)-N-(3-(2-(2-(3-chloroindazol-6-yloxy)ethylamino)-1-hydroxyethyl)phenyl)-2-fluorobenzenesulfonamide;
(R)-2-fluoro-N-(3-(1-hydroxy-2-(2-(3-(trifluoromethyl)-indazol-6-yloxy)ethylamino)ethyl)phenyl)benzenesulfonamide; and
(R)-2,5-difluoro-N-(3-(1-hydroxy-2-(2-(3-methylindazol-6-yloxy)ethylamino)ethyl)phenyl)benzenesulfonamide.
The following compounds and salts thereof can be given as more preferred concrete examples of compounds of the present invention.
(R)-3-amino-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide;
(R)-2-fluoro-N-(3-(1-hydroxy-2-(2-(3-methoxyindazol-6-yloxy)ethylamino)ethyl)phenyl)benzenesulfonamide;
(R)-5-amino-2-fluoro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-3-sulfonamide;
(R)-N-(3-(1-hydroxy-2-(2-(3-methoxyindazol-6-yloxy)ethylamino)ethyl)phenyl)pyridine-3-sulfonamide;
(R)-3-chloro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)cyclopropane sulfonamide;
(R)-3-amino-N-(3-(1-hydroxy-2-(2-(3-methylindazol-6-yloxy)ethylamino)ethyl)phenyl)benzenesulfonamide;
(R)-3-chloro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-methoxybenzenesulfonamide;
(R)-N-(3-(1-hydroxy-2-(2-(3-methylindazol-6-yloxy)ethylamino)ethyl)phenyl)pyridine-3-sulfonamide;
(R)-N-(3-(1-hydroxy-2-(2-(3-methylindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)thiophene-3-sulfonamide;
(R)-N-(3-(1-hydroxy-2-(2-(3-methylindazol-6-yloxy)ethylamino)ethyl)phenyl)cyclopropane sulfonamide;
(R)-N-(3-(1-hydroxy-2-(2-(3-methylindazol-6-yloxy)ethylamino)ethyl)phenyl)-3-methylbenzenesulfonamide;
(R)-2-amino-N-(3-(1-hydroxy-2-(2-(3-methylindazol-6-yloxy)ethylamino)ethyl)phenyl)benzenesulfonamide;
(R)-5-amino-2-fluoro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-1-methylpyrazole-4-sulfonamide;
(R)-N-(3-(1-hydroxy-2-(2-(3-methylindazol-6-yloxy)ethylamino)ethyl)phenyl)pyrazole-4-sulfonamide;
(R)-3-amino-N-(3-(2-(2-(3-(difluoromethoxy)-indazol-6-yloxy)ethylamino)-1-hydroxyethyl)phenyl)benzenesulfonamide;
(R)-N-(3-(2-(2-(3-(difluoromethoxy)-indazol-6-yloxy)ethylamino)-1-hydroxyethyl)phenyl)pyrazole-4-sulfonamide;
(R)-3-amino-N-(3-(2-(2-(3-ethylindazol-6-yloxy)ethylamino)-1-hydroxyethyl)phenyl)benzenesulfonamide;
(R)-N-(3-(2-(2-(3-ethylindazol-6-yloxy)ethylamino)-1-hydroxyethyl)phenyl)cyclopropane sulfonamide;
(R)-N-(3-(2-(2-(3-ethylindazol-6-yloxy)ethylamino)-1-hydroxyethyl)phenyl)-3-methylbenzenesulfonamide;
(R)-N-(3-(2-(2-(3-ethylindazol-6-yloxy)ethylamino)-1-hydroxyethyl)phenyl)-1-methylpyrazole-4-sulfonamide;
(R)-N-(3-(2-(2-(3-ethylindazol-6-yloxy)ethylamino)-1-hydroxyethyl)phenyl)pyrazole-4-sulfonamide;
(R)-N-(3-(2-(2-(3-chloroindazol-6-yloxy)ethylamino)-1-hydroxyethyl)phenyl)cyclopropane sulfonamide;
(R)-3-amino-N-(3-(2-(2-(3-chloroindazol-6-yloxy)ethylamino)-1-hydroxyethyl)phenyl)benzenesulfonamide;
(R)-N-(3-(1-hydroxy-2-(2-(3-(trifluoromethyl)-indazol-6-yloxy)ethylamino)ethyl)phenyl)cyclopropane sulfonamide;
(R)-N-(3-(2-(2-(3-cyclopropylindazol-6-yloxy)ethylamino)-1-hydroxyethyl)phenyl)cyclopropane sulfonamide;
(R)-3-amino-N-(3-(1-hydroxy-2-(2-(3-isopropylindazol-6-yloxy)ethylamino)ethyl)phenyl)benzenesulfonamide;
(R)-3-(6-(2-(2-(3-(3-amino-phenylsulfonamide)phenyl)-2-hydroxyethylamino)ethoxy)indazol-3-yl)-N,N-dimethyl propanamide;
(R)-2-fluoro-N-(3-(1-hydroxy-2-(2-(3-methylindazol-6-yloxy)ethylamino)ethyl)phenyl)benzenesulfonamide;
(R)-3-hydroxy-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-(methylamino)-benzenesulfonamide; and
(R)-2-hydroxy-N-(3-(1-hydroxy-2-(2-(3-methylindazol-6-yloxy)ethylamino)ethyl)phenyl)benzenesulfonamide.
The following compounds and salts thereof can be given as even more preferred concrete examples of compounds of the present invention.
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide;
(R)-2-fluoro-N-(3-(1-hydroxy-2-(2-(3-methylindazol-6-yloxy)ethylamino)ethyl)phenyl)benzenesulfonamide;
The compounds of the present invention are novel compounds not described in the literature. Compounds of the present invention shown by the general formula (I) can be manufactured, for example, by the methods described in schemes 1-12 below. However, methods of manufacturing the compounds of the present invention are not limited to the following methods.

In each of the reactions, the reaction time is not particularly restricted. However, since the progress of the reaction can be traced easily by the analysis means discussed below, the reaction may be terminated at the point when the yield of the target compound reaches its peak. Each reaction may also be carried out as needed in an inert gas atmosphere, such as a nitrogen stream or an argon stream. Protective groups may also be introduced and removed as needed in each reaction. The protective groups that can be used in each reaction and methods of protection and deprotection are not restricted as long as they are protective groups and methods of protection and deprotection used in ordinary organic synthesis. For example, known protective groups and methods of protection and deprotection described, for example, in Protective Groups in Organic Synthesis, John Wiley and Sons, 2007 edition may be selected as is appropriate. Protection and deprotection may also be implemented any number of times as needed in any steps in the production process of a compound shown by the general formula (I).

The use of free compounds is described for the sake of convenience, unless specifically stated otherwise, in the following methods. However, manufacture is also possible in some cases using salts of the free compounds.

Examples of the protective groups used in the present invention include indazole (-NH-) protective groups, hydroxyl group (-OH) protective groups, methanesulfonamide group (-NHSO₂Me) protective groups, amino group (-NH- or -NH₂) protective groups, and the like.

Examples of indazole (-NH-) protective groups include a trityl group, benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, phenacyl group, acetyl group, trifluoroacetyl group, pivaloyl group, benzoyl group, methoxycarbonyl group, ethoxycarbonyl group, allyloxycarbonyl (Alloc) group, 2,2,2-trichloroethoxycarbonyl group, benzyloxycarbonyl (Cbz) group, tert-butoxycarbonyl (Boc) group, 1-methyl-1-(4-biphenyl)ethoxycarbonyl (Bpoc) group, 9-fluorenylmethoxycarbonyl group, N,N-dimethylsulfonyl group, methanesulfonyl group, benzenesulfonyl group, p-toluenesulfonyl group, mesitylenesulfonyl group, p-methoxyphenylsulfonyl group, tetrahydrofuranyl (THP) group, tetrahydrofuryl group, allyl group, methoxymethyl (MOM) group, methoxyethoxymethyl (MEM) group, benzyloxymethyl (BOM) group, 2-(trimethylsilyl)ethoxymethyl (SEM) group, and the like.
Examples of hydroxyl group (-OH) protective groups include a C₁₋₄ alkyl group, C₂₋₄ alkenyl group, C₁₋₄ alkyl group substituted by a C₁₋₄ alkoxy group, C₁₋₄ alkyl group substituted by 1-3 halogen atoms, silyl group substituted by three same or different C₁₋₄ alkyl groups or phenyl groups, tetrahydropyranyl group, tetrahydrofuryl group, propargyl group, trimethylsilylethyl group, and the like. Concrete examples include a methyl group, ethyl group, tert-butyl group, allyl group, methoxymethyl (MOM) group, methoxyethoxymethyl (MEM) group, trichloroethyl group, phenyl group, methylphenyl group, chlorophenyl group, benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, phenacyl group, trityl group, 1-ethoxyethyl (EE) group, tetrahydropyranyl (THP) group, tetrahydrofuryl group, propargyl group, trimethylsilyl (TMS) group, triethylsilyl (TES) group, tert-butyldimethylsilyl (TBDMS) group, tert-butyldiphenylsilyl (TBDPS) group, acetyl (Ac) group, pivaloyl group, benzoyl group, allyloxycarbonyl (Alloc) group, 2,2,2-trichloroethoxycarbonyl (Troc) group, and the like.
Examples of protective groups for methanesulfonamide groups (-NHSO₂Me) include a methoxycarbonyl group, ethoxycarbonyl group, tert-butoxycarbonyl (Boc) group, benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, tert-butyl group, diphenylmethyl group, methoxyphenyl group, and the like.

Examples of amino group (-NH- or -NH₂) protective groups include a benzyl group, methylbenzyl group, chlorobenzyl group, dichlorobenzyl group, fluorobenzyl group, trifluoromethylbenzyl group, nitrobenzyl group, methoxyphenyl group, N-methylaminobenzyl group, N,N-dimethylaminobenzyl group, phenacyl group, acetyl group, trifluoroacetyl group, pivaloyl group, benzoyl group, allyloxycarbonyl group, 2,2,2-trichloroethoxycarbonyl group, benzyloxycarbonyl group, tert-butoxycarbonyl (Boc) group, 1-methyl-1-(4-biphenyl)ethoxycarbonyl (Bpoc) group, 9-fluorenylmethoxycarbonyl group, 2-nitrobenzenesulfonyl group, 4-nitrobenzenesulfonyl group, 2,4-dinitrobenzenesulfonyl group, benzyloxymethyl (BOM) group, 2-(trimethylsilyl)ethoxymethyl (SEM) group, and the like.

Removal of the protective groups permits conversion into the target compound during the production process or simultaneously or sequentially with production in the final step. Protection and deprotection reactions may be carried out in accordance with known methods, for example, the methods described in Protective Groups in Organic Synthesis, John Wiley and Sons, 2007 edition, for example, by the methods given as examples in (1)-(3) below.

(1) A deprotection reaction under acidic conditions can be carried out, for example, at a temperature of from -10 to 100°C in an organic acid, Lewis acid, inorganic acid, or mixture of these in an inert solvent. The amount of acid used is preferably from a one-fold molar quantity to a large excess. There are also methods that add ethanethiol, 1,2-ethanedithiol, or the like as an additive.

Examples of inert solvents include dichloromethane, chloroform, 1,4-dioxane, ethyl acetate, methyl-tert-butyl ether, tetrahydrofuran, anisole, and the like. Examples of organic acids include acetic acid, trifluoroacetic acid, methanesulfonic acid, p-toluenesulfonic acid, and the like. Examples of Lewis acids include boron tribromide, boron trifluoride, aluminum bromide, aluminum chloride, and the like. Examples of inorganic acids include hydrochloric acid, hydrogen chloride-1,4-dioxane, hydrogen chloride-ethyl acetate, hydrobromic acid, sulfuric acid, and the like. Examples of organic acids, Lewis acids, inorganic acids, or mixtures of these include hydrogen bromide/acetic acid and the like.

(2) A deprotection reaction by hydrogenolysis can be carried out, for example, at a temperature of from -10 to 70°C in the presence of hydrogen gas, ammonium formate, hydrazine hydrate, or another such hydrogen source under normal pressure or increased pressure with 0.1-300 wt% of a catalyst added in an inert solvent. The reaction can also be carried out by adding from a 0.05-fold molar quantity to a large excess of an inorganic acid to the above reaction solution.

Examples of inert solvents include tetrahydrofuran, dioxane, dimethoxyethane, diethyl ether, and other such ethers; methanol, ethanol, and other such alcohols; benzene, toluene, and other such benzenes; acetone, methyl ethyl ketone, and other such ketones; acetonitrile, and other such nitriles; dimethylformamide, and other such amides; ethyl acetate and other such esters; water, acetic acid, and the like alone or mixtures of these. Examples of catalysts include palladium-carbon powder, platinum oxide (PtO₂), activated nickel, and the like. Examples of inorganic acids include hydrochloric acid, sulfuric acid, and the like.

(3) A deprotection reaction of a silyl group can be carried out, for example, at a temperature of from -10 to 60°C using fluoride ion or the like in a water-miscible organic solvent.

Examples of organic solvents include tetrahydrofuran, acetic acid, acetonitrile, and the like. Tetra-n-butyl ammonium fluoride, hydrofluoric acid, hydrogen fluoride-pyridine complex, hydrogen fluoride-triethylamine complex, and the like may be used, for example, as the fluoride ion.

In the schemes discussed below, "STEP" means step. For example, "STEP 1-1" means step 1-1.

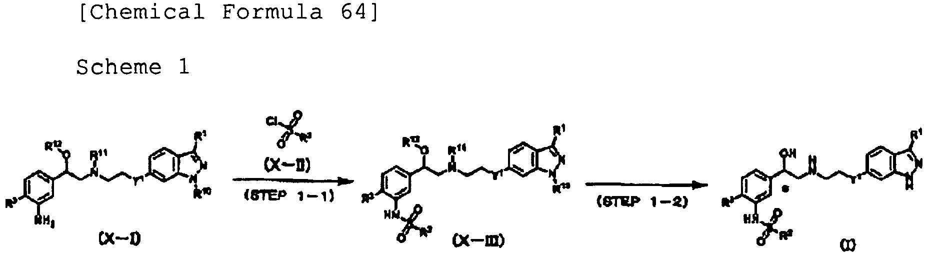

In scheme 1, R¹, R², R³, and Y¹ in each general formula are as defined above; R¹⁰ is an above-mentioned amino group protective group, R¹¹ is an above-mentioned amino group protective group, and R¹² is an above-mentioned hydroxyl group protective group.

R¹⁰ in each general formula in scheme 1 is preferably a benzyl group, tert-butoxycarbonyl group, or tetrahydropyranyl group. R¹¹ is preferably a tert-butoxycarbonyl group. R¹² is preferably triethylsilyl group.

### Step 1-1 (STEP 1-1)

A compound shown by the general formula (X-III) is obtained by reacting a compound shown by the general formula (X-I) and a compound shown by the general formula (X-II) by adding a base in an inert solvent.

Examples of inert solvents include methyl isobutyl ketone and other such ketone organic solvents; toluene and other such hydrocarbon solvents; dichloromethane, chloroform, 1,2-dichloroethane, and other such halogenated hydrocarbons; acetonitrile, and the like. Dichloromethane is preferred. Examples of bases include 1,8-diazabicyclo[5,4,0]-undecene, trimethylamine, N,N-diisopropylethylamine, triethylamine, and other such organic tertiary amines; pyridine, 4-dimethylaminopyridine, and other such organic bases; potassium carbonate, sodium bicarbonate, and other such inorganic bases. Pyridine or 1,8-diazabicyclo[5,4,0]-undecene is preferred.

The amount of base used is a 1- to 10-fold molar quantity, for example, versus the compound shown by the general formula (X-I); a 1- to 5-fold molar quantity is preferred. The amount of compound shown by the general formula (X-II) used is usually a 1- to 10-fold molar quantity, for example, versus the compound shown by the general formula (X-I); a 1- to 5-fold molar quantity is preferred.

The reaction temperature is from -10 to 60°C; -10 to 30°C is preferred. The reaction time is 0.1-48 hours; 0.2-24 hours is preferred.

### Step 1-2 (STEP 1-2)

A compound shown by the general formula (X-III) is subjected to a deprotection reaction in accordance with known methods, for example, by methods described in Protective Groups in Organic Synthesis, John Wiley and Sons, 2007 edition, and a compound shown by the general formula (I) can be produced.

Suitable examples include conducting a deprotection reaction under acidic conditions as above, conducting a deprotection reaction by hydrogenolysis as above, or using a combination. In any case, a deprotection reaction suited to the various protective groups present in the compound shown by the general formula (X-III) should be selected.

For example, in a deprotection reaction under acidic conditions, a compound shown by the general formula (I) is obtained by reacting by adding an acid in an inert solvent.

Examples of inert solvents include ethyl acetate, 1,4-dioxane, MTBE, and the like. Examples of acids include hydrochloric acid-1,4-dioxane solution, hydrochloric acid-ethyl acetate solution, and the like. The reaction temperature is from -20 to 60°C; 0-40°C is preferred. The reaction time is 0.1-24 hours; 1-20 hours is preferred.

Before conducting step 1-2, one may conduct conversion to an amino group by reduction of the nitro group as in Working Example 1, acetylation of the amino group as in Working Example 240, or conversion to a carboxylate group by hydrolysis of the ester group followed by conversion to a hydroxymethyl group by reduction of the carboxylic acid as in Reference Examples 119-122.

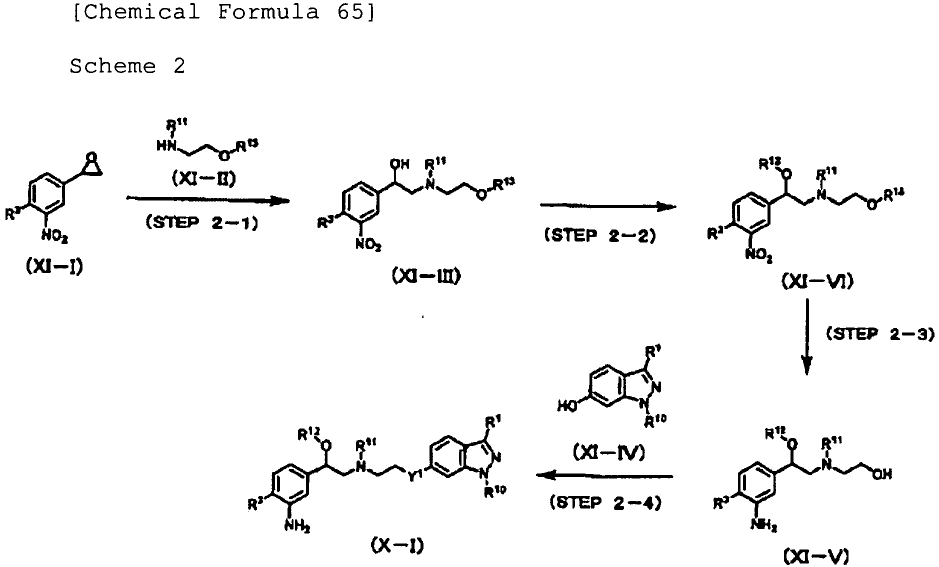

When Y¹ is an oxygen atom, R¹, R³, R¹⁰, R¹¹, and R¹² in each general formula in scheme 2 are as defined above, and R¹³ is an above-mentioned hydroxyl group protective group.

R¹⁰ in each general formula in scheme 2 is preferably a benzyl group, tert-butoxycarbonyl group, or tetrahydropyranyl group, more preferably a tert-butoxycarbonyl group. R¹¹ is preferably a benzyl group or tert-butoxycarbonyl group. R¹² is preferably a triethylsilyl group, and R¹³ is preferably a benzyl group.

### Step 2-1 (STEP 2-1)

A compound shown by the general formula (XI-III) is obtained by reacting a compound shown by the general formula (XI-I) and a compound shown by the general formula (XI-II) in an inert solvent.

Examples of inert solvents include methanol, ethanol, 1-butanol, 2-butanol, 2-propanol, and other such alcohols; N,N-dimethylformamide, N,N-dimethylacetamide, dimethyl sulfoxide, acetonitrile, and the like, individually or in mixture. 2-Propanol is preferred.

The molar ratio of the compound shown by the general formula (XI-I) and the compound shown by the general formula (XI-II) is compound shown by the general formula (XI-I)/compound shown by the general formula (XI-II) = 0.2- to 5-fold molar quantity, preferably 0.75- to 1.5-fold molar quantity. The reaction temperature is from -10°C to reflux, preferably 60°C to reflux. The reaction time is 0.5-48 hours, preferably 12-48 hours.

A Lewis acid catalyst may be added as needed.

### Step 2-2 (STEP 2-2)

A compound shown by the general formula (XI-VI) can be obtained by carrying out a hydroxyl group protection reaction of the compound shown by the general formula (XI-III) by a known method, for example, in accordance with a method described in Protective Groups in Organic Synthesis, John Wiley and Sons, 2007 edition.

As an appropriate example, a compound shown by the general formula (XI-VI) is obtained by adding a base to a compound shown by the general formula (XI-III) in an inert solvent and reacting with a silylating agent.

Examples of inert solvents include N,N-dimethylformamide. Examples of bases include imidazole. Examples of silylating agents include triethylchlorosilane and tert-butyldimethylchlorosilane. The reaction temperature is from - 20 to 60°C, preferably 0-30°C. The reaction time is 0.5-48 hours, preferably 1-24 hours.

### Step 2-3 (STEP 2-3)

A compound shown by the general formula (XI-V) is obtained by adding a catalyst to the compound shown by the general formula (XI-VI) in an insert solvent and reacting in the presence of hydrogen gas.

Examples of insert solvents include methanol, ethanol, 1-butanol, 2-butanol, 2-propanol, and other such alcohols; tetrahydrofuran, diethyl ether, and other such ethers, used individually or in mixture. Ethanol is preferred. Examples of catalysts include palladium-carbon powder, platinum oxide (PtO₂), activated nickel, and the like. Palladium-carbon powder is preferred. The reaction temperature is from 0°C to reflux, preferably 0-60°C. The reaction time is 0.5-48 hours, preferably 1-24 hours.

A switch may also be made to an appropriate protective group, as in Reference Example 87.

### Step 2-4 (STEP 2-4)

A compound shown by the general formula (X-I) can be obtained by reacting a compound shown by the general formula (XI-V) and a compound shown by the general formula (XI-IV) by adding phosphines and an azo compound in an inert solvent.

Examples of inert solvents include diethyl ether, tetrahydrofuran, dimethoxyethane, and other such ethers; methylene chloride, and other such halogen solvents; benzene, toluene, xylene, and other such benzenes. Toluene and tetrahydrofuran are preferred. Examples of phosphines include triphenyl phosphine and tributyl phosphine. Triphenyl phosphine is preferred. Examples of azo compounds include diethyl azodicarboxylate, diisopropyl azodicarboxylate, N,N,N',N'-tetramethylazodicarboxamide, 1,1'-(azodicarbonyl)dipiperidine, N,N,N',N'-tetraisopropylcarboxamide, di-2-methoxyethylazodicarboxylate, and the like. Diisopropyl azodicarboxylate, N,N,N',N'-tetramethylazodicarboxamide, and di-2-methoxyethylazodicarboxylate are preferred.

The amount of phosphines used is, for example, a 1- to 10-fold molar quantity versus the compound shown by the general formula (XI-IV); a 1- to 5-fold molar quantity is preferred. The amount of azo compound used is, for example, a 1- to 10-fold molar quantity versus the compound shown by the general formula (XI-IV); a 1- to 5-fold molar quantity is preferred. The molar ratio of the compound shown by the general formula (XI-V) and the compound shown by the general formula (XI-IV) is, for example, compound shown by the general formula (XI-V)/compound shown by the general formula (XI-IV) = 0.2-to 5-fold molar quantity; a 0.75- to 1.5-fold molar quantity is preferred. The reaction temperature is usually from -20°C to reflux; 20-70°C is preferred. The reaction time is 0.5-48 hours; 1-24 hours is preferred.

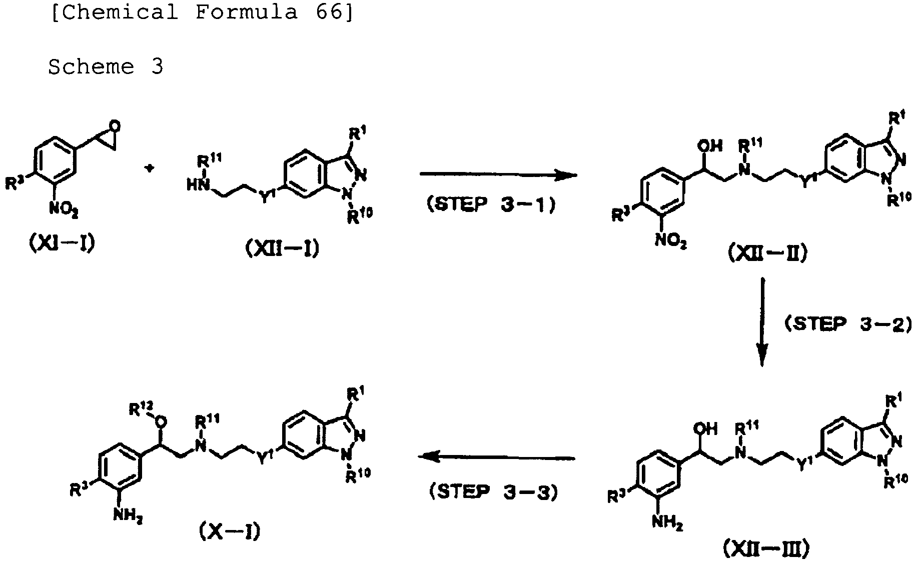

In scheme 3, R¹, R³, R¹⁰, R¹¹, R¹², Y¹ in each general formula are as defined above.

R¹⁰ in each general formula in scheme 3 is preferably a benzyl group, tert-butoxycarbonyl group, or tetrahydropyranyl group, more preferably a tert-butoxycarbonyl group. R¹¹ is preferably a benzyl group or tert-butoxycarbonyl group. R¹² is preferably a triethylsilyl group.

### Step 3-1 (STEP 3-1)

A compound shown by the general formula (XII-II) is obtained by reacting a compound shown by the general formula (XI-I) and a compound shown by the general formula (XII-I) in an inert solvent.

Examples of inert solvents include methanol, ethanol, 1-butanol, 2-butanol, 2-propanol, and other such alcohols; N,N-dimethylformamide, N,N-dimethylacetamide, dimethyl sulfoxide, acetonitrile, and the like, used individually or in mixture. 2-Propanol is preferred.

The molar ratio of the compound shown by the general formula (XI-I) and the compound shown by the general formula (XII-I) is compound shown by the general formula (XI-I)/compound shown by the general formula (XII-I) = 0.2- to 5-fold molar quantity; a 0.75- to 1.5-fold molar quantity is preferred. The reaction temperature is from -10°C to reflux, preferably 60°C to reflux. The reaction time is 0.5-48 hours, preferably 12-48 hours.

### Step 3-2 (STEP 3-2)

A compound shown by the general formula (XII-III) is obtained by adding a catalyst to a compound shown by the general formula (XII-II) in an inert solvent and reacting in the presence of hydrogen gas.

Examples of inert solvents include methanol, ethanol, 1-butanol, 2-butanol, 2-propanol, and other such alcohols; tetrahydrofuran, diethyl ether, and other such ethers, used individually or in mixture. Ethanol or a tetrahydrofuran-methanol mixture is preferred. Examples of catalysts include palladium-carbon powder, platinum oxide (PtO₂) CM-101 catalyst available from N. E. Chemcat Corporation and the like, activated nickel, and the like. Palladium-carbon powder or CM-101 is preferred. The reaction temperature is from 0°C to reflux, preferably 0-60°C. The reaction time is from 0.5 hour to three days, preferably from one hour to three days.

A switch may also be made to an appropriate protective group, as in Reference Examples 93 and 96.

### Step 3-3 (STEP 3-3)

A compound shown by the general formula (X-I) can be obtained by carrying out a hydroxyl group protection reaction of the compound shown by the general formula (XII-III) by a known method, for example, in accordance with a method described in Protective Groups in Organic Synthesis, John Wiley and Sons, 2007 edition.

As an appropriate example, a compound shown by the general formula (X-I) is obtained by adding a base to a compound shown by the general formula (XII-III) in an inert solvent and reacting with a silylating agent.

Examples of inert solvents include N,N-dimethylformamide. Example of bases include imidazole. Examples of silylating agents include triethylchlorosilane and tert-butyldimethylchlorosilane.

The reaction temperature is from -20 to 60°C, preferably 0-30°C. The reaction time is 0.5-48 hours, preferably 1-24 hours.

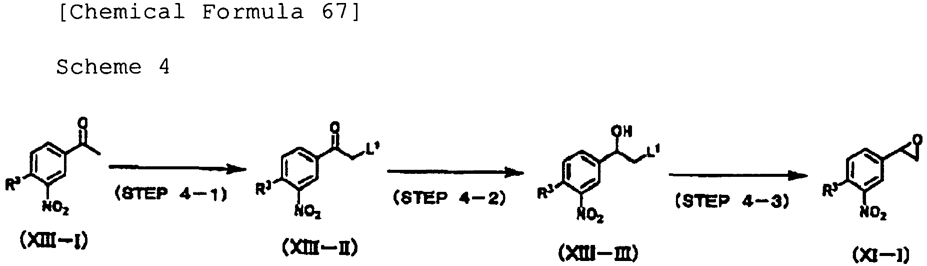

In scheme 4, R³ in each of the general formulae is as defined above, and L¹ is a leaving group. For L¹ in each of the general formulae in scheme 4 there may be cited a chlorine atom, bromine atom, iodine atom, p-toluenesulfonyloxy group, methanesulfonyloxy group, or the like. A chlorine atom is preferred.

When R³ in the general formula (XI-I) is a hydrogen atom, the compound can be manufactured by a method such as that of Working Example 6 of International Patent Publication No. WO01/17962 (incorporated herein by reference). When R³ in the general formula (XIII-I) is a fluorine atom, the compound can be obtained from Aldrich Co. or another supplier. When R³ in the general formula (XI-I) is a chlorine atom, the compound can be manufactured by a method such as that of Working Example 19 of International Patent Publication No. WO01/17962.

### Step 4-1 (STEP 4-1)

A compound shown by the general formula (XIII-II) is obtained by adding a halogenating agent to a compound shown by the general formula (XIII-I) in an inert solvent, also adding methanol if necessary, and reacting.

Examples of inert solvents include dichloromethane, 1,2-dichloroethane, chloroform, and other such halogenated hydrocarbons; dichloromethane is preferred. Examples of halogenating agents include chlorine gas, bromine gas, sulfuryl chloride, and the like. Sulfuryl chloride is preferred.

The amount of halogenating agent used is preferably a 1-to 3-fold molar quantity versus the compound shown by the general formula (XIII-I). The amount of methanol used is a 0-to 5-fold molar quantity versus the compound shown by the general formula (XIII-I), preferably a 0.1- to 3-fold molar quantity. The reaction temperature is preferably from -10 to 50°C. The reaction time is preferably 1-10 hours, including the dropwise addition time of the halogenating agent and methanol.

### Step 4-2 (STEP 4-2)

A compound shown by the general formula (XIII-III) is obtained by reacting a compound shown by the general formula (XIII-II) with a reducing agent in an organic solvent.

Examples of organic solvents include methanol, ethanol, and other such alcohols; tetrahydrofuran, and other such ethers. Examples of reducing agents include sodium borohydride and the like.

Unless one is specifically conducting an asymmetric reduction reaction, the compound shown by the general formula (XIII-III) obtained by this reduction reaction is obtained as a racemic mixture.

Examples of means of obtaining an optically active compound include means of conducting an asymmetric reduction reaction. An asymmetric reduction reaction can be carried out in accordance with ordinary chemical references, for example, by the methods described in Experimental Chemistry Lectures, 5th edition (edited by the Chemical Society of Japan, published by Maruzen), Vol. 19, pp. 65-171 or references described in the literature.

As an appropriate example, a compound shown by the general formula (XIII-III) can be obtained by adding an optically active ligand and a reducing agent to a compound shown by the general formula (XIII-II) in an inert solvent and reacting.

Examples of inert solvents include dichloromethane and other such halogen solvents; toluene and other such hydrocarbon solvents; tetrahydrofuran and other such ether solvents, used individually or in mixture. A mixture of toluene and tetrahydrofuran is preferred. Examples of optically active ligands include (R)-2-methyl-CBS-oxazoborolidine, (R)-2-n-butyl-CBS-oxazaborolidine, and the like. (R)-2-methyl-CBS-oxazaborolidine-toluene solution purchased from Aldrich Co. or another supplier is preferred. Examples of reducing agents include borane tetrahydrofuran complex, borane dimethyl sulfide complex, catecholborane, and the like. Borane dimethyl sulfide complex is preferred.

The amount of optically active ligand used is preferably a 0.05- to 1-fold molar quantity versus the compound shown by the general formula (XIII-II). The amount of reducing agent used is preferably a 1- to 10-fold molar quantity versus the compound shown by the general formula (XIII-II). The reaction temperature is from -78 to 50°C, preferably from -10 to 30°C. The reaction time is 0.1-12 hours, preferably 1-12 hours.

### Step 4-3 (STEP 4-3)

A compound shown by the general formula (XI-I) is obtained by adding a base to a compound shown by the general formula (XIII-III) in an inert solvent and reacting.

Examples of inert solvents include water, methanol, 2-propanol, ethanol, and other such alcohols; N,N-dimethylformamide, tetrahydrofuran, 1,4-dioxane, acetone, 2-butanone, dimethyl sulfoxide, acetonitrile, and the like, used individually or in mixture. 2-Propanol is preferred. Examples of bases include potassium carbonate, sodium carbonate, cesium carbonate, sodium bicarbonate, potassium hydroxide, sodium hydroxide, sodium methoxide, 28% sodium methoxide-methanol solution, potassium t-butoxide, and other such alkali metal compounds; pyridine, 4-dimethylaminopyridine, 1,8-diazabicyclo[5,4,0]-undecene, trimethylamine, triethylamine, and other such organic tertiary amines. Sodium hydroxide is preferred.

The amount of base used is preferably a 1- to 10-fold molar quantity versus the compound shown by the general formula (XIII-III). The reaction temperature is from -40°C to reflux, preferably from -10 to 50°C. The reaction time is 0.1-48 hours, preferably 0.1-12 hours.

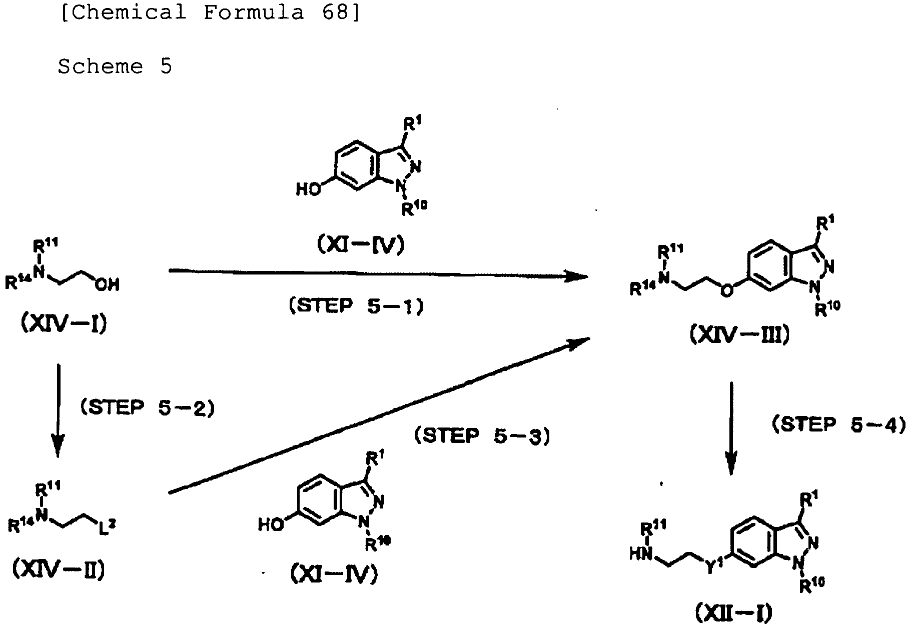

When Y¹ is an oxygen atom, R¹, R¹⁰, and R¹¹ in each general formula in scheme 5 are as defined above. R¹⁴ is an above-mentioned amino group protective group. L² is a leaving group.

In scheme 5, R¹⁴ in each general formula is preferably a benzyl group. Examples of L² include a chlorine atom, bromine atom, iodine atom, p-toluenesulfonyloxy group, methanesulfonyloxy group, and the like. A methanesulfonyloxy group is preferred.

Compounds in which R¹¹ of the general formula (XIV-I) is a benzyl group and R¹⁴ is a benzyl group can be purchased from Tokyo Chemical Industry Co., Ltd. or another supplier.

### Step 5-1 (STEP 5-1)

A compound shown by the general formula (XIV-III) can be obtained by adding phosphines and an azo compound to a compound shown by the general formula (XIV-I) and a compound shown by the general formula (XI-IV) in an inert solvent and reacting.

Examples of inert solvents include diethyl ether, tetrahydrofuran, dimethoxyethane, and other such ethers; methylene chloride and other such halogen solvents; benzene, toluene, xylene, and other such benzenes. Toluene or tetrahydrofuran is preferred. Examples of phosphines include triphenyl phosphine and tributyl phosphine. Triphenyl phosphine is preferred. Examples of azo compounds include diethyl azodicarboxylate, diisopropyl azodicarboxylate, N,N,N',N'-tetramethylazodicarboxamide, 1,1'-(azodicarbonyl)dipiperidine, N,N,N',N'-tetraisopropylcarboxamide, and the like. N,N,N',N'-tetramethylazodicarboxamide is preferred.

The amount of phosphine used is a 1- to 10-fold molar quantity versus the compound shown by the general formula (XI-IV), preferably a 1- to 5-fold molar quantity. The amount of azo compound used is a 1- to 10-fold molar quantity versus the compound shown by the general formula (XI-IV), preferably a 1-to 5-fold molar quantity. The amount of compound shown by the general formula (XIV-I) used is a 1- to 10-fold molar quantity versus the compound shown by the general formula (XI-IV), preferably a 1- to 5-fold molar quantity. The reaction temperature is usually from -20°C to reflux, preferably 0-30°C. The reaction time is 1-48 hours, preferably 3-24 hours.

### Step 5-2 (STEP 5-2)

A compound shown by the general formula (XIV-II) is obtained by adding a base and sulfonylation reagent to a compound shown by the general formula (XIV-I) in an inert solvent and reacting.

Examples of inert solvents include dichloromethane, chloroform, and other such halogenated hydrocarbons; tetrahydrofuran and other such ethers, used individually or in mixture. Examples of bases include pyridine, triethylamine, diisopropylethylamine, 1,8-diazabicyclo[5,4,0]-undecene, and other such organic tertiary amines; potassium carbonate, sodium carbonate, cesium carbonate, sodium bicarbonate, and other such alkali metal compounds. Triethylamine is preferred. Examples of sulfonylation reagents include p-toluenesulfonyl chloride, methanesulfonyl chloride, and the like.

The amount of sulfonylation reagent used is a 1- to 10-fold molar quantity versus the compound shown by the general formula (XIV-I), preferably a 1- to 2-fold molar quantity. The amount of base used is a 1- to 10-fold molar quantity versus the compound shown by the general formula (XIV-I), preferably a 1- to 2-fold molar quantity. The reaction temperature is from -20°C to reflux, preferably from -10 to 50°C. The reaction time is usually 0.1-24 hours, preferably 1-10 hours, including the reagent dropwise addition time.

A compound shown by the general formula (XIV-II) can also be obtained by reacting a compound shown by the general formula (XIV-I) in accordance with ordinary chemical references, for example, by the methods described in Experimental Chemistry Lectures, 4th edition (edited by the Chemical Society of Japan, published by Maruzen), Vol. 19, pp. 438-446 or references described in the literature. As an appropriate example, a compound shown by the general formula (XIV-I) is obtained by adding a halogenation reagent and phosphines to a compound shown by the general formula (XIV-I) in an inert solvent and reacting.

Examples of inert solvents include dichloromethane, chloroform, and other such halogenated hydrocarbons; tetrahydrofuran, and other such ethers; benzene, toluene, and other such hydrocarbon solvents, used individually or in mixture. Examples of halogenation reagents include carbon tetrachloride, N-chlorosuccinimide, N-bromosuccinimide, carbon tetrabromide, N-iodosuccinimide, and the like. Examples of phosphines include triphenyl phosphine and n-butyl phosphine. Triphenyl phosphine is preferred.

The amount of halogenation reagent used is preferably a 1- to 10-fold molar quantity versus the compound shown by the general formula (XIV-I). The amount of phosphines used is preferably a 1- to 10-fold molar quantity versus the compound shown by the general formula (XIV-I). The reaction temperature is from -10°C to reflux, preferably from -10 to 40°C. The reaction time is 0.1-24 hours, preferably 0.5-12 hours.

As another method, a compound shown by the general formula (XIV-II) can also be obtained by adding a base, if needed, to a compound shown by the general formula (XIV-I) in an inert solvent and reacting with a halogenation reagent.

Examples of inert solvents include dichloromethane, chloroform, and other such halogenated hydrocarbons; tetrahydrofuran and other such ethers; benzene, toluene, and other such hydrocarbon solvents, used individually or in mixture.
Examples of halogenation reagents include thionyl chloride, thionyl bromide, phosphorus tribromide, and the like. Examples of bases include pyridine, 4-dimethylaminopyridine, triethylamine, diisopropylethylamine, 1,8-diazabicyclo[5,4,0]-undecene, and other such organic tertiary amines.
The amount of halogenation reagent used is preferably a 1- to 10-fold molar quantity versus the compound shown by the general formula (XIV-I). The amount of base used is a 0- to 10-fold molar quantity versus the compound shown by the general formula (XIV-I), preferably a 1- to 10-fold molar quantity. The reaction temperature is from -10°C to reflux, preferably from -10 to 40°C. The reaction time is 0.1-24 hours, preferably 0.5-12 hours.

### Step 5-3 (STEP 5-3)

A compound shown by the general formula (XIV-III) is obtained by adding a base to a compound shown by the general formula (XIV-II) and a compound shown by the general formula (XI-IV) in an inert solvent and reacting.
Examples of inert solvents include tetrahydrofuran, N,N-dimethylformamide, N,N-dimethylacetamide, dimethyl sulfoxide, acetonitrile, and the like, used individually or in mixture. Examples of bases include potassium carbonate, sodium carbonate, cesium carbonate, sodium bicarbonate, potassium hydroxide, sodium hydroxide, sodium methoxide, 28% sodium methoxide-methanol solution, potassium t-butoxide, and other such alkali metal compounds; pyridine, 4-dimethylaminopyridine, 1,8-diazabicyclo[5,4,0]-undecene, trimethylamine, triethylamine, and other such organic tertiary amines.

The amount of base used is a 1- to 10-fold molar quantity versus the compound shown by the general formula (XI-IV), preferably a 1- to 5-fold molar quantity. The amount of compound shown by the general formula (XIV-II) used is a 1- to 10-fold molar quantity versus the compound shown by the general formula (XI-IV), preferably a 1- to 3-fold molar quantity. The reaction temperature is from -20°C to reflux, preferably 0-60°C. The reaction time is 0.1-48 hours, preferably 2-24 hours, including the reagent dropwise addition time.

If the reaction progresses slowly, a 0.1- to 1.5-fold molar quantity of potassium iodide, sodium iodide, or another such catalyst may be added as needed versus the compound shown by the general formula (XIV-II).

### Step 5-4 (STEP 5-4)

When it is necessary to remove the protective groups of the compound shown by the general formula (XIV-III), a deprotection reaction of R¹⁴ for R¹⁰ and R¹¹ may be performed selectively in accordance with known methods, for example, methods described in Protective Groups in Organic Synthesis, John Wiley and Sons, 2007 edition. There is also another embodiment in which a deprotection reaction of R¹¹ for R¹⁰ and R¹⁴ is performed selectively. For example, when R¹¹ and R¹⁴ are both benzyl groups in the general formula (XIV-III), there are conditions whereby only one of the benzyl groups of R¹¹ or R¹⁴ is deprotected selectively. Such conditions include a method for obtaining a compound shown by the general formula (XII-I) by reacting under control by adding a catalyst and hydrochloric acid in an inert solvent in the presence of normal-pressure or increased-pressure hydrogen gas.

Examples of inert solvents include methanol, ethanol, and other such alcohol solvents. Ethanol is preferred. Palladium-carbon powder is preferred as the catalyst.

The amount of catalyst used is 1-40 wt% versus the compound shown by the general formula (XIV-III), preferably 5-40 wt%. The amount of hydrochloric acid used is a 0.05- to 3-fold molar quantity versus the compound shown by the general formula (XIV-III), preferably a 0.1- to 1-fold molar quantity. The reaction temperature is 0-60°C, preferably 0-40°C. The reaction time is 0.1-24 hours, preferably 0.1-12 hours.

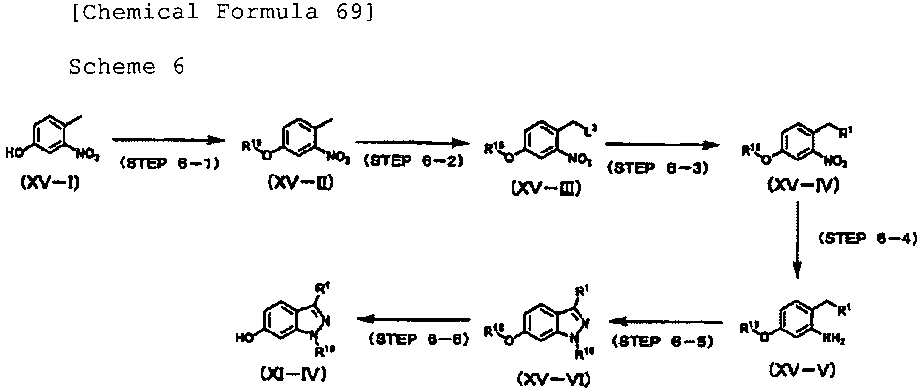

In each general formula in scheme 6, R¹ is -OR⁴ or a group other than -(CH₂)ₙCONR⁷⁻¹R⁷⁻² in the above definition. R¹⁰ is as defined above. L³ represents a leaving group. R¹⁵ is a hydroxyl group protective group.

In each general formula in scheme 6, L³ is preferably a bromine atom or iodine atom. R¹⁵ is preferably a methyl group.

Compounds in which R¹⁵ is a methyl group among compounds shown by the general formula (XV-II) can be purchased from Aldrich Co. or another supplier.

### Step 6-1 (STEP 6-1)

A compound shown by the general formula (XV-II) is obtained by conducting a hydroxyl group protection reaction of a compound (XV-I) which can be purchased from Tokyo Chemical Industry Co., Ltd. or another supplier by a known method, for example, in accordance with methods described in Protective Groups in Organic Synthesis, John Wiley and Sons, 2007 edition.

### Step 6-2 (STEP 6-2)

A compound shown by the general formula (XV-III) is obtained by reacting a compound shown by the general formula (XV-II) in accordance with ordinary chemical references, for example, by the methods described in Experimental Chemistry Lectures, 4th edition (edited by the Chemical Society of Japan, published by Maruzen), Vol. 19, pp. 416-482 or references described in the literature. As an appropriate example, a compound shown by the general formula (XV-III) is obtained by adding a halogenation reagent and a radical initiator to a compound shown by the general formula (XV-II) in an inert solvent and reacting. This reaction may also be carried out under photoirradiation instead of adding a radical initiator.

Examples of inert solvents include benzene, chlorobenzene, and other such benzenes; carbon tetrachloride and other such halogenated hydrocarbons, and the like, used individually or in mixture. Carbon tetrachloride is preferred. Examples of halogenation reagents include bromine, N-bromosuccinimide, and the like. N-bromosuccinimide is preferred. Examples of radical initiators include azobisbutyronitrile, benzoyl peroxide, and the like. Benzoyl peroxide is preferred.

The amount of halogenation reagent used is preferably a 1- to 10-fold molar quantity versus the compound shown by the general formula (XV-II). The amount of radical initiator used is preferably a 1- to 10-fold molar quantity versus the compound shown by the general formula (XV-II). The reaction temperature is from -20°C to reflux, preferably 40°C to reflux. The reaction time is 0.1-48 hours, preferably 5-48 hours.

### Step 6-3 (STEP 6-3)

A compound shown by the general formula (XV-IV) is obtained by adding a nucleophilic reagent to introduce an R¹ group to a compound shown by the general formula (XV-III) in an inert solvent, adding a catalyst as needed, and reacting.

A commercial Grignard reagent, organic zinc reagent, organic boronic acid reagent, organic boronic acid ester reagent, organic lithium reagent, organic copper reagent, or organic tin reagent is purchased or one can be prepared by the usual methods as the nucleophilic reagent to introduce an R¹ group. Examples of catalysts include a palladium complex, nickel complex, copper complex, copper salt, copper powder, lithium salt, and the like.

When R¹ is a trifluoromethyl group in a compound shown by the general formula (XV-IV), a compound shown by the general formula (XV-IV) is obtained by reacting a compound shown by the general formula (XV-III) in accordance with ordinary chemical references, for example, methods described in Organofluorine Chemistry (by Kenji Uneyama, published by Blackwell), pp. 292-300 or references described in the literature. As an appropriate example, a compound shown by the general formula (XV-IV) is obtained by adding a trifluoromethylation reagent and a catalyst to a compound shown by the general formula (XV-III) in an inert solvent and reacting.

Examples of inert solvents include N,N-dimethylformamide, N,N-dimethylacetamide, dimethyl sulfoxide, N-methylpyrrolidone, and other such aprotic polar solvents. N,N-dimethylformamide is preferred. Examples of trifluoromethylation reagents include trifluoromethyl iodide, sodium trifluoroacetate, methyl 2,2-difluoro-2-(fluorosulfonyl)acetate, trifluoromethyl-trimethylsilane, trifluoromethyl-triethylsilane, methyl chlorodifluoroacetate-potassium fluoride, and the like. Methyl 2,2-difluoro-2-(fluorosulfonyl)acetate is preferred. Examples of catalysts include copper iodide, copper bromide, or other such copper salts, and copper powder. Copper iodide is preferred.

The amount of trifluoromethylation reagent used is preferably a 1- to 10-fold molar quantity versus the compound shown by the general formula (XV-III). The amount of catalyst used is a 0.001- to 10-fold molar quantity versus the compound shown by the general formula (XV-III), preferably a 0.1- to 1-fold molar quantity. The reaction temperature is from 0°C to reflux, preferably 40-130°C. The reaction time is 0.1-48 hours, preferably 1-12 hours.

### Step 6-4 (STEP 6-4)

A compound shown by the general formula (XV-V) is obtained by reacting a compound shown by the general formula (XV-IV) in accordance with ordinary chemical references, for example, by the methods described in Experimental Chemistry Lectures, 4th edition (edited by the Chemical Society of Japan, published by Maruzen), Vol. 26, pp. 159-266 or references described in the literature. As an appropriate example, a compound shown by the general formula (XV-V) is obtained by adding a catalyst and a hydrogen source to a compound shown by the general formula (XV-IV) in an inert solvent and reacting.

Examples of inert solvents include methanol, ethanol, and other such alcohols. Methanol is preferred. Examples of catalysts include Raney nickel, palladium-carbon, and the like. Palladium-carbon is preferred. Examples of hydrogen sources include hydrogen gas, formic acid-ammonium, and the like. Hydrogen gas is preferred.

The amount of catalyst used is 0.1-10 wt% versus the compound shown by the general formula (XV-IV), preferably 1-10 wt%. The amount of hydrogen source used is from 1 to a large excess by molar quantity versus the compound shown by the general formula (XV-IV), preferably a 2- to 40-fold molar quantity. When hydrogen gas is used as the hydrogen source, it may be under normal pressure or increased pressure. The reaction temperature is from -20°C to reflux, preferably 0°C to reflux. The reaction time is 0.1-24 hours, preferably 0.5-12 hours.

### Step 6-5 (STEP 6-5)

A compound shown by the general formula (XV-VI) is obtained by reacting in accordance with ordinary chemical references, for example, by the methods described in the new edition of Heterocyclic Compounds, Oyo edition (by H. Yamanaka, T. Hino, M. Nakagawa H. Sakamoto, published by Kodansha), pp. 41-63 or references described in the literature. As an appropriate example, a compound shown by the general formula (XV-VI) is obtained by adding a diazonium reagent or nitroso reagent and, if needed, acetic anhydride, to a compound shown by the general formula (XV-V) in an inert solvent and reacting.

Examples of inert solvents include acetic acid, benzene, toluene, monochlorobenzene, or other such benzenes, used individually or in mixture. Monochlorobenzene is preferred. Examples of diazonium reagents and nitroso reagents include sodium nitrite, tert-butyl nitrite, isoamyl nitrite, and the like. Isoamyl nitrite is preferred.

The amount of diazonium reagent or nitroso reagent used is preferably a 1- to 10-fold molar quantity versus the compound shown by the general formula (XV-V). The amount of acetic anhydride used is a 0- to 10-fold molar quantity versus the compound shown by the general formula (XV-V), preferably a 1- to 10-fold molar quantity. The reaction temperature is from -20°C to reflux, preferably 40°C to reflux. The reaction time is 0.1-24 hours, preferably 1-20 hours.

### Step 6-6 (STEP 6-6)

When it is necessary to remove the protective groups of a compound shown by the general formula (XV-VI), a compound shown by the general formula (XI-IV) is obtained by a known method, for example, by a method described in Protective Groups in Organic Synthesis, John Wiley and Sons, 2007 edition.
The method described in Reference Example 50 can be given as an appropriate example. A switch may also be made to appropriate protective groups as in Reference Examples 46-49.

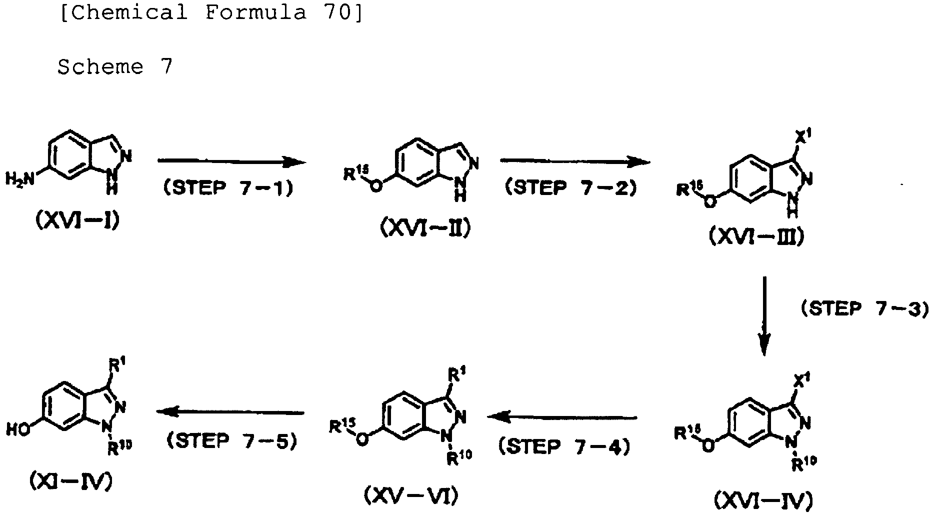

In each general formula in scheme 7, R¹ is as defined above and shows -OR⁴ or a group other than -(CH₂)ₙCONR⁷⁻¹R⁷⁻². R¹⁰ is as defined above. R¹⁵ is a hydrogen atom or hydroxyl group protective group. X¹ is a halogen atom.
In each general formula in scheme 7, X¹ is preferably a chlorine atom, bromine atom, or iodine atom, and R¹⁵ is preferably an acetyl group or tert-butyldiphenylsilyl group.

When R¹ is a chlorine atom, step 7-4 is omitted, and it is easily interpreted by those skilled in the art that X¹ = R¹ = chlorine atom.

### Step 7-1 (STEP 7-1)

A compound shown by the general formula (XVI-II) is obtained by reacting a compound (XVI-I), which can be purchased from Tokyo Chemical Industry Co., Ltd. or another supplier, in accordance with ordinary chemical references, for example, by methods described in Experimental Chemistry Lectures, 4th edition (edited by the Chemical Society of Japan, published by Maruzen), Vol. 20, pp. 112-114 or references described in the literature. As an appropriate example, a compound shown by the general formula (XVI-II) is obtained by reacting with acetic acid or the like after deriving a diazonium salt of a compound (XVI-I) by adding a diazonium reagent or nitroso reagent and an acid to a compound (XVI-I) in an inert solvent and reacting.

Water is preferred as the inert solvent. Examples of diazonium reagents or nitroso reagents include sodium nitrite, tert-butyl nitrite, isoamyl nitrite, and the like. Sodium nitrite is preferred. Examples of acids include hydrochloric acid, sulfuric acid, tetrafluoroboric acid, and the like. Tetrafluoroboric acid is preferred.

The amount of diazonium reagent or nitroso reagent used is preferably a 1- to 10-fold molar quantity versus compound (XVI-I). The amount of acid used is preferably from 1-fold to a large excess molar quantity versus compound (XVI-I). The reaction temperature is preferably from -20 to 100°C. The reaction time is 0.1-48 hours, preferably 1-24 hours.

Hydroxyl group protection can be carried out when R¹⁵ is a hydrogen atom. This protection reaction can be conducted by known methods, for example, in accordance with methods described in Protective Groups in Organic Synthesis, John Wiley and Sons, 2007 edition.

### Step 7-2 (STEP 7-2)

A compound shown by the general formula (XVI-III) is obtained by adding a halogenation catalyst and, if necessary, a base to a compound shown by the general formula (XVI-II) in an inert solvent and reacting.

Examples of inert solvents include diethyl ether, tetrahydrofuran, dimethoxyethane, and other such ethers; dichloromethane, chloroform, 1,2-dichloroethane, and other such halogenated hydrocarbons; benzene, toluene, xylene, and other such benzenes; acetonitrile, and the like, used individually or in mixture. Tetrahydrofuran or acetonitrile is preferred. Examples of halogenation reagents include chlorine gas, bromine, iodine, N-bromosuccinimide, N-chlorosuccinimide, N-iodosuccinimide, and the like. N-chlorosuccinimide, N-bromosuccinimide, or iodine is preferred. Examples of bases include potassium carbonate, sodium carbonate, cesium carbonate, sodium bicarbonate, potassium hydroxide, sodium hydroxide, sodium methoxide, potassium t-butoxide, and other such alkali metal compounds; pyridine, 4-dimethylaminopyridine, 1,8-diazabicyclo[5,4,0]-undecene, trimethylamine, triethylamine, or other such organic tertiary amines. Potassium t-butoxide is preferred.

The amount of halogenation reagent used is preferably a 1- to 10-fold molar quantity versus the compound shown by the general formula (XVI-II). The amount of base used in a 0- to 10-fold molar quantity versus the compound shown by the general formula (XVI-II), preferably a 0- to 5-fold molar quantity. The reaction temperature is from -20°C to reflux, preferably 0°C to reflux. The reaction time is 0.1-24 hours, preferably 0.1-12 hours.

### Step 7-3 (STEP 7-3)

When it is necessary to remove the protective groups of a compound shown by the general formula (XVI-III), the above amino group protective groups are selected, and a compound shown by the general formula (XVI-IV) can be obtained by a known method, for example, by a method described in Protective Groups in Organic Synthesis, John Wiley and Sons, 2007 edition. As an appropriate example, a compound shown by the general formula (XVI-IV) is obtained by adding a protective reagent and, if necessary, a base or catalyst to a compound shown by the general formula (XVI-III) in an inert solvent and reacting.

Examples of inert solvents include diethyl ether, tetrahydrofuran, dimethoxyethane, and other such ethers; dichloromethane, 1,2-dichloroethane, and other such halogenated hydrocarbons; benzene, toluene, xylene, and other such benzenes; acetonitrile, and the like, individually or in mixture. Examples of protective reagents include dihydropyran, di-tert-butyl carbonate, and the like. Examples of bases include potassium carbonate, sodium carbonate, cesium carbonate, sodium bicarbonate, potassium hydroxide, sodium hydroxide, sodium methoxide, potassium t-butoxide, and other such alkali metal compounds; pyridine, 4-dimethylaminopyridine, 1,8-diazabicyclo[5,4,0]-undecene, trimethylamine, triethylamine, and other such organic tertiary amines. An acid catalyst or base catalyst may be used in accordance with the protection reaction as the catalyst. Examples of acid catalysts include hydrochloric acid, p-toluenesulfonyl acid, and the like. Examples of base catalysts include 4-dimethylaminopyridine.

The amount of protective reagent used is a 1- to 10-fold molar quantity versus the compound shown by the general formula (XVI-III), preferably a 1- to 5-fold molar quantity. The amount of base used is a 0- to 10-fold molar quantity versus the compound shown by the general formula (XVI-III), preferably a 0- to 5-fold molar quantity. The amount of catalyst used is a 0.001- to 1-fold molar quantity versus the compound shown by the general formula (XVI-III), preferably a 0.01- to 0.5-fold molar quantity. The reaction temperature is from -20°C to reflux, preferably 0-100°C. The reaction time is 0.1-48 hours, preferably 1-24 hours.

### Step 7-4 (STEP 7-4)

A compound shown by the general formula (XV-VI) is obtained by adding a nucleophilic reagent to introduce an R¹ and, if necessary, a catalyst to a compound shown by the general formula (XVI-IV) in an inert solvent and reacting.

A commercial Grignard reagent, organic zinc reagent, organic boronic acid reagent, organic boronic acid ester reagent, organic lithium reagent, organic copper reagent, or organic tin reagent is purchased or one can be prepared by a usual method as the nucleophilic reagent to introduce an R¹. Examples of catalysts include a palladium complex, nickel complex, copper complex, copper salt, copper powder, lithium salt, and the like.

When R¹ is a trifluoromethyl group in the general formula (XV-VI), a compound shown by the general formula (XV-VI) is obtained by reacting a compound shown by the general formula (XVI-IV) in accordance with ordinary chemical references, for example, methods described in Organofluorine Chemistry (by Kenji Uneyama, published by Blackwell), pp. 292-300 or references described in the literature. As an appropriate example, a compound shown by the general formula (XV-VI) is obtained by adding a trifluoromethylation reagent and a catalyst to a compound shown by the general formula (XVI-IV) in an inert solvent and reacting.

Examples of inert solvents include N,N-dimethylformamide, N,N-dimethylacetamide, dimethyl sulfoxide, N-methylpyrrolidone, and other such aprotic polar solvents. N-methylpyrrolidone is preferred. Examples of trifluoromethylation reagents include trifluoromethyl iodide, sodium trifluoroacetate, methyl 2,2-difluoro-2-(fluorosulfonyl)acetate, trifluoromethyl-trimethylsilane, trifluoromethyl-triethylsilane, methyl chlorodifluoroacetate-potassium fluoride, and the like. Methyl 2,2-difluoro-2-(fluorosulfonyl)acetate is preferred. Examples of catalysts include a copper complex, copper iodide, copper bromide, and other such copper salts, and copper powder. Copper iodide is preferred.

The amount of trifluoromethylation reagent used is a 1-to 10-fold molar quantity versus the compound shown by the general formula (XVI-IV), preferably a 1- to 5-fold molar quantity. The amount of catalyst used is a 0.001- to 10-fold molar quantity versus the compound shown by the general formula (XVI-IV), preferably a 0.1- to 5-fold molar quantity. The reaction temperature is from 0°C to reflux, preferably 60°C to reflux. The reaction time is 0.1-48 hours, preferably 1-24 hours.

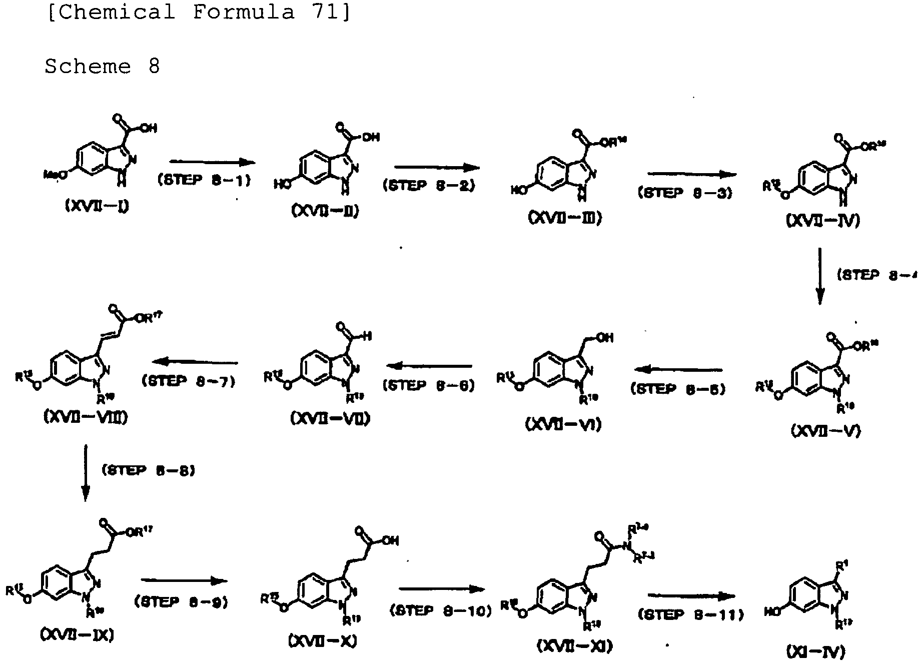

In each general formula in scheme 8, R¹, R⁷⁻¹, R⁷⁻², and R¹⁰ are as defined above. R¹⁵ is a hydroxyl group protective group. R¹⁶ and R¹⁷ are C1-C6 alkyl groups.

In each general formula in scheme 8, R¹⁵ is preferably a tert-butyldiphenylsilyl group or benzyl group. R¹⁶ is preferably a methyl group or ethyl group. R¹⁷ is preferably an ethyl group.

### Step 8-1 (STEP 8-1)

A compound shown by the general formula (XVII-II) is obtained by reacting a compound (XVII-I), which can be purchased from Chem. Pacific Co. or another supplier, by a known method, for example, in accordance with methods described in Protective Groups in Organic Synthesis, John Wiley and Sons, 2007 edition.

The method described in Reference Example 69 can be given as an appropriate example.

### Step 8-2 (STEP 8-2)

A compound (XVII-II) can be converted into a compound shown by the general formula (XVII-III) by a method of converting a carboxylic acid into an ester, for example, in accordance with methods described in Protective Groups in Organic Synthesis, John Wiley and Sons, 2007 edition. As an appropriate example, a compound shown by the general formula (XVII-III) is obtained by adding an acid catalyst or thionyl chloride to compound (XVII-II) in an alcohol solvent and reacting.

Methanol, ethanol, n-propanol, n-butanol, or the like should be selected in accordance with the type of R¹⁶ introduced as the alcohol solvent. Examples of acid catalysts include hydrochloric acid, sulfuric acid, p-toluenesulfonic acid, trifluoroacetic acid, and the like.

The amount of acid catalyst used is a 0.01- to 10-fold molar quantity versus compound (XVII-II). The amount of thionyl chloride used is a 1- to 10-fold molar quantity versus compound (XVII-II), preferably a 1- to 5-fold molar quantity. The reaction temperature is from 0°C to reflux, more preferably 40°C to reflux. The reaction time is 0.1-48 hours, preferably 1-24 hours.

### Step 8-3 (STEP 8-3)

When it is necessary to remove the hydroxyl group protective groups of a compound shown by the general formula (XVII-III), the above hydroxyl group protective groups are selected, and a compound shown by the general formula (XVII-IV) is obtained by a known method, for example, by a method described in Protective Groups in Organic Synthesis, John Wiley and Sons, 2007 edition. As an appropriate example, a compound shown by the general formula (XVII-IV) is obtained by adding a base to a compound shown by the general formula (XVII-III) in an inert solvent and reacting with a silylating agent.

Examples of inert solvents include N,N-dimethylformamide. Examples of bases include imidazole. Examples of silylating agents include triethylchlorosilane, tert-butyldimethylchlorosilane, and the like.

The amount of silylating agent used is a 1- to 10-fold molar quantity versus the compound shown by the general formula (XVII-III), preferably a 1- to 5-fold molar quantity. The amount of base used is a 1- to 10-fold molar quantity versus the compound shown by the general formula (XVII-III), preferably a 1- to 5-fold molar quantity. The reaction temperature is from -20°C to reflux, preferably 0-40°C. The reaction time is 0.1-48 hours, preferably 0.1-12 hours.

### Step 8-4 (STEP 8-4)

When a compound shown by the general formula (XVII-IV) requires protective groups, the above amino group protective groups are selected, and a compound shown by the general formula (XVII-V) is obtained by a known method, for example, by a method described in Protective Groups in Organic Synthesis, John Wiley and Sons, 2007 edition. As an appropriate example, a compound shown by the general formula (XVII-V) is obtained by adding a protective reagent and, if necessary, a base or acid catalyst to a compound shown by the general formula (XVII-IV) in an inert solvent and reacting.

Examples of inert solvents include diethyl ether, tetrahydrofuran, dimethoxyethane, and other such ethers; dichloromethane, 1,2-dichloroethane, and other such halogenated hydrocarbons; benzene, toluene, xylene, and other such benzenes; acetonitrile, and the like, used individually or in mixture. Examples of protective reagents include dihydropyran, chloromethyl methyl ether, 2-(chloromethoxy)ethoxytrimethylsilane, and the like. Examples of bases include potassium carbonate, sodium carbonate, cesium carbonate, sodium bicarbonate, potassium hydroxide, sodium hydroxide, sodium methoxide, potassium t-butoxide, and other such alkali metal compounds; pyridine, 4-dimethylaminopyridine, 1,8-diazabicyclo[5,4,0]-undecene, trimethylamine, triethylamine, and other such organic tertiary amines. Examples of acid catalysts include hydrochloric acid, trifluoroacetic acid, p-toluenesulfonyl acid, and the like.

The amount of protective reagent used is a 1- to 10-fold molar quantity versus the compound shown by the general formula (XVII-V), preferably 1- to 5-fold molar quantity. The amount of base used is a 0- to 10-fold molar quantity versus the compound shown by the general formula (XVII-V), preferably a 0- to 5-fold molar quantity. The amount of catalyst used is a 0.001- to 1-fold molar quantity versus the compound shown by the general formula (XVII-V), preferably a 0.01- to 0.5-fold molar quantity. The reaction temperature is from -20°C to reflux, preferably 0-100°C. The reaction time is 0.1-48 hours, preferably 1-24 hours.

### Step 8-5 (STEP 8-5)

A compound shown by the general formula (XVII-VI) is obtained by reacting a compound shown by the general formula (XVII-V) in accordance with ordinary chemical references, for example, by methods described in Experimental Chemistry Lectures, 4th edition (edited by the Chemical Society of Japan, published by Maruzen), Vol. 26, pp. 159-266 or references described in the literature. As an appropriate example, a compound shown by the general formula (XVII-VI) is obtained by adding a reducing agent to a compound shown by the general formula (XVII-V) in an inert solvent and reacting.

Examples of inert solvents include diethyl ether, tetrahydrofuran, dimethoxyethane, and other such ethers; benzene, toluene, xylene, and other such benzenes; dichloromethane, chloroform, 1,2-dichloroethane, and other such halogenated hydrocarbons, used individually or in mixture. Examples of reducing agents include lithium aluminum hydride, diisobutyl aluminum hydride, lithium borohydride, bis(2-methoxyethoxy)aluminum sodium hydride, and the like.

The amount of reducing agent used is preferably a 1- to 10-fold molar quantity versus the compound shown by the general formula (XVII-V), preferably a 1- to 5-fold molar quantity. The reaction temperature is from -20°C to reflux, preferably 0-50°C. The reaction time is 0.1-48 hours, preferably 0.1-12 hours.

### Step 8-6 (STEP 8-6)

A compound shown by the general formula (XVII-VII) is obtained by reacting a compound shown by the general formula (XVII-VI) in accordance with ordinary chemical references, for example, by methods described in Experimental Chemistry Lectures, 4th edition (edited by the Chemical Society of Japan, published by Maruzen), Vol. 21, pp. 1-23 or references described in the literature. As an appropriate example, a compound shown by the general formula (XVII-VII) is obtained by adding an oxidizer to a compound shown by the general formula (XVII-VI) in an inert solvent and reacting.

Examples of inert solvents include diethyl ether, tetrahydrofuran, 1,4-dioxane, dimethoxyethane, and other such ethers; benzene, toluene, xylene, and other such benzenes; dichloromethane, chloroform, 1,2-dichloroethane, and other such halogenated hydrocarbons, used individually or in mixture. A mixture of dichloromethane and tetrahydrofuran is preferred.

Examples of oxidizers include 1,1,1-triacetoxy-1,1-dihydro-1,2-benziodoxol-3(1H)-one, 2-iodoxybenzoic acid, pyridinium chlorochromate, active manganese dioxide, dimethyl sulfoxide-dicyclohexylcarbodiimide, dimethyl sulfoxide-acetic anhydride, dimethyl sulfoxide-trifluoroacetic anhydride, dimethyl sulfoxide-thionyl chloride, dimethyl sulfoxide-oxalyl chloride, dimethyl sulfoxide-N-chlorosuccinimide, dimethyl sulfoxide-chlorine gas, oxoammonium salt, and super-ruthenium tetrapropyl ammonium. Active manganese dioxide is preferred.

The amount of oxidizer needed is a 1- to 10-fold molar quantity versus the compound shown by the general formula (XVII-VI). The reaction temperature is from -20°C to reflux, preferably from -20 to 40°C. The reaction time is 0.1-48 hours, preferably 0.1-12 hours.

The amount of the above oxidizer can also be reduced to a catalytic quantity by causing the above oxidizer to be present together with 4-methylmorpholine-N-oxide or another such reoxidizer.

### Step 8-7 (STEP 8-7)

A compound shown by the general formula (XVII-VIII) is obtained by reacting a compound shown by the general formula (XVII-VII) in accordance with ordinary chemical references, for example, by methods described in Experimental Chemistry Lectures, 4th edition (edited by the Chemical Society of Japan, published by Maruzen), Vol. 19, pp. 53-101 or references described in the literature. As an appropriate example, a compound shown by the general formula (XVII-VIII) is obtained by adding the following phosphorus ylide or phosphonate and, if necessary, a base to a compound shown by the general formula (XVII-VII) in an inert solvent and reacting.

Examples of inert solvents include diethyl ether, tetrahydrofuran, dimethoxyethane, and other such ethers; benzene, toluene, xylene, and other such benzenes; dichloromethane, chloroform, 1,2-dichloroethane, and other such halogenated hydrocarbons; dimethyl sulfoxide and other such aprotic polar solvents, used individually or in mixture. Tetrahydrofuran is preferred. A phosphorus ylide can be prepared by a usual method. For example, it can be prepared by a usual method such as adding methyl α-bromoacetate, ethyl α-bromoacetate, benzyl α-bromoacetate, tert-butyl α-bromoacetate, n-propyl α-bromoacetate, or another such α-bromoacetic acid ester and triphenyl phosphine in the above inert solvent. Ethyl 2-(diethoxyphosphoryl)acetate is preferred as a phosphonate. Examples of bases include potassium carbonate, sodium carbonate, cesium carbonate, sodium bicarbonate, potassium hydroxide, cesium hydroxide, sodium hydroxide, barium hydroxide, sodium methoxide, sodium hydride, potassium hydride, potassium t-butoxide, and other such alkali metal compounds. Sodium hydride is preferred.

The amount of phosphorus ylide or phosphonate used is a 1- to 10-fold molar quantity versus the compound shown by the general formula (XVII-VII), preferably a 1- to 5-fold molar quantity. The amount of base used is a 0- to 10-fold molar quantity versus the compound shown by the general formula (XVII-VII), preferably a 0- to 5-fold molar quantity. The reaction temperature is from -20°C to reflux, preferably from -20 to 40°C. The reaction time is 0.1-48 hours, preferably 0.1-12 hours.

### Step 8-8 (STEP 8-8)

A compound shown by the general formula (XVII-IX) is obtained by reacting a compound shown by the general formula (XVII-VIII) in accordance with ordinary chemical references, for example, by methods described in Experimental Chemistry Lectures, 4th edition (edited by the Chemical Society of Japan, published by Maruzen), Vol. 26, pp. 159-266 or references described in the literature.
As an appropriate example, a compound shown by the general formula (XVII-IX) is obtained by adding a reducing agent and, if necessary, a base to a compound shown by the general formula (XVII-VIII) in an inert solvent and reacting.

Examples of inert solvents include water and diethyl ether, tetrahydrofuran, dimethoxyethane, and other such ethers, used individually or in mixture. A mixture of dimethoxyethane and water is preferred. p-Toluenesulfonyl hydrazide is preferred as the reducing agent. Sodium acetate is preferred as the base.

The amount of reducing agent used is a 1- to 30-fold quantity versus the compound shown by the general formula (XVII-VIII), preferably a 1- to 20-fold quantity. The amount of base used is a 1- to 30-fold quantity versus the compound shown by the general formula (XVII-VIII), preferably a 1- to 20-fold quantity. The reaction temperature is from -20°C to reflux, preferably from 40°C to reflux. The reaction time is 0.1-48 hours, preferably 1-24 hours.

### Step 8-9 (STEP 8-9)

A compound shown by the general formula (XVII-X) is obtained by reacting a compound shown by the general formula (XVII-IX) in accordance with ordinary chemical references, for example, by methods described in Experimental Chemistry Lectures, 4th edition (edited by the Chemical Society of Japan, published by Maruzen), Vol. 22, pp. 1-43 or references described in the literature. A compound shown by the general formula (XVII-X) can also be obtained by reacting a compound shown by the general formula (XVII-IX) in accordance with methods described in Protective Groups in Organic Synthesis, John Wiley and Sons, 2007 edition, and the like. As an appropriate example, a compound shown by the general formula (XVII-X) is obtained by adding a base to a compound shown by the general formula (XVII-IX) in an inert solvent and reacting.

Examples of inert solvents include water and methanol, ethanol, and other such alcohols, used individually or in mixture. A mixture of methanol and water is preferred. Examples of bases include potassium carbonate, cesium carbonate, potassium hydroxide, cesium hydroxide, sodium hydroxide, barium hydroxide, sodium methoxide, sodium hydride, potassium hydride, potassium t-butoxide, and other such alkali metal compounds. Sodium hydroxide is preferred.

The amount of base used is a 1- to 20-fold molar quantity versus the compound shown by the general formula (XVII-IX), preferably a 1- to 10-fold molar quantity. The reaction temperature is from -20°C to reflux, preferably from 0°C to reflux. The reaction time is 0.1-48 hours, preferably 0.1-12 hours.

### Step 8-10 (STEP 8-10)

A compound shown by the general formula (XVII-XI) is obtained by reacting a compound shown by the general formula (XVII-X) in accordance with ordinary chemical references, for example, by methods described in Experimental Chemistry Lectures, 4th edition (edited by the Chemical Society of Japan, published by Maruzen), Vol. 22, pp. 137-173 or pp. 258-309 or references described in the literature. As an appropriate example, a compound shown by the general formula (XVII-XI) is obtained by adding an alkyloxychloride or acid chloride and a base to a compound shown by the general formula (XVII-X) in an inert solvent and reacting, then adding (R⁷⁻¹)(R⁷⁻²)NH after forming a mixed acid anhydride.

Examples of inert solvents include diethyl ether, tetrahydrofuran, dimethoxyethane, and other such ethers; benzene, toluene, xylene, and other such benzenes; dichloromethane, chloroform, 1,2-dichloroethane, and other such halogenated hydrocarbons, dimethyl sulfoxide, N,N-dimethylformamide, N,N-dimethylacetamide, acetonitrile, and other such aprotic polar solvents, used individually or in mixture. Tetrahydrofuran is preferred. Examples of the alkyloxychloride or acid chloride include isobutyloxycarbonyl chloride, diethylacetyl chloride, pivaloyl chloride, and the like. Pivaloyl chloride is preferred. Examples of bases include pyridine, 4-dimethylaminopyridine, 1,8-diazabicyclo[5,4,0]-undecene, trimethylamine, triethylamine, and other such organic tertiary amines. Triethylamine is preferred.

The amount of alkyloxychloride or acid chloride used is a 1- to 5-fold molar quantity versus the compound shown by the general formula (XVII-X). The amount of base used is a 1- to 10-fold molar quantity versus the compound shown by the general formula (XVII-X). The amount of (R⁷⁻¹)(R⁷⁻²)NH used is preferably a 1- to 10-fold molar quantity. The reaction temperature is from -20°C to reflux, preferably 0-40°C. The reaction time is 0.1-48 hours, preferably 0.1-12 hours.

### Step 8-11 (STEP 8-11)

When it is necessary to remove protective groups of a compound shown by the general formula (XVII-XI), a compound shown by the general formula (XI-IV) is obtained by a known method, for example, in accordance with methods described in Protective Groups in Organic Synthesis, John Wiley and Sons, 2007 edition.

The method described in Reference Example 83 can be given as an appropriate example.

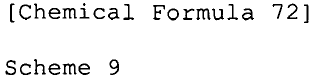

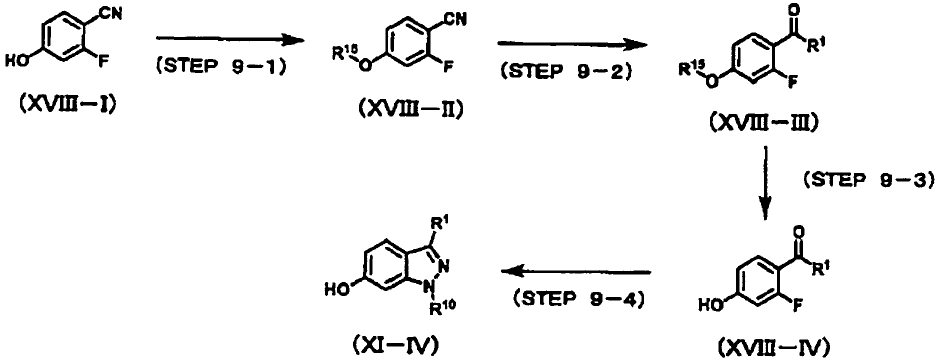

In each formula in scheme 9, R¹⁰ is as defined above, R¹ is a group excluding a perfluoroalkyl group, -OR⁴, -CH(R⁵)OR⁶, and -(CH₂)ₙCONR⁷⁻¹R⁷⁻² from the groups defined above, and R¹⁵ is a hydroxyl group protective group.

In each formula in scheme 9, R¹⁵ is preferably a methoxymethyl group, benzyl group, or tert-butyldimethylsilyl group.

When R¹ of a compound shown by the general formula (XVIII-IV) is a methyl group, the compound can be purchased from Tokyo Chemical Industry Co., Ltd. or another supplier.

### Step 9-1 (STEP 9-1)

A compound shown by the general formula (XVIII-II) is obtained by conducting a protection reaction of the hydroxyl groups of a compound (XVIII-I) which can be purchased from Wako Pure Chemical Industries Co., Ltd. or another supplier by a known method, for example, in accordance with a method described in Protective Groups in Organic Synthesis, John Wiley and Sons, 2007 edition.

As an appropriate example, a compound shown by the general formula (XVIII-II) is obtained by adding a base and protective reagent to compound (XVIII-I) in an inert solvent and reacting.

Examples of inert solvents include dichloromethane, chloroform, 1,2-dichloroethane, and other such halogenated hydrocarbons, N,N-dimethylformamide and other such aprotic polar solvents, used individually or in mixture. Examples of bases include triethylamine, diisopropylethylamine, 1,8-diazabicyclo[5,4,0]-undecene, and other such organic tertiary amines; potassium carbonate, sodium carbonate, cesium carbonate, sodium bicarbonate, and other such alkali metal compounds. Triethylamine, diisopropylethylamine, potassium carbonate, and imidazole are preferred. Examples of protective reagents include tert-butyldimethylchlorosilane, methoxymethyl chloride, benzyl chloride, benzyl bromide, and the like.

The amount of base used is, for example, a 1- to 5-fold molar quantity versus compound (XVIII-I). The protective reagent is, for example, a 1- to 5-fold molar quantity versus compound (XVIII-I). The reaction temperature is from -20°C to reflux, preferably 0-40°C. The reaction time is 0.1-48 hours, preferably 0.1-12 hours.

### Step 9-2 (STEP 9-2)

A compound shown by the general formula (XVIII-III) is obtained by reacting a compound shown by the general formula (XVIII-II) in accordance with ordinary chemical references, for example, by methods described in Experimental Chemistry Lectures, 4th edition (edited by the Chemical Society of Japan, published by Maruzen), Vol. 25, pp. 59-82 or references described in the literature.

As an appropriate example, a compound shown by the general formula (XVIII-III) is obtained by adding a Grignard reagent to introduce an R¹ group and, if necessary, a catalyst to a compound shown by the general formula (XVIII-II) in an inert solvent and reacting, then adding an acidic aqueous solution and hydrolyzing after forming an imine.

Examples of inert solvents include diethyl ether, tetrahydrofuran, dimethoxyethane, and other such ethers; benzene, toluene, xylene, and other such benzenes, used individually or in mixture. Diethyl ether and tetrahydrofuran are preferred. Examples of the Grignard reagent of R¹ include commercial Grignard reagent, Grignard reagent prepared in accordance with the above chemical references or in accordance with methods described in the literature, and Grignard reagent prepared by known methods other than these. For example, cyclobutyl magnesium bromide can be prepared by adding magnesium, a small amount of iodine, and bromocyclobutane to a dehydrated diethyl ether solvent. Examples of catalysts include lithium chloride and other such lithium salts, copper cyanide, copper chloride, copper bromide, copper bromide dimethyl sulfide complex, copper iodide, and other such copper salts and copper complexes. Copper bromide is preferred.

The amount of Grignard reagent used is, for example, a 1-to 5-fold molar quantity versus the compound shown by the general formula (XVIII-II). The catalyst ratio is, for example, compound shown by the general formula (XVIII-II)/catalyst = S/C = 1- to 10,000-fold molar quantity, preferably S/C = 10- to 1000-fold molar quantity. The reaction temperature is from -20°C to reflux, preferably 0°C to reflux. The reaction time is 0.1-48 hours, preferably 0.1-12 hours.

### Step 9-3 (STEP 9-3)

When it is necessary to remove the protective groups of a compound shown by the general formula (XVIII-III), a compound shown by the general formula (XVIII-IV) is obtained by a known method, for example, by a method described in Protective Groups in Organic Synthesis, John Wiley and Sons, 2007 edition. Step 9-2 and step 9-3 can also be carried out continuously through proper selection of R¹⁵.

### Step 9-4 (STEP 9-4)

A compound shown by the general formula (XI-IV) is obtained by adding hydrazines and, if necessary, adding a base to a compound shown by the general formula (XVIII-IV) in an inert solvent and reacting.

Examples of inert solvents include methanol, ethanol, 1-butanol, 2-butanol, and other such alcohols; tetrahydrofuran, dimethoxyethane, and other such ethers; benzene, toluene, xylene, and other such benzenes, used individually or in mixture. Xylene is preferred. Examples of hydrazines include benzyl hydrazine, benzylhydrazine-monohydrochloride, benzylhydrazine-dihydrochloride, hydrazine-monohydrate, and hydrazine-hydrate. Benzylhydrazine-monohydrochloride is preferred. Examples of bases include sodium acetate, potassium carbonate, sodium carbonate, cesium carbonate, sodium bicarbonate, and other such alkali metal compounds. Sodium acetate is preferred.

The amount of hydrazines used is a 1- to 5-fold molar quantity versus the compound shown by the general formula (XVIII-IV), preferably a 1- to 3-fold molar quantity. The amount of base used is a 0- to 10-fold molar quantity versus the compound shown by the general formula (XVIII-IV), more preferably a 1- to 5-fold molar quantity. The reaction temperature is from 0°C to reflux, more preferably from 50°C to reflux. The reaction time is 0.1-48 hours, preferably 3-24 hours.

If the reaction progresses slowly, the interior of the reaction system may be placed in pressurized state by sealing the reactor. In this case, the reaction temperature can exceed the reflux temperature of the solvent, for example, from reflux to 250°C, preferably reflux to 200°C.

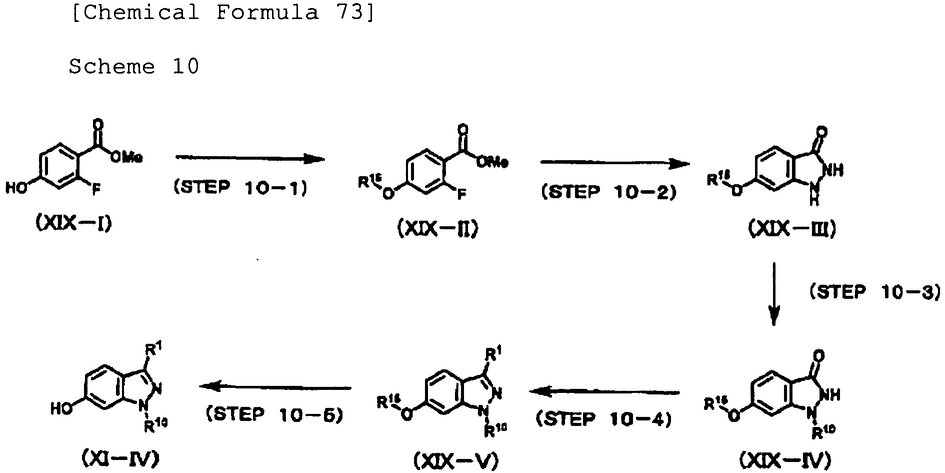

In each general formula in scheme 10, R¹ is -OR⁴ (R⁴ is as defined above) among the above definitions. R¹⁰ is an above-mentioned indazole protective group, and R¹⁵ is an above-mentioned hydroxyl group protective group.

In each general formula in scheme 10, R¹⁰ is preferably a tert-butoxycarbonyl group, and R¹⁵ is preferably a benzyl group.

### Step 10-1 (STEP 10-1)

When it is necessary to protect the hydroxyl groups of a compound (XIX-I) which can be purchased from Chanzou Fine Chem. Co. or another supplier, a compound shown by the general formula (XIX-II) is obtained by a known method, for example, in accordance with a method described in Protective Groups in Organic Synthesis, John Wiley and Sons, 2007 edition.

As an appropriate example, a compound shown by the general formula (XIX-II) is obtained by reacting a compound (XIX-I) with a benzylation reagent and a base in an inert solvent.

Examples of inert solvents include acetone, methyl ethyl ketone, and other such ketones; tetrahydrofuran, diethyl ether, and other such ethers; N,N-dimethylformamide, and other such inert solvents, used individually or in mixture. Acetone is preferred. Examples of benzylation reagents include benzyl chloride, benzyl bromide, and the like. Benzyl bromide is preferred. Examples of bases include potassium carbonate, sodium carbonate, cesium carbonate, sodium bicarbonate, potassium hydroxide, sodium hydroxide, sodium methoxide, potassium t-butoxide, and other such inorganic bases, pyridine, 4-dimethylaminopyridine, 1,8-diazabicyclo[5,4,0]-undecene, trimethylamine, triethylamine, and other such organic amines. Potassium carbonate is preferred.

The amount of base used is preferably a 1- to 10-fold molar quantity versus compound (XIX-I). The benzylation reagent is preferably a 1- to 10-fold molar quantity versus compound (XIX-I).

The reaction temperature is from -20°C to reflux, preferably 0-70°C. The reaction time is 0.1-48 hours, preferably 1-24 hours.

### Step 10-2 (STEP 10-2)

A compound shown by the general formula (XIX-III) is obtained by adding hydrazines and, if necessary, a base, to a compound shown by the general formula (XIX-II) in an inert solvent and reacting.

Examples of inert solvents include methanol, ethanol, 1-butanol, 2-butanol, and other such alcohols; tetrahydrofuran, dimethoxyethane, and other such ethers; benzene, toluene, xylene, and other such benzenes, used individually or in mixture. 1-Butanol is preferred. Examples of hydrazines include hydrazine-monohydrate, hydrazine-monohydrochloride, hydrazine-dihydrochloride, and hydrazine-hydrate. Hydrazine-monohydrate is preferred. Examples of bases include sodium acetate, potassium carbonate, sodium carbonate, cesium carbonate, sodium bicarbonate, and other such inorganic bases, and the like.

Hydrazines are, for example, a 1- to 20-fold molar quantity versus the compound shown by the general formula (XIX-II), more preferably a 1- to 15-fold molar quantity. The base is preferably a 0- to 10-fold molar quantity versus the compound shown by the general formula (XIX-II).

The reaction temperature is from 0°C to reflux. The reaction can also be carried out so that the reaction temperature exceeds the reflux temperature of the solvent by reacting in a sealed microwave reactor. In this case, 100-200°C is preferred. The reaction time is 0.1-48 hours, preferably 0.1-12 hours.

### Step 10-3 (STEP 10-3)

When a compound shown by the general formula (XIX-III) requires amine protective groups, a compound shown by the general formula (XIX-IV) is obtained by a known method, for example, in accordance with a method described in Protective Groups in Organic Synthesis, John Wiley and Sons, 2007 edition.

As an appropriate example, a compound shown by the general formula (XIX-IV) is obtained by adding Boc₂O, a base, and, if necessary, a catalyst to a compound shown by the general formula (XIX-III) in an inert solvent and reacting.

Examples of inert solvents include diethyl ether, tetrahydrofuran, dimethoxyethane, and other such ethers; dichloromethane, 1,2-dichloroethane, and other such halogenated hydrocarbons; benzene, toluene, xylene, and other such benzenes; acetonitrile, and other such inert solvents, used individually or in mixture. Dichloromethane is preferred. Examples of bases include potassium carbonate, sodium carbonate, cesium carbonate, sodium bicarbonate, potassium hydroxide, sodium hydroxide, sodium methoxide, potassium t-butoxide, and other such inorganic bases; pyridine, 4-dimethylaminopyridine, 1,8-diazabicyclo[5,4,0]-undecene, trimethylamine, triethylamine, and other such organic tertiary amines. Triethylamine is preferred. Examples of catalysts include 4-N,N-dimethylaminopyridine and the like.

Boc₂O is preferably a 1- to 10-fold molar quantity versus the compound shown by the general formula (XIX-III). The base is preferably a 1- to 10-fold molar quantity versus the compound shown by the general formula (XIX-III). The catalyst is preferably a 0.001- to 1-fold molar quantity versus the compound shown by the general formula (XIX-III). The reaction temperature is from -20 to 100°C, preferably 0-50°C. The reaction time is 0.1-48 hours, preferably 1-24 hours.

### Step 10-4 (STEP 10-4)

A compound shown by the general formula (XIX-V) is obtained by reacting a compound shown by the general formula (XIX-IV) in accordance with ordinary chemical references, for example, by methods described in Experimental Chemistry Lectures, 5th edition (edited by the Chemical Society of Japan, published by Maruzen), Vol. 14, pp. 239-314 or references described in the literature.

As an appropriate example, a compound shown by the general formula (XIX-V) in which R¹ is -OR⁴ (R⁴ is a lower alkyl group or optionally substituted cyclic lower alkyl group) is obtained by reacting a compound shown by the general formula (XIX-IV) with an alkylation reagent and a base in an inert solvent.

Examples of inert solvents include benzene, toluene, xylene, and other such benzenes; water; N,N-dimethylformamide, N,N-dimethylacetamide, dimethyl sulfoxide, N-methylpyrrolidone, acetonitrile, or other such aprotic polar solvents, used individually or in mixture. Toluene is preferred. Examples of alkylation reagents include methyl iodide, dimethylsulfuric acid, and the like when R⁴ is a methyl group. Methyl iodide is preferred. Examples of bases include potassium carbonate, silver carbonate, sodium carbonate, cesium carbonate, sodium bicarbonate, potassium hydroxide, sodium hydroxide, sodium methoxide, potassium t-butoxide, and other such inorganic bases; pyridine, 4-dimethylaminopyridine, 1,8-diazabicyclo[5,4,0]-undecene, trimethylamine, triethylamine, and other such organic tertiary amines. Silver carbonate is preferred.

The alkylation reagent is preferably a 1- to 10-fold molar quantity versus the compound shown by the general formula (XIX-IV). The base is preferably a 1- to 10-fold molar quantity versus the compound shown by the general formula (XIX-IV). The reaction temperature is from -20 to 100°C, preferably 20-100°C. The reaction time is 0.1-48 hours, preferably 1-24 hours.
A compound shown by the general formula (XIX-V) can also be obtained by reacting a compound shown by the general formula (XIX-IV) in accordance with ordinary chemical references, for example, methods described in Organofluorine Chemistry (by Kenji Uneyama, published by Blackwell), pp. 257-292 or p. 310, or references described in the literature.

As an appropriate example, a compound shown by the general formula (XIX-V) in which R¹ is -OR⁴ (R⁴ is a difluoromethyl group or trifluoromethyl group) is obtained by reacting a compound shown by the general formula (XIX-IV) with a fluoroalkylation reagent and a base in an inert solvent.

Examples of inert solvents include water and N,N-dimethylformamide, N,N-dimethylacetamide, dimethyl sulfoxide, N-methylpyrrolidone, acetonitrile, and other such aprotic polar solvents, used individually or in mixture. N,N-dimethylformamide is preferred.

Examples of fluoroalkylation reagents include chlorodifluoromethane, sodium chlorodifluoroacetate, tert-butyl chlorodifluoroacetate, 2-chloro-2,2-difluoroacetophenone, 2,2-difluoro-2-(fluorosulfonyl)acetic acid, methyl chlorodifluoroacetate, and the like. Sodium chlorodifluoroacetate is preferred.

Examples of bases include inorganic bases, potassium carbonate, sodium carbonate, cesium carbonate, sodium bicarbonate, potassium hydroxide, sodium hydroxide, sodium methoxide, potassium t-butoxide, and other such alkali metal compounds. Potassium carbonate is preferred.
The difluoromethylation reagent is a 1- to 20-fold molar quantity versus the compound shown by the general formula (XIX-IV), preferably a 1- to 10-fold molar quantity. The base is a 1- to 20-fold molar quantity versus the compound shown by the general formula (XIX-IV), preferably a 1- to 10-fold molar quantity. The reaction temperature is from 25°C to reflux, preferably 25-100°C. The reaction time is 0.1-48 hours, preferably 1-24 hours.

### Step 10-5 (STEP 10-5)

When it is necessary to remove the protective groups of a compound shown by the general formula (XIX-V), a compound shown by the general formula (XI-IV) is obtained by a known method, for example, in accordance with a method described in Protective Groups in Organic Synthesis, John Wiley and Sons, 2007 edition.

As an appropriate example, a compound shown by the general formula (XI-IV) is obtained by adding a catalyst to a compound shown by the general formula (XIX-V) in an inert solvent and reacting in the presence of hydrogen gas.

Examples of inert solvents include methanol, ethanol, 1-butanol, 2-butanol, 2-propanol, and other such alcohols; tetrahydrofuran, diethyl ether, and other such ethers, used individually or in mixture. Tetrahydrofuran is preferred. Examples of catalysts include palladium carbon powder. The reaction temperature is from 0°C to reflux, preferably 0-60°C. The reaction time is 0.5-48 hours, preferably 1-24 hours.

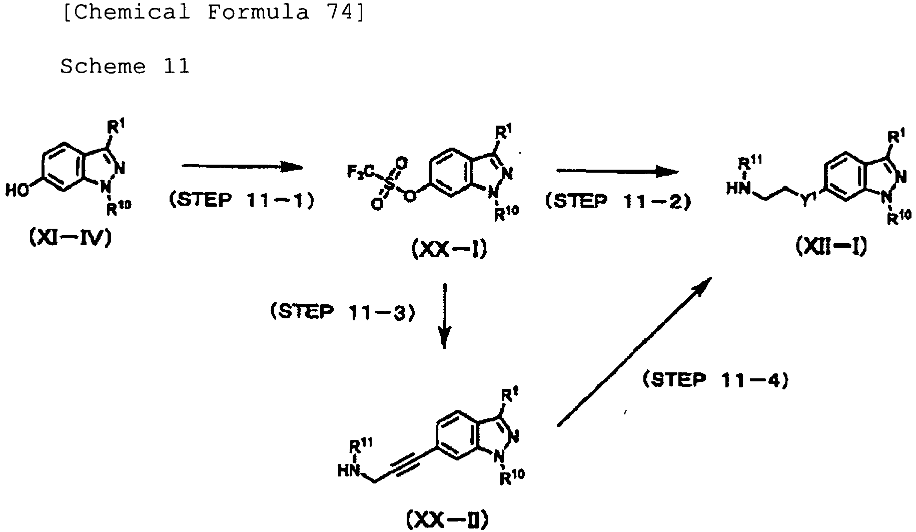

In each general formula in scheme 11, R¹ is as defined above, Y¹ is an atom excluding an oxygen atom from the above definitions, R¹⁰ is an above-mentioned indazole protective group, and R¹¹ is an above-mentioned amino group protective group.

### Step 11-1 (STEP 11-1)

A compound shown by the general formula (XX-I) can be obtained by adding trifluoromethanesulfonic anhydride to a compound shown by the general formula (XI-IV) in an inert solvent in the presence of a base.

Examples of inert solvents include diethyl ether, tetrahydrofuran, dimethoxyethane, and other such ethers; dichloromethane, 1,2-dichloroethane, and other such halogenated hydrocarbons; benzene, toluene, xylene, and other such benzenes; and other such inert solvents, used individually or in mixture. Examples of bases include pyridine, 2,6-dimethylpyridine, 4-dimethylaminopyridine, 1,8-diazabicyclo[5,4,0]-undecene, trimethylamine, triethylamine, and other such organic tertiary amines.

Trifluoromethanesulfonic anhydride is a 1- to 10-fold molar quantity versus the compound shown by the general formula (XI-IV). The base is a 1- to 10-fold molar quantity versus the compound shown by the general formula (XI-IV). The reaction temperature is from -20 to 100°C. The reaction time is 0.1-48 hours.

### Step 11-2 (STEP 11-2)

A compound shown by the general formula (XII-I) is obtained by reacting a compound shown by the general formula (XX-I) in accordance with ordinary chemical references, for example, by the methods described in Metal-Catalyzed Cross-Coupling Reactions (edited by Armin de Meijere, Francois Diederich), pp. 699-760 or references described in the literature.

R¹¹ may be converted to a suitable protective group as needed.

### Step 11-3 (STEP 11-3)

A compound shown by the general formula (XX-II) is obtained by reacting a compound shown by the general formula (XX-I) in accordance with ordinary chemical references, for example, by the methods described Metal-Catalyzed Cross-Coupling Reactions (edited by Armin de Meijere, Francois Diederich), pp. 317-394 or references described in the literature.

R¹¹ may be converted to a suitable protective group as needed.

### Step 11-4 (STEP 11-4)

A compound shown by the general formula (XII-I) is obtained by reacting a compound shown by the general formula (XX-II) in accordance with ordinary chemical references, for example, by methods described in Experimental Chemistry Lectures, 4th edition (edited by the Chemical Society of Japan, published by Maruzen), Vol. 26, pp. 159-266 or references described in the literature.

R¹¹ may be converted to a suitable protective group as needed.

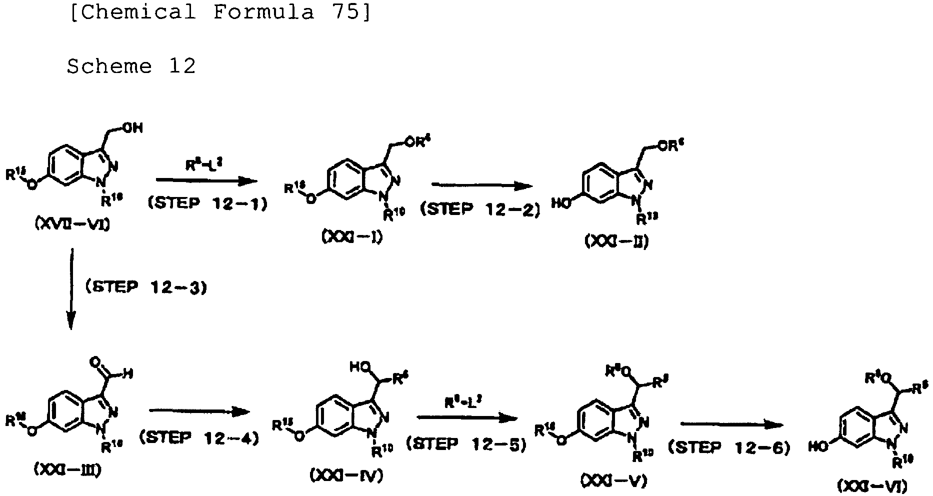

In each general formula in scheme 12, R⁵, R⁶, R¹⁰, R¹⁵, and L² are as defined above.

### Step 12-1 (STEP 12-1)

A compound shown by the general formula (XXI-I) is obtained by reacting a compound shown by the general formula (XVII-VI) in accordance with ordinary chemical references, for example, by methods described in Experimental Chemistry Lectures, 4th edition (edited by the Chemical Society of Japan, published by Maruzen), Vol. 20, pp. 187-200 or references described in the literature. As an appropriate example, a compound shown by the general formula (XXI-I) is obtained by adding a base and R⁶-L² to a compound shown by the general formula (XVII-VI) in an inert solvent and reacting.

Examples of inert solvents include diethyl ether, tetrahydrofuran, 1,4-dioxane, dimethoxyethane, and other such ethers; N,N-dimethylformamide, and other such aprotic polar solvents, used individually or in mixture. N,N-dimethylformamide is preferred. Examples of bases include potassium carbonate, sodium carbonate, cesium carbonate, sodium bicarbonate, potassium hydroxide, cesium hydroxide, sodium hydroxide, barium hydroxide, sodium methoxide, sodium hydride, potassium hydride, potassium t-butoxide, and other such alkali metal compounds; pyridine, 4-dimethylaminopyridine, 1,8-diazabicyclo[5,4,0]-undecene, trimethylamine, triethylamine, and other such organic tertiary amines. Sodium hydride is preferred. An alkylating agent can be used as R⁶-L² When this alkyl is a methyl group, examples of methylating agents include dimethyl sulfate, methyl iodide, and the like. Methyl iodide is preferred.

The amount of base used is preferably a 1- to 5-fold molar quantity versus the compound shown by the general formula (XXI-I). The amount of R⁶-L² used is preferably a 1-to 5-fold molar quantity versus the compound shown by the general formula (XXI-I). The reaction temperature is from - 20°C to reflux, preferably from -20 to 40°C. The reaction time is 0.1-48 hours, preferably 0.1-12 hours.

### Step 12-2 (STEP 12-2)

When it is necessary to remove the protective groups of a compound shown by the general formula (XXI-I), a compound shown by the general formula (XXI-II)is obtained by a known method, for example, in accordance with a method described in Protective Groups in Organic Synthesis, John Wiley and Sons, 2007 edition.

### Step 12-3 (STEP 12-3)

A compound shown by the general formula (XXI-III) is obtained by reacting a compound shown by the general formula (XVII-VI) in accordance with ordinary chemical references, for example, by methods described in Experimental Chemistry Lectures, 4th edition (edited by the Chemical Society of Japan, published by Maruzen), Vol. 21, pp. 1-23 or references described in the literature. As an appropriate example, a compound shown by the general formula (XXI-III) is obtained by adding an oxidizer to a compound shown by the general formula (XVII-VI) in an inert solvent and reacting.

Examples of inert solvents include diethyl ether, tetrahydrofuran, 1,4-dioxane, dimethoxyethane, and other such ethers; benzene, toluene, xylene, and other such benzenes; dichloromethane, chloroform, 1,2-dichloroethane, and other such halogenated hydrocarbons, used individually or in mixture. A mixture of dichloromethane and tetrahydrofuran is preferred.

Examples of oxidizers include 1,1,1-triacetoxy-1,1-dihydro-1,2-benziodoxol-3(1H)-one, 2-iodoxybenzoic acid, pyridinium chlorochromate, active manganese dioxide, dimethyl sulfoxide-dicyclohexylcarbodiimide, dimethyl sulfoxide-acetic anhydride, dimethyl sulfoxide-trifluoroacetic anhydride, dimethyl sulfoxide-thionyl chloride, dimethyl sulfoxide-oxalyl chloride, dimethyl sulfoxide-N-chlorosuccinimide, dimethyl sulfoxide-chlorine gas, oxoammonium salt, and super-ruthenium tetrapropyl ammonium. Active manganese dioxide is preferred.

The amount of oxidizer needed is a 1- to 10-fold molar quantity versus the compound shown by the general formula (XVII-VI). The reaction temperature is from -20°C to reflux, preferably from -20 to 40°C. The reaction time is 0.1-48 hours, preferably 0.1-12 hours.
The amount of the above oxidizer can also be reduced to a catalytic quantity by causing the above oxidizer to be present together with 4-methylmorpholine-N-oxide or another such reoxidizer.

### Step 12-4 (STEP 12-4)

A compound shown by the general formula (XXI-IV) is obtained by reacting a compound shown by the general formula (XXI-III) in accordance with the methods described in Experimental Chemistry Lectures, 4th edition (edited by the Chemical Society of Japan, published by Maruzen), Vol. 25, pp. 60-72 or references described in the literature. As an appropriate example, a compound shown by the general formula (XXI-IV) is obtained by adding Grignard reagent to introduce R⁵ to a compound shown by the general formula (XXI-III) in an inert solvent and reacting.
Examples of inert solvents include diethyl ether, tetrahydrofuran, dimethoxyethane, and other such ethers; benzene, toluene, xylene, and other such benzenes, used individually or in mixture. It is preferable to use a commercial Grignard reagent or to prepare it by a usual method as the Grignard reagent to introduce R⁵.

The amount of Grignard reagent used to introduce R⁵ is preferably a 1- to 5-fold molar quantity versus the compound shown by the general formula (XXI-III). The reaction temperature is from -20°C to reflux, preferably from -20 to 40°C. The reaction time is 0.1-48 hours, preferably 0.1-12 hours.

Unless one is specifically conducting an asymmetric carbon-carbon bond synthesis reaction, the compound shown by the general formula (XXI-IV) obtained by this reduction reaction is obtained as a racemic mixture.

Methods of conducting an asymmetric carbon-carbon bond synthesis reaction can be given as examples of methods of obtaining an optically active compound. An asymmetric carbon-carbon bond synthesis reaction can be carried out in accordance with ordinary chemical references, for example, by methods described in Experimental Chemistry Lectures, 4th edition (edited by the Chemical Society of Japan, published by Maruzen), Vol. 26, pp. 68-158 or references described in the literature.

### Step 12-5 (STEP 12-5)

A compound shown by the general formula (XXI-V) is obtained by reacting a compound shown by the general formula (XXI-IV) in accordance with ordinary chemical references, for example, by methods described in Experimental Chemistry Lectures, 4th edition (edited by the Chemical Society of Japan, published by Maruzen), Vol. 20, pp. 187-200 or references described in the literature. As an appropriate example, a compound shown by the general formula (XXI-V) is obtained by adding a base and R⁶-L² to a compound shown by the general formula (XXI-IV) in an inert solvent and reacting.

Examples of inert solvents include diethyl ether, tetrahydrofuran, 1,4-dioxane, dimethoxyethane, and other such ethers; N,N-dimethylformamide, and other such aprotic polar solvents, used individually or in mixture. N,N-dimethylformamide is preferred. Examples of bases include potassium carbonate, sodium carbonate, cesium carbonate, sodium bicarbonate, potassium hydroxide, cesium hydroxide, sodium hydroxide, barium hydroxide, sodium methoxide, sodium hydride, potassium hydride, potassium t-butoxide, and other such alkali metal compounds; pyridine, 4-dimethylaminopyridine, 1,8-diazabicyclo[5,4,0]-undecene, trimethylamine, triethylamine, and other such organic tertiary amines. Sodium hydride is preferred. An alkylating agent can be used as R⁶-L². When this alkyl is a methyl group, examples of methylating agents include dimethylsulfuric acid, methyl iodide, and the like. Methyl iodide is preferred.

The amount of base used is preferably a 1- to 5-fold molar quantity versus the compound shown by the general formula (XXI-IV). The amount of R⁶-L² used is preferably a 1-to 5-fold quantity versus the compound shown by the general formula (XXI-IV). The reaction temperature is from -20°C to reflux, preferably from -20 to 40°C. The reaction time is 0.1-48 hours, preferably 0.1-12 hours.

### Step 12-6 (STEP 12-6)

When it is necessary to remove the protective groups of a compound shown by the general formula (XXI-V), a compound shown by the general formula (XXI-VI) is obtained by known methods, for example, in accordance with methods described in Protective Groups in Organic Synthesis, John Wiley and Sons, 2007 edition.

The compounds of the present invention obtained in this way and their respective raw material compounds and intermediates can be isolated and refined by extraction, distillation, chromatography, recrystallization, and other such ordinary methods.

A method of using a commercial raw material compound (or one that can be prepared by a known method or in accordance with a known method) that is optically active in advance in the part corresponding to the asymmetric carbon can be given as an example of a method of producing compounds of the present invention that contain an asymmetric carbon, aside from the previously mentioned production method by asymmetric reduction. The compounds of the present invention or precursors thereof can also be isolated as optically active isomers by the usual methods. Such methods include high-performance liquid chromatography (HPLC) using an optically active column; traditional optical fractional crystallization in which a salt is formed with an optically active reagent, and separation is performed using fractional crystallization or another technique, whereupon the formed salt is resolved; condensation with an optically active reagent, isolation and purification of the diastereomer produced, followed again by decomposition; and other such methods. When a precursor is isolated and made into an optically active compound, an optically active compound of the present invention can then be produced by implementing the production processes shown above.

The compounds of the present invention are non-toxic and are useful as drugs. For example, they can be utilized as drugs used in the treatment and prevention of diseases related to the β3-adrenergic receptors since they have β3-adernergic receptor agonist activity. "Diseases related to the β3-adrenergic receptors" is a general descriptor for diseases that can be improved by agonist activity mediated by these receptors. Examples include overactive bladder, urinary incontinence, interstitial cystitis, diabetes, obesity, hyperlipidemia, fatty liver, gastrointestinal diseases (preferably abnormal movement or ulcers of the gastrointestinal tract), depression, cholelithiasis, diseases caused by biliary hyperkinesia, diseases associated with decreased lacrimation, and the like. The use of the drug of the present invention is even more preferred in the treatment and/or prevention of overactive bladder or urinary incontinence, and the use of the drug of the present invention is especially preferred for the treatment of overactive bladder. The use of the drug of the present invention to treat urinary incontinence is another especially preferred embodiment.

The definition of overactive bladder according to the International Continence Society (ICS) is "characterized by urgency as the main symptom, usually with frequent urination and nocturia, with or without incontinence." The definition of urinary incontinence according to the ICS is "objectively demonstrable involuntary loss of urine posing a social and hygienic problem."

The compounds of the present invention are also useful as β3/α1-adrenergic receptor selective agonists. In particular, the compounds of the present invention are desirable in that they have substantially no effect on the α1-adrenergic receptors of a patient even when the compound is administered to a patient with the intention of acting on the β3-adrenergic receptors.

Here, one of the preferred embodiments of a compound that "acts selectively on the β3/α1-adrenergic receptors" is a compound in which the intrinsic activity (I.A. (%)) ratio, that is, the value obtained by dividing the I.A. (%) on the α1-adrenergic receptors by the I.A. (%) on the β3-adrenergic receptors, is 0.8 or lower, preferably 0.7 or lower, more preferably 0.5 or lower, and ideally 0.3 or lower, in the "Test Case 4-A] discussed below. Compounds in which the above I.A. ratio is 0.15 or lower are even more preferred.

Another preferred embodiment of a compound that "acts selectively on the β3/α1-adrenergic receptors" is when the above I.A. ratio is 0.8 or lower and the EC50 ratio, that is, the value obtained by dividing the EC50 of the compound on the α1-adrenergic receptors by the EC50 on the β3-adrenergic receptors is 5 or higher. Another preferred embodiment is when the above I.A. ratio is 0.5 or lower and the above EC50 ratio is 5 or higher. Another preferred embodiment is when the above I.A. ratio is 0.3 or lower and the above EC50 ratio is 5 or higher. Another preferred embodiment is when the above I.A. ratio is 0.15 or lower and the above EC50 ratio is 5 or higher.

Another preferred embodiment of a compound that "acts selectively on the β3/α1-adrenergic receptors" is when the above I.A. ratio is 0.8 or lower and the EC50 ratio is 10 or higher. Another preferred embodiment is when the above I.A. ratio is 0.5 or lower and the EC50 ratio is 10 or higher. Another preferred embodiment is when the above I.A. ratio is 0.3 or lower and the EC50 ratio is 10 or higher. Another preferred embodiment is when the above I.A. ratio is 0.15 or lower and the EC50 ratio is 10 or higher.

Another preferred embodiment of a compound that "acts selectively on the β3/α1-adrenergic receptors" is when the above I.A. ratio is 0.8 or lower and the EC50 ratio is 15 or higher. Another preferred embodiment is when the above I.A. ratio is 0.5 or lower and the above EC50 ratio is 15 or higher. Another preferred embodiment is when the above I.A. ratio is 0.3 or lower and the above EC50 ratio is 15 or higher. Another preferred embodiment is when the above I.A. ratio is 0.15 or lower and the above EC50 ratio is 15 or higher.

The phrase "substantially does not act on the α1-adrenergic receptors" means that, again in the [Test Case 4-A], the compound presents an I.A. of 55% or lower, preferably 45% or lower, more preferably 35% or lower, even more preferably 25% or lower, especially preferably 15% or lower, and ideally 5% or lower, on the α1-adrenergic receptors and that in [Test Case 4-B] discussed below the compound exhibits an agonist response of 30% or lower, preferably 25% or lower, more preferably 20% or lower, even more preferably 15% or lower, especially preferably 10% or lower, and ideally 5% or lower, versus an α1-adrenergic receptor agonist (norepinephrine).

To continue the explanation, the compounds of the present invention have excellent safety (various types of toxicity and safety pharmacology) and pharmacokinetics and can be confirmed to be useful as the active ingredient of drugs.

Tests relating to safety include but are not limited to the following. Cytotoxicity tests (tests using HL60 cells and hepatocytes, and the like), genotoxicity tests (Ames test, mouse lymphoma TK test, chromosomal aberration test, micronucleus test, and the like), skin sensitization tests (Buehler method, GPMT method, APT method, LLNA test, and the like), skin photosensitization tests (adjuvant and strip method and the like), ocular irritation tests (single instillation, short-term continuous instillation, repeated instillation, and the like), safety pharmacology tests on the cardiovascular system (measurement of ECG, heart rate and blood pressure by telemetry, APD method, hERG inhibition assay, and the like), safety pharmacology tests on the central nervous system (FOB method, Irwin method, and the like), safety pharmacology tests on the respiratory system (measurement by an instrument that measures respiratory performance (plethysmography), measurement by blood gas analysis, and the like), general toxicity tests, reproductive and developmental toxicity tests, and the like are included.

Tests relating to pharmacokinetics include but are not limited to the following. Cytochrome P450 enzyme inhibition or induction tests, cell permeability tests (tests using CaCO-2 cells and MDCK cells and the like), drug transporter ATPase assay, oral absorption tests, measurement of changes in blood concentration, metabolic studies (stability tests, metabolic molecular species tests, reactivity tests, and the like), solubility tests (solubility tests by turbidimetry and the like), and the like are included.

The utility of the compounds of the present invention as the active ingredient of drugs can be confirmed by conducting cytotoxicity tests, for example. Cytotoxicity tests include methods that use various types of cultured cells, for example, HL-60 cells which are human pre-leukemia cells, primary isolated cultured cells of hepatocytes, a neutrophil fraction prepared from human peripheral blood, and the like. These tests can be conducted by the methods discussed below, but are not limited to this description alone. The cells are prepared as a cell suspension of from 10⁶ to 10⁷ cells/mL, and from 0.01 to 1 mL of the suspension is dispensed into microtubes or microplates. From 1 to 100% of the amount of the cell suspension of a solution obtained by dissolving the test compound is added thereto and cultured for from 30 minutes to several days at 37°C under 5% CO₂. After culture has been completed, the cell survival rate is evaluated using the MTT method or WST-1 method (Ishiyama, M., et al., In Vitro Toxicology, 8, p. 187, 1995) or the like. Measuring the cytotoxicity of the compounds of the present invention for cells makes it possible to confirm their utility as the active ingredient of drugs.

The utility of the compounds of the present invention as the active ingredient of drugs can be confirmed by conducting genotoxicity tests, for example. Genotoxicity tests include the Ames test, mouse lymphoma TK test, chromosomal aberration test, micronucleus test, and the like. The Ames test is a method of evaluating back mutations using Salmonella and *Escherichia coli* by culturing the bacteria on culture plates and the like that contain the test compound (II-1. Genotoxicity test according to the 1999 Pharmaceutical Affairs Bureau Notification No. 1604, "Genotoxicity test guidelines"). The mouse lymphoma TK test is a test to detect potential gene mutation ability targeting the thymidine kinase gene of mouse lymphoma L5178Y cells (II-3. Mouse lymphoma TK test according to the 1999 Pharmaceutical Affairs Bureau Notification No. 1604, "Genotoxicity test guidelines;" Clive D., et al., Mutat. Res., 31, pp. 17-29, 1975; Cole, M., et al., Mutat. Res., 111, pp. 371-386, 1983). The chromosomal aberration test is a method of assessing activity that causes aberrations in chromosomes by culturing mammalian cultured cells and the test compound together, fixing the cells, and staining and examining the chromosomes (II-2. Chromosomal aberration test using mammalian cultured cells according to the 1999 Pharmaceutical Affairs Bureau Notification No. 1604, "Genotoxicity test guidelines"). The micronucleus test evaluates the micronucleus formation capacity that causes chromosomal aberrations and includes methods using rodents (in vivo test) (II-4. Micronucleus test using rodents according to the 1999 Pharmaceutical Affairs Bureau Notification No. 1604, "Genotoxicity test guidelines;" Hayashi, M., et al., Mutat. Res., 312, pp. 293-304, 1994; Hayashi, M., et al.,. Environ. Mol. Mutagen., 35, pp. 234-252, 2000) and methods using cultured cells (in vitro test) (Fenech, M., et al., Mutat. Res., 147, pp. 29-36, 1985; Miller, B., et al., Mutat. Res., 392, pp. 45-59, 1997). Their utility as an active ingredient of drugs can be confirmed by clarifying the genotoxicity of the compounds of the present invention by using any one or more of these methods.

The utility of the compounds of the present invention as the active ingredient of drugs can be confirmed by conducting skin sensitization tests, for example. Skin sensitization tests include the Buehler method (Buehler, E.V., Arch. Dermatol., 91- pp. 171-177, 1965), GPMT method (maximization method (Magnusson, B., et al., J. Invest. Dermatol, 52, pp. 268-276, 1969)), APT method (adjuvant and patch method (Sato, Y., et al., Contact Dermatitis, 7, pp. 225-237, 1981)), and the like as skin sensitization tests that use guinea pigs. They also include LLNA (local lymph node assay) (OECD Guideline for the testing of chemicals 429, skin sensitization 2002; Takeyoshi, M., et al., Toxicol. Lett., 119(3), pp. 203-8, 2001; Takeyoshi, M., et al., J. Appl. Toxicol., 25(2), pp. 129-34, 2005) as a skin sensitization test using mice. Their utility as an active ingredient of drugs can be confirmed by clarifying the skin sensitization capacity of the compounds of the present invention by using any one or more of these methods.

The utility of the compounds of the present invention as the active ingredient of drugs can be confirmed by conducting skin photosensitization tests, for example. Skin photosensitization tests include skin photosensitization tests using guinea pigs ("Explanation of non-clinical testing guidelines for drug products," Yakuji Nippo Ltd., 2002 1-9: Skin photosensitization test). Methods include the adjuvant and strip method (Ichikawa, H., et al., J. Invest. Dermatol., 76, pp. 498-501, 1981), Harber method (Harber, L.C., Arch. Dermatol., 96, pp. 646-653, 1967), Horio method (Horio, T., J. Invest. Dermatol., 67, pp. 591-593, 1976), Jordan method (Jordan, W.P., Contact Dermatitis, 8, pp. 109-116, 1982), Kochever method (Kochever, I.E., et al., J. Invest. Dermatol., 73, pp. 144-146, 1979), Maurer method (Maurer, T., et al., Br. J. Dermatol., 63, pp. 593-605, 1980), Morikawa method (Morikawa, F., et al., "Sunlight and man," Tokyo Univ. Press, Tokyo, pp. 529-557, 1974), Vinson method (Vinson, L. J., J. Soc. Cosm. Chem., 17, pp. 123-130, 1966), and the like. Their utility as an active ingredient of drugs can be confirmed by clarifying the skin photosensitization capacity of the compounds of the present invention by using any one or more of these methods.

The utility of the compounds of the present invention as the active ingredient of drugs can be confirmed by conducting ocular irritation tests, for example. Ocular irritation tests include single-instillation test methods using the eyes of rabbits, monkeys, and the like (one-time-only instillation), short-term continuous instillation test methods (instillation multiple times within a certain short period of time), and repeated instillation test methods (repeated instillation over a period of from several days to several tens of days), and methods of evaluation of improvement of the ocular irritation symptoms within a certain period of time after instillation based on the Draize score (Fukui, N., et al., Gendai no Rinsho, 4(7), pp. 277-289, 1970), and the like. Their utility as an active ingredient of drugs can be confirmed by clarifying the ocular irritation capacity of the compounds of the present invention by using any one or more of these methods.

The utility of the compounds of the present invention as the active ingredient of drugs can be confirmed by conducting safety pharmacology tests on the cardiovascular system, for example. Safety pharmacology tests on the cardiovascular system include telemetry methods (methods of measuring the effects of administration of a test compound in an awake state on the ECG, heart rate, blood pressure, blood flow, and the like (edited by S. Sugano, H. Tsubone, Y. Nakada, Animal ECG, echocardiography, blood pressure, and pathology tests for basic and clinical trials, 2003, Maruzen Co., Ltd.), APD method (method for measuring the action potential duration of myocardial cells) (Muraki, K., et al., AM. J. Physiol., 269, H524-532, 1995; Ducic, I., et al., J. Cardiovasc. Pharmacol., 30(1), pp. 42-54, 1997), hERG inhibition assay (patch clamp method (Chacin, M., et al., Nippon Yakurigaku Zasshi, 119, pp. 345-351, 2002), binding assay method (Gilbert, J.D., et al., J. Pharm. Tox. Methods, 50, pp. 187-199, 2004), Rb+ efflux assay (Cheng, C.S., et al., Drug Develop. Indust. Pharm., 28, pp. 177-191, 2002), membrane potential assay (Dorn A., et al., J. Biomol. Screen., 10, pp. 339-347, 2005), and the like. Their utility as an active ingredient of drugs can be confirmed by clarifying the effects of the compounds of the present invention on the cardiovascular system by using any one or more of these methods.

The utility of the compounds of the present invention as the active ingredient of drugs can be confirmed by conducting safety pharmacology tests on the central nervous system, for example. Safety pharmacology tests on the central nervous system include the FOB method (functional observation battery (Mattson, J.L., et al., J. American College.of Technology, 15(3), pp. 239-254, 1996)), Irwin modified method (method of evaluating general symptoms and behavior observations (Irwin, S., Comprehensive Observational Assessment (Berl.) 13, pp. 222-257, 1968)), and the like. Their utility as an active ingredient of drugs can be confirmed by clarifying the effects of the compounds of the present invention on the central nervous system by using any one or more of these methods.

The utility of the compounds of the present invention as the active ingredient of drugs can be confirmed by conducting safety pharmacology tests on the respiratory system, for example. Safety pharmacology tests on the respiratory system include measurement by an instrument that measures respiratory performance (measurement of respiratory rate, tidal volume, minute volume, and the like)(Drorbaugh, J.E., et al., Pediatrics, 16, pp. 81-87, 1955; Epstein, M.A., et al., Respir. Physiol., 32, pp. 105-120, 1978), measurement by a blood gas analysis instrument (measurement of blood gases, hemoglobin oxygen saturation, and the like) (Matsuo, S., Medicina, 40, pp. 188-, 2003), and the like. Their utility as an active ingredient of drugs can be confirmed by clarifying the effects of the compounds of the present invention on the respiratory system by using any one or more of these methods.

The utility of the compounds of the present invention as the active ingredient of drugs can be confirmed by conducting general toxicity tests, for example. General toxicity tests are methods of observing the general condition of the animals administered, clinical chemical changes, histopathological changes, and the like by single-dose or repeated-dose (over multiple days) oral or intravenous administration of a test compound dissolved or suspended in a suitable solvent using rats, mice, and other such rodents or monkeys, dogs, or other such non-rodents. Their utility as an active ingredient of drugs can be confirmed by clarifying the general toxicity of the compounds of the present invention by using these methods.

The utility of the compounds of the present invention as the active ingredient of drugs can be confirmed by conducting reproductive and developmental toxicity tests, for example. Reproductive and developmental toxicity tests are tests that study negative effects of a test compound on the reproductive and developmental processes using rats, mice, and other such rodents and monkeys, dogs, and other such non-rodents (refer to "Explanation of non-clinical testing guidelines for drug products 2002" Yakuji Nippo Ltd., 2002 1-6: Reproductive and developmental toxicity tests, and the like). Reproductive and developmental toxicity tests include tests relating to fertility and early embryonic development up to implantation, tests relating to prenatal and postnatal development and maternal function, tests relating to embryonic and fetal development ([3] Reproductive and developmental toxicity tests according to the 2000 Pharmaceutical Affairs Bureau Notification No. 1834 attachment "Drug product toxicity test methods guidelines"), and the like. Their utility as an active ingredient of drugs can be confirmed by clarifying the reproductive and developmental toxicity of the compounds of the present invention by using these methods.

The utility of the compounds of the present invention as the active ingredient of drugs can be confirmed, for example, by conducting cytochrome P450 inhibition and induction tests (Gomez-Lechon, M.J., et al., Curr. Drug Meta. 5(5), pp. 443-462, 2004). These include methods that measure if a test compound inhibits enzyme activity in vitro using human P450-expressing microsomes or cytochrome P450 of various molecular species prepared by purification from cells or using genetic recombinants (Miller, V.P., et al., Ann. N. Y. Acad. Sci., 919, pp. 26-32, 2000), methods that measure changes in the expression or activity of cytochrome P450 of various molecular species using human liver microsomes or cell lysate (Hengstler, J. G., et al., Drug Metab. Rev., 32, pp. 81-118, 2000), methods that investigate the enzyme induction capacity of a test compound by extracting RNA from human hepatocytes that have been exposed to the test compound and comparing the mRNA expression level with that of a control (Kato, M., et al., Drug. Metab. Pharmacokinet., 20 (4), pp. 236-243, 2005), and the like. Their utility as an active ingredient of drugs can be confirmed by clarifying the enzyme-inhibiting and enzyme-inducing effects of the compounds of the present invention on cytochrome P450 by using any one or more of these methods.

The utility of the compounds of the present invention as the active ingredient of drugs can be confirmed, for example, by conducting cell permeability tests. These include methods of measuring the cell membrane permeability of a test compound in an in vitro cell culture system using CaCO-2 cells (Delie, F., et al., Crit. Rev. Ther. Drug Carrier Syst., 14, pp. 221-286, 1997; Yamashita, S., et al., Eur. J. Pharm. Sci., 10, pp. 195-204, 2000; Ingels, F.M., et al., J. Pharm. Sci., 92, pp. 1545-1558, 2003), methods of measuring the cell membrane permeability of a test compound in an in vitro cell culture system using MDCK cells (Irvine, J. D., et al., J. Pharm. Sci., 88, pp. 28-33, 1999), and the like. Their utility as an active ingredient of drugs can be confirmed by clarifying the cell permeability of the compounds of the present invention by using any one or more of these methods.

The utility of the compounds of the present invention as the active ingredient of drugs can be confirmed, for example, by conducting drug transporter ATPase assay as an ATP-binding cassette (ABC) transporter. Examples of drug transporter ATPase assays include methods of investigating whether or not a test compound is a substrate of P-glycoprotein (P-gp) using a P-gp baculovirus expression system (Germann, U. A., Methods Enzymol, 292, pp. 427-41, 1998) and the like. For example, confirmation can be obtained by conducting a transport test using oocytes collected from *Xenopus laevis* as a solute carrier transporter (SLC) transporter. Examples include methods of investigating whether or not a test compound is a substrate of OATP2 using oocytes that express OATP2 as a transport system (Tamai, I. et al., Pharm. Res. 2001, Sep; 18(9):1262-2169). Their utility as an active ingredient of drugs can be confirmed by clarifying the effect of the compounds of the present invention on ABC transporters or SLC transporters by using these methods.

The utility of the compounds of the present invention as the active ingredient of drugs can be confirmed, for example, by conducting oral absorption tests. Oral absorption tests include methods of evaluation using rodents, monkeys, dogs, or the like by dissolving or suspending a certain amount of a test compound in a suitable solvent and measuring the blood concentration over time after oral administration, LC-MS/MS methods to track the blood migration of a test compound by oral administration (edited by K. Harada et al., "Latest mass spectrometry for the life sciences," Kodansha Scientific, 2002), and the like. Their utility as an active ingredient of drugs can be confirmed by clarifying the oral absorption of the compounds of the present invention by using these methods.

The utility of the compounds of the present invention as the active ingredient of drugs can be confirmed, for example, by conducting tests that measure changes in the blood concentration. Tests that measure changes in the blood concentration include LC-MS/MS methods of measuring the changes in the concentration of a test compound in the blood after administration of a test compound to rodents, monkeys, dogs, or the like (edited by K. Harada et al., "Latest mass spectrometry for the life sciences," Kodansha Scientific, 2002) and the like. Their utility as an active ingredient of drugs can be confirmed by clarifying the changes in the blood concentration of the compounds of the present invention by using these methods.

The utility of the compounds of the present invention as the active ingredient of drugs can be confirmed, for example, by conducting metabolic tests. Metabolic tests include methods of testing stability in the blood (methods of predicting the metabolic clearance in vivo from the metabolic rate of a test compound in liver microsomes from humans or other animal species (Shou, W. Z., et al., J. Mass Spectrum., 40(10), pp. 1347-1356, 2005; Li, C., et al., Drug Metab. Dispos., 34(6), 901-905, 2006)), metabolic molecular species test methods, reactive metabolite test methods, and the like. Their utility as an active ingredient of drugs can be confirmed by clarifying the metabolic profile of the compounds of the present invention by using any one or more of these methods.

The utility of the compounds of the present invention as the active ingredient of drugs can be confirmed, for example, by conducting solubility tests. Solubility tests include methods of testing solubility by turbidimetry (Lipinski, C. A., et al., Adv. Drug Deliv. Rev., 23, pp. 3-26, 1997; Bevan, C. D., et al., Anal. Chem., 72, pp. 1781-1787, 2000), and the like. Their utility as an active ingredient of drugs can be confirmed by clarifying the solubility of the compounds of the present invention by using these methods.

The utility of the compounds of the present invention as the active ingredient of drugs can be confirmed, for example, by investigating upper gastrointestinal disorders, renal dysfunction, and the like. Effects on the gastric mucosa can be investigated using a fasted rat gastric mucosa damage model as a pharmacology test of the upper gastrointestinal tract. Examples of pharmacology tests of renal function include measurement of renal blood flow, glomerular filtration [Physiology, 18th edition (Bunkodo), 1986, Chapter 17], and the like. Their utility as an active ingredient of drugs can be confirmed by clarifying the effect of the compounds of the present invention on the upper gastrointestinal tract and renal function by using any one or more of these methods.

The drug of the present invention can be administered orally in the form of a tablet, powder, granules, capsule, sugar-coated tablet, liquid, syrup, or the like or can be administered parenterally in the form of an injection, drip, suppository, percutaneous or absorbable agent, or the like. Inhalation in the form of an aerosol, dry powder, or other such spray can also be given as an example of a preferred form of administration.

The dosing period of a drug of the present invention is not particularly restricted. As a general rule, the time when clinical symptoms of a disease are judged to have appeared can be selected as the dosing period when the drug is administered for the purpose of treatment. Administration usually continues for a period of several weeks to one year, but continuous administration is also possible in accordance with the condition. Continuous administration after recovery from the clinical symptoms is also possible. Furthermore, prophylactic administration is also possible at the discretion of the clinician even if clinical symptoms have not appeared. The dose of the drug of the present invention is not particularly restricted. For example, 0.01-2000 mg of active ingredient can be administered from once to divided over several times as an ordinary adult daily dose. The frequency of administration can be from once a month to every day, preferably from once/week to three times/week, or five times/week, or daily. The daily dose, dosing period, and frequency of administration may also be increased or decreased as is appropriate depending on the patient's age, weight, physical state of health, disease to be treated, severity thereof, and other such factors.

As shall be apparent, the drug of the present invention can be administered together with another drug for preventing or treating various abnormalities and diseases other than those intended in the prophylactic and/or treatment drug of the present invention.

### WORKING EXAMPLES

The present invention is explained further below through working examples, reference examples, and test cases. However, the scope of the present invention is not limited to the following examples.

Various analyses were performed as follows in the following working examples.

(1) Precoated silica gel 60 F254 (made by Merck, product No. 5715-1M) was used in thin-layer chromatography (TLC). After development by chloroform:methanol (1:0-1:1) or ethyl acetate:hexane (1:0-0:1) or the like, spots were confirmed by coloration by UV (254 nm or 365 nm) irradiation, iodine solution, potassium permanganate aqueous solution, phosphomolybdic acid (ethanol solution), ninhydrin, dinitrophenylhydrazine hydrochloric acid solution, or the like.

(2) Column chromatography was conducted by the following methods.
"Column A": A Multi Prep YFLC (made by Yamazen Corp.) was used, and a Hi-Flash™ Column-Silica Gel series made by the same company was used as the column.
"Column B": A Multi Prep YFLC (made by Yamazen Corp.) was used, and a Purif Pack-Si series made by Moritex Corp. was used as the column.
"Column C": A 2 ch parallel purification system "Purif-α2 (50F)" made by Moritex Corp. was used, and a Purif Pack-Si series made by the same company was used as the column.
"Column D": A 2 ch parallel purification system "Purif-α2 (50F)" made by Moritex Corp. was used, and a Hi-Flash™ Column-Silica gel series made by Yamazen Corp. was used as the column.
"Column E": Silica Gel 60N (spherical, neutral, 40 to 100 µm, made by Kanto Chemical Co., Inc.) was used.
"Column F": A BOND ELUT series (MEGA BE-Si; made by Varian, Inc.) was used.
"Column G": A Quad 1 preparative system (made by Biotage) was used, and one or several cartridge columns of any of KP-Sil-12M, 40S, or 40M made by the same company were used in accordance with the amount of sample.
"Column H": Silica gel (made by Merck) was used.
"Column I": BONDESIL-SCX 40UM (made by Varian, Inc.) was used.

(3) In HPLC purification, an LCMS preparative system (made by Waters Corp.) was used, and an ODS column was used. A water-acetonitrile solvent containing 0.1% of acetic acid was used as the eluent. In the case of HPLC purification, unless particularly stated otherwise, a target substance was collected using the molecular weight as a trigger, and the solvent was removed by freeze-drying.

(4) In measurement of the nuclear magnetic resonance spectrum (NMR), measurement was carried out using an AL-300 (FT-NMR, made by JEOL, Ltd.), Gemini-300 (FT-NMR, made by Varian, Inc.) or LA-400 (FT-NMR, made by JEOL, Ltd.). The chemical shift, using tetramethylsilane (TMS) as an internal standard, was expressed as δ(ppm), and the coupling constant was expressed as J (Hz). The symbols of the splitting pattern are as follows: s: singlet, d: doublet, t: triplet, q: quartet, qu: quintet, dd: doublet doublet, td: triplet doublet, m: multiplet, brs: broad singlet, brd: broad doublet, brdd: broad doublet doublet, and brddd: broad doublet doublet doublet.

(5) For "LCMS," the mass spectrum was measured by liquid chromatography-mass spectrometry (LC-MS).
"LCMS Condition: A": A platform-LC type mass spectrometer [made by Micromass, Ltd.] was used as the mass spectrometer, and measurement was conducted by an electrospray ionization (ESI) method. A liquid chromatography apparatus made by Gilson, Inc. was used. The separating column used was a Develosil C30-UG-5 (50 x 4.6 mm) [made by Nomura Chemical Co., Ltd.]. Elution was generally carried out at a flow rate of 2 mL/min, using solution A = water [containing 0.1% (v/v) of acetic acid] and solution B = acetonitrile [containing 0.1% (v/v) of acetic acid] as solvents. Measurement was conducted under the conditions in which a 5-98% (v/v) linear gradient of solution B was run from 0 to 4 minutes, followed by elution by 98% solution B up to 6 minutes.

"LCMS Condition: B": A platform-LC type mass spectrometer [made by Micromass, Ltd.] was used as the mass spectrometer, and measurement was conducted by an electrospray ionization (ESI) method. A liquid chromatography apparatus made by Gilson, Inc. was used. The separating column used was a Develosil C30-UG-5 (50 x 4.6 mm) [made by Nomura Chemical Co., Ltd.]. Elution was generally carried out at a flow rate of 2 mL/min, using solution A = water [containing 0.1% (v/v) of acetic acid] and solution B = acetonitrile [containing 0.1% (v/v) of acetic acid]. Measurement was conducted under conditions in which a 5-100% (v/v) linear gradient of solution B was run from 0 to 5 minutes, followed by elution by 100% solution B up to 9 minutes, and then elution by 5% solution B from 9.01 to 10 minutes.
"LCMS Condition: C": A single quadrupole mass spectrometer UPLC/SQD system [made by Waters Corp.] was used as the mass spectrometer, and measurement was conducted by an electrospray ionization (EST) method. An Acquity Ultra Performance LC system made by Waters Corp. was used as the liquid chromatography apparatus. The separating column used was ACQUITY UPLC BEH C18 2.1 x 50 mm, 1.7 µm [made by Waters Corp.]. Elution was generally carried out at a flow rate of 0.6 mL/min, using solution A = water [containing 0.1% (v/v) of acetic acid] and solution B = acetonitrile [containing 0.1% (v/v) of acetic acid]. Measurement was conducted under conditions in which a 5-90% (v/v) linear gradient of solution B was run from 0 to 2.0 minutes, a 90-98% (v/v) linear gradient of solution B was run from 2.0 minutes to 2.5 minutes, and then elution was carried out using 5% solution B from 2.6 minutes to 2.8 minutes.
"LCMS Condition: D": A single quadrupole mass spectrometer UPLC/SQD system [made by Waters Corp.] was used as the mass spectrometer, and measurement was conducted by an electrospray ionization (ESI) method. An Acquity Ultra Performance LC system made by Waters Corp. was used as the liquid chromatography apparatus. The separating column used was ACQUITY UPLC BEH C18 2.1 x 50 mm, 1.7 µm [made by Waters Corp.]. Elution was generally carried out at a flow rate of 0.6 mL/min, using solution A = water [containing 0.1% (v/v) of acetic acid] and solution B = acetonitrile [containing 0.1% (v/v) of acetic acid]. Measurement was made under conditions in which a 50-90% (v/v) linear gradient of solution B was run from 0 to 2.0 minutes, a 90-98% (v/v) linear gradient of solution B was run from 2.0 minutes to 2.5 minutes, and then elution was carried out using 50% solution B from 2.6 minutes to 2.8 minutes.
"LCMS Condition: E": A single quadrupole mass spectrometer UPLC/SQD system [made by Waters Corp.] was used as the mass spectrometer, and measurement was conducted by an electrospray ionization (ESI) method. An Acquity Ultra Performance LC system made by Waters Corp. was used as the liquid chromatography apparatus. The separating column used was ACQUITY UPLC BEH C18 2.1 x 50 mm, 1.7 µm [made by Waters Corp.]. Elution was generally carried out at a flow rate of 0.6 mL/min, using solution A = water [containing 0.1% (v/v) of acetic acid] and solution B = acetonitrile [containing 0.1% (v/v) of acetic acid]. Measurement was conducted under the conditions in which a 70-90% (v/v) linear gradient of solution B was run from 0 to 2.0 minutes, a 90-98% (v/v) linear gradient of solution B was run from 2.0 minutes to 2.5 minutes, and then elution was carried out using 50% solution B from 2.6 minutes to 2.8 minutes.

(6) For ion chromatography, anion measurement was carried out using an IonPac AS14 (made by Nippon Dionex) as the column. The mobile phase was a 1.0 mmol/L aqueous solution of sodium bicarbonate containing 3.5 mmol/L sodium carbonate at a flow rate of 1.2 mL/min. The column temperature was 30°C, and the detector used was an electrical conductivity detector. Mixed anion standard solution mixed anion standard solution IV (made by Kanto Chemical Co., Inc.) was used as the standard solution. Cation measurement was carried out using IonPac CS14 (made by Nippon Dionex) as the column. The mobile phase used was a 10 mmol/L aqueous solution of methanesulfonic acid at a flow rate of 1.0 mL/min. The column temperature was 30°C, and the detector used was an electrical conductivity detector. Mixed cation standard solution mixed anion standard solution II (made by Kanto Chemical Co., Inc.) was used as the standard solution.

(7) Sealed reactions in microwaves were conducted using Discover (made by CEM Corp.).

The following abbreviations and terms are used in the following working examples.
THF: Tetrahydrofuran
Boc₂O: di-tert-butyl dicarbonate
DMF: N,N-dimethylformamide
TBDMSCl: tert-Butyldimethylsilyl chloride
TBDPSCl: tert-Butyldiphenylsilyl chloride
DMAP: 4-Dimethylaminopyridine
TBAF: Tetra-n-butylammonium fluoride
TMAD: N,N,N',N'-tetramethylazodicarboxamide
MTBE: Methyl-tert-butyl ether
NBS: N-bromosuccinimide
NCS: N-chlorosuccinimide
DBU: 1,8-Diazabicyclo[5,4,0]-undecene
DIAD: Diisopropyl azodicarboxylate
Et₂O: Diethyl ether
(R)-CBS: (R)-5,5-diphenyl-2-methyl-3,4-propano-1,3,2-oxazaborolidine
The following abbreviations and terms are used in chemical formulas that give chemical structural formulas.
Bn: Benzyl group
Boc: tert-Butoxycarbonyl group
TBDMSO: tert-Butyldimethylsilyloxy group
TBDPSO: tert-Butyldiphenylsilyloxy group
THP: Tetrahydro-2H-pyranyl group
Cbz: Benzyloxycarbonyl group
Intermediates for which synthesis methods and references are not listed in the working examples and reference examples are listed below together with references that describe their synthesis methods.

### 1-Benzyl-3-methylindazol-6-ol: Reference Example 11 of International Patent Publication No. WO03/035620 (incorporated herein by reference)

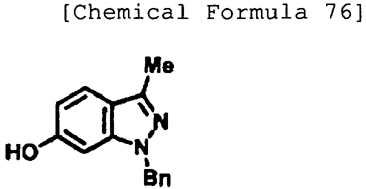

### (R)-2-(3-nitrophenyl)oxirane: Working Example 6 of International Patent Publication No. WO01/17962

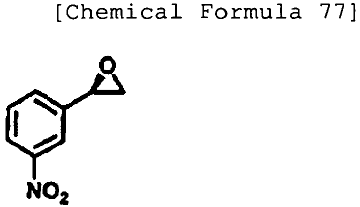

### (R)-2-(4-chloro-3-nitrophenyl)oxirane: Working Example 19 of International Patent Publication No. WO01/17962

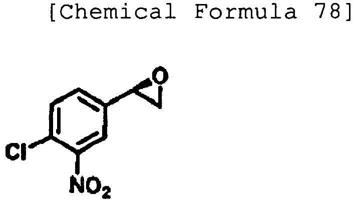

### [Reference Example 1]

### (R)-tert-butyl 6-(2-((2-(3-aminophenyl)-2-(triethylsilyloxy)ethyl)(tert-butoxycarbonyl)amino)ethoxy)-3-methylindazole-1-carboxylate

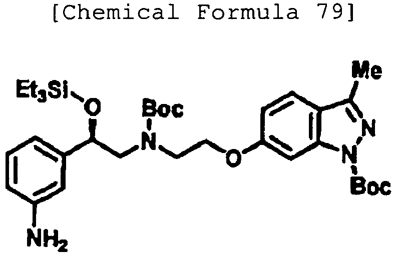

(R)-tert-butyl 2-(3-aminophenyl)-2-(triethylsilyloxy)ethyl(2-hydroxyethyl)carbamate (3.9587 g), which can be manufactured by the method described in Reference Example 87 and the like, tert-butyl 6-hydroxy-3-methylindazole-1-carboxylate (2.0894 g), which can be manufactured by the method described in Reference Example 28 and the like, and triphenyl phosphine (2.7559 g; made by Kanto Chemical Co., Inc.) were dissolved in dehydrated toluene (50 mL). DIAD (2.12 mL; made by Aldrich) was added and stirred overnight at room temperature. The reaction solution was crudely purified by column chromatography ("Column A;" n-hexane: ethyl acetate = 77:23→56:44). The crudely purified product (6.3754 g) was dissolved in CH₂Cl₂ (50 mL). MP-carbonate [8.8 g (2.73 mol/g); made by Argonaut] was added and stirred overnight at room temperature. After filtering the reaction solution, the filtrate was concentrated under reduced pressure. The resulting residue was purified three times by column chromatography ("Column A;" n-hexane: ethyl acetate = 77:23→56:44), and the title compound (3.5065 g) was obtained.
¹H-NMR (300MHz, CDC l₃) ; δ (ppm) 0. 48-0. 5 6 (6 H, m), 0. 8 4-0. 9 1 (9H, m), 1. 48 (9H, s) , 1. 7 0 (9H, s), 2. 52-2. 53 (3H, m), 3. 18-3 .73 (4H, m), 4. 04-4. 13 (2H, m), 4. 80-4. 9 9 (1 H, m), 6.56-6.89 (4H, m), 7. 09 (1 H, t, J=7. 6), 7.46 (1H, dd, J=2. 1, 8. 7), 7. 54 ( 1 H, s)
LCMS: 641 [M + H]; Retention time: 1.71 min; LCMS conditions: E

### [Reference Example 2]

### (R)-tert-butyl 6-(2-((2-(3-aminophenyl)-2-(triethylsilyloxy)ethyl)(tert-butoxycarbonyl)amino)ethoxy)-3-methoxyindazole-1-carboxylate

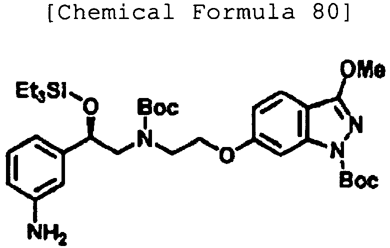

The title compound (1.577 g) was obtained by the same method as in Reference Example 1 except that tert-butyl 6-hydroxy-3-methoxyindazole-1-carboxylate (1.0539 g), which can be manufactured by the method described in Reference Example 26 and the like, was used instead of tert-butyl 6-hydroxy-3-methylindazole-1-carboxylate.
¹H-NMR (300MHz, CDCl₃); *δ* (p pm) 0.48-0.5 7 (6 H, m), 0.88 (9H, t, J=8. 0), 1.47-1.48 (9H, m), 1. 6 8 (9 H, s), 3.14-3.74(4H, m), 4.00-4.10 (2H, m), 4. 1 3 (3H, s), 4.79-4. 99 (1H, m), 6.56-6.84 (4H, m), 7.08 (1 H, t , J=7. 6), 7.41 (1 H, s), 7. 47 (1H, dd, J=2. 9 8 7)
LCMS: 657 [M + H]; Retention time: 1.88 min; LCMS conditions: E

### [Reference Example 3]

### (R)-tert-butyl 6-(2-((2-(3-aminophenyl)-2-(triethylsilyloxy)ethyl)(tert-butoxycarbonyl)amino)ethoxy)-3-(difluoromethoxy)-indazole-1-carboxylate

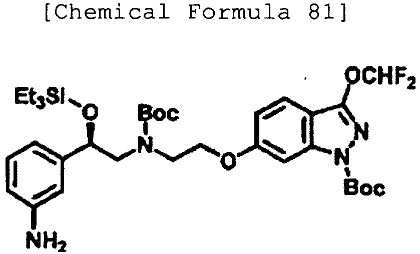

The title compound (2.0404 g) was obtained by the same method as in Reference Example 1 except that tert-butyl 3-difluoromethoxy-6-hydroxyindazole-1-carboxylate (1.4889 g), which can be manufactured by the method described in Reference Example 30 and the like, was used instead of tert-butyl 6-hydroxy-3-methylindazole-1-carboxylate.
¹H-NMR (300MHz, CDCl₃); *δ* (ppm) 0. 55 (6H. q, J=8.0), 0.88 (9 H, t, J=8.0), 1.48-1.4 9 (9 H, m), 1.67 (9 H, s), 3. 14-3. 7 5 (4 H, m) 4. 01-4. 11 (2H, m), 4. 80-4, 9 9 (1H, m), 6 .57-6. 92 (4H, m), 7. 09 (1 H, t, J=7. 6), 7. 35 (1 H. t. J=72. 2), 7.48-7.54 (2H. m)
LCMS: 693 [M + H]; Retention time: 7.24 min; LCMS conditions: B

### [Reference Example 4]

### (R)-tert-butyl 6-(2-((2-(3-aminophenyl)-2-(triethylsilyloxy)ethyl)(tert-butoxycarbonyl)amino)ethoxy)-3-ethylindazole-1-carboxylate

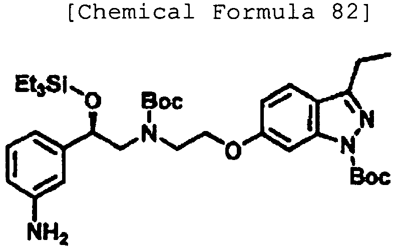

The title compound (4.4487 g) was obtained by the same method as in Reference Example 1 except that tert-butyl 6-hydroxy-3-ethylindazole-1-carboxylate (2.5983 g), which can be manufactured by the method described in Reference Example 37 and the like, was used instead of tert-butyl 6-hydroxy-3-methylindazole-1-carboxylate.
¹H-NMR (300MHz, CDC l₃); *δ* (ppm) 0. 53 (6 H, q, J=7.6), 0.88 (9H, t, J=7. 6), 1.38 (3H, t, J=7. 6), 1.48 (9 H, s), 1.70 (9H, s), 2. 9 5 (2H, q, J=7. 6), 3. 20-3.73 (4H, m), 4. 04 -4.10 (2 H, m), 4.86-4.96 (1H, m), 6.56-6 88 (4H, m), 7. 09 (1H, t, J=7.6), 7.48-7. 5 2 (2H, m)
LCMS: 655 [M + H]; Retention time: 1.94 min; LCMS conditions: E

### [Reference Example 5]

### (R)-tert-butyl 6-(2-((2-(3-aminophenyl)-2-(triethylsilyloxy)ethyl)(tert-butoxycarbonyl)amino)ethoxy)-3-chloroindazole-1-carboxylate

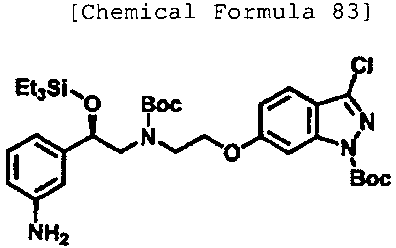

The title compound (1.862 g) was obtained by the same method as in Reference Example 1 except that tert-butyl 3-chloro-6-hydroxyindazole-1-carboxylate (1.3084 g), which can be manufactured by the method described in Reference Example 42 and the like, was used instead of tert-butyl 6-hydroxy-3-methylindazole-1-carboxylate.
¹H-NMR (300MHz, CDC l₃); *δ* (ppm) 0.56 (6 H, q, J=8.0), 0.88 (9H, t, J=8.0), 1.48 (9 H, s), 1.69 (9H, s), 3.15-3.77 (4 H, m), 4. 0 6 -4.13 (2 H, m), 4. 80-4. 99 (1H, m), 6. 58-6 79 (3H, m), 6.92-6.95 (1 H. m), 7. 09 (1 H. t, J=7.6), 7.50 (1 H, dd, J=2.9, 8.7), 7.5 7 (1 H. s)
LCMS: 661 [M + H]; Retention time: 2.22 min; LCMS conditions: E

### [Reference Example 6]

### (R)-tert-butyl6-(2-((2-(3-aminophenyl)-2-(triethylsilyloxy)ethyl)(tert-butoxycarbonyl)amino)ethoxy)-3-(trifluoromethyl)-indazole-1-carboxylate

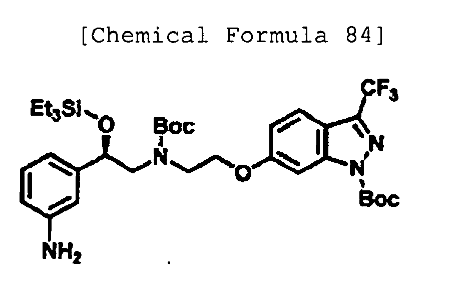

The title compound (1.7128 g) was obtained by the same method as in Reference Example 1 except that tert-butyl 6-hydroxy-3-(trifluoromethyl)-indazole-1-carboxylate (1.6977 g), which can be manufactured by the method described in Reference Example 50 and the like, was used instead of tert-butyl 6-hydroxy-3-methylindazole-1-carboxylate.
¹H-NMR (300MHz, CDCl₃); *δ* (ppm) 0.53 (6H. q, J=8. 0), 0.88 (9H, t, J=8.0), 1.48 (9 H, s), 1.71 (9H, s), 3. 15-3.78 (4H, m), 4. 0 3 -4.14 (2H, m), 4.80-4.99 (1H, m), 6.58-6. .7 9 (3 H, m), 6. 9 9 (1H, dd, J = 1. 8, 8.7), 7. 09 (1H, t, J=7.6), 7. 60-7. 66 (2H, m)
LCMS: 695 [M + H]; Retention time: 2.03 min; LCMS conditions: E

### [Reference Example 7]

### (R)-tert-butyl 6-(2-((2-(3-aminophenyl)-2-(triethylsilyloxy)ethyl)(tert-butoxycarbonyl)amino)ethoxy)-3-cyclopropylindazole-1-carboxylate

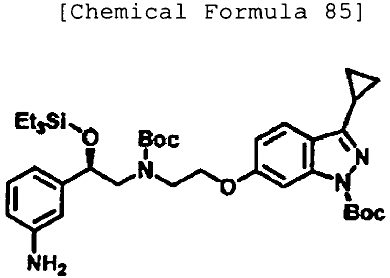

The title compound (1.0644 g) was obtained by the same method as in Reference Example 1 except that tert-butyl 6-hydroxy-3-cyclopropylindazole-1-carboxylate (924 mg), which can be manufactured by the method described in Reference Example 56 and the like, was used instead of tert-butyl 6-hydroxy-3-methylindazole-1-carboxylate.
¹H-NMR (300MHz, CDCl₃); *δ* (ppm) 0. 53 (6H, q, J=8. 0), 0.88 (9H, t, J=8.0), 1.02-1.0 9 (2H, m), 1.15-1.20 (2H, m), 1. 4 7 (9H, s) , 1.68 (9H, s), 2.12-2.19 (1H, m), 3.37-3. 77 (4H, m), 4.03-4.11 (2H, m), 4.79-4.9 9 (1 H. m), 6.56-6.87 (4H, m), 7. 08 (1H, t. J = 7.6), 7.49-7.52 (2H, m)
LCMS: 667 [M + H]; Retention time: 2.06 min; LCMS conditions: E

### [Reference Example 8]

### (R)-tert-butyl 6-(2-((2-(3-aminophenyl)-2-(triethylsilyloxy)ethyl)(tert-butoxycarbonyl)amino)ethoxy)-3-cyclobutylindazole-1-carboxylate

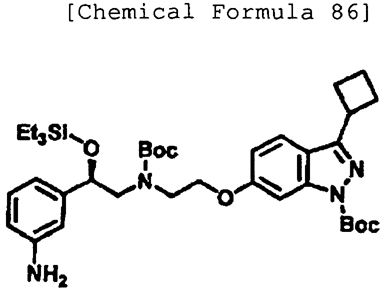

The title compound (619.1 mg) was obtained by the same method as in Reference Example 1 except that tert-butyl 6-hydroxy-3-cyclobutylindazole-1-carboxylate (434.5 mg), which can be manufactured by the method described in Reference Example 62 and the like, was used instead of tert-butyl 6-hydroxy-3-methylindazole-1-carboxylate.
¹H-NMR (300MHz. CDCl₃); *δ* (ppm) 0.48-0.5 7 (6H, m), 0. 87 (9H, t, J=8.0), 1.47 (9H, s ), 1.69 (9H, s), 2.01 - 2. 1 7 (2H, m), 2.42-2. 5 9 (4H, m), 3.20-3.72 (4H, m), 3. 8 7 (1H , q u, J=8.7), 4.03-4.12 (2H, m), 4.79-4. 96 (1 H, m), 6.56-6.87 (4H, m), 7. 08 (1H, t , J=7.6), 7.50-7.53 (2H, m)
LCMS: 681 [M + H]; Retention time: 2.28 min; LCMS conditions: E

### [Reference Example 9]

### (R)-tert-butyl 6-(2-((2-(3-aminophenyl)-2-(triethylsilyloxy)ethyl)(tert-butoxycarbonyl)amino)ethoxy)-3-isopropylindazole-1-carboxylate

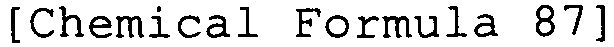

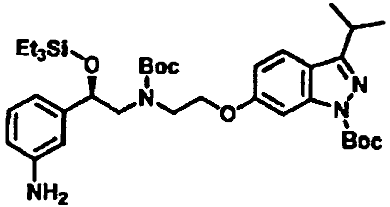

The title compound (633.1 mg) was obtained by the same method as in Reference Example 1 except that tert-butyl 6-hydroxy-3-isopropylindazole-1-carboxylate (419.7 mg), which can be manufactured by the method described in Reference Example 68 and the like, was used instead of tert-butyl 6-hydroxy-3-methylindazole-1-carboxylate.
¹H-NMR (300MHz, CDCl₃); *δ* (ppm) 0.49-0.5 7 (6H, m), 0.8 8 (9H, t, J=8.0), 1.44 (6H, d , J=6.9), 1.47 (9H, s), 1. 74 (9H, s), 3. 1 5 -3.7 8 (5H, m), 4.02-4.12 (2H, m), 4.79-4 .99 (1H, m), 6.56-6.87 (4H, m), 7. 08 (1H, t, J=7.6), 7.51 (1H, s), 7. 57 (1 H, d, J=8. 7)
LCMS: 669 [M + H]; Retention time: 2.18 min; LCMS conditions: E

### [Reference Example 10]

### (R)-tert-butyl 6-(2-((2-(3-aminophenyl)-2-(triethylsilyloxy)ethyl)(tert-butoxycarbonyl)amino)ethoxy)-3-(3-(dimethylamino)-3-oxopropyl)indazole-1-carboxylate

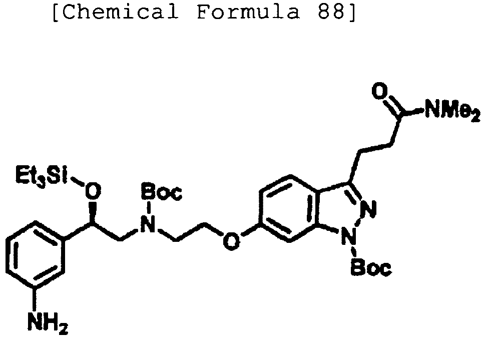

The title compound (453 mg) was obtained by the same method as in Reference Example 1 except that tert-butyl 3-(3-(dimethylamino)-3-oxopropyl)-6-hydroxyindazole-1-carboxylate (502.1 mg), which can be manufactured by the method described in Reference Example 83 and the like, was used instead of tert-butyl 6-hydroxy-3-methylindazole-1-carboxylate.
¹H-NMR (300MHz, CDCl₃); *δ* (ppm) 0.49-0.5 7 (6H, m), 0.88 (9H, t, J=8.0), 1.47 (9H, s ), 1. 6 9 (9H, s), 2.88 (2H, t. J=6.9), 2. 9 5 (3H, s), 3.01 (3H, s), 3.18 - 3. 7 2 (4 H, m), 3. 2 6 (2H, t, J=6.9), 4.03-4.12 (2H, m), 4 .7904. 9 8 (1H, m), 6.56-6.88 (4H, m), 7.0 8 (1H, t, J = 7.3), 7. 48 (1 H. s), 7. 48-7.53 (1 H, m)
LCMS: 726 [M + H]; Retention time: 6.46 min; LCMS conditions: B

### [Reference Example 11]

### (R)-tert-butyl 6-(2-((2-(3-amino-4-fluorophenyl)-2-(triethylsilyloxy)ethyl)(tert-butoxycarbonyl)amino)ethoxy)-3-methylindazole-1-carboxylate

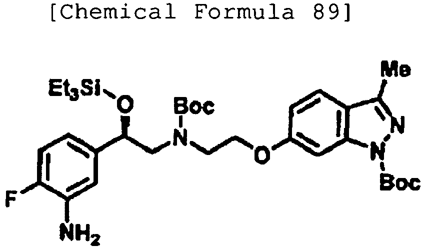

(R)-tert-butyl 6-(2-((2-(3-amino-4-fluorophenyl)-2-hydroxyethyl)(tert-butoxycarbonyl)amino)ethoxy)-3-methylindazole-1-carboxylate (1.1753 g), which can be manufactured by the method described in Reference Example 93 and the like, and imidazole (578.2 mg; made by Tokyo Chemical Industry Co., Ltd.) were dissolved in dehydrated DMF (10 mL). Chlorotriethylsilane (1.40 mL; made by Shin-Etsu Chemical Co., Ltd.) was added and stirred for 30 minutes at room temperature. The reaction solution was poured into saturated sodium bicarbonate and extracted once with ethyl acetate. The organic layer was washed once with water and once with brine, and the solvent was distilled off under reduced pressure. The resulting residue was purified by column chromatography ("Column B;" n-hexane: ethyl acetate = 84:16→64:36), and the title compound (1.2861 g) was obtained.
¹H-NMR (300MHz, CDCl₃) ; δ (ppm) 0. 52 (6H, q, J=8. 0), 0.88 (9H, t, J = 8.0), 1.47 (9H. s), 1.70 (9H, s) 2.53 (3H, s), 3.13-3.76 (4 H, m), 4. 0 4-4.12 (2H, m), 4.77 - 4. 9 6 (1 H, m), 6. 57-6. 9 4 (4H, m), 7. 4 6 (1H, d, J = 8 .7), 7.55 (1H, s)
LCMS: 659 [M + H]; Retention time: 1.87 min; LCMS conditions: E

### [Reference Example 12]

### (R)-tert-butyl 6-(2-((2-(3-amino-4-chlorophenyl)-2-(triethylsilyloxy)ethyl)(tert-butoxycarbonyl)amino)ethoxy)-3-methylindazole-1-carboxylate

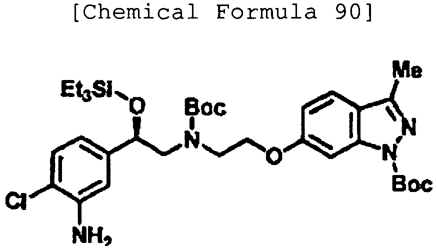

(R)-tert-butyl 6-(2-((2-(3-amino-4-chlorophenyl)-2-hydroxyethyl)(tert-butoxycarbonyl)amino)ethoxy)-3-methylindazole-carboxylate (1.5310 g), which can be manufactured by the method described in Reference Example 96 and the like, was dissolved in dehydrated DMF (12 mL). Imidazole (751 mg; made by Tokyo Chemical Industry Co., Ltd.) and chlorotriethylsilane (1.84 mL; made by Shin-Etsu Chemical Co., Ltd.) were added and stirred for 50 minutes at room temperature. The reaction solution was poured into saturated sodium bicarbonate and extracted once with ethyl acetate. The organic layer was washed once with water and once with brine, and the solvent was distilled off under reduced pressure. The resulting residue was purified by column chromatography ("Column B;" n-hexane: ethyl acetate = 88:12→67:33), and the title compound (1.6137 g) was obtained. ¹H-NMR (300MHz, CDCl₃) ; δ (ppm) 0. 53 (6H, q, J=7.6), 0. 88 (9H, t, J=7. 6), 1.46-1.4 7 (9H, m), 1. 7 0 (9H, s), 2. 5 3 (3H, s), 3. 1 2 - 3. 7 7 (4 H, m), 4.03-4.1 2 (4H, m), 4.77-4 .98 (1H, m), 6. 59-6.89 (3H, m), 7. 1 7 (1H, d, J=8. 0), 7. 46 (1H, d, J=8. 7), 7. 55 (1H, s)

### [Reference Example 13]

### (R)-tert-butyl 6-(2-((2-(3-amino-4-fluorophenyl)-2-(triethylsilyloxy)ethyl)(tert-butoxycarbonyl)amino)ethoxy)-3-methoxyindazole-1-carboxylate

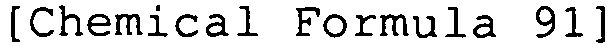

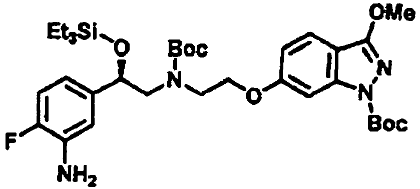

The title compound (1.0262 g) was obtained by the same method as in Reference Example 11 using (R)-tert-butyl 6-(2-((2-(3-amino-4-fluorophenyl)-2-hydroxyethyl)(tert-butoxycarbonyl)amino)ethoxy)-3-methoxyindazole-1-carboxylate (1.0634 g), which can be manufactured by the method described in Reference Example 100 and the like, instead of (R)-tert-butyl 6-(2-((2-(3-amino-4-fluorophenyl)-2-hydroxyethyl)(tert-butoxycarbonyl)amino)ethoxy)-3-methylindazole-carboxylate.
¹H-NMR (300MHz, CDCl₃) ; δ (p pm) 0. 5 1 (6H, q, J=8. 0), 0.87 (9H, t, J=8. 0), 1. 4 6 (9H, s), 1. 6 8 (9H, s) . 3. 12-3.75 (4H, m), 4. 0 5 -4.12 (2H, m), 4. 1 3 (3H, s), 4. 76-4.96 (1 H, m), 6. 57-6.94 (4H, m), 7.42 (1H, brs), 7. 47 (1H, dd, J=2. 1, 8.7)
LCMS: 675 [M + H]; Retention time: 2.03 min; LCMS conditions: E

### [Reference Example 14]

### (R)-tert-butyl 6-(2-((2-(3-amino-4-chlorophenyl)-2-(triethylsilyloxy)ethyl)(tert-butoxycarbonyl)amino)ethoxy)-3-methoxyindazole-1-carboxylate

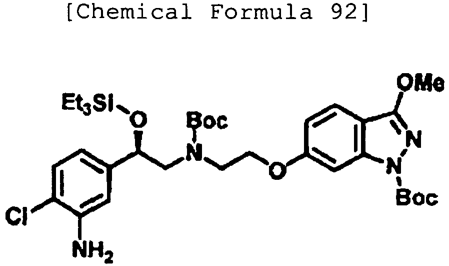

The title compound (2.1204 g) was obtained by the same method as in Reference Example 12 using (R)-tert-butyl 6-(2-((2-(3-amino-4-chlorophenyl)-2-hydroxyethyl)(tert-butoxycarbonyl)amino)ethoxy)-3-methoxyindazole-carboxylate (1.9428 g), which can be manufactured by the method described in Reference Example 103 and the like, instead of (R)-tert-butyl 6-(2-((2-(3-amino-4-chlorophenyl)-2-hydroxyethyl)(tert-butoxycarbonyl)amino)ethoxy)-3-methylindazole-carboxylate.
¹H-NMR (300MHz, CDCl₃) ; δ (ppm) 0.52 (6H. q, J=7. 6), 0.88 (9H, t, J = 7.6), 1.46 (9H, s), 1.67 (9H, s) . 3. 1 2-3.7 6 (4H, m), 4. 0 2 -4.10 (2H, m), 4.13 (3H, s), 4. 76-4.97 (1 H, m), 6.58-6.83 (3H, m), 7. 1 7 (1H, d, J=8 .0), 7. 42 (1H, brs), 7. 47 (1 H, dd, J = 1.8, 8. 4)

### [Reference Example 15]

### (R)-tert-butyl 6-(2-((2-(3-amino-4-fluorophenyl)-2-(triethylsilyloxy)ethyl)(tert-butoxycarbonyl)amino)ethoxy)-3-chloroindazole-1-carboxylate

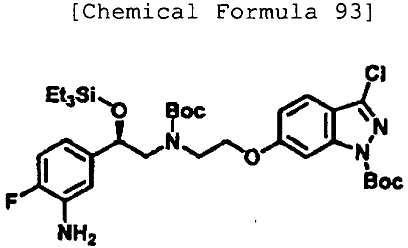

(R)-tert-butyl 6-(2-((2-(3-amino-4-fluorophenyl)-2-hydroxyethyl)(tert-butoxycarbonyl)amino)ethoxy)-3-chloroindazole-1-carboxylate (601.2 mg), which can be manufactured by the method described in Reference Example 107 and the like, and imidazole (290.3 mg; made by Tokyo Chemical Industry Co., Ltd.) were dissolved in dehydrated DMF (5 mL). Chlorotriethylsilane (705 µL; made by Shin-Etsu Chemical Co., Ltd.) was added and stirred for three hours at room temperature. The reaction solution was poured into saturated sodium bicarbonate and extracted twice with ethyl acetate. The organic layer was washed twice with water and once with brine. After drying had been performed using anhydrous sodium sulfate, the solvent was distilled off under reduced pressure. The resulting residue was purified by column chromatography ("Column B;" n-hexane: ethyl acetate = 84:16→64:36), and the title compound (480 mg) was obtained.
¹H-NMR (300MHz, CDCl₃); δ (ppm) 0. 52 (6H, q, J = 8.0), 0.88 (9 H, q, J=8.0), 1. 47 (9H, s), 1.69 (9H, s), 3. 12-3.7 6 (4 H, m), 4. 0 6 -4.1 2 (2H, m), 4.77-4.9 7 (1H, m), 6.57-6 .96 (4H, m), 7.51 (1H, dd, J=2.1, 8. 4), 7. 58 (1H, s)
LCMS: 679 [M + H]; Retention time: 2.29 min; LCMS conditions: E

### [Reference Example 16]

### (R)-tert-butyl 6-(2-((2-(3-amino-4-chlorophenyl)-2-(triethylsilyloxy)ethyl)(tert-butoxycarbonyl)amino)ethoxy)-3-chloroindazole-1-carboxylate

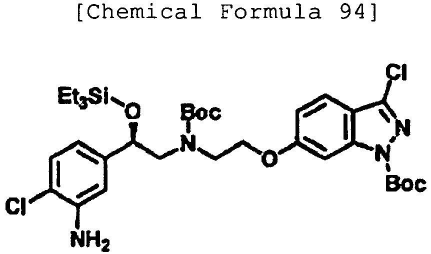

(R)-tert-butyl 6-(2-((2-(3-amino-4-chlorophenyl)-2-hydroxyethyl)(tert-butoxycarbonyl)amino)ethoxy)-3-chloroindazole-carboxylate (404.3 mg), which can be manufactured by the method described in Reference Example 110 and the like, was dissolved in dehydrated DMF (5 mL). Imidazole (197.8 mg; made by Tokyo Chemical Industry Co., Ltd.) and chlorotriethylsilane (470 µL; made by Shin-Etsu Chemical Co., Ltd.) were added and stirred for six hours at room temperature. The reaction solution was poured into saturated sodium bicarbonate and extracted once with ethyl acetate. The organic layer was washed once with water and once with brine, dried using anhydrous sodium sulfate, and the solvent was distilled off under reduced pressure. The resulting residue was purified by column chromatography ("Column B;" n-hexane: ethyl acetate = 92:8→71:29), and the title compound (278.7 mg) was obtained.
¹H-NMR (300MHz, CDCl₃) ; δ (ppm) 0.53 (6H, q, J=7. 6), 0. 88 (9H, t, J = 7.6), 1.46-1.4 7 (9H, m), 1.69 (9H, s), 3.11-3.77 (4 H, m) , 4. 03-4. 1 2 (2H, m), 4. 77-4. 9 8 (1H, m), 6 . 59-6.9 5 (3H, m), 7. 1 7 (1 H, d, J=8.0), 7. 5 1 (1H, dd, J=1. 8, 8.7), 7. 5 8 (1H, s)
LCMS: 694 [M + H]; Retention time: 2.56 min; LCMS conditions: E

### [Reference Example 17]

### (R)-tert-butyl 6-(2-((2-(3-amino-4-fluorophenyl)-2-(triethylsilyloxy)ethyl(tert-butoxycarbonyl)amino)ethoxy)-3-(trifluoromethyl)-indazole-1-carboxylate

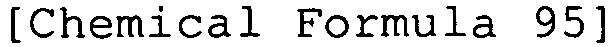

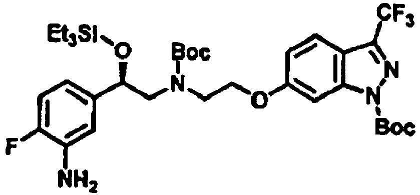

The title compound (709.9 mg) was obtained by the same method as in Reference Example 11 using (R)-tert-butyl 6-(2-((2-(3-amino-4-fluorophenyl)-2-hydroxyethyl)(tert-butoxycarbonyl)amino)ethoxy)-3-(trifluoromethyl)-indazole-1-carboxylate (680.5 mg), which can be manufactured by the method described in Reference Example 114 and the like, instead of (R)-tert-butyl 6-(2-((2-(3-amino-4-fluorophenyl)-2-hydroxyethyl)(tert-butoxycarbonyl)amino)ethoxy)-3-methylindazole-carboxylate.
¹H-NMR (300MHz, CDCl₃) ; δ (ppm) 0.52 (6H, q, J = 7.6), 0.88 (9 H, t, J=7.6), 1.47 (9H, s), 1. 7 1 (9H, s), 3.12-3.77 (6H, m), 4. 0 6 -4.13 (2H, m), 4. 77-4.9 7 (1H, m), 6. 57-7 .01 (4H, m), 7.61 (1H, s), 7. 6 5 (1H, d, J = 8 7)

### [Reference Example 18]

### (R)-tert-butyl 6-(2-((2-(3-amino-4-chlorophenyl)-2-(triethylsilyloxy)ethyl)(tert-butoxycarbonyl)amino)ethoxy)-3-(trifluoromethyl)-indazole-1-carboxylate

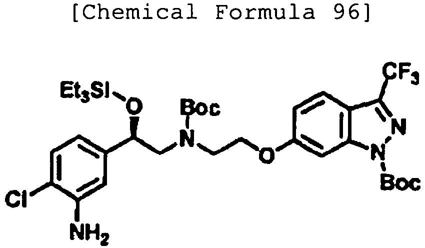

The title compound (778.1 mg) was obtained by the same method as in Reference Example 12 using (R)-tert-butyl 6-(2-((2-(3-amino-4-chlorophenyl)-2-hydroxyethyl)(tert-butoxycarbonyl)amino)ethoxy)-3-(trifluoromethyl)-indazole-1-carboxylate (741 mg), which can be manufactured by the method described in Reference Example 117 and the like, instead of (R)-tert-butyl 6-(2-((2-(3-amino-4-chlorophenyl)-2-hydroxyethyl)(tert-butoxycarbonyl)amino)ethoxy)-3-methylindazole-carboxylate.
¹H-NMR (300MHz, CDCl₃) ; δ (ppm) 0.53 (6H, q, J = 7.6), 0.89 (9H, t, J=7. 6), 1. 46-1.4 8 (9H, m), 1.71 (9H, s), 3. 12-3.80 (4H, m) , 4. 05-4.14 (2 H, m), 4. 78-5.0 4 (1H, m), 6 . 54-6.80 (2H, m), 6.96-7.01 (1 H, m), 7. 1 8 (1 H, d, J=8. 4), 7.61 (1H, d, J=1.8), 7.6 5 (1H, d, J=8. 7)

### [Reference Example 19]

### Cyclobutanesulfonyl chloride

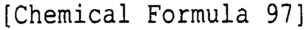

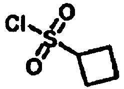

[Solution 19-1]: Bromocyclobutane (939.4 mg; made by Aldrich) was dissolved in dehydrated diethyl ether (6 mL) in an argon atmosphere. After cooling to -78°C, 1.77 mol/L tert-butyllithium-n-pentane solution (7.9 mL; made by Kanto Chemical Co., Inc.) was added dropwise. The reaction solution was warmed to -35°C and stirred for 1.5 hours at -35°C.

[Solution 19-2]: Sulfuryl chloride (600 µL) was dissolved in n-hexane (20 mL) in an argon atmosphere and cooled to - 45°C.

"Solution 19-1" was added dropwise to "solution 19-2" at -45°C, and the reaction solution was warmed to 0°C and stirred for one hour at 0°C. The reaction solution was poured into water and extracted once by ethyl acetate. The organic layer was dried by anhydrous magnesium sulfate, and the solvent was distilled off under reduced pressure. The title compound was obtained as a crude product (1.0238 g). This crude product was used in the next reaction without any particular purification.

### [Reference Example 20]

### 2-Fluoropyridine-3-sulfonyl chloride

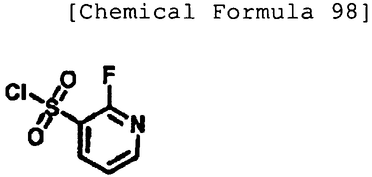

"Solution 20-1": 2-Fluoropyridine-3-amine (1.1132 g; made by Matrix) was dissolved in acetic acid (1 mL; made by Aldrich), and concentrated hydrochloric acid (2.5 mL; made by Kanto Chemical Co., Inc.) was added. After cooling to 0°C, sodium nitrite aqueous solution [2.5 mL; solution prepared by dissolving sodium nitrite (786.4 mg; made by Wako Pure Chemical Industries Co., Ltd.) in water (2.5 mL)] was added and stirred for 20 minutes at 0°C.
"Solution 20-2": Acetic acid (10 mL; made by Aldrich) was cooled to 0°C. After bubbling for 10 minutes with SO₂ gas, copper(II) chloride dihydrate (183.5 mg; made by Wako Pure Chemical Industries Co., Ltd.) was added.

"Solution 20-1" was added to "solution 20-2" at 0°C and stirred for three hours at 0°C. CH₂Cl₂ was added to the reaction solution, and it was washed once with water. The organic layer was dried using anhydrous sodium sulfate solution, and the solvent was then distilled off under reduced pressure. A crude product of the title compound (270.6 mg) was obtained. This crude product was used in the next reaction without any particular purification.

### [Reference Example 21]

### 3-(Benzyloxy)benzene-1-sulfonyl chloride

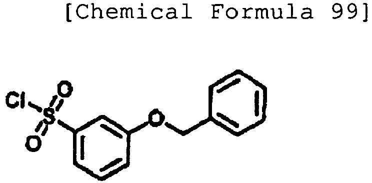

Magnesium (172.4 mg; made by Wako Pure Chemical Industries Co., Ltd.), dehydrated THF (5 mL), dibromoethane (20 µL; made by Tokyo Chemical Industry Co., Ltd.), and 1-(benzyloxy)-3-bromobenzene-THF solution [15 mL; solution prepared by dissolving 1-(benzyloxy)-3-bromobenzene (1.3146 g; made by Tokyo Chemical Industry Co., Ltd.) in dehydrated THF (15 mL)] were added and stirred for one hour by reflux in a nitrogen atmosphere. The reaction solution was cooled to - 78°C and bubbled for 20 minutes by SO₂ gas. The reaction solution was brought to room temperature, bubbled for 10 minutes with nitrogen gas, and filtered. The filtrate was concentrated, and the residue obtained was dissolved in CH₂Cl₂ (25 mL), then cooled to 0°C. SO₂Cl₂ (0.6 mL; made by Wako Pure Chemical Industries Co., Ltd.) was added to this solution and stirred overnight while warming to room temperature. Water was added to the reaction solution. After filtering by Celite, the filtrate was washed with brine. The organic layer was dried by anhydrous magnesium sulfate, and the solvent was distilled off under reduced pressure. The title compound was obtained as a crude product (1.3811 g). This crude product was used in the next reaction without any particular purification.

### [Reference Example 22]

### Methyl 4-(benzyloxy)-2-fluorobenzoate

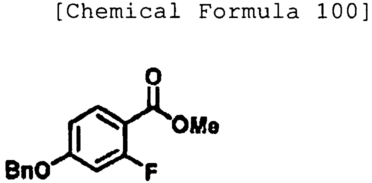

Methyl 2-fluoro-4-hydroxybenzoate (1.4685 g; made by Chanzou Fine Chem. Co.) and potassium carbonate (3.6917 g; made by Aldrich) were suspended in dehydrated DMF (21 mL; made by Kanto Chemical Co., Inc.). Benzyl bromide (1.22 mL; made by Wako Pure Chemical Industries Co., Ltd.) was added and stirred overnight at 50°C. The reaction solution was cooled to room temperature, and then poured into water and extracted twice using ethyl acetate. The organic layer was washed twice with water and once with brine. After drying had been performed using anhydrous sodium sulfate, the solvent was distilled off under reduced pressure. The residue was purified by column chromatography ("Column B;" n-hexane: ethyl acetate = 97:3→77:23), and the title compound (2.2207 g) was obtained.
¹H-NMR (300MHz, CDCl₃) ; *δ* (ppm) 3. 89 (3H, s), 5. 0 9 (2H, s), 6. 7 0 (1H, dd, J=2.3, 1 2. 6), 6.78 (1H, dd, J=2.3, 8.8), 7.31-7.41 (5H, m), 7. 89 (1H, t, J=8. 6)

### [Reference Example 23]

### 6-(Benzyloxy)-1,2-dihydroindazol-3-one

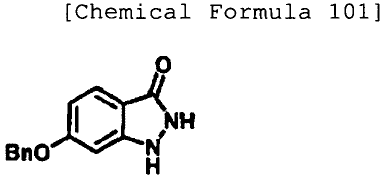

4-(Benzyloxy)-2-fluorobenzoate (52.4 mg), which can be manufactured by the method described in Reference Example 22 and the like, was dissolved in n-butanol (1 mL; made by Kanto Chemical Co., Inc.). Hydrazine monohydrate (96 µL; made by Aldrich) was added and stirred for one hour at 160°C in a sealed microwave reactor. After filtering the precipitate of the reaction solution, washing was performed using n-butanol, and the title compound (39.6 mg) was obtained.
¹H-NMR (300MHz, DMSO-d₆) ; δ (ppm) 5.13 (2 H, s), 6. 6 6 (1H, d d, J=2.0, 8.6), 6.74 (1H , d, J=2.0), 7.30-7.48 (6 H, m)
LCMS: 241 [M + H]; Retention time: 3.18 min; LCMS conditions: A

### [Reference Example 24]

### tert-Butyl 6-(benzyloxy)-3-oxo-2,3-dihydroindazole-1-carboxylate

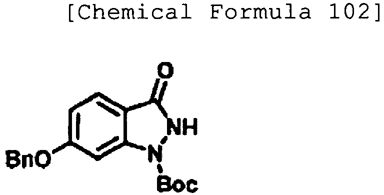

6-(Benzyloxy)-1,2-dihydroindazol-3-one (1.9209 g), which can be manufactured by the method described in Reference Example 23 and the like, was suspended in dichloromethane (80 mL; made by Wako Pure Chemical Industries Co., Ltd.). Triethylamine (2.78 mL; made by Kokusan Chemical Co., Ltd.), BoC₂O (4.6 mL: made by Wako Pure Chemical Industries Co., Ltd.), and DMAP (0.4947 g; made by Wako Pure Chemical Industries Co., Ltd.) were added. Nitrogen purging was carried out, and [the contents] were stirred overnight at room temperature. The reaction solution was washed twice with 1 mol/L hydrochloric acid and once with water. The organic layer was dried using magnesium sulfate, and the solvent was distilled off under reduced pressure. The residue was dissolved in methanol (64 mL; made by Wako Pure Chemical Industries Co., Ltd.), and 7 mol/L ammonia-methanol solution (16 mL; made by Aldrich) was added and stirred for four hours at room temperature. After the reaction solution had been concentrated under reduced pressure, ethanol was added to the residue. The precipitate was filtered, and the title compound (1.5822 g) was obtained. After also concentrating the filtrate under reduced pressure, ethanol was added to the residue. The precipitate was again filtered out, and the title compound (0.3956 g) was obtained.
¹H-NMR (300MHz, CDCl₃) ; δ (ppm) 1. 70 (9H, s), 5. 1 5 (2 H, s), 6. 96 (1H, dd, J=2.0, 8.6 ), 7.32-7.60 (6H, m), 7. 6 8 (1H, d, J=8. 6)
LCMS: 341 [M + H]; Retention time: 4.57 min; LCMS conditions: A

### [Reference Example 25]

### tert-Butyl 6-(benzyloxy)-3-methoxyindazole-1-carboxylate

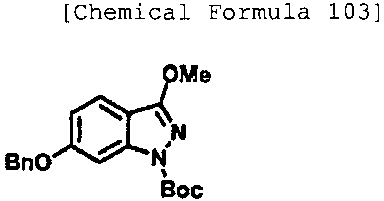

After suspending tert-butyl 6-(benzyloxy)-3-oxo-2,3-dihydroindazole-1-carboxylate (207.1 mg), which can be manufactured by the method described in Reference Example 24 and the like, and Ag₂CO₃ (509.1 mg; made by Kanto Chemical Co., Inc.) in dehydrated toluene (6 mL), methyl iodide (373 µL; made by Tokyo Chemical Industry Co., Ltd.) was added and stirred for two hours at 60°C in a sealed microwave reactor. After the reaction solution had been cooled to room temperature, the insoluble matter was filtered out. The filtrate was concentrated under reduced pressure, and the residue obtained was purified by column chromatography ("Column B;" n-hexane: ethyl acetate = 99:1→78:22), and the title compound (153.5 mg) was obtained.
¹H-NMR (300MHz, CDCl₃) ; δ (p pm) 1.68 (9H. s), 4. 1 4 (3H, s) . 5. 1 4 (2H, s), 6.94 (1H, d d, J = 2.2, 8. 6), 7.31-7.46 (5H, m), 7.51 ( 1 H, d. J=8.6), 7.60 (1H, brs)
LCMS: 355 [M + H]; Retention time: 5.79 min; LCMS conditions: A

### [Reference Example 26]

### tert-Butyl 6-hydroxy-3-methoxyindazole-1-carboxylate

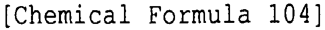

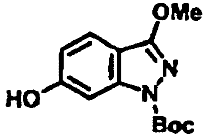

After suspending tert-butyl 6-(benzyloxy)-3-methoxyindazole-1-carboxylate (206 mg), which can be manufactured by the method described in Reference Example 25 and the like, and 5% palladium carbon (STD-type, containing 50% water) (113 mg/ made by N. E. Chemcat Corporation) in THF (5.8 mL), the interior of the reaction system was purged using hydrogen to create a hydrogen atmosphere, and [the contents] were stirred overnight at room temperature. After the interior of the reaction system had been purged using nitrogen, [the contents] were filtered. The filtrate was concentrated under reduced pressure, and the title compound (163.5 mg) was obtained.
¹H-NMR (300MHz, CDCl₃); δ (ppm) 1. 64 (9H. s), 4. 1 0 (3 H, s), 6. 6 0 (1 H, brs), 6. 8 3 (1H , dd, J=1.8, 8.4), 7.43 (1H, brs), 7. 48 (1 H, d, J=8.4)
LCMS: 265 [M + H]; Retention time: 3.74 min; LCMS conditions: A

### [Reference Example 27]

### 3-Methylindazol-6-ol

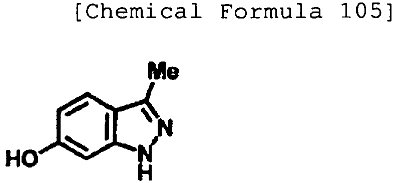

After suspending 1-benzyl-3-methylindazol-6-ol (16.7410 g) and 10% palladium carbon (PE-type, containing 50% water) (5.1164 g; made by N. E. Chemcat Corporation) in ethanol (166 mL), concentrated hydrochloric acid (5.83 mL; made by Kanto Chemical Co., Inc.) was added. The interior of the reaction system was purged using hydrogen to create a hydrogen atmosphere, and [the contents] were stirred for 10 hours at 60°C. After the reaction solution had been cooled to room temperature, the interior of the reaction system was purged by nitrogen. 10% palladium carbon (PE-type, containing 50% water) (1.5059 g; made by N. E. Chemcat Corporation) was added. The interior of the reaction system was purged using hydrogen to create a hydrogen atmosphere, and [the contents] were stirred for five hours at 60°C. The reaction solution was cooled to room temperature. After the interior of the reaction system had been purged using nitrogen, [the contents] were filtered. The filtrate was placed under reduced pressure, and the solvent was distilled off. Ethyl acetate was added to the residue obtained, washing was performed once with saturated sodium bicarbonate aqueous solution. The organic layer was washed once with saturated sodium bicarbonate and dried using sodium sulfate. The organic layer was placed under reduced pressure, and the solvent was distilled off. The title compound was obtained as a crude product (10.816 g).
¹H-NMR (300MHz. DMSO-d₆); *δ* (ppm) 2.38 (3 H, s), 6.58 (1H, dd, J=2.2, 8.4), 6.67 (1H d, J=2.2), 7.44 (1H, d, J=8.4), 9.47 (1H brs), 12.09 (1H, brs)
LCMS: 149 [M + H]; Retention time: 7.48 min; LCMS conditions: A

### [Reference Example 28]

### tert-Butyl 6-hydroxy-3-methylindazole-1-carboxylate

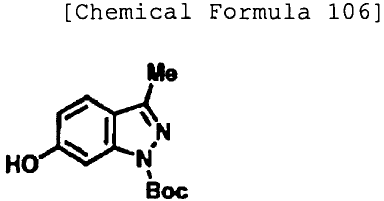

3-Methylindazol-6-ol (10.72 g), which can be manufactured by the method described in Reference Example 27 and the like, and imidazole (9.5492 g; made by Tokyo Chemical Industry Co., Ltd.) were dissolved in dehydrated DMF (140 mL). TBDPSCI (38.5301 g; made by Wako Pure Chemical Industries Co., Ltd.) was added and stirred overnight at room temperature. The reaction solution was poured into water and extracted twice using ethyl acetate. The organic layer was washed twice with water and once with brine. After drying using sodium sulfate had been performed, the solvent was distilled off under reduced pressure. The residue (41.3621 g) obtained was dissolved in CH₂Cl₂ (350 mL). Triethylamine (8.5155 g; made by Kokusan Chemical Co., Ltd.), Boc₂O (18.3611 g; made by Wako Pure Chemical Industries Co., Ltd.), and 4-N,N-dimethylaminopyridine (846.7 mg) were added and stirred overnight at room temperature. The reaction solution was washed twice with 1 mol/L hydrochloric acid water and once with brine. After drying had been performed using anhydrous sodium sulfate, the solvent was distilled off under reduced pressure. The residue (52.566 g) obtained was dissolved in dehydrated THF (350 mL). 1 mol/L TBAF-THF solution (140 mL; made by Tokyo Chemical Industry Co., Ltd.) was added and [the contents] were stirred for one hour at room temperature. Ethyl acetate was added to the reaction solution, and it was washed once with brine, once with water, and once with brine. After the organic layer had been dried using magnesium sulfate, the solvent was distilled off under reduced pressure. The resulting residue was purified by column chromatography ("Column A;" n-hexane: ethyl acetate = 74:26→47:53), and the title compound (10.934 g) was obtained.
¹H-NMR (300MHz, CDCl₃); *δ* (ppm) 1.66 (9H, s), 2.52 (3H, s), 6.42 (1H, brs), 6.88 (1H dd. J=2.2, 8.4), 7.48 (1H, d, J=8.4), 7. 57 (1H, s)
LCMS: 249 [M + H]; Retention time: 1.29 min; LCMS conditions: C

### [Reference Example 29]

### tert-Butyl 6-(benzyloxy)-3-difluoromethoxyindazaole-1-carboxylate

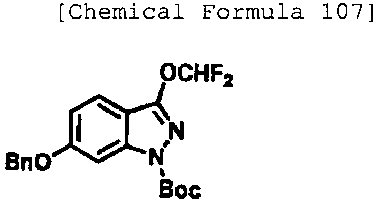

tert-Butyl 6-(benzyloxy)-3-oxo-2,3-dihydroindazole-1-carboxylate (342 mg), which can be manufactured by the method described in Reference Example 24 and the like, and potassium carbonate (2.0887 g; made by Aldrich) were suspended in dehydrated DMF (10 mL; made by Kanto Chemical Co., Inc.). Sodium chlorodifluoroacetate (853 mg; made by Tokyo Chemical Industry Co., Ltd.) was added and stirred for 12 hours at 80°C. The reaction solution was poured into water and extracted twice using ethyl acetate. The organic layer was washed twice with water and once with brine. After drying using sodium sulfate had been performed, the solvent was distilled off under reduced pressure. The residue was purified by column chromatography ("Column A;" n-hexane: ethyl acetate = 100:0→87:13), and the title compound (264.7 mg) was obtained.
¹H-NMR (CDCl₃); *δ* (ppm) 1.68 (9H. s), 5.15 (2H, s), 7.02 (1H, dd, J=2.2, 8.8), 7.32-7.60 (5H, m), 7.36 (1H, t, J=72. 0), 7.56 ( 1H, d, J=8.8), 7.63 (1H, brs)
LCMS: 391 [M + H]; Retention time: 5.97 min; LCMS conditions: A

### [Reference Example 30]

### tert-Butyl 3-difluoromethoxy-6-hydroxyindazole-1-carboxylate

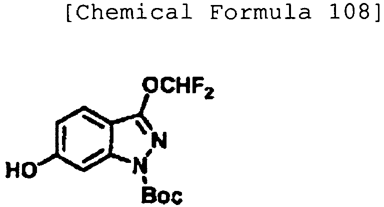

tert-Butyl 6-(benzyloxy)-3-difluoromethoxyindazole-1-carboxylate (262.5 mg), which can be manufactured by the method described in Reference Example 29 and the like, and 5% palladium carbon (STD-type, containing 50% water) (109.9 mg; made by N. E. Chemcat Corporation) were suspended in dehydrated THF (3.4 mL; made by Kanto Chemical Co., Inc.), purged using hydrogen, and stirred overnight at room temperature. The reaction solution was purged by nitrogen and filtered. The filtrate was concentrated under reduced pressure, and the title compound (197.2 mg) was obtained.
¹H-NMR (CDCl₃); *δ* (ppm) 1.68 (9H, s), 6.08 (1H, brs), 6.89 (1H, dd, J=2.2, 8.6), 7.3 4 (1H, t, J=72.0), 7.48 (1H, brs), 7.54 (1 H, d, J=8.6)
LCMS: 301 [M + H]; Retention time: 4.04 min; LCMS conditions: A

### [Reference Example 31]

### 4-(tert-Butyldimethylsilyloxy)-2-fluorobenzonitrile

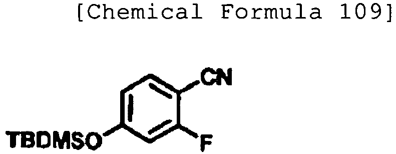

2-Fluoro-4-hydroxybenzonitrile (30.1 g; made by Wako Pure Chemical Industries Co., Ltd.) and imidazole (18.3 g; made by Tokyo Chemical Industry Co., Ltd.) were dissolved in dehydrated DMF (436 mg; made by Kanto Chemical Co., Inc.). After cooling to 0°C, TBDMSCI (48.3 g; made by Tokyo Chemical Industry Co., Ltd.) was added and [the contents] were stirred for one hour while being warmed to room temperature. After distilling off the solvent under reduced pressure, water was added to the reaction solution, and extraction was performed twice using ethyl acetate. The organic layer was washed twice with water and with brine. After drying had been performed using anhydrous sodium sulfate, the solvent was distilled off under reduced pressure, followed by purification by column chromatography ("Column A;" n-hexane: ethyl acetate = 100:0→94:6), and the title compound (40.3 g) was obtained. ¹H-NMR (300MHz, CDCl₃); *δ* (ppm) 0.25 (3H, s), 0.25 (3H, s), 0.98 (9H, s), 6.62-6.70 (2H, m), 7.44-7.50 (1H, m)

### [Reference Example 32]

### 1-(2-Fluoro-4-hydroxyphenyl)propan-1-one

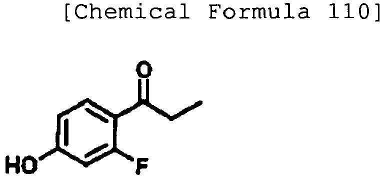

4-(tert-Butyldimethylsilyloxy)-2-fluorobenzonitrile (10.06 g), which can be manufactured by the method described in Reference Example 31 and the like, was dissolved in dehydrated diethyl ether (100 mL; made by Kanto Chemical Co., Inc.) in an argon atmosphere, and 3 mol/L ethyl magnesium bromide-diethyl ether solution (35 mL; made by Kanto Chemical Co., Inc.) was added dropwise. After dropwise addition had been completed, the reaction solution was stirred for 20 minutes at room temperature and for 1.5 hours under reflux. The reaction solution was cooled to 0°C, and water (35.98 mL) and 5 mol/L hydrochloric acid (35.98 mL) were added. After stirring overnight under reflux, the reaction solution was cooled to room temperature and extracted three times using ethyl acetate. The organic layer was washed with water and brine. After drying had been performed using anhydrous sodium sulfate, the solvent was distilled off under reduced pressure. The residue was dissolved in dehydrated THF (100 mL; made by Kanto Chemical Co., Inc.). 1 mol/L TBAF-THF solution (31.5 mL; made by Tokyo Chemical Industry Co., Ltd.) was added and stirred for 20 minutes at room temperature. Water and brine were added to the reaction solution, and extraction was performed three times using ethyl acetate. The organic layer was washed with water and brine. After drying had been performed using anhydrous sodium sulfate, the solvent was distilled off under reduced pressure. The residue was dissolved in diethyl ether and extracted by 2 mol/L sodium hydroxide aqueous solution. The water layer was washed with diethyl ether. 2 mol/L hydrochloric acid was added to the water layer, and extraction was performed twice using ethyl acetate. The organic layer was washed with water and brine. After drying had been performed using anhydrous sodium sulfate, the solvent was distilled off under reduced pressure, and the title compound (5.63 g) was obtained.
¹H-NMR (300MHz, CDCl₃); *δ* (ppm) 1.14 (3H, t, J=7.3), 2.86-2.95 (2H, m), 6.55 (1H, d d, J=2.2, 13.2), 6.67 (1H, dd, J=2.2, 8.8 ), 7.71-7.77 (1H, m), 10.22 (1H, brs)
LCMS: 167 [M - H]; Retention time: 3.31 min; LCMS conditions: B

### [Reference Example 33]

### 1-Benzyl-3-ethylindazol-6-ol

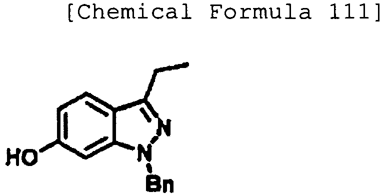

1-(2-fluoro-4-hydroxyphenyl)propan-1-one (5.37 g), which can be manufactured by the method described in Reference Example 32 and the like, sodium acetate (12.75 g; made by Wako Pure Chemical Industries Co., Ltd.), and benzyl hydrazine dihydrochloride (9.452 g; made by Aldrich) were suspended in xylene (76 mL) and stirred overnight under reflux using a Dean-Stark apparatus. After cooling to room temperature, water was added to the reaction solution, and extraction was performed twice using ethyl acetate. The organic layer was washed twice with water and with brine. After drying had been performed using anhydrous sodium sulfate, the solvent was distilled off under reduced pressure. The precipitate was filtered out before the solvent had been completely removed. The solid was washed with n-hexane, and the title compound (4.76 g) was obtained. The filtrate was further concentrated under reduced pressure, and the solid that precipitated during concentration was also filtered out, and the title compound (3.44 g) was obtained.
¹H-NMR (300MHz, DMSO-d₆); *δ* (ppm) 1.28 (3 H, t, J=7.7) 2.84 (2H, q, J=7.7), 5.42 (2 H, s), 6.62 (1H, dd, J=1.8, 8.4), 6.72 (1H d, J=1.8) 7.14-7.33 (5H, m), 7.51 (1H, d J=8.8), 9.58 (1H, brs)
LCMS: 253 [M + H]; Retention time: 3.78 min; LCMS conditions: A

### [Reference Example 34]

### 3-Ethylindazol-6-ol

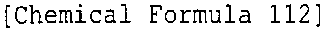

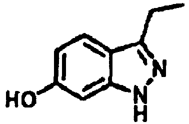

1-Benzyl-3-ethylindazol-6-ol (4.780 g), which can be manufactured by the method described in Reference Example 33 and the like, and 10% palladium carbon (PE-type, containing 50% water) (1.953 g; made by N. E. Chemcat Corporation) were suspended in ethanol (189 mL; made by Wako Pure Chemical Industries Co., Ltd.). Concentrated hydrochloric acid (1.579 mL; made by Kanto Chemical Co., Inc.) was added, and the interior of the reaction system was purged using hydrogen to create a hydrogen atmosphere and stirred for 1.2 hours at 60°C. After the reaction solution had been cooled to room temperature, it was purged by nitrogen and filtered. The filtrate was concentrated under reduced pressure, and a hydrochloride of the title compound (3.918 g) was obtained.
¹H-NMR (300MHz. DMSO-d₆); 1.31 (3H, t, J= 7.7), 2.95 (2H, q, J=7.7), 6.68-6.78 (2H m), 7.59-7.67 (1H, m)
LCMS: 163 [M + H]; Retention time: 2.76 min; LCMS conditions: A

### [Reference Example 35]

### 6-(tert-Butyldiphenylsilyloxy)-3-ethylindazole

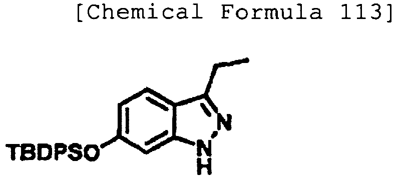

3-Ethylindazol-6-ol hydrochloride (3.76 g), which can be manufactured by the method described in Reference Example 34 and the like, and imidazole (4.510 g; made by Kanto Chemical Co., Inc.) were dissolved in dehydrated DMF (122 mL; made by Kanto Chemical Co., Inc.) and cooled to 0°C. TBDPSCI (17.01 mL; made by Tokyo Chemical Industry Co., Ltd.) was added. After being stirred overnight while warmed to room temperature, [the contents] were stirred for one hour at 30°C. More imidazole (1.289 g; made by Tokyo Chemical Industry Co., Ltd.) and TBDPSCI (4.862 mL; made by Tokyo Chemical Industry Co., Ltd.) were added and stirred for 2.5 hours at 30°C. Water was added to the reaction solution, and extraction was performed twice using ethyl acetate. The organic layer was washed twice with water and once with brine. After the organic layer was dried using anhydrous sodium sulfate, the solvent was distilled off under reduced pressure. The residue was purified by column chromatography ("Column A;" n-hexane: ethyl acetate = 88:12→67:33), and the title compound (5.9788 g) was obtained.
¹H-NMR (300MHz, CDCl₃); *δ* (ppm) 1.11 (9H, s), 1.35 (3H, t, J=7.7), 2. 90 (2H, q, J=7. 7), 6.61 (1H, d, J=1.5), 6.74 (1H, dd, J=2 . 2, 8.8) 7.33-7.45 (7H, m), 7.72-7.76 (4 H, m)
LCMS: 401 [M + H]; Retention time: 6.23 min; LCMS conditions: B

### [Reference Example 36]

### tert-Butyl 6-(tert-butyldiphenylsilyloxy)-3-ethylindazole-1-carboxylate

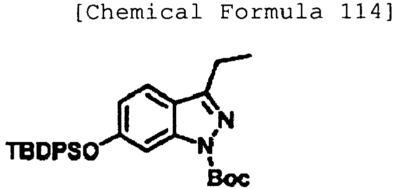

6-(tert-Butyldiphenylsilyloxy)-3-ethylindazole (5.98 g), which can be manufactured by the method described in Reference Example 35 and the like, was dissolved in dehydrated THF (150 mL; made by Kanto Chemical Co., Inc.). Triethylamine (2.50 mL; made by Kokusan Chemical Co., Ltd.), DMAP (1.01 g; made by Wako Pure Chemical Industries Co., Ltd.), and Boc₂O (4.11 mL; made by Peptide Institute, Inc.) were added and stirred overnight at room temperature. After the reaction solution had been concentrated under reduced pressure, ethyl acetate was added to the residue, and washing was performed twice using 1 mol/L hydrochloric acid. After the organic layer was dried using anhydrous sodium sulfate, the solvent was distilled off under reduced pressure, and the title compound (8.02 g) was obtained.
LCMS: 501 [M + H]; Retention time: 7.48 min; LCMS conditions: B

### [Reference Example 37]

### tert-Butyl 6-hydroxy-3-ethylindazole-1-carboxylate

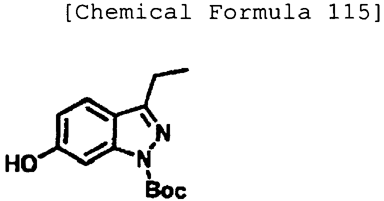

tert-Butyl 6-(tert-butyldiphenylsilyloxy)-3-ethylindazole-1-carboxylate (8.02 g), which can be manufactured by the method described in Reference Example 36 and the like, was dissolved in dehydrated THF (53 mL). 1 mol/L TBAF-THF solution (31.5 mL; made by Tokyo Chemical Industry Co., Ltd.) was added and stirred for 0.5 hour at room temperature. Water and brine were added to the reaction solution, and extraction was performed three times using ethyl acetate. The organic layer was washed with water and brine. After drying had been performed using anhydrous sodium sulfate, the solvent was distilled off under reduced pressure. The residue was purified by column chromatography ("Column B;" n-hexane: ethyl acetate = 95:5→74:26), and the title compound (3.269 g) was obtained.
¹H-NMR (300MHz. CDCl₃) ; *δ* (ppm) 1.37 (3H, t, J=7.7), 1.63 (9H, s), 2.94 (2H, q, J=7. 7), 6.89 (1H, dd, J=2.2, 8.4), 6.91-6.93 (1H, m) 7.47-7.61 (2H, m)
LCMS: 263 [M + H]; Retention time: 3.74 min; LCMS conditions: B

### [Reference Example 38]

### Indazol-6-ol

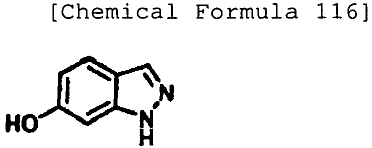

Indazol-6-amine (24.33 g; made by Tokyo Chemical Industry Co., Ltd.) was dissolved in water (100 mL) and 48 wt% tetrafluoroboric acid aqueous solution (242 mL; made by Aldrich). After cooling to 0°C, a sodium nitrite aqueous solution [20 mL; solution prepared by dissolving sodium nitrite (13.87 g; made by Kanto Chemical Co., Inc.) in water (20 mL)] was added dropwise over 10 minutes and [the contents] were stirred for 30 minutes at 0°C. The precipitate of the reaction solution was filtered out and washed with chloroform. The precipitate obtained was dissolved in acetic acid (250 mL) and stirred for 10 minutes at 50°C, 10 minutes at 110°C, and 10 minutes at 130°C. The reaction solution was cooled, saturated sodium carbonate aqueous solution was added, and [the reaction solution] was extracted using ethyl acetate. The organic layer was washed with brine. After drying had been performed using anhydrous magnesium sulfate, the solvent was distilled off under reduced pressure. The resulting residue was dissolved in ethanol (240 mL). 2 mol/L sodium hydroxide aqueous solution (365 mL) was added and stirred for one hour at room temperature. The reaction solution was concentrated under reduced pressure. After adding 2 mol/L hydrochloric acid (200 mL), water, and saturated ammonium chloride aqueous solution to the residue to bring the pH to approximately 7, extraction was performed using ethyl acetate. The organic layer was washed with brine. After drying had been performed using anhydrous magnesium sulfate, the solvent was distilled off under reduced pressure. Chloroform was added to the residue, and the insoluble matter was filtered out. After washing with chloroform, a crude product of the title compound (13.5401 g) was obtained.
¹H-NMR (DMSO-d₆): *δ* (ppm) 6.64 (1H. dd, J= 1.8, 8.8), 6.78 (1H, d d, J=0.7, 1.8), 7.5 2 (1H, d, J=8.8), 7.86 (1H, d, J=0.7), 9.5 4 (1H,s), 12.56 (1H, s)
LCMS: 134 [M + H]; Retention time: 0.72 min; LCMS conditions: C

### [Reference Example 39]

### 6-tert-Butyldiphenylsilyloxyindazole

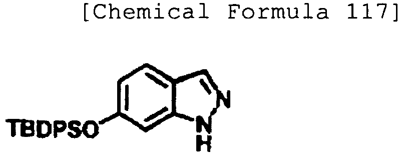

Indazol-6-ol (4.029 g), which can be manufactured by the method described in Reference Example 38 and the like, was dissolved in dehydrated DMF (60 mL; made by Kanto Chemical Co., Inc.). Imidazole (4.49 g; made by Tokyo Chemical Industry Co., Ltd.) and TBDPSCI (17.1 mL; made by Tokyo Chemical Industry Co., Ltd.) were added and [the contents] were stirred overnight at room temperature. The reaction solution was poured into water and extracted three times using ethyl acetate. The organic layer was washed three times with water. After drying had been performed using anhydrous magnesium sulfate, the solvent was distilled off under reduced pressure. The resulting residue was purified by column chromatography ("Column A;" n-hexane: ethyl acetate = 92:8→71:29), and the title compound (9.214 g) was obtained.
¹H-NMR (CDCl₃) ; *δ*(ppm) 1.11 (9H, s), 6.66 -6.67 (1H,m), 6.78 (1H,dd, J=2.0, 8.8), 7.33-7.45 (6H, m), 7.48 (1H, dd, J=0.5, 8 .8), 7.71-7.74 (4H, m), 7.88 (1H, s)
LCMS: 373 [M + H]; Retention time: 5.88 min; LCMS conditions: A

### [Reference Example 40]

### 6-(tert-Butyldiphenylsilyloxy)-3-chloroindazole

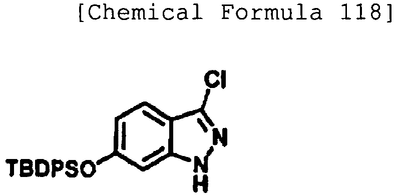

6-tert-Butyldiphenylsilyloxyindazole (29.246 g), which can be manufactured by the method described in Reference Example 39 and the like, was dissolved in dehydrated THF (200 mL) in a nitrogen atmosphere. After cooling to 0°C, potassium tert-butoxide (18.2190 g; made by Kanto Chemical Co., Inc.) and N-chlorosuccinimide (17.0497 g; made by Kanto Chemical Co., Inc.) were added and [the contents] were stirred for four hours while warming from 0°C to room temperature. The reaction solution was poured into saturated ammonium chloride aqueous solution and extracted twice using ethyl acetate. After washing once with brine, the organic layer was dried using magnesium sulfate, and the solvent was distilled off under reduced pressure. The resulting residue was purified by column chromatography ("Column A;" n-hexane: ethyl acetate = 88:12→67:33), and the title compound (18.592 g) was obtained.
¹H-NMR (CDCl₃); *δ* (ppm) 1.11 (9H, s), 6.60 (1H, d, J=2.1), 6.83 (1H, dd, J=2.1, 8.7) 7.33-7.46 (6H, m), 7.69-7.74 (5H, m), 9 .53 (1H, brs)
LCMS: 407 [M + H]; Retention time: 2.44 min; LCMS conditions: C

### [Reference Example 41]

### tert-Butyl 6-(tert-butyldiphenylsilyloxy)-3-chloroindazole-1-carboxylate

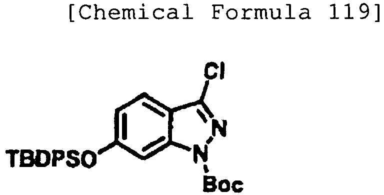

6-(tert-Butyldiphenylsilyloxy)-3-chloroindazole (18.458 g), which can be manufactured by the method described in Reference Example 40 and the like, was dissolved in dehydrated THF (200 mL). Triethylamine (7.67 mL; made by Wako Pure Chemical Industries Co., Ltd.), Boc₂O (made by Wako Pure Chemical Industries Co., Ltd.), and 4-N,N-dimethylaminopyridine (550 mg; made by Wako Pure Chemical Industries Co., Ltd.) were added and [the contents] were stirred overnight at room temperature. Ethyl acetate was added to the reaction solution. The organic layer was washed twice with 1 mol/L hydrochloric acid and once with brine, then dried using magnesium sulfate and the solvent distilled off under reduced pressure. The resulting residue was purified by column chromatography ("Column A;" n-hexane: ethyl acetate = 97:3→80:20), and the title compound (17.513 g) was obtained.
¹H-NMR (CDCl₃); *δ* (ppm) 1.11 (9H, s), 1.71 (9H, s), 6.82 (1H, d, J=8.7), 7.34-7.43 ( 6H, m), 7.69-7.72 (6H, m)

### [Reference Example 42]

### tert-Butyl 3-chloro-6-hydroxyindazole-1-carboxylate

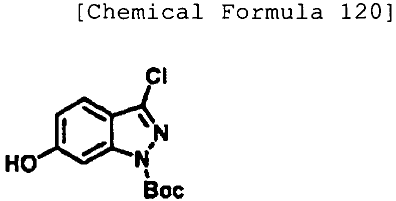

tert-Butyl 6-(tert-butyldiphenylsilyloxy)-3-chloroindazole-1-carboxylate (17.415 g), which can be manufactured by the method described in Reference Example 41 and the like, was dissolved in dehydrated THF (150 mL). 1 mol/L TBAF-THF solution (42 mL; made by Tokyo Chemical Industry Co., Ltd.) was added and [the contents] were stirred overnight at room temperature. Ethyl acetate was added to the reaction solution. The organic layer was washed once with brine, once with water, and once with brine. After drying had been performed using anhydrous sodium sulfate, the solvent was distilled off under reduced pressure. n-Hexane (150 mL) was added to the residue obtained. After ultrasonicating the suspension, the precipitate was filtered out, and the title compound (6.3815 g) was obtained.
¹H-NMR (CDCl₃) ; *δ* (ppm) 1.68 (9H, s), 6.03 (1H, s), 6.95 (1H, dd, J=2.1, 8.7), 7.53 ( 1H, d, J=8.7), 7.60 (1H, d, J=2.1)
LCMS: 269 [M + H]; Retention time: 1.60 min; LCMS conditions: C

### [Reference Example 43]

### 1-(Bromomethyl)-4-methoxy-2-nitrobenzene

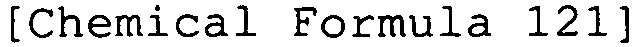

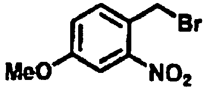

4-Methyl-3-nitroanisole (2.7 mL; made by Aldrich) was dissolved in carbon tetrachloride (20 mL; made by Wako Pure Chemical Industries Co., Ltd.). NBS (4.0520 g; made by Tokyo Chemical Industry Co., Ltd.) and hydrated benzoyl peroxide (348 mg; made by Aldrich) were added. Nitrogen purge was conducted, and [the contents] were stirred for three hours under reflux. After filtering the reaction solution, the filtrate was washed with a NaHSO₃ aqueous solution and water. After drying had been performed using anhydrous sodium sulfate, the solvent was distilled off under reduced pressure. The residue was purified by column chromatography ("Column B;" n-hexane: ethyl acetate = 94:6→73:27), and the title compound (3.2549 g) was obtained.
¹H-NMR (300MHz, CDCl₃); *δ* (ppm) 3.88 (3H, s), 4.80 (2H, s), 7.13 (1H, dd, J=2.5, 8.9 ), 7.45 (1H, d, J=8.4), 7.55 (1H, d, J=2.5 )

### [Reference Example 44]

### 4-Methoxy-2-nitro-1-(2,2,2-trifluoroethyl)benzene

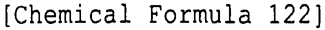

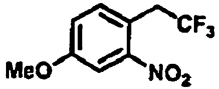

1-(Bromomethyl)-4-methoxy-2-nitrobenzene (2.7123 g), which can be manufactured by the method described in Reference Example 43 and the like, was dissolved in dehydrated DMF (22 mL; made by Kanto Chemical Co., Inc.). Copper iodide (524 mg; made by Wako Pure Chemical Industries Co., Ltd.) and methyl 2,2-difluoro-2-(fluorosulfonyl)acetate (3.06 mL; made by Tokyo Chemical Co.,) were added. Nitrogen purge was conducted, and [the contents] were stirred for four hours at 100°C. Ethyl acetate was added to the reaction solution. The organic layer was washed with ammonia water, water, and brine. After drying had been performed using anhydrous magnesium sulfate, the solvent was distilled off under reduced pressure. The residue was purified by column chromatography ("Column B;" n-hexane: ethyl acetate = 97:3→77:23), and the title compound (1.579 g) was obtained.
¹H-NMR (300MHz, CDCl₃) ; *δ* (ppm) 3. 83 (2H, q, J=10.2), 3.88 (3H, s), 7.14 (1H, dd, J= 2.5, 8.4), 7.35 (1H, d, J=8.4), 7.51 (1H, d, J=2.9)

### [Reference Example 45]

### 5-Methoxy-2-(2,2,2-trifluoroethyl)benzeneamine

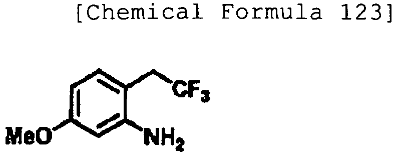

4-Methoxy-2-nitro-1-(2,2,2-trifluoroethyl)benzene (1.8153 g), which can be manufactured by the method described in Reference Example 44 and the like, and 5% palladium carbon (STD-type, containing 50% water) (926 mg; made by N. E. Chemcat Corporation) were suspended in methanol (30 mL; made by Kanto Chemical Co., Inc.). The interior of the reaction system was purged using hydrogen to create a hydrogen atmosphere, and [the contents] were stirred overnight at room temperature. After the reaction solution had been purged using nitrogen, 5% palladium carbon (STD-type, containing 50% water) (926 mg; made by N. E. Chemcat Corporation) was added. The interior of the reaction system was purged using hydrogen to create a hydrogen atmosphere, and [the contents] were stirred for nine hours at room temperature. After the reaction solution had been purged using nitrogen, filtration was performed and the filtrate was concentrated under reduced pressure. The residue was dissolved in chloroform. After drying had been performed using anhydrous magnesium sulfate, the solvent was distilled off under reduced pressure, and the title compound (1.4096 g) was obtained.
LCMS: 206 [M + H]; Retention time: 1.41 min; LCMS conditions: C

### [Reference Example 46]

### 1-(6-Methoxy-3-(trifluoromethyl)-indazol-1-yl)ethanone

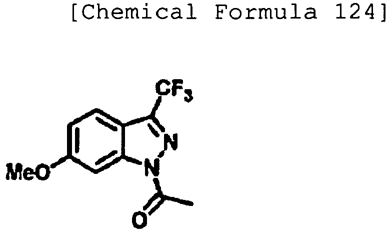

5-Methoxy-2-(2,2,2-trifluoroethyl)benzeneamine (1.4013 g), which can be manufactured by the method described in Reference Example 45 and the like, was dissolved in monochlorobenzene (23 mL; made by Kanto Chemical Co., Inc.). Potassium acetate (1.6884 g; made by Wako Pure Chemical Industries Co., Ltd.) and acetic anhydride (3.26 mL; made by Wako Pure Chemical Industries Co., Ltd.) were added and stirred for five minutes at 80°C. Isoamyl nitrite (2.75 mL; made by Tokyo Chemical Industry Co., Ltd.) was added to the reaction solution and stirred for 15 hours at 80°C. The reaction solution was cooled to room temperature. After standing for two days, isoamyl nitrite (1 mL; made by Tokyo Chemical Industry Co., Ltd.) was added and stirred for four hours at 80°C. The reaction solution was cooled to room temperature, and ethyl acetate was added. The organic layer was washed with saturated aqueous sodium bicarbonate and brine. After drying had been performed using anhydrous sodium sulfate, the solvent was distilled off under reduced pressure. The residue was purified by column chromatography ("Column B;" n-hexane: ethyl acetate = 100:0→80:20), and the title compound (1.6105 g) was obtained.
¹H-NMR (300 MHz. CDCl₃) ; *δ* (ppm) 2.81 (3H, s), 3.93 (3H, s), 7.06 (1H, dd, J=2.2, 8.7 ), 7.67 (1H, d, J=8.7), 7.90 (1H, d, J=2.2 )
LCMS: 259 [M + H]; Retention time: 1.90 min; LCMS conditions: C

### [Reference Example 47]

### 3-(Trifluoromethyl)-indazol-6-ol

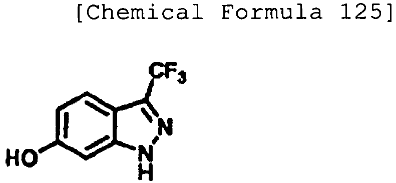

1-(6-Methoxy-3-trifluoromethylindazol-1-ol)ethanone (2.582 g), which can be manufactured by the method described in Reference Example 46 and the like, was added to hydrobromic acid (100 mL; made by Wako Pure Chemical Industries Co., Ltd.) and stirred overnight at 110°C. After cooling the reaction solution to 0°C, it was neutralized to approximately pH 7 by 5 mol/L sodium hydroxide aqueous solution and extracted once by ethyl acetate. The organic layer was washed with brine. After drying had been performed using anhydrous magnesium sulfate, the solvent was distilled off under reduced pressure, and the title compound (1.8583 g) was obtained.
¹H-NMR (300MHz, DMSO-d₆); *δ* (ppm) 6.85 (1 H, dd, J=1.8, 8.7), 6.87 (1H, d, J=1.8), 7 .57 (1H, d, J=8.7), 9.98 (1H, brs), 13.45 (1H, brs)
LCMS: 202 [M + H]; Retention time: 1.23 min; LCMS conditions: C

### [Reference Example 48]

### 6-(tert-Butyldiphenylsilyloxy)-3-(trifluoromethyl)-indazole

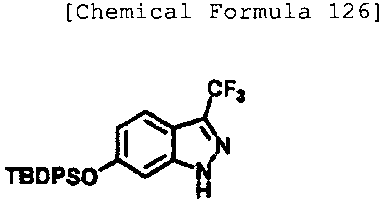

3-Trifluoromethylindazol-6-ol (1.818 g), which can be manufactured by the method described in Reference Example 47 and the like, and imidazole (1.363 g; made by Tokyo Chemical Industry Co., Ltd.) were dissolved in dehydrated DMF (22 mL, made by Kanto Chemical Co., Inc.). TBDPSCI (5.14 mL; made by Tokyo Chemical Industry Co., Ltd.) was added and stirred overnight at room temperature. Ethyl acetate was added to the reaction solution. The organic layer was washed twice with water and once with brine. After drying had been performed using anhydrous magnesium sulfate, the solvent was distilled off under reduced pressure. The residue was purified by column chromatography ("Column A;" n-hexane: ethyl acetate = 97:3→76:24). The low-purity fraction was again purified by column chromatography ("Column A;" n-hexane: ethyl acetate = 97:3→76:24) The low-purity fraction was again purified by column chromatography ("Column B;" n-hexane: ethyl acetate = 97:3→76:24), and the title compound (3.367 g) was obtained.
¹H-NMR (300MHz. DMSO-d₆) : *δ* (ppm) 6.75 (1 H, d, J=1.8), 6.93 (1H, dd, J=2.2, 8.7), 7 .37-7.53 (6H, m), 7.62 (1H, d, J=8.7), 7. 69-7.72 (4H, m) 13.49 (1H, brs)

### [Reference Example 49]

### tert-Butyl 6-(tert-butyldiphenylsilyloxy)-3-(trifluoromethyl)-indazole-1-carboxylate

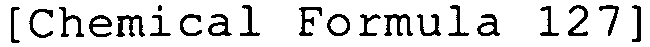

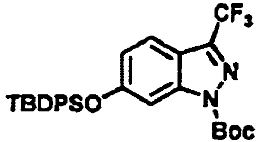

6-(tert-Butyldiphenylsilyloxy)-3-trifluoromethylindazole (3.3513 g), which can be manufactured by the method described in Reference Example 48 and the like, was dissolved in dehydrated THF (35 mL; made by Kanto Chemical Co., Inc.). Boc₂O (2.09 mL; made by Wako Pure Chemical Industries, Ltd., Ltd.), triethylamine (1.28 mL; made by Kokusan Chemical Co., Ltd.) and DMAP (93.1 mg; made by Wako Pure Chemical Industries Co., Ltd.) were added and stirred overnight at room temperature. Ethyl acetate was added to the reaction solution. The organic layer was washed twice with 1 mol/L hydrochloric acid, once with water, and once with brine. After drying had been performed using anhydrous magnesium sulfate, the solvent was distilled off under reduced pressure, and a crude product of the title compound (4.4733 g) was obtained.
¹H-NMR (300MHz, DMSO-d₆); *δ* (ppm) 1.06 (9 H, s), 1.36 (9H, s), 7.11 (1H, dd, J=2.2, 8 .7) 7.33 (1H, d, J=1.8), 7.38-7.53 (6H, m), 7.63-7.72 (4H,m), 7.75 (1H, d, J=8.7 )

### [Reference Example 50]

### tert-Butyl 6-hydroxy-3-(trifluoromethyl)-indazole-1-carboxylate

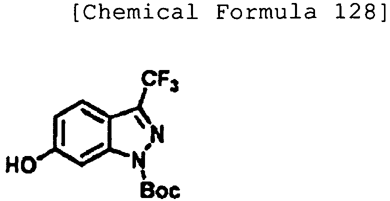

A crude product of tert-butyl 6-(tert-butyldiphenylsilyloxy)-3-trifluoromethylindazole-1-carboxylate (4.4773 g), which can be manufactured by the method described in Reference Example 49 and the like, was dissolved in dehydrated THF (40 mL; made by Kanto Chemical Co., Inc.). 1 mol/L TBAF-THF solution (12 mL; made by Tokyo Chemical Industry Co., Ltd.) was added and stirred for 20 minutes at room temperature. The reaction solution was poured into brine and extracted once with ethyl acetate. The organic layer was washed twice with water and once with brine. After drying had been performed using anhydrous magnesium sulfate, the solvent was distilled off under reduced pressure. The residue was purified by column chromatography ("Column B;" n-hexane: ethyl acetate = 86:14→65:35), and the title compound (1.6508 g) was obtained.
¹H-NMR (300MHz, CDCl₃) ; *δ* (ppm) 1.72 (9H, s), 5.39 (1H, s), 6.98 (1H, dd, J=2.1, 8.7 ), 7.62 (1H, d, J=2.1), 7.68 (1H, d, J=8.7 )
LCMS: 301 [M + H]; Retention time: 1.73 min; LCMS conditions: C

### [Reference Example 51]

### Cyclopropyl(2-fluoro-4-hydroxyphenyl)methanone

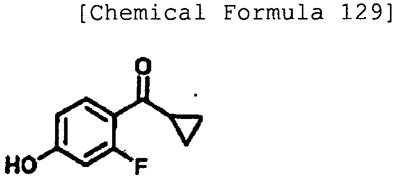

4-(tert-Butyldimethylsilyloxy)-2-fluorobenzonitrile (10.00 g), which can be manufactured by the method described in Reference Example 31 and the like, was dissolved in dehydrated THF (30 mL; made by Kanto Chemical Co., Inc.) in an argon atmosphere. After cooling to 0°C, 1 mol/L cyclopropyl magnesium bromide-THF solution (80 mL; made by Tokyo Chemical Industry Co., Ltd.) was added dropwise. After dropwise addition had been completed, the reaction solution was stirred for 10 minutes at 0°C and stirred for 1.5 hours under reflux. The reaction solution was cooled to 0°C. Water (50 mL) and 5 mol/L hydrochloric acid (50 mL) were added. After stirring overnight under reflux, the reaction solution was cooled to room temperature and extracted three times using ethyl acetate. The organic layer was washed with water and brine. After drying had been performed using anhydrous magnesium sulfate, the solvent was distilled off under reduced pressure. The residue was dissolved in dehydrated THF (100 mL; made by Kanto Chemical Co., Inc.). 1 mol/L TBAF-THF solution (31.5 mL; made by Tokyo Chemical Industry Co., Ltd.) was added and stirred for 20 minutes at room temperature. Water and brine were added to the reaction solution, and extraction was performed three times using ethyl acetate. The organic layer was washed with water and brine. After drying had been performed using anhydrous magnesium sulfate, the solvent was distilled off under reduced pressure. n-Hexane was added to the residue. After suspending the insoluble matter using ultrasound, the insoluble matter was filtered out, and a crude product of the title compound (6.5156 g) was obtained.
¹H-NMR (300MHz, DMSO-d₆); δ (ppm) 0.97-1 .04 (4H, m), 2.56-2.65 (1H, m), 6.61-6.7 2 (2H, m), 7.64-7.72 (1H, m)
LCMS: 179 [M - H]; Retention time: 3.20 min; LCMS conditions: B

### [Reference Example 52]

### 1-Benzyl-3-cyclopropylindazol-6-ol

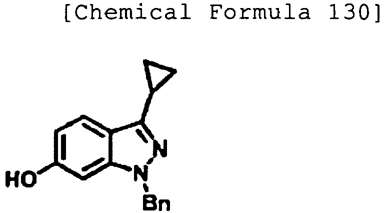

Cyclopropyl(2-fluoro-4-hydroxyphenyl)methanone (6.51 g), which can be manufactured by the method described in Reference Example 51 and the like, sodium acetate (14.3163 g; made by Wako Pure Chemical Industries Co., Ltd.), and benzyl hydrazine dihydrochloride (10.72 g; made by Aldrich) were suspended in xylene (180 mL) and stirred overnight under reflux using a Dean-Stark apparatus. After the reaction solution had been cooled to room temperature, water was added, and extraction was performed twice using ethyl acetate. The organic layer was washed twice with water and with brine. After drying had been performed using anhydrous sodium sulfate, the solvent was distilled off under reduced pressure. n-Hexane was added to the residue. The insoluble mater was filtered out, and a crude product of the title compound (10.97 g) was obtained.
¹H-NMR (300MHz, DMSO-d₆); *δ* (ppm) 0.87-0 .99 (4H, m), 2.14-2.26 (1H, m), 5.38 (2H, s), 6.61 (1H, dd, J=2.0, 8.8), 6.69 (1H, d J=1.8), 7.11-7.31 (5H, m), 7.53 (1H, d, J=8.8), 9.57 (1H, brs)
LCMS: 265 [M + H]; Retention time: 4.02 min; LCMS conditions: A

### [Reference Example 53]

### 3-Cyclopropylindazol-6-ol

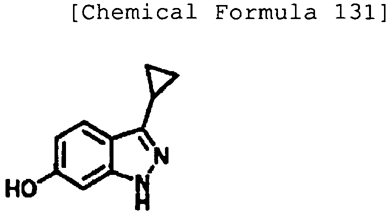

1-Benzyl-3-cyclopropylindazol-6-ol (6.68 g), which can be manufactured by the method described in Reference Example 52 and the like, and 10% palladium carbon (PE-type, containing 50% water) (2.68 g; made by N. E. Chemcat Corporation) were suspended in ethanol (246 mL; made by Wako Pure Chemical Industries Co., Ltd.). Concentrated hydrochloric acid (2.05 mL; made by Kanto Chemical Co., Inc.) was added. The interior of the reaction system was purged using hydrogen to create a hydrogen atmosphere, and [the contents] were stirred for three hours at 60°C. After the reaction solution had been cooled to room temperature, it was purged by nitrogen and filtered. The filtrate was concentrated under reduced pressure, and a hydrochloride of the title compound was obtained as a crude product (5.82 g).
LCMS: 175 [M + H]; Retention time: 2.85 min; LCMS conditions: A

### [Reference Example 54]

### 6-(tert-Butyldiphenylsilyloxy)-3-cyclopropylindazole

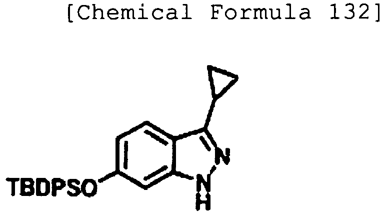

3-Cyclopropylindazol-6-ol hydrochloride (5.18 g), which can be manufactured by the method described in Reference Example 53 and the like, and imidazole (4.21 g; made by Tokyo Chemical Industry Co., Ltd.) were dissolved in dehydrated DMF (122 mL; made by Kanto Chemical Co., Inc.). TBDPSCI (15.67 mL; made by Tokyo Chemical Industry Co., Ltd.) was added and stirred overnight at 20°C. Imidazole (1.8 g; made by Tokyo Chemical Industry Co., Ltd.) and TBDPSCI (6.27 mL; made by Tokyo Chemical Industry Co., Ltd.) were added to the reaction solution and stirred for two hours at 20°C. Water was added to the reaction solution, and it was extracted twice with ethyl acetate. The organic layer was washed twice with water and once with brine. After the organic layer was dried using anhydrous sodium sulfate, the solvent was distilled off under reduced pressure. The residue was purified by column chromatography ("Column A;" n-hexane: ethyl acetate = 95:5→74:26), and the title compound (4.71 g) was obtained.
¹H-NMR (300MHz, CDCl₃); *δ* (ppm) 0.95-1.0 0 (4H, m), 1.11 (9H, s), 2.04-2.14 (1H, m) , 6.59 (1H, d, J=2.2), 6.73 (1H, dd, J=2.2 , 8.8), 7.33-7.49 (7H, m), 7.72-7.75 (4H , m)
LCMS: 413 [M + H]; Retention time: 6.23 min; LCMS conditions: B

### [Reference Example 55]

### tert-Butyl 6-(tert-butyldiphenylsilyloxy)-3-cyclopropylindazole-1-carboxylate

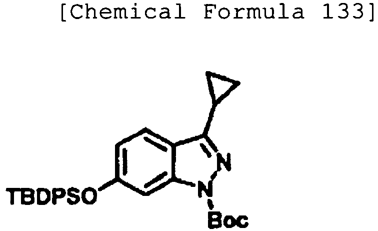

6-(tert-Butyldiphenylsilyloxy)-3-cyclopropylindazole (4.70 g), which can be manufactured by the method described in Reference Example 54 and the like, was dissolved in dehydrated THF (113 mL; made by Kanto Chemical Co., Inc.). Triethylamine (1.905 mL; made by Kokusan Chemical Co., Ltd.), DMAP (0.721 g; made by Wako Pure Chemical Industries Co., Ltd.), and Boc₂O (3.14 mL) were added and stirred overnight at room temperature. After the reaction solution had been concentrated under reduced pressure, the residue was purified by column chromatography ("Column A;" n-hexane: ethyl acetate = 100:0→90:10), and the title compound (8.02 g) was obtained.
¹H-NMR (300MHz, CDCl₃); *δ* (ppm) 0.97-1.2 8 (13H, m), 1.41 (9H, s), 2.07-2.15 (1H, m ), 6.78 (1H, dd, J=2.2, 8.4), 7.33-7.45 ( 8H, m), 7.66-7.74 (4H, m)
LCMS: 513 [M + H]; Retention time: 7.59 min; LCMS conditions: B

### [Reference Example 56]

### tert-Butyl 6-hydroxy-3-cyclopropylindazole-1-carboxylate

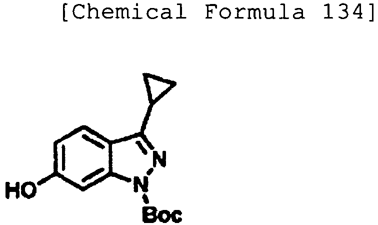

tert-Butyl 6-(tert-butyldiphenylsilyloxy)-3-cyclopropylinidazole-1-carboxylate (5.08 g), which can be manufactured by the method described in Reference Example 55 and the like, was dissolved in THF (53 mL; made by Kanto Chemical Co., Inc.). 1 mol/L TBAF-THF solution (19.82 mL) was added and stirred for 0.5 hour at room temperature. Water and brine were added to the reaction solution, and extraction was performed three times using ethyl acetate. The organic layer was washed with water and brine. After drying had been performed using anhydrous sodium sulfate, the solvent was distilled off under reduced pressure. The residue was purified by column chromatography ("Column B;" n-hexane: ethyl acetate = 95:5→74:26), and the title compound (2.54 g) was obtained.
¹H-NMR (300MHz, CDCl₃); *δ* (ppm) 0.96-1.2 3 (4H, m), 1.64 (9H, s), 2.12-2.22 (1H, m) , 6.26 (1H, brs), 6.87 (1H, dd, J=2.2, 8.8 ), 7.50-7.61 (2H, m)
LCMS: 275 [M + H]; Retention time: 4.08 min; LCMS conditions: B

### [Reference Example 57]

### Cyclobutyl(2-fluoro-4-hydroxyphenyl)methanone

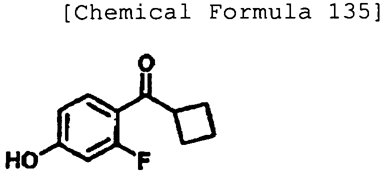

4-(tert-Butyldimethylsilyloxy)-2-fluorobenzonitrile (9.49 g), which can be manufactured by the method described in Reference Example 31 and the like, was dissolved in dehydrated THF (30 mL; made by Kanto Chemical Co., Inc.) in a nitrogen atmosphere and 0.78 mol/L cyclobutyl magnesium bromide-diethyl ether solution [60 mL; magnesium (9.18 g) was suspended in dehydrated diethyl ether (20 mL; made by Kanto Chemical Co., Inc.); after adding a small amount of iodine, [the mixture] was stirred for 15 minutes at room temperature; dehydrated diethyl ether (10 mL; made by Kanto Chemical Co., Inc.) was added to the reaction solution, and bromocyclobutane (6.974 mL) dissolved in dehydrated diethyl ether (50 mL; made by Kanto Chemical Co., Inc.) was added dropwise. After dropwise addition had been completed, stirring was conducted for one hour at room temperature, and a solution was prepared; part of the solution was sampled and titrated by 0.1 mol/L hydrochloric acid, and the concentration was verified to be 0.78 mol/L] was added dropwise. After dropwise addition had been completed, the reaction solution was stirred for 15 minutes at room temperature. Copper bromide (95.4 mg) was added and [the contents] were stirred for 0.5 hour under reflux. The reaction solution was cooled to 0°C, and water (30 mL) and 5 mol/L hydrochloric acid (30 mL) were added. After stirring for one hour under reflux, the reaction solution was cooled to room temperature and extracted three times using ethyl acetate. The organic layer was washed with water and brine. After drying had been performed using anhydrous sodium sulfate, the solvent was distilled off under reduced pressure. The residue was dissolved in dehydrated THF (76 mL; made by Kanto Chemical Co, Ltd.). 1 mol/L TBAF-THF solution (38 mL) was added and stirred for 5 minutes at room temperature. Water and brine were added to the reaction solution, and extraction was performed twice using ethyl acetate. The organic layer was washed with water and brine. After drying had been performed using anhydrous sodium sulfate, the solvent was distilled off under reduced pressure. The residue was dissolved in diethyl ether and extracted by 2 mol/L sodium hydroxide aqueous solution. The water layer was washed six times using diethyl ether. 2 mol/L hydrochloric acid was added to the water layer. It was extracted twice with ethyl acetate, and the organic layer was washed with water and brine. After drying had been performed using anhydrous sodium sulfate, the solvent was distilled off under reduced pressure, and the title compound (6.967 g) was obtained.
¹H-NMR (300MHz, DMSO-d₆); *δ* (ppm) 1.71-1 .80 (1H, m),1.92-2.04 (1H, m), 2.15-2.2 3 (4H, m), 3.79-3.85 (1H, m), 6.61 (1H, dd , J=2.0, 13.5), 6.72 (1H, dd, J=2.0, 8.42 ), 7.70-7.76 (1H, m), 10.80 (1H, brs)
LCMS: 195 [M + H]; Retention time: 3.68 min; LCMS conditions: B

### [Reference Example 58]

### 1-Benzyl-3-cyclobutylindazol-6-one

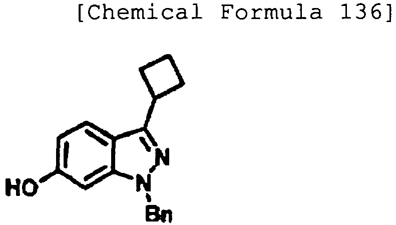

Cyclobutyl(2-fluoro-4-hydroxyphenyl)methanone (6.967 g), which can be manufactured by the method described in Reference Example 57 and the like, sodium acetate (14.16 g; made by Kanto Chemical Co., Inc.), and benzyl hydrazine dihydrochloride (10.55 g; made by Aldrich) were suspended in xylene (85 mL; made by Wako Pure Chemical Industries Co., Ltd.) and stirred overnight under reflux using a Dean-Stark apparatus. After the reaction solution had been cooled to room temperature, the precipitate was filtered out. The solid obtained was dissolved in water and ethyl acetate. The water layer was extracted twice using ethyl acetate. The organic layers were combined and washed twice with water and with brine. After drying had been performed using anhydrous sodium sulfate, the solvent was distilled off under reduced pressure, and the title compound (8.003 g) was obtained.
¹H-NMR (300MHz, DMSO-d₆); *δ* (ppm) 1.89-2 .10 (2H, m), 2.32-2.41(4H, m), 3.78-3.8 4 (1H, m), 5.44 (2H, s), 6.62 (11H, dd, J=1. 8, 8.4), 6.72 (1H, d, J=1.8), 7.14-7.32( 5H, m), 7.51(1H, d, J=8.4), 10.33 (1H, br s)
LCMS: 279 [M + H]; Retention time: 4.25 min; LCMS conditions: B

### [Reference Example 59]

### 3-Cyclobutylindazol-6-ol

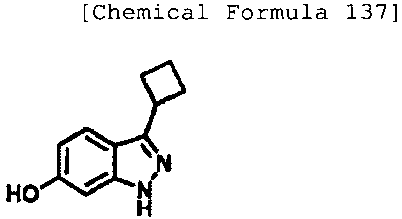

1-Benzyl-3-cyclobutylindazol-6-ol (8 g), which can be manufactured by the method described in Reference Example 58 and the like, and 10% palladium carbon (PE-type, containing 50% water) (3.22 g; made by N. E. Chemcat Corporation) were suspended in ethanol (287.4 mL). Concentrated hydrochloric acid (2.40 mL) was added. The interior of the reaction system was purged using hydrogen to create a hydrogen atmosphere, and [the contents] were stirred for 1.5 hours at 60°C. After the reaction solution had been cooled to room temperature, it was purged by nitrogen and filtered. The filtrate was concentrated under reduced pressure, and a hydrochloride of the title compound (6.5 g) was obtained as a crude product.
¹H-NMR (300MHz. DMSO-d₆) ; 1.87-2.20 (2H , m), 2.25-2.42 (4H, m), 3.43-3.92 (1H, m ), 6.65 (1H, dd, J=2.0, 8.8), 6.73 (1H, d, J=2.0), 7.56 (1H, d, J=8.8)
LCMS: 189 [M + H]; Retention time: 3.06 min; LCMS conditions: A

### [Reference Example 60]

### 6-(tert-Butyldiphenylsilyloxy)-3-cyclobutylindazole

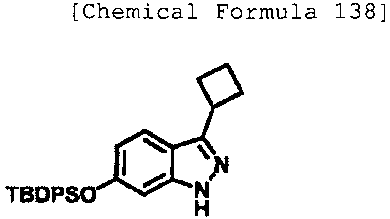

3-Cyclobutylindazol-6-ol hydrochloride (6.45 g), which can be manufactured by the method described in Reference Example 59 and the like, and imidazole (5.039 g) were dissolved in DMF (100 mL; made by Kanto Chemical Co., Inc.). TBDPSCI (18.44 mL) was added and stirred overnight at room temperature. Water was added to the reaction solution, and extraction was performed twice using ethyl acetate. The organic layer was washed twice with water and once with brine. After the organic layer was dried using anhydrous sodium sulfate, the solvent was distilled off under reduced pressure. The residue was purified by column chromatography ("Column A;" n-hexane: ethyl acetate = 95:5→74:26), and the title compound (9.93 g) was obtained.
¹H-NMR (300MHz, CDCl₃) ; *δ* (ppm) 1.11 (9H, s), 1.94-2.16 (2H, m), 2.39-2.49 (4H, m) , 3.80-3.86 (1H, m), 6.61 (1H, d, J=1.8), 6.72 (1H, dd, J=1.8, 8.4) 7.33-7.47 (7H, m), 7.72-7.75 (4H, m)
LCMS: 427 [M + H]; Retention time: 6.63 min; LCMS conditions: B

### [Reference Example 61]

### tert-Butyl 6-(tert-butyldiphenylsilyloxy)-3-cyclobutylindazole-1-carboxylate

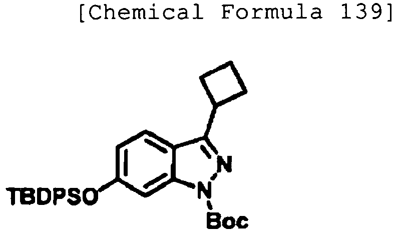

6-(tert-Butyldiphenylsilyloxy)-3-cyclobutylindazole (9.93 g), which can be manufactured by the method described in Reference Example 60 and the like, was dissolved in dehydrated THF (200 mL; made by Kanto Chemical Co., Inc.). Triethylamine (3.90 mL), DMAP (1.51 g), and Boc₂O (3.14 mL) were added and stirred for four hours at room temperature. After the reaction solution had been concentrated under reduced pressure, ethyl acetate was added to the residue, and washing was performed twice using 1 mol/L hydrochloric acid. After the organic layer was dried using anhydrous sodium sulfate, the solvent was distilled off under reduced pressure, and a crude product of the title compound (12.92 g) was obtained.
¹H-NMR (300MHz, CDCl₃); *δ* (ppm) 1.11 (9H, s), 1.42 (9H, s), 1.98-2.13 (2H, m), 2.38 -2.57 (4H, m), 3.79-3.85 (1H, m), 6.77 (1 H, dd, J=2.0, 8.6), 7.32-7.44 (8H, m), 7. 70-7.74 (4H, m)
LCMS: 527 [M + H]; Retention time: 7.99 min; LCMS conditions: B

### [Reference Example 62]

### tert-Butyl 6-hydroxy-3-cyclobutylindazole-1-carboxylate

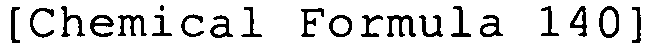

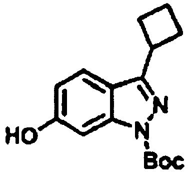

tert-Butyl 6-(tert-butyldiphenylsilyloxy)-3-cyclobutylindazole-1-carboxylate (12.26 g), which can be manufactured by the method described in Reference Example 61 and the like, was dissolved in THF (83 mL; Kanto Chemical Co., Inc.). 1 mol/L TBAF-THF solution (46 mL) was added and stirred for one hour at room temperature. Water and brine were added to the reaction solution, and extraction was performed three times using ethyl acetate. The organic layer was washed with water and brine. After drying had been performed using anhydrous sodium sulfate, the solvent was distilled off under reduced pressure. The residue was purified by column chromatography ("Column B;" n-hexane: ethyl acetate = 95:5→74:26), and the title compound (6.4457 g) was obtained.
¹H-NMR (300MHz. CDCl₃); *δ* (ppm) 1.63 (9H, s), 1.94-2.17 (2H, m), 2.37-2.59 (4H, m) , 3.87-3.89 (1H, m), 6.86 (1 H, dd, J=1.8, 8.4), 7.53-7.55 (2H, m)
LCMS: 289 [M + H]; Retention time: 4.42 min; LCMS conditions: B

### [Reference Example 63]

### 1-(2-Fluoro-4-hydroxyphenyl)-2-methylpropan-1-one

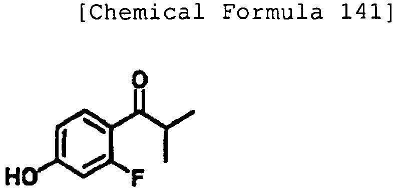

Dehydrated THF (5 mL; made by Kanto Chemical Co., Inc.) was added to 4-(tert-butyldimethylsilyloxy)-2-fluorobenzonitrile (14.02 g), which can be manufactured by the method described in Reference Example 31 and the like, in an argon atmosphere. After cooling to 0°C, 0.78 mol/L isopropyl magnesium bromide-THF solution (89 mL; made by Kanto Chemical Co., Inc.) was added dropwise. After dropwise addition had been completed, stirring was carried out for 20 minutes while warming the reaction solution to room temperature. Copper bromide (140 mg; made by Wako Pure Chemical Industries Co., Ltd.) was added and stirred for 1.5 hours at 60°C. The reaction solution was cooled to 0°C, and water (21.4 mL) and 5 mol/L hydrochloric acid (21.4 mL) were added. After stirring for six hours at 60°C, more 5 mol/L hydrochloric acid (21 mL) was added and stirred for 13 hours at 60°C. The reaction solution was cooled to room temperature and extracted three times using ethyl acetate. The organic layer was washed with water and brine. After drying had been performed using anhydrous sodium sulfate, the solvent was distilled off under reduced pressure. n-Hexane was added to the residue. The precipitate was filtered out, and the title compound (8.69 g) was obtained.
¹H-NMR (300MHz, DMSO-d₆) ; *δ* (ppm) 1.06 (6 H, d, J=6.6), 3.27-3.33 (1H, m), 6.62 (1H , dd, J=2.2, 13.6), 6.70 (1H, dd, J=2.2, 8 .6), 7.66-7.72 (1H, m)
LCMS: 181 [M - H]; Retention time: 3.70 min; LCMS conditions: A

### [Reference Example 64]

### 1-Benzyl-3-isopropylindazol-6-ol

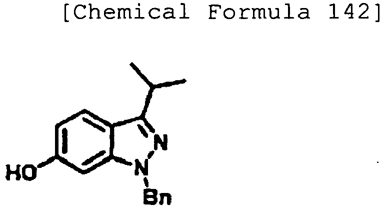

1-(2-Fluoro-4-hydroxyphenyl)-2-methylpropan-1-one (8.69 g), which can be manufactured by the method described in Reference Example 63, sodium acetate (18.90 g; made by Kanto Chemical Co., Inc.), and benzyl hydrazine dihydrochloride (13.97 g; made by Aldrich Co.) were suspended in xylene (200 mL; made by Wako Pure Chemical Industries Co., Ltd.) and stirred overnight under reflux using a Dean-Stark apparatus. After the reaction solution had been cooled to room temperature, water was added, and [the reaction solution] was extracted twice using ethyl acetate. The organic layer was washed twice with water and with brine. After drying had been performed using anhydrous sodium sulfate, the solvent was distilled off under reduced pressure. n-Hexane was added to the residue. The precipitate was filtered out, and a crude product of the title compound (14.09 g) was obtained.
¹H-NMR (300MHz, DMSO-d₆) ; *δ* (ppm) 1.35 (6 H, d, J=6.8), 3.25-3.29 (1H, m), 5.42 (2H ,s), 6.61(1H, dd, J=2.0, 8.6), 6.70 (1H, d, J=1.6), 7.12-7.57 (5H, m), 7.56 (1H, d , J=8.6), 10.32 (1H, brs)
LCMS: 267 [M + H]; Retention time: 3.99 min; LCMS conditions: A

### [Reference Example 65]

### 3-Isopropylindazol-6-ol

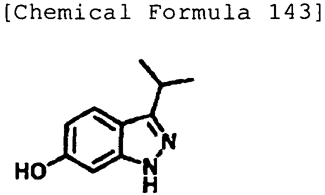

1-Benzyl-3-isopropylindazol-6-ol (6.51 g), which can be manufactured by the method described in Reference Example 64 and the like, and 10% palladium carbon (PE-type, containing 50% water) (2.69 g; made by N. E. Chemcat Corporation) were suspended in ethanol (244 mL; made by Wako Pure Chemical Industries Co., Ltd.). Concentrated hydrochloric acid (2.03 mL; made by Wako Pure Chemical Industries Co., Ltd.) was added. The interior of the reaction system was purged using hydrogen to create a hydrogen atmosphere, and [the contents] were stirred for 1.5 hours at 60°C. After the reaction solution had been cooled to room temperature, it was purged by nitrogen and filtered. The filtrate was concentrated under reduced pressure, and a hydrochloride of the title compound was obtained as a crude product (6.17 g).
¹H-NMR (300MHz, DMSO-d₆); *δ* (ppm) 1.35 (6 H, d, J=7.0), 3.32-3.36 (1H, m), 6.69 (1H ,dd, J=2.0, 8.8), 6.75 (1H, d, J=2.0),7. 64 (1H, d, J=8.8)
LCMS: 177 [M + H]; Retention time: 2.83 min; LCMS conditions: A

### [Reference Example 66]

### 6-(tert-Butyldiphenylsilyloxy)3-isopropylindazole

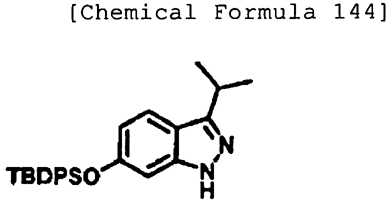

3-Isopropylindazol-6-ol hydrochloride (6.17 g), which can be manufactured by the method described in Reference Example 65 and the like, and imidazole (4.15 g; made by Tokyo Chemical Industry Co., Ltd.) were dissolved in dehydrated DMF (122 mL; made by Kanto Chemical Co., Inc.) and cooled to 0°C. TBDPSCI (15.67 mL; made by Tokyo Chemical Industry Co., Ltd.) was added. After stirring was performed overnight while [the contents] were warmed to room temperature, more imidazole (2.47 g; made by Tokyo Chemical Industry Co., Ltd.) and TBDPSCI (9.4 mL; made by Tokyo Co., Ltd.) were added and [the contents] were stirred for three hours at 20°C. Water was added to the reaction solution, and extraction was performed twice using ethyl acetate. The organic layer was washed twice with water and once with brine. After the organic layer was dried using anhydrous sodium sulfate, the solvent was distilled off under reduced pressure. The residue was purified by column chromatography ("Column A;" n-hexane: ethyl acetate = 95:5→74:26), and the title compound (6.65 g) was obtained.
¹H-NMR (300MHz, CDCl₃) ; *δ* (ppm) 1.11 (9H, s), 1.39 (6H, d, J=7.0), 3.24-3.38 (1H, m ), 6.61 (1H, d, J=8.8), 6.73 (1H, dd, J=2. 0, 8.8), 7.34-7.48 (7H, m), 7.72-7.76 (4 H, m)
LCMS: 415 [M + H]; Retention time: 6.40 min; LCMS conditions: B

### [Reference Example 67]

### tert-Butyl 6-(tert-butyldiphenylsilyloxy)-3-isopropylindazole-1-carboxylate

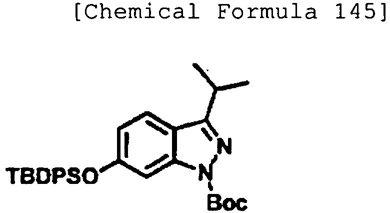

6-(tert-Butyldiphenylsilyloxy)-3-isopropylindazole (6.65 g), which can be manufactured by the method described in Reference Example 66, was dissolved in dehydrated acetonitrile (160 mL; made by Kanto Chemical Co., Inc.). Triethylamine (2.68 mL; made by Kokusan Chemical Co., Ltd.), DMAP (0.98 g; made by Wako Pure Chemical Industries Co., Ltd.), and Boc₂O (4.426 g; made by Peptide Institute, Inc.) were added and [the contents] were stirred for 13 hours at room temperature. After the reaction solution had been concentrated under reduced pressure, the residue was crudely purified by column chromatography ("Column B;" n-hexane: ethyl acetate = 100:0→90:10), and a crude product of the title compound (8.12 g) was obtained.
¹H-NMR (300MHz, CDCl₃); 1.11 (9H, s), 1.3 9-1.52 (15H, m), 3.26-3.35 (1H, m), 6.89 (1H, dd, J=2.2, 8.6), 7.33-7.46 (8H, m), 7.71-7.74 (4H, m)
LCMS: 515 [M + H]; Retention time: 7.93 min; LCMS conditions: B

### [Reference Example 68]

### tert-Butyl 6-hydroxy-3-isopropylindazole-1-carboxylate

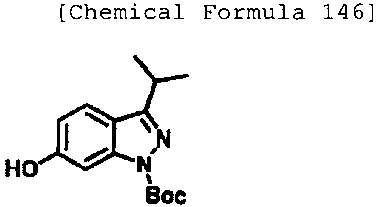

tert-Butyl 6-(tert-butyldiphenylsilyloxy)-3-isoproppylindazole-1-carboxylate (8.12 g), which can be manufactured by the method described in Reference Example 67 and the like, was dissolved in dehydrated THF (56.4 mL; made by Kanto Chemical Co., Inc.). After cooling to 0°C, 1 mol/L TBAF-THF solution (31.5 mL; made by Aldrich Co.) was added and stirred for one hour at room temperature. Water and brine were added to the reaction solution, and extraction was performed three times using ethyl acetate. The organic layer was washed with water and brine. After drying had been performed using anhydrous sodium sulfate, the solvent was distilled off under reduced pressure. The residue was purified by column chromatography ("Column B;" n-hexane: ethyl acetate = 95:5→74:26), and the title compound (3.39 g) was obtained.
¹H-NMR (300MHz, CDCl₃); *δ* (ppm) 1.43 (6H, d, J=7.0), 1.64 (9H, s), 3.28-3.40 (1H, m ), 6.22 (1H, brs), 6.86 (1H, dd, J=2.2, 8. 4), 7.53-7.54 (1H, m), 7.59 (1H, d, J=8.4 )
LCMS: 277 [M + H]; Retention time: 4.08 min; LCMS conditions: B

### [Reference Example 69]

### 6-Hydroxyindazol-3-carboxylic acid

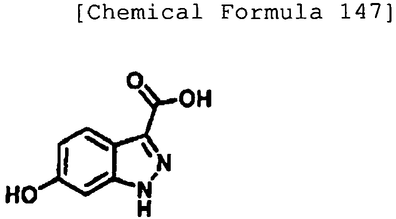

6-Methoxyindazole-3-carboxylic acid (1.015 g; made by Chem. Pacific) was dissolved in hydrobromic acid (52 mL; made by Kanto Chemical Co., Inc.) and [the solution] was stirred overnight under reflux. After [the contents] had been cooled to room temperature, the disappearance of the raw material and the title compound were confirmed using LCMS. The solvent was distilled off under reduced pressure, and a crude product of the title compound (1.504 g) was obtained.
LCMS: 179 [M + H]; Retention time: 1.94 min; LCMS conditions: A

### [Reference Example 70]

### Ethyl 6-hydroxyindazole-3-carboxylate

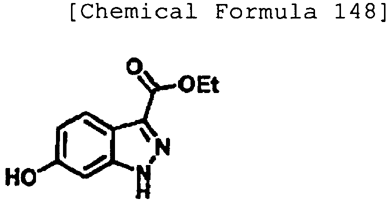

A crude product of 6-hydroxyindazole-3-carboxylic acid (1.504 g), which can be manufactured by the method described in Reference Example 69 and the like, was dissolved in ethanol. After cooling to 0°C, thionyl chloride (7.6 mL; made by Wako Pure Chemical Industries Co., Ltd.) was added dropwise. After the reaction solution had been stirred overnight at 60°C, it was cooled to room temperature. The disappearance of the raw material and the title compound were confirmed using LCMS, and the solvent was distilled off under reduced pressure. Ethanol (50 mL) was added to the residue, and the solvent was distilled off under reduced pressure. THF (50 mL) was added to the residue, and the solvent was again distilled off under reduced pressure, and a crude product of the title compound (1.457 g) was obtained.
LCMS: 207 [M + H]; Retention time: 2.80 min; LCMS conditions: A

### [Reference Example 71]

### Ethyl 6-tert-butyldiphenylsilyloxyindazole-3-carboxylate

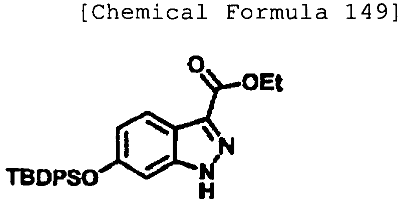

Ethyl 6-hydroxyindazole-3-carboxylate (1.457 g), which can be manufactured by the method described in Reference Example 70 and the like, was dissolved in dehydrated DMF (15.6 mL; made by Kanto Chemical Co., Inc.). Imidazole (1.425 g; made by Tokyo Chemical Industry Co., Ltd.) and TBDPSCI (4.06 mL) were added and stirred overnight at room temperature. The reaction solution was poured into a saturated sodium bicarbonate aqueous solution and extracted twice using ethyl acetate. The organic layer was washed with brine, and the insoluble matter was filtered out using Celite. The organic layer was washed twice with water. After drying had been performed using anhydrous magnesium sulfate, the solvent was distilled off under reduced pressure. The residue was purified by column chromatography ("Column A;" n-hexane: ethyl acetate = 81:19→60:40), and the title compound (1.467 g) was obtained.
¹H-NMR (300MHz, CDCl₃) ; *δ* (ppm) 1.14 (9H, s), 1.42 (3H, t, J=6.9), 1.47-2.18 (5H, m ), 3.46-3.54 (1H, m), 3.84-3.87 (1H, m), 4.44 (2H, q, J=7.1), 5.47 (1H, dd, J=2.7, 9.9), 6.86 (1H, d, J=2.0), 7.33-7.46 (6H , m), 7.71-7.76 (4H, m), 790 (1H, d, J=8. 6)
LCMS: 445 [M + H]; Retention time: 6.09 min; LCMS conditions: B

### [Reference Example 72]

### Ethyl 6-(tert-butyldiphenylsilyloxy)-1-(tetrahydro-2H-pyran-2-yl)indazole-3-carboxylate

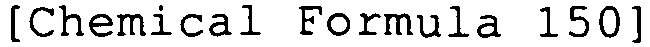

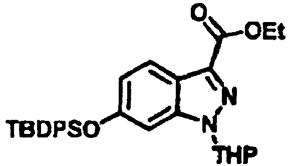

6-tert-Butyldiphenylsilyloxyindazole-3-carboxylate (1.461 g), which can be manufactured by the method described in Reference Example 71 and the like, was dissolved in toluene (16.5 mL; made by Wako Pure Chemical Industries Co., Ltd.). 3,4-Dihydro-2H-pyran (0.6 mL; made by Tokyo Chemical Industry Co., Ltd.) and toluenesulfonic acid monohydrate (0.1293 g) were added and [the contents] were stirred overnight at 60°C in a nitrogen atmosphere. The reaction solution was poured into a saturated sodium bicarbonate aqueous solution and extracted once by ethyl acetate. The organic layer was washed twice by water and once by brine. After drying had been performed using anhydrous magnesium sulfate, the solvent was distilled off under reduced pressure. The residue was purified by column chromatography ("Column A;" n-hexane: ethyl acetate = 96:4→75:25), and the title compound (1.3354 g) was obtained.
¹H-NMR (300MHz, CDCl₃) ; *δ* (p pm) 1.14 (9H, s), 1.42 (3H, t, J=6.9), 1.47-2.18 (5H, m ), 3.46-3.54 (1H. m), 3.84-3.87 (1H, m). 4.44 (2H, q, J=7.1), 5.47 (1H, dd, J=2.7, 9.9), 6.86 (1H, d, J=2.0), 7.33-7.46 (6H , m), 7.71-7.76 (4H, m), 7.90 (1H, d, J=8. 6)
LCMS: 529 [M + H]; Retention time: 6.83 min; LCMS conditions: B

### [Reference Example 73]

### Ethyl 6-hydroxy-1-(tetrahydro-2H-pyran-2-yl)indazole-3-carboxylate

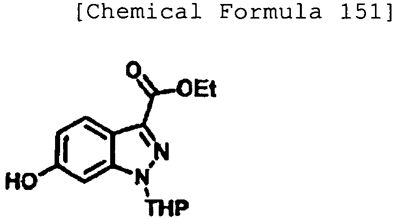

6-(tert-Butyldiphenylsilyloxy)-1-(tetrahydro-2H-pyran-2-yl)indazole-3-carboxylate (1.299 g), which can be manufactured by the method described in Reference Example 72 and the like, was dissolved in dehydrated THF (12.3 mL; made by Kanto Chemical Co., Inc.). 1 mol/L TBAF-THF solution (3.69 mL; made by Aldrich Co.) was added and [the contents] were stirred for two hours at room temperature in a nitrogen atmosphere. Ethyl acetate was added to the reaction solution, and it was washed three times using brine. After drying had been performed using anhydrous magnesium sulfate, the solvent was distilled off under reduced pressure. The residue was purified by column chromatography ("Column A;" n-hexane: ethyl acetate = 100:0→81:19), and the title compound (0.660 g) was obtained.
¹H-NMR (300MHz, CDCl₃); *δ* (ppm) 1.45 (3H. t, J=7.1), 1.60-1.76 (3H, m), 2.03-2.08 (2H, m), 2.42-2.53 (1H, m), 3.67-3.75 (1 H, m), 4.01-4.05 (1H, m), 4.48 (2H, q, J=7 .1), 5.40 (1H, brs), 5.71 (1H, dd, J=2.7, 9.7), 6.88 (1H, dd, J=2.0, 8.8), 7.07 (1H , d, J=2. 0), 8. 03 (1H, d, J=8.8)
LCMS: 291 [M + H]; Retention time: 3.69 min; LCMS conditions: A

### [Reference Example 74]

### Ethyl 6-benzyloxy-1-(tetrahydro-2H-pyran-2-yl) indazole-3-carboxylate

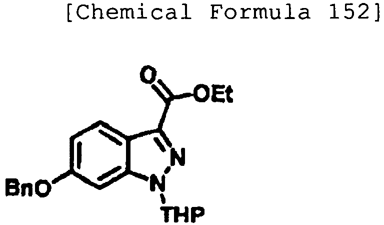

Ethyl 6-hydroxy-1-(tetrahydro-2H-pyran-2-yl) indazole-3-carboxylate (148 mg), which can be manufactured by the method described in Reference Example 73 and the like, was dissolved in dehydrated DMF (5.2 mL; made by Kanto Chemical Co., Inc.). Potassium carbonate (227 mg; made by Aldrich Co.) and benzyl bromide (73.6 µg; made by Wako Pure Chemical Industries Co., Ltd.) were added and [the contents] were stirred overnight at 60°C. The reaction solution was cooled to room temperature, and then poured into water and extracted twice using ethyl acetate. The organic layer was washed twice using water and once using brine. After drying had been performed using anhydrous sodium sulfate, the solvent was distilled off under reduced pressure. The residue was purified by column chromatography ("Column A;" n-hexane: ethyl acetate = 95:5→74:26), and the title compound (187 mg) was obtained.
¹H-NMR (300MHz. CDCl₃) ; *δ* (ppm) 1.46 (3H, t, J=7.2), 1.67-1.78 (3H, m), 2.04-2.12 (2H, m), 2.42-2.49 (1H, m), 3.69-3.75 (1 H, m), 4.01-4.05 (1H, m), 4.49 (2H, q, J=7 .2), 5.16 (2H, s), 5.75 (1H, dd, J=2.6, 9. 2), 7.04 (1H, dd, J=2.2, 8.8), 7.11 (1H, d , J=1.7), 7.32-7.49 (5H, m), 8.06 (1H, d, J=8.9)

### [Reference Example 75]

### (6-(Benzyloxy) -1- (tetrahydro-2H-pyran-2-yl) indazol-3-yl)methanol

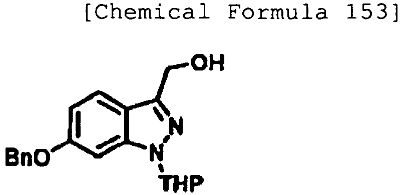

Ethyl 6-benzyloxy-1-(tetrahydro-2H-pyran-2-yl) indazole-3-carboxylate (182 mg), which can be manufactured by the method described in Reference Example 74 and the like, was dissolved in dehydrated THF (4.78 mL; made by Kanto Chemical Co., Inc.) and purged by nitrogen. LiAlH₄ (54 mg) was added at 0°C and stirred for one hour while warming to room temperature. After cooling the reaction solution to 0°C, THF/water = 1/1 (5 mL), Rochelle salt (; Kanto Chemical Co., Inc.) and saturated sodium bicarbonate aqueous solution were added, and [the reaction solution] was extracted twice using ethyl acetate. The organic layer was washed twice with water and once with brine. After drying had been performed using anhydrous sodium sulfate, the solvent was distilled off under reduced pressure. Hexane was added to the residue. The solvent was distilled off under reduced pressure, and the title compound (155 mg) was obtained.
¹H-NMR (300MHz. CDCl₃); *δ* (ppm) 1.54-1.7 9 (3H, m), 1.98-2.14 (2H, m), 2.05 (1H, t, J=5.9), 2.46-2.59 (1H, m), 3.72-3.77 (1 H, m), 4.02-4.06 (1H, m), 4.98 (2H, d, J=5 .9), 5.15 (2H, s), 5.58 (1H, dd, J=2.8, 9. 5), 6.91 (1H, dd, J=2.2, 8.8), 6.98 (1H, d , J=2.0), 7.32-7.49 (5H, m), 7.65 (1H, d, J=8.6)
LCMS: 339 [M + H]; Retention time: 4.02 min; LCMS conditions: A

### [Reference Example 76]

### (6-(Benzyloxy)-1-(tetrahydro-2H-pyran-2-yl) indazole-3-carbaldehyde

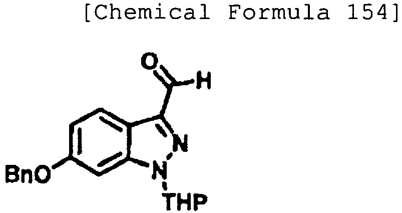

(6-(Benzyloxy)-1-(tetrahydro-2H-pyran-2-yl) indazol-3-yl)methanol (1.70 g), which can be manufactured by the method described in Reference Example 75 and the like, was dissolved in dichloromethane (25 mL; made by Kanto Chemical Co., Inc.) and THF (25 mL; made by Kanto Chemical Co., Inc.). Active manganese dioxide (7.48 g; made by Aldrich Co.) was added and [the contents] were stirred overnight at room temperature. The reaction solution was filtered using thin, solidified anhydrous magnesium sulfate. After again filtering the filtrate using a membrane filter (0.2 µm, Advantec), the solvent was distilled off under reduced pressure. The residue was purified by column chromatography ("Column B;" n-hexane: ethyl acetate = 100:0→82:18), and the title compound (1.20 g) was obtained.
¹H-NMR (300MHz. CDCl₃) ; *δ* (ppm) 1.66-1.8 7 (3H, m), 2.04-2.22 (2H, m), 2.49-2.61 ( 1H, m), 3.71-3.79 (1H, m), 3.96-4.01 (1H , m), 5.16 (2H, s), 5.74 (1H, dd, J=3.1, 8. 8), 7.06-7.10 (2H, m), 7.32-7.49 (5H, m) , 8.15 (1H, d, J=9.4), 10.19 (1H, s)
LCMS: 337 [M + H]; Retention time: 5.14 min; LCMS conditions: A

### [Reference Example 77]

### (E)-ethyl 3- (6- (benzyloxy) -1- (tetrahydro-2H-pyran-2-yl)indazol-3-yl)acrylate

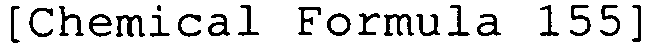

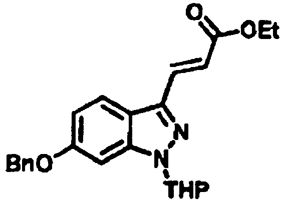

Ethyl 2-(diethoxyphosphoryl)acetate (0.595 mL; made by Tokyo Chemical Industry Co., Ltd.) was dissolved in dehydrated THF (10 mL; made by Kanto Chemical Co., Inc.). Sodium hydride-60% oil (126 mg; made by Kanto Chemical Co., Inc.) was added. After stirring for 10 minutes at room temperature, (6-(benzyloxy)-1-(tetrahydro-2H-pyran-2-yl)indazole-3-carbaldehyde (849 mg), which can be manufactured by the method described in Reference Example 76 and the like, was added and [the contents] were stirred for five hours at room temperature. Water was added to the reaction solution, and extraction was performed twice using ethyl acetate. The organic layer was washed twice with water and once with brine. After drying had been performed using anhydrous sodium sulfate, the solvent was distilled off under reduced pressure, and a crude product of the title compound (1.10 g) was obtained. ¹H-NMR (300MHz, CDCl₃) ; *δ* (ppm) 1.35 (3H, t, J=7.2), 1.68-1.75 (3H, m), 2.07-2.16 (2H, m), 2.50-2.54 (1H, m), 3.70-3.77 (1 H, m), 3.96-4.00 (1H, m), 4.28 (2H, q, J=7 .2), 5.15 (2H, s), 5.66 (1H, dd, J=2.9, 8. 8), 6.73 (1H, d, J=16.3), 6.99 (1H, dd, J= 2.2, 8.8), 7.05 (1H, d, J=1.8), 7.32-7.4 9 (5H, m), 7.79 (1H, d, J=9.0), 7.94 (1H, d , J=16.3)
LCMS: 407 [M + H]; Retention time: 5.72 min; LCMS conditions: B

### [Reference Example 78]

### Ethyl 3-(6-(benzyloxy)-1-(tetrahydro-2H-pyran-2-yl)indazol-3-yl)propanoate

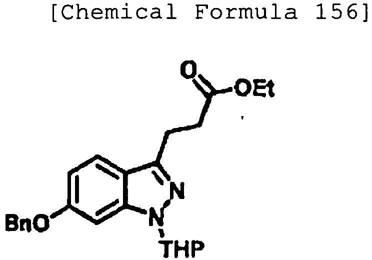

(E)-ethyl 3-(6-(benzyloxy)-1-(tetrahydro-2H-pyran-2-yl)indazol-3-yl)acrylate (1.02 g), which can be manufactured by the method described in Reference Example 77 and the like, and p-toluenesulfonyl hydrazide (4.70 g; made by Tokyo Chemical Industry Co., Ltd.) were dissolved in ethylene glycol dimethyl ether (12.5 mL; made by Aldrich Co.). After warming to 95°C, sodium acetate aqueous solution (prepared from sodium acetate (4.11 g; made by Wako Pure Chemical Industries Co., Ltd.) and water (6.25 mL)) was added dropwise and stirred for 5.5 hours at 95°C. The reaction solution was cooled to room temperature, and then poured into saturated sodium bicarbonate aqueous solution that contained ice and extracted twice using ethyl acetate. The organic layer was washed twice with water and once with brine. After drying had been performed using anhydrous sodium sulfate, the solvent was distilled off under reduced pressure. The residue was purified by column chromatography ("Column A;" n-hexane: ethyl acetate = 96:4→75:25), and the title compound (0.809 g) was obtained.
¹H-NMR (300MHz, CDCl₃) ; *δ* (ppm) 1.24 (3H, t, J=7.2), 1.54-1.76 (3H, m), 1.96-2.01 (1H, m), 2.11-2.14 (1H, m), 2.45-2.56 (1 H, m), 2.81-2.86 (2H, m), 3.20-3.25 (2H, m), 3.67-3.75 (1H, m), 4.00-4.03 (1H, m) , 4.13 (2H, q, J=7.2), 5.13 (2H, s), 5.54 ( 1H, dd, J=2.8, 9.4), 6.87 (1H, dd, J=2.2, 8.8), 6.96 (1H, d, J=2.0), 7.31-7.55 (6H , m)
LCMS: 409 [M + H]; Retention time: 2.04 min; LCMS conditions: C

### [Reference Example 79]

### 3-(6-(Benzyloxy)-1-(tetrahydro-2H-pyran-2-yl) indazol-3-yl)propanoic acid

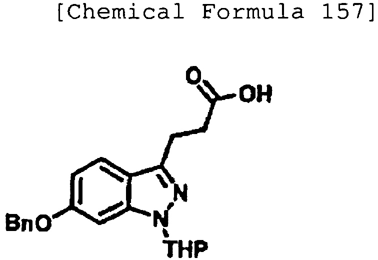

Ethyl 3-(6-(benzyloxy)-1-(tetrahydro-2H-pyran-2-yl)indazol-3-yl)propanoate (0.809 g), which can be manufactured by the method described in Reference Example 78 and the like, was dissolved in methanol (8 mL; made by Kanto Chemical Co., Inc.). 2 mol/L sodium hydroxide aqueous solution (1.68 mL) was added and [the contents] were stirred overnight at 40°C. After the reaction solution had been cooled to room temperature, the solvent was distilled off under reduced pressure. Water (10 mL), 2 mol/L hydrochloric acid (1.78 mL), and water (20 mL) were added to the residue. The precipitate was filtered out, and the title compound (0.630 g) was obtained.
¹H-NMR (300MHz, CDCl₃) ; *δ* (ppm) 1.64-1.7 6 (3H, m), 1.99-2.12 (2H, m), 2.43-2.54 ( 1H, m), 2.92 (2H, t, J=7.3) 1, 3.24 (2H, t, J =7.3), 3.68-3.75 (1H, m), 4.00-4.03 (1H , m), 5.14 (2H, s), 5.56 (1H, dd, J=2.6, 9. 4), 6.88 (1H, dd, J=2.2, 8.4), 6.96 (1H, d , J=1.5), 7.31-7.54 (6H, m)
LCMS: 381 [M + H]; Retention time: 4.34 min; LCMS conditions: A

### [Reference Example 80]

### 3-(6-(Benzyloxy)-1-(tetrahydro-2H-pyran-2-yl) indazole-N,N-dimethylpropanamide

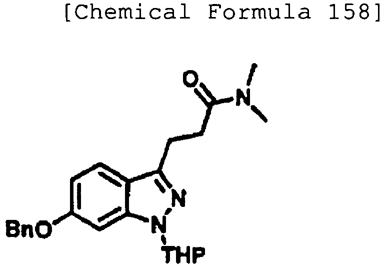

3-(6-(Benzyloxy)-1-(tetrahydro-2H-pyran-2-yl) indazol-3-yl)propanoic acid (570 mg), which can be manufactured by the method described in Reference Example 79 and the like, was dissolved in dehydrated THF (7 mL; made by Kanto Chemical Co., Inc.). Triethylamine (0.289 mL; made by Wako Pure Chemical Industries Co., Ltd.) and pivaloyl chloride (0.204 mL; made by Kanto Chemical Co., Inc.) were added and [the contents] were stirred for 15 minutes at room temperature. 2 mol/L dimethylamine-THF solution (24.84 mL) was added to the reaction solution and stirred overnight at room temperature. The reaction solution was poured into water and extracted twice using ethyl acetate. The organic layer was washed twice with water and once with brine. After drying had been performed using anhydrous sodium sulfate, the solvent was distilled off under reduced pressure. The residue was purified by column chromatography ("Column B;" n-hexane: ethyl acetate = 54:46→33:67), and the title compound (597 mg) was obtained.
¹H-NMR (300MHz, CDCl₃) ; *δ* (ppm) 1.61-1.7 7 (3H, m), 1.96-2.13 (2H, m), 2.50-2.53 ( 1H, m), 2.81-2.86 (2H, m), 2.95 (3H, s), 2 .97 (3H, s), 3.24-3.29 (2H, m), 3.72-3.7 5 (1H, m), 4.04-4.08 (1H, m), 5.14 (2H, s) 5.53 (1H. dd. J=2.6, 9.7), 6.86 (1H, dd. J=2.2, 8.8), 6.95 (1H, d, J=2.0), 7.31-7 .57 (6H, m)
LCMS: 408 [M + H]; Retention time: 4.28 min; LCMS conditions: B

### [Reference Example 81]

### 3-(6-(Benzyloxy)indazol-3-yl)-N,N-dimethylpropanamide

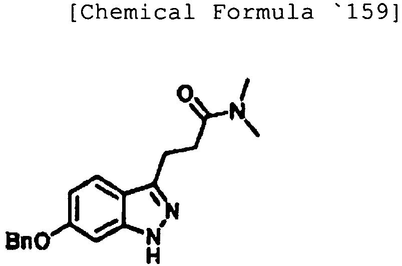

3-(6-Benzyloxy)-1-(tetrahydro-2H-pyran-2-yl) indazol-3-yl)-N,N-dimethylpropanamide (7.4931 g), which can be manufactured by the method described in Reference Example 80 and the like, 4 mol/L hydrochloric acid-1,4-dioxane solution (360 mL; made by Kokusan Chemical Co., Ltd.), and dehydrated methanol (4 mL; made by Kanto Chemical Co., Inc.) were added and [the contents] were stirred for 16 hours at 50°C. After filtering out the precipitate in the reaction solution, the solid obtained was washed with MTBE and introduced into aqueous sodium bicarbonate. The water layer was extracted once with ethyl acetate and once with chloroform. After drying the organic layer by magnesium sulfate, concentrating was performed under reduced pressure, and the title compound (5.5850 g) was obtained.
¹H-NMR (300MHz. DMSO-d₆) ; *δ* (ppm) 2.73 (2 H, t, J=8.0), 2.81 (3H, s), 2.94 (3H, s), 3 .05 (2H, t, J=8.0), 5.15 (2H, s), 6.77 (1H , dd, J=2.1, 8.7), 6.91 (1H, d, J=2.1), 7. 30-7.48 (5H, m), 7.59 (1H, d, J=8.7), 12. 39 (1H, s)
LCMS: 324 [M + H]; Retention time: 1.37 min; LCMS conditions: C

### [Reference Example 82]

### tert-Butyl 6-(benzyloxy)-3-(3-(dimethylamino)-3-oxopropyl) indazole-1-carboxylate

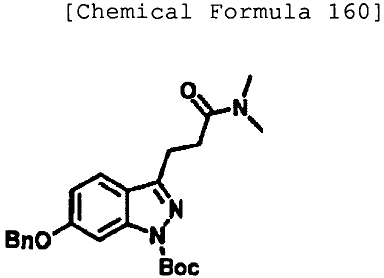

3-(6-(Benzyloxy)indazol-3-yl)-N,N-dimethylpropanamide (5.289 g), which can be manufactured by the method described in Reference Example 81 and the like, was dissolved in dehydrated THF. Boc₂O (4.5 mL; made by Wako Pure Chemical Industries Co., Ltd.), triethylamine (2.75 mL; made by Kokusan Chemical Co., Ltd.), and 4-N,N-dimethylaminopyridine (203 mg) were added and [the contents] were stirred overnight at room temperature. Ethyl acetate was added to the reaction solution. The organic layer was washed twice with 1 mol/L aqueous hydrochloric acid, once with water, and once with brine. After drying was performed using magnesium sulfate, the solvent was distilled off under reduced pressure, and the title compound (7.066 g) was obtained.
LCMS: 424 [M + H]; Retention time: 1.85 min; LCMS conditions: C

### [Reference Example 83]

### tert-Butyl 3-(3-(dimethylamino)-3-oxopropyl)-6-hydroxyindazole-1-carboxylate

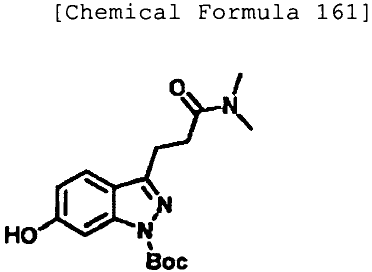

tert-Butyl 6-(benzyloxy)-3-(3-(dimethylamino)-3-oxopropyl) indazole-1-carboxylate (6.9915 g), which can be manufactured by the method described in Reference Example 82 and the like, and 5% palladium carbon (STD-type, containing 50% water) (3.5517 g; made by N. E. Chemcat Corporation) were suspended in THF (81.5 mL). The interior of the reaction system was then purged using hydrogen to create a hydrogen atmosphere, and [the contents] were stirred overnight at room temperature. After purging the interior of the reaction system with nitrogen, [the reaction solution] was filtered. The filtrate was concentrated under reduced pressure, and the title compound (5.7091 g) was obtained.
¹H-NMR (300MHz, CDCl₃) ; *δ* (ppm) 1.62 (9H, s), 2.94 (2H, t, J=6.5), 2.97 (3H, s), 3.0 5 (3H, s), 3.24 (2H, t, J=6.5), 6.69 (1H, d d, J=1.8, 8.4), 7.26-7.29 (1H, m), 7.41 ( 1H, s), 8.65 (1H, s)
LCMS: 334 [M + H]; Retention time: 1.17 min; LCMS conditions: C

### [Reference Example 84]

### N-benzyl-2-(benzyloxy)ethanamine

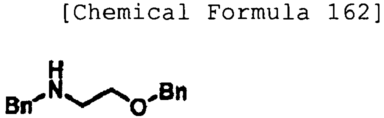

2-(Benzyloxy)ethanamine (12.3146 g; made by Bionet Co., Ltd.) was dissolved in CH₂Cl₂ (150 mL). Benzaldehyde (8.7219 g; made by Kanto Chemical Co., Inc.) and anhydrous sodium sulfate (67.7879 g; made by Wako Pure Chemical Industries Co., Ltd.) were added and [the contents] were stirred overnight at room temperature. After filtering the reaction solution, the filtrate was concentrated under reduced pressure. The resulting residue was dissolved in methanol (150 mL). Sodium borohydride (3.4129 g; made by Kanto Chemical Co., Inc.) was added and [the contents] were stirred for two hours at room temperature. The reaction solution was concentrated under reduced pressure. After adding water, it was extracted twice using ethyl acetate. The organic layer was washed twice with water and once with brine, and dried using anhydrous sodium sulfate. The organic layer was concentrated under reduced pressure, and the title compound (20.188 g) was obtained. ¹H-NMR (300MHz, CDCl₃) ; *δ* (ppm) 2.84 (2H, t, J=5.1), 3.62 (2H, t, J=5.1), 3.80 (2H. s), 4.52 (2H, s), 7.20-7.37 (10H, m)

### [Reference Example 85]

### (R) -2- (benzyl (2- (benzyloxy) ethyl) amino) -1- (3-nitrophenyl)ethanol

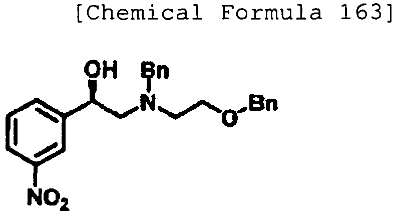

N-benzyl-2-(benzyloxy)ethanamine (13.6532 g), which can be manufactured by the method described in Reference Example 84 and the like, (R)-2-(3-nitrophenyl)oxirane (20.21 g), and 2-propanol (205 mL) were added and [the contents] were stirred for 36 hours under reflux. The reaction solution was cooled to room temperature. After concentrating under reduced pressure, toluene (100 mL) was added to the residue, which was then concentrated under reduced pressure. The resulting residue was purified by column chromatography ("Column D;" n-hexane: ethyl acetate = 85:15→80:20), and the title compound (30.761 g) was obtained.
¹H-NMR (300MHz, CDCl₃) (ppm) 2.61 (1H, dd, J=3.2, 10.2), 2.75-3.01 (3H, m), 3.5 1-3.63 (2H, m), 3.78 (2H, dd, J=13.5, 68. 9), 4.53 (2H, s), 4.70 (1H, dd, J=3.2, 10. 2), 7.27-7.39 (10H, m), 7.44 (1H, t, J=8. 0), 7.59 (1H, d, J=8.0), 8.08 (1H, qd, J=1 .1, 8.0), 8.16 (1H, d, J=1.1)
LCMS: 407 [M + H]; Retention time: 1.31 min; LCMS conditions: C

### [Reference Example 86]

### (R)-N-benzyl-N-(2-(benzyloxy)ethyl)-2-(3-nitrophenyl)-2-(triethylsilyloxy)ethanamine

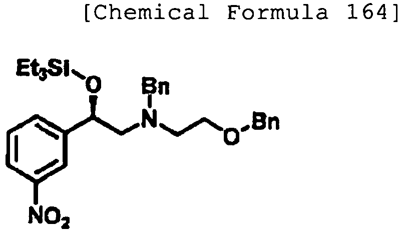

(R) -2- (benzyl (2- (benzyloxy) ethyl) amino) -1- (3-nitrophenyl)ethanol (30.371 g), which can be manufactured by the method described in Reference Example 85 and the like, and imidazole (6.1318 g; made by Tokyo Chemical Industry Co., Ltd.) were dissolved in dehydrated DMF (150 mL). Chlorotriethylsilane (15.1 mL; made by Shin-Etsu Chemical Co., Ltd.) was added and [the contents] were stirred overnight at room temperature. The reaction solution was poured into water and extracted twice using ethyl acetate. The organic layer was washed twice with water and once with brine. After drying was performed using magnesium sulfate, the solvent was distilled off under reduced pressure. The resulting residue was purified by column chromatography ("Column A;" n-hexane: ethyl acetate = 100:0→87:13), and the title compound (36.655 g) was obtained.
¹H-NMR (300MHz, CDCl₃) ; *δ* (ppm) 0.44-0.5 3 (6H, m), 0.85 (9H, t, J=8.0), 2.67-2.85 (4H, m), 3.40-3.45 (2H, m), 3.62 (2H, dd. J=13.5, 42.8), 4.42 (2H, s). 4.68 (1H, dd , J=7.3), 7.05-7.36 (10H, m), 7.56 (1H, d , J=7.6), 8.02-8.06 (1H, m), 8.11-8.12 ( 1H, m)

### [Reference Example 87]

### (R)-tert-butyl 2-(3-aminophenyl)-2-(triethylsilyloxy)ethyl (2-hydroxyethyl)carbamate

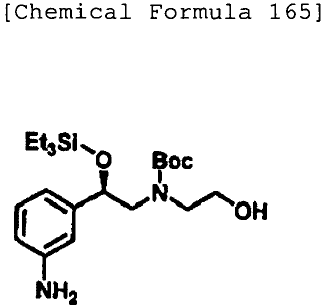

(R)-N-benzyl-N-(2-(benzyloxy)ethyl)-2-(3-nitrophenyl)-2-(triethylsilyloxy)ethanamine (36.455 g), which can be manufactured by the method described in Reference Example 86 and the like, and 10% palladium carbon (PE-type, containing 50% water) (15.1241 g; made by N. E. Chemcat Corporation) were suspended in ethanol (175 mL). A hydrogen atmosphere was created by purging the interior of the reaction system with hydrogen, and [the contents] were stirred for nine hours at 50°C. The reaction solution was again purged with hydrogen to create a hydrogen atmosphere and stirred for four hours at 50°C. After the reaction solution had been cooled to room temperature, it was purged by nitrogen and filtered. After the reaction solution had been concentrated under reduced pressure, the residue obtained (25.083 g) was dissolved in THF (175 mL). Boc₂O (14.6029 g; made by Wako Pure Chemical Industries Co., Ltd.) was added and [the contents] were stirred for 1.5 hours at room temperature. The reaction solution was concentrated under reduced pressure. 20% Palladium hydroxide carbon (containing 50% water) (15.0214 g; made by N. E. Chemcat Corporation), THF (80 mL), and methanol (80 mL) were added to the residue obtained to suspend it. The interior of the reaction system was purged using hydrogen to create a hydrogen atmosphere, and [the contents] were stirred for eight hours at 50°C. After the reaction solution had been cooled to room temperature, it was purged by nitrogen and filtered. After the reaction solution had been concentrated under reduced pressure, the residue obtained was purified by column chromatography ("Column A;" n-hexane: ethyl acetate = 75:25→54:46), and the title compound (16.918 g) was obtained.
¹H-NMR (300MHz, CDCl₃) ; *δ* (ppm) 0. 43-0.5 7 (6H, m), 0.87 (9H, t, J=8.0), 1.48-1.50 (9H, m), 2.04-3.86 (6H, m), 4.08-5.19 (1 H, m), 6.57-6.77 (3H, m), 7.09 (1H, t, J=7 .6)
LCMS: 411 [M + H]; Retention time: 2.03 min; LCMS conditions: C

### [Reference Example 88]

### 2-Chloro-1-(4-fluoro-3-nitrophenyl)ethanone

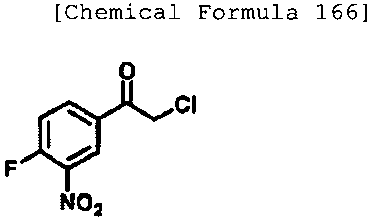

1-(4-Fluoro-3-nitrophenhyl)ethanone (18.42549 g; made by Aldrich Co.) was dissolved in CH₂Cl₂ (400 mL). Methanol (3.04 mL) was added,[and the system was] purged by nitrogen and cooled to 0°C. SO₂Cl₂-CH₂Cl₂ solution [109.32 mL; solution prepared by dissolving SO₂Cl₂ (9.32 mL; made by Wako Pure Chemical Industries Co., Ltd.) in CH₂Cl₂ (100 mL)] was added dropwise over 30 minutes and stirred overnight while warming to room temperature. After cooling the reaction solution to 0°C and adding methanol (1.52 mL), SO₂Cl₂-CH₂Cl₂ solution [64.66 mL, a solution prepared by dissolving SO₂Cl₂ (4.66 mL; made by Wako Pure Chemical Industries Co., Ltd.) in CH₂Cl₂ (60 mL)] was added dropwise and [the contents were] stirred for three hours while warming to room temperature. The reaction solution was washed once with saturated aqueous sodium carbonate and once with brine. The organic layer was dried using anhydrous sodium sulfate. After distilling off the solvent under reduced pressure, the crystals solidified in the silica gel while purifying the residue obtained by column chromatography ("Column A;" n-hexane: ethyl acetate = 88:12→67:33) were extracted by ethyl acetate and filtered out. The solvent was distilled off by placing the filtrate under reduced pressure, and the title compound (6.403 g) was obtained.
¹H-NMR (300MHz, CDCl₃) ; *δ* (ppm) 4.66 (2H, s), 7.46 (1H, dd. J=8.7, 9.8), 8.28 (1H, d dd, J=2.1, 4.0, 8.7), 8.67 (1H, dd, J=2.1 , 7.3)

### [Reference Example 89]

### (R)-2-(4-fluoro-3-nitrophenyl)oxirane

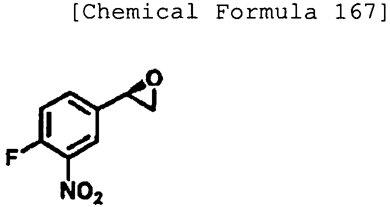

2-Chloro-1-(4-fluoro-3-nitrophenyl)ethanone (5.4667 g), which can be manufactured by the method described in Reference Example 88 and the like, was dissolved in dehydrated THF (100 mL) in a nitrogen atmosphere. 1 mol/L (R)-CBS-toluene solution (7.5 mL; made by Aldrich Co.) was added and cooled to 0°C. 2 mol/L BH₃•SMe₂-toluene solution (10 mL; made by Aldrich Co.) was added to this solution dropwise over 10 minutes and stirred for two hours at 0°C. Aqueous ammonium chloride was added to the reaction solution, and extraction was performed twice using ethyl acetate. After washing the organic layer once with brine, drying was performed using magnesium sulfate, and the solvent was distilled off under reduced pressure. 2-Propanol (100 mL) and 1 mol/L aqueous sodium hydroxide (25 mL; made by Kanto Chemical Co., Inc.) were added to the residue obtained and stirred for 10 minutes at 0°C. The reaction solution was poured into water and extracted twice using ethyl acetate. After washing the organic layer once with brine, drying was performed using anhydrous sodium sulfate and concentrating under reduced pressure was performed. The resulting residue was purified by column chromatography ("Column B;" n-hexane: ethyl acetate = 88:12→67:33), and the title compound (4.2885 g, optical purity: 92% ee) was obtained.
[Optical purity measurement conditions]: Column: As-H (made by Daicel Corporation], mobile layer; hexane: ethanol = 90: 10, flow rate: 0.5 mL/min, UV detection: 254 nm, temperature: 40°C
¹H-NMR (300MHz, CDCl₃) ; *δ* (ppm) 2.76 (1H, dd, J=2.5, 5.4), 3.20 (1H. dd, J=4.0, 5.4 ), 3.92 (1H, dd, J=2.5, 4.0), 7.28 (1H, dd , J=8.4, 10.2), 7.54 (1H, ddd, J=2.1, 4.0 , 8.4), 7.99 (1H. dd. J=2.1, 6.9)

### [Reference Example 90]

### tert-Butyl 6-(2-(dibenzylamino)ethoxy) 3-methylindazole-1-carboxylate

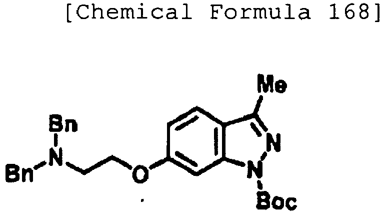

tert-Butyl 6-hydroxy-3-methylindazole-1-carboxylate (4.9962 g), which can be manufactured by the method described in Reference Example 28 and the like, and 2-(dibenzylamino)ethanol (5.0 mL; made by Tokyo Chemical Industry Co., Ltd.) were dissolved in dehydrated THF (100 mL). Triphenyl phosphine (10.5872 g; made by Tokyo Chemical Industry Co., Ltd.) and TMAD (6.9197 g; made by Masuda Chemical Industry Co., Ltd) were added and [the contents] were stirred overnight at room temperature. After filtering the reaction solution, the filtrate was concentrated under reduced pressure. Toluene was added to the residue obtained. After filtering out the insoluble matter, the filtrate was concentrated under reduced pressure. The resulting residue was purified by column chromatography ("Column D;" n-hexane: ethyl acetate = 90:10→75:25), and the title compound (9.6091 g) was obtained.
¹H-NMR (300MHz, CDCl₃) ; *δ* (ppm) 1.71 (9H, s), 2.53 (3H, s), 2.95 (2H, t, J=5.8), 3.7 3 (4H, s), 4.13 (2H, t, J=5.8), 6.87 (1H, d d, J=1.4, 8.7), 7.20-7.41 (10H, m), 7.46 (1H, d. J=8.7), 7.56 (1H, d. J=1.4)
LCMS: 472 [M + H]; Retention time: 2.04 min; LCMS conditions: C

### [Reference Example 91]

### tert-Butyl 6-(2-(benzylamino)ethoxy)-3-methylindazole-1-carboxylate

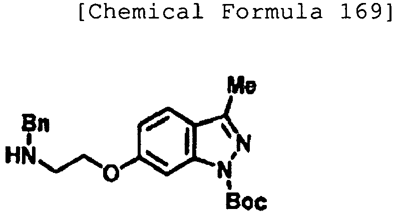

tert-Butyl 6-(2-(dibenzylamino)ethoxy) 3-methylindazole-1-carboxylate (10.84 g), which can be manufactured by the method described in Reference Example 90 and the like, and 5% palladium carbon (STD-type, containing 50% water) (2.0643 g; made by N. E. Chemcat Corporation) were suspended in methanol (115 mL). Concentrated hydrochloric acid (1.91 mL; made by Kanto Chemical Co., Inc.) was then added. The interior of the reaction system was purged using hydrogen to create a hydrogen atmosphere, and [the contents] were stirred for three hours at room temperature. After the interior of the reaction system had been purged using nitrogen, [the contents were] filtered. The filtrate was concentrated under reduced pressure, and the title compound (8.4593 g) was obtained.
¹H-NMR (300MHz, CDCl₃) ; *δ* (ppm) 1.71 (9H, s), 2.53 (3H, s), 3.07 (2H, t, J=5.4), 3.8 9 (2H, s), 4.19 (2H, t, J=5.4), 6.91 (1H, d d, J=1.8, 8.4), 7.27-7.37 (5H, m), 7.48 ( 1H, d, J=8.4), 7.61 (1H, d, J=1.8)
LCMS: 382 [M + H]; Retention time: 1.09 min; LCMS conditions: C

### [Reference Example 92]

### (R)-tert-butyl 6-(2-(benzyl(2-(4-fluoro-3-nitrophenyl)-2-hydroxyethyl) amino) ethoxy) -3-methylindazole-1-carboxylate

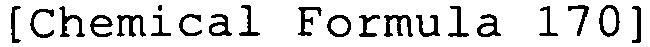

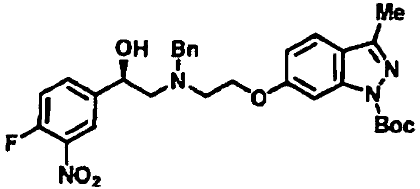

tert-Butyl 6-(2-(benzylamino) ethoxy)-3-methylindazole-1-carboxylate (1.4963 g), which can be manufactured by the method described in Reference Example 91 and the like, (R)-2-(4-fluoro-3-nitrophenyl)oxirane (723.2 mg), which can be manufactured by the method described in Reference Example 89 and the like, and 2-propanol (8 mL) were added and [the contents] were stirred overnight under reflux. After the reaction solution had been cooled to room temperature, concentrating was performed under reduced pressure. The residue obtained residue obtained was purified by column chromatography ("Column B;" n-hexane: ethyl acetate = 71:29→50:50), and the title compound (1.3571 g) was obtained.
¹H-NMR (300MHz, CDC l₃) ; *δ* (ppm) 1.71 (9H, s), 2.55 (3H, s), 2.65 (1H, dd, J=10.2, 13 .1), 2.91 (1H, dd, J=3.6, 13.1), 3.01-3. 23 (2H, m), 3.86 (2H, dd, 13.1, 67.4), 4.1 0-4.20 (2H, m), 4.71 (1H, dd, J=3.2, 10.2 ), 6.94 (1H, dd, J=2.1, 8.4), 7.18-7.33 ( 6H, m), 7.51 (1H, d, J=8.4), 7.56 (1H, ddd , J=2.1, 4.0, 8.4), 7.62 (1H, s), 8.00 (1H , dd, J=2.1, 7.3)
LCMS: 565 [M + H]; Retention time: 1.87 min; LCMS conditions: C

### [Reference Example 93]

### (R)-tert-butyl 6-(2-((2-(3-amino-4-fluorophenyl)-2-hydroxyethyl)(tert-butoxycarbonyl)amino)ethoxy)-3-methylindazole-1-carboxylate

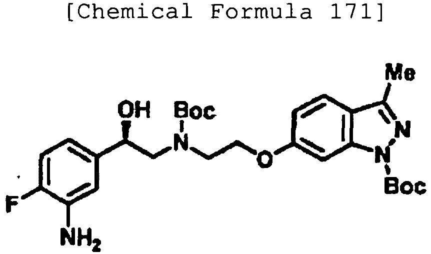

(R)-tert-butyl 6-(2-(benzyl(2-(4-fluoro-3-nitrophenyl)-2-hydroxyethyl)amino)ethoxy) 3-methylindazole-1-carboxylate (1.2046 g), which can be manufactured by the method described in Reference Example 92 and the like, and 10% palladium carbon (PE-type, containing 50% water) (0.2793 g; made by N. E. Chemcat Corporation) were suspended in 0.1 mol/L hydrochloric acid-ethanol solution (42.6 mL; made by Kanto Chemical Co., Inc.). The interior of the reaction system was then purged using hydrogen to create a hydrogen atmosphere, and [the contents] were stirred for one hour at room temperature. After the interior of the reaction system had been purged using nitrogen, [the contents were] filtered. After adding triethylamine (1.18 mL; made by Kanto Chemical Co., Inc.) to the filtrate, concentrating was performed under reduced pressure. The resulting residue was dissolved in methanol (20 mL). Boc₂O (458 µL; made by Wako Pure Chemical Industries Co., Ltd.) was added and [the contents] were stirred for five days at room temperature. After the reaction solution had been concentrated under reduced pressure, the residue obtained was designated as "residue A." (R)-tert-butyl 6-(2-(benzyl(2-(4-fluoro-3-nitrophenyl)-2-hydroxyethyl)amino)ethoxy) 3-methylindazole-1-carboxylate (119.8 mg), which can be manufactured by the method described in Reference Example 92 and the like, and 10% palladium carbon (PE-type, containing 50% water) (21.7 g; made by N. E. Chemcat Corporation) were suspended in 0.1 mol/L hydrochloric acid-ethanol solution (4 mL; made by Kanto Chemical Co., Inc.). The interior of the reaction system was then purged using hydrogen to create a hydrogen atmosphere, and [the contents] were stirred for one hour at room temperature. After the interior of the reaction system had been purged using nitrogen, [the contents were] filtered. After adding triethylamine (111 µL; made by Kanto Chemical Co., Inc.) to the filtrate, concentrating was performed under reduced pressure. The resulting residue was dissolved in CH₂Cl₂ (4 mL) and methanol (2 µL). Boc₂O (41 µL; made by Wako Pure Chemical Industries Co., Ltd.) was added, stirred overnight at room temperature, and allowed to stand for 12 days. After the reaction solution had been concentrated under reduced pressure, the residue obtained was designated as "residue B." "Residue A" and "residue B" were combined and purified by column chromatography ("Column B;" n-hexane: ethyl acetate = 64:36→43:57), and the title compound (1.1753 g) was obtained.
LCMS: 545 [M + H]; Retention time: 1.85 min; LCMS conditions: C

### [Reference Example 94]

### (R)-tert-butyl 6-(2-(benzyl(2-(4-chloro-3-nitrophenyl)-2-hydroxyethyl)amino)ethoxy)-3-methylindazole-1-carboxylate

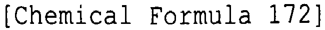

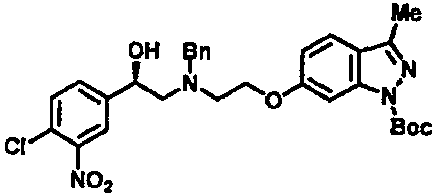

tert-Butyl 6-(2-(benzylamino)ethoxy)-3-methylindazole-1-carboxylate (3.0074 g), which can be manufactured by the method described in Reference Example 91 and the like, (R)-2-(4-chloro-3-nitrophenyl)oxirane (1.6147 g), and 2-propanol (8 mL) were added and [the contents] were stirred overnight under reflux. After the reaction solution had been cooled to room temperature, concentrating was performed under reduced pressure. The resulting residue was purified by column chromatography ("Column B;" n-hexane: ethyl acetate = 71:29→50:50), and the title compound (3.307 g) was obtained.
¹H-NMR (300MHz, CDCl₃); *δ* (ppm) 1.71 (9H, s), 2.55 (3H, s), 2.65 (1H, dd, J=10.2, 12 .8), 2.91 (1H, dd, J=3.2, 12.8), 3.01-3. 22 (2H, m), 3.85 (2H, dd, J=13.5, 66.3), 4 .13-4.21 (3H, m), 4.70 (1H, dd, J=3.2, 9. 8), 6.94 (1H, dd, J=2.1, 8.4), 7.27-7.35 (5H, m), 7.42-7.53 (2H, m), 7.52 (1H, d, J =8.4), 7.61 (1H, d, J=1.4), 7.83 (1H, d, J =1.4)
LCMS: 581 [M + H]; Retention time: 2.01 min; LCMS conditions: C

### [Reference Example 95]

### (R)-tert-butyl 6-(2-((2-(3-amino-4-chlorophenyl)-2-hydroxyethyl)(benzyl)amino)ethoxy)-3-methylindazole-1-carboxylate

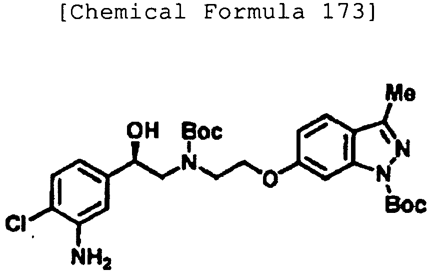

(R)-tert-butyl 6-(2-(benzyl(2-(4-chloro-3-nitrophenyl)-2-hydroxyethyl)amino)ethoxy) 3-methylindazole-1-carboxylate (3.2091 g), which can be manufactured by the method described in Reference Example 94 and the like, and CM-101 catalyst (6.6902 g; made by N. E. Chemcat Corporation) were suspended in methanol (25 mL) and THF (25 mL). The interior of the reaction system was then purged using hydrogen to create a hydrogen atmosphere, and [the contents] were stirred for four days at room temperature. After the interior of the reaction system had been purged using nitrogen, [the contents were] filtered. After the reaction solution had been concentrated under reduced pressure, CH₂Cl₂ was added to the residue obtained. The organic layer was dried using anhydrous sodium sulfate, the solvent was distilled off under reduced pressure, and the title compound (2.2493 g) was obtained.
LCMS: 551 [M + H]; Retention time: 1.53 min; LCMS conditions: C

### [Reference Example 96]

### (R)-tert-butyl 6-(2-((2-(3-amino-4-chlorophenyl)-2-hydroxyethyl)(tert-butoxycarbonyl)amino)ethoxy)-3-methylindazole-carboxylate

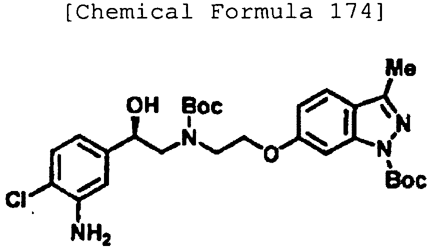

(R)-tert-butyl 6-(2-((2-(3-amino-4-chlorophenyl)-2-hydroxyethyl)(benzyl)amino)ethoxy-3-methylindazole-1-carboxylate (2.1101 g), which can be manufactured by the method described in Reference Example 95 and the like, and 10% palladium carbon (PE-type, containing 50% water) (417.6 mg/made by N. E. Chemcat Corporation) were suspended in 0.1 mol/L hydrochloric acid-ethanol solution (76.6 mL; made by Kanto Chemical Co., Inc.). The interior of the reaction system was then purged using hydrogen to create a hydrogen atmosphere, and [the contents] were stirred for 40 minutes at room temperature. After the interior of the reaction system had been purged using nitrogen, [the contents were] filtered. After adding triethylamine (2.12 mL; made by Kanto Chemical Co., Inc.) to the filtrate, concentrating was performed under reduced pressure. The resulting residue was dissolved in CH₂Cl₂ (20 mL). Boc₂O (792 µL; made by Wako Pure Chemical Industries Co., Ltd.) and triethylamine (0.5 mL; made by Kanto Chemical Co., Inc.) were added and [the contents] were stirred overnight at room temperature. After the reaction solution had been concentrated under reduced pressure, the residue obtained was purified by column chromatography ("Column B;" n-hexane: ethyl acetate = 63:37→42:58), and the title compound (1.5424 g) was obtained.
¹H-NMR (300MHz, CDCl₃) ; *δ* (ppm) 1.48 (9H, s), 1.70 (9H, s), 2.54 (3H, s), 3.22-3.78 (4H, m), 4.06-4.34 (5H, m), 4.91 (1H, brs ), 6.68-6.91 (3H, m), 7.19 (1H, d, J=8.0) 7.48 (1H, d, J=8.7), 7.60 (1H, brs)

### [Reference Example 97]

### tert-Butyl 6-(2-(dibenzylamino)ethoxy) 3-methoxyindazole-1-carboxylate

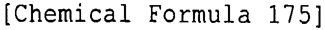

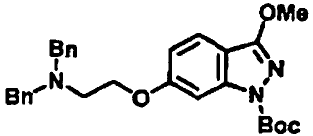

The title compound (9.456 g) was obtained by the same method as in Reference Example 90 using tert-butyl 6-hydroxy-3-methoxyindazole-1-carboxylate (5.3121 g), which can be manufactured by the method described in Reference Example 26 and the like, instead of tert-butyl 6-hydroxy-3-methylindazole-1-carboxylate.
¹H-NMR (300MHz, CDCl₃) ; *δ* (ppm) 1.69 (9H, s), 2.94 (2H, t, J=5.8), 3.73 (4H, s), 4.1 0 (2H, t, J=5.8), 4.14 (3H, s), 6.8 (1H, d d, J=2.1, 8.4), 7.20-7.40 (11H, m), 7.47 (1H, d, J=8.4)
LCMS: 488 [M + H]; Retention time: 2.10 min; LCMS conditions: C

### [Reference Example 98]

### tert-Butyl 6-(2-(benzylamino)ethoxy) 3-methoxyindazole-1-carboxylate

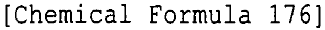

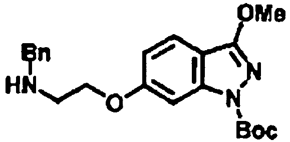

The title compound (7.8617 g) was obtained by the same method as in Reference Example 91 using tert-butyl 6-(2-(dibenzylamino)ethoxy 3-methoxyindazole-1-carboxylate (9.45 g), which can be manufactured by the method described in Reference Example 97 and the like, instead of tert-butyl 6-(2-(dibenzylamino)ethoxy) 3-methylindazole-1-carboxylate.
¹H-NMR (300MHz, CDCl₃) ; *δ* (ppm) 1.69 (9H, s), 3.06 (2H, t, J=5.1), 3.89 (2H, s), 4. 1 4 (3H, s), 4.16 (2H, t, J=5.1), 6.86 (1H, d d, J=2.1, 8.7), 7.27-7.37 (5H, m), 7.47-7.50 (2H, m)
LCMS: 398 [M + H]; Retention time: 1.18 min; LCMS conditions: C

### [Reference Example 99]

### (R)-tert-butyl 6-(2-(benzyl(2-(4-fluoro-3-nitrophenyl)-2-hydroxyethyl)amino)ethoxy)-3-methoxyindazole-1-carboxylate

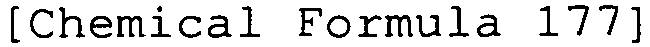

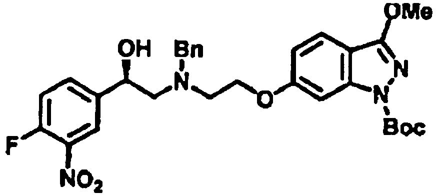

The title compound (1.5167 g) was obtained by the same method as in Reference Example 92 using tert-butyl 6-(2-(benzylamino)ethoxy 3-methoxyindazole-1-carboxylate (1.6037 g), which can be manufactured by the method described in Reference Example 98 and the like, instead of tert-butyl 6-(2-(benzylamino)ethoxy) 3-methylindazole-1-carboxylate.
¹H-NMR (300MHz, CDCl₃) ; *δ* (ppm) 1.69 (9H, s), 2.65 (1H, dd, J=10.2, 12.8), 2.91 (1H , dd, J=3.2, 12.8), 3.02-3.22 (2H, m), 3. 86 (2H, dd, J=13.5, 66.7), 4.10-4.13 (2H .m), 4.15 (3H, s), 4.71 (1H, dd, J=3.2, 10 .2), 6.88 (1H, dd, J=2.1, 8.7), 7.20 (1H, d, J=8.4), 7.27-7.33 (5H, m), 7.49 (1H, s ), 7.52 (1H, d, J=8.7), 7.57 (1H, ddd, J=2 .1, 4.0, 8.4), 8.00 (1H, dd, J=2.1, 6.9)
LCMS: 581 [M + H]; Retention time: 1.99 min; LCMS conditions: C

### [Reference Example 100]

### (R)-tert-butyl 6-(2-((2-(3-amino-4-fluorophenyl)-2-hydroxyethyl)(tert-butoxycarbonyl)amino)ethoxy)-3-methoxyindazole-1-carboxylate

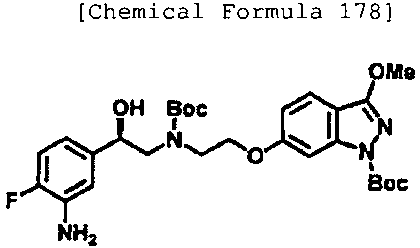

The title compound (1.0652 g) was obtained by the same method as in Reference Example 93 using (R)-tert-butyl 6-(2-(benzyl(2-(4-fluoro-3-nitrophenyl)-2-hydroxyethyl)amino)ethoxy)-3-methoxyindazole-1-carboxylate (1.5021 g), which can be manufactured by the method described in Reference Example 99 and the like, instead of (R)-tert-butyl 6-(2-(benzyl(2-(4-fluoro-3-nitrophenyl)-2-hydroxyethyl)amino)ethoxy)-3-methylindazole-1-carboxylate.
¹H-NMR (300MHz, CDCl₃) ; *δ* (ppm) 1.48 (9H, s), 1.68 (9H, s), 3.17-3.89 (4H, m), 4.14 -4.38 (5H, m), 4.89 (1H, brs), 6.67-6.97 (4H, m), 7.48-7.51 (2H, m)
LCMS: 561 [M + H]; Retention time: 1.93 min; LCMS conditions: C

### [Reference Example 101]

### (R)-tert-butyl 6-(2-(benzyl(2-(4-chloro-3-nitrophenyl)-2-hydroxyethyl)amino)ethoxy)-3-methoxyindazole-1-carboxylate

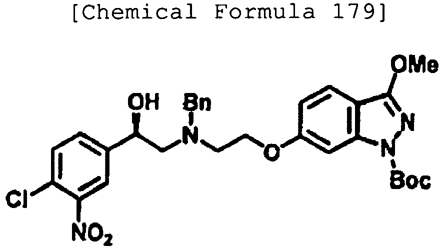

The title compound (3.6878 g) was obtained by the same method as in Reference Example 94 using tert-butyl 6-(2-(benzylamino)ethoxy) 3-methoxyindazole-1-carboxylate (3.1935 g), which can be manufactured by the method described in Reference Example 98 and the like, instead of tert-butyl 6-(2-(benzylamino)ethoxy) 3-methylindazole-1-carboxylate.
¹H-NMR (300MHz, CDCl₃) ; *δ* (ppm) 1. 69 (9H, s), 2.64 (1H, dd, J=10.2, 12.8), 2.92 (1H , dd, J=3.2, 12.8), 3.00-3.21 (2H, m), 3. 85 (2H, dd, J=13.5, 64.5), 4.08-4.13 (2H , m), 4.14 (3H, s), 4.70 (1H, dd, J=3.2, 10 , 2), 6.88 (1H, dd, J=2.1, 8.4), 7.27-7.3 5 (5H, m), 7.44-7.48 (2H, m), 7.52 (1H, d, J=8.4), 7.83 (1H, d, J=1.4)
LCMS: 597 [M + H]; Retention time: 2.08 min; LCMS conditions: C

### [Reference Example 102]

### (R)-tert-butyl 6-(2-((2-(3-amino-4-chlorophenyl)-2-hydroxyethyl)(benzyl)amino)ethoxy)-3-methoxyindazole-1-carboxylate

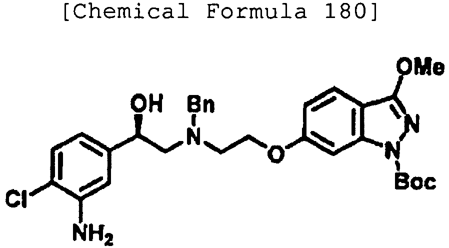

The title compound was obtained as a crude product (2.818 g) by the same method as in Reference Example 95 using (R)-tert-butyl 6-(2-(benzyl(2-(4-chloro-3-nitrophenyl)-2-hydroxyethyl)amino)ethoxy)-3-methoxyindazole-1-carboxylate (3.6514 g), which can be manufactured by the method described in Reference Example 101 and the like, instead of (R)-tert-butyl 6-(2-(benzyl(2-(4-chloro-3-nitrophenyl)-2-hydroxyethyl)amino)ethoxy)-3-methylindazole-1-carboxylate.
LCMS: 567 [M + H]; Retention time: 1.73 min; LCMS conditions: C

### [Reference Example 103]

### (R)-tert-butyl 6-(2-((2-(3-amino-4-chlorophenyl)-2-hydroxyethyl)(tert-butoxycarbonyl)amino)ethoxy)-3-methoxyindazole-carboxylate

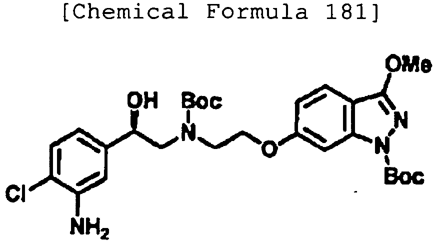

The title compound (1.9703 g) was obtained by the same method as in Reference Example 96 using (R)-tert-butyl 6-(2-((2-(3-amino-4-chlorophenyl)-2-hydroxyethyl)(benzylamino)ethoxy)-3-methoxyindazole-1-carboxylate (2.818 g), which can be manufactured by the method described in Reference Example 102 and the like, instead of (R)-tert-butyl 6-(2-((2-(3-amino-4-chlorophenyl)-2-hydroxyethyl)(benzyl)amino)ethoxy)-3-methylindazole-1-carboxylate.
¹H-NMR (300MHz, CDCl₃); *δ* (ppm) 1.48 (9H, s), 1.68 (9H, s), 3.19-3.64 (4H, m), 4.05 -4.29 (5H, m), 4.90 (1H, brs), 6.68-6.84 (3H, m), 7.20 (1H, d, J=8.4), 7.49-7.51 ( 2H, m)
LCMS: 577 [M + H]; Retention time: 2.03 min; LCMS conditions: C

### [Reference Example 104]

### tert-Butyl 3-chloro-6-(2-dibenzylamino)ethoxy)indazole-1-carboxylate

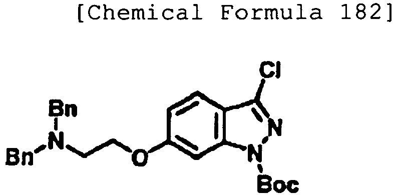

tert-Butyl 3-chloro-6-hydroxyindazole-1-carboxylate (4.3611 g), which can be manufactured by the method described in Reference Example 42 and the like, and 2-(dibenzylamino)ethanol (4.1529 g; made by Masuda Chemical Co., Ltd.) were dissolved in dehydrated THF (100 mL). Triphenyl phosphine (7.9370 g; made by Kanto Chemical Co., Inc.) and TMAD (5.2270 g; made by Masuda Chemical Co., Ltd.) were added and [the contents] were stirred overnight at room temperature. The reaction solution was filtered, and the filtrate was concentrated under reduced pressure. The resulting residue was dissolved in toluene, and the solvent was distilled off under reduced pressure. The resulting residue was purified by column chromatography ("Column D;" n-hexane: ethyl acetate = 90:10→75:25), and the title compound (7.1028 g) was obtained.
LCMS: 492 [M + H]; Retention time: 2.29 min; LCMS conditions: C

### [Reference Example 105]

### tert-Butyl 6-(2-benzylamino)ethoxy) 3-chloroindazole-1-carboxylate

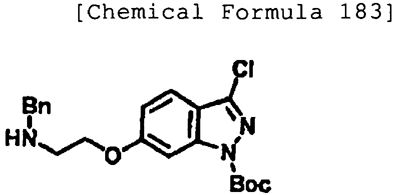

tert-Butyl 3-chloro-6-(2-(dibenzylamino)ethoxy)indazole-1-carboxylate (7.010 g), which can be manufactured by the method described in Reference Example 104 and the like, and 5% palladium carbon (STD-type, containing 50% water) (1.8534 g; made by N. E. Chemcat Corporation) were suspended in methanol (20 mL). 5 mol/L hydrochloric acid (2.9 mL; made by Kanto Chemical Co., Inc.) was then added. The interior of the reaction system was purged using hydrogen to create a hydrogen atmosphere, and [the contents] were stirred for 10 min at room temperature. After the interior of the reaction system had been purged using nitrogen, ethanol (20 mL) and water (10 mL) were added. The interior of the reaction system was purged using hydrogen to create a hydrogen atmosphere, and [the contents] were stirred for one hour at room temperature. After the interior of the reaction system had been purged using nitrogen, [the contents were] filtered. The filtrate was concentrated under reduced pressure, and a crude product of the title compound (3.6082 g) was obtained.
¹H-NMR (300MHz, CDCl₃); δ (ppm) 1.70 (9H, s), 3.08 (2H, t, J=5.1), 3.89 (2H, s), 4.1 7-4.21 (2H, m), 6.98 (1H, dd, J=1.8, 8.7) , 7.27-7.37 (5H, m), 7.53 (1H, d, J=8.7), 7.63 (1H, d, J=1.8)
LCMS: 402 [M + H]; Retention time: 1.29 min; LCMS conditions: C

### [Reference Example 106]

### (R)-tert-butyl 6-(2-(benzyl(2-(4-fluoro-3-nitrophenyl)-2-hydroxyethyl)amino)ethoxy)-3-chloroindazole-1-carboxylate

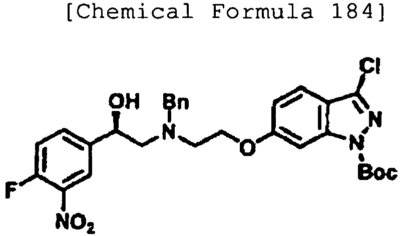

tert-Butyl 6-(2-benzylamino)ethoxy) 3-chloroindazole-1-carboxylate (1.6144 g), which can be manufactured by the method described in Reference Example 105 and the like, (R)-2-(4-fluoro-3-nitrophenyl)oxirane (732 mg), which can be manufactured by the method described in Reference Example 89 and the like, and 2-propanol (8 mL) were added and [the contents] were stirred overnight under reflux. After the reaction solution had been cooled to room temperature, concentrating was performed under reduced pressure. The resulting residue was purified by column chromatography ("Column C;" n-hexane: ethyl acetate = 75:25→70:30), and the title compound (0.7936 g) was obtained.
¹H-NMR (300MHz, CDCl₃) ; *δ* (ppm) 1.70 (9H, s), 2.65 (1H, dd, J=10.2, 12.8), 2.92 (1H , dd, J=3.2, 12.8), 3.03-3.23 (2H, m), 3. 86 (2H, dd, J=13.5, 68.9), 4.08-4.18 (2H , m), 4.71 (1H, dd, J=3.2, 10.2), 7.00 (1H , dd, J=1.8, 8.7), 7.21 (1H, dd, J=1.8, 8. 7), 7.27-7.33 (5H, m), 7.54-7.63 (3H, m) , 8.00 (1H, dd, J=2.1, 6.9)
LCMS: 585 [M + H]; Retention time: 2.05 min; LCMS conditions: C

### [Reference Example 107]

### (R)-tert-butyl 6-(2-((2-(3-amino-4-fluorophenyl)-2-hydroxyethyl)(tert-butoxycarbonyl)amino)ethoxy)-3-chloroindazole-1-carboxylate

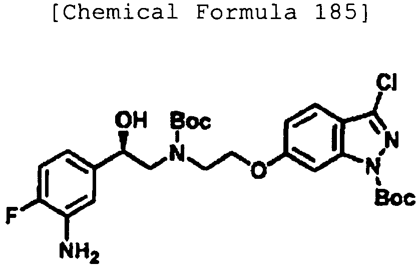

(R)-tert-butyl 6-(2-(benzyl(2-(4-fluoro-3-nitrophenyl)-2-hydroxyethyl)amino)ethoxy)-3-chloroindazole-1-carboxylate (785.3 mg), which can be manufactured by the method described in Reference Example 106 and the like, and 10% palladium carbon (PE-type, containing 50% water) (175.8 mg; made by N. E. Chemcat Corporation) were suspended in 0.1 mol/L hydrochloric acid-ethanol solution (27 mL; made by Kanto Chemical Co., Inc.). The interior of the reaction system was then purged using hydrogen to create a hydrogen atmosphere, and [the contents] were stirred for one hour at room temperature. After the interior of the reaction system had been purged using nitrogen, [the contents were] filtered. After adding triethylamine (752 µL; made by Kanto Chemical Co., Inc.) to the filtrate, concentrating was performed under reduced pressure. The resulting residue was dissolved in CH₂Cl₂ (10 mL) and methanol (10 mL). Boc₂O (294 µL; made by Wako Pure Chemical Industries Co., Ltd.) was added and [the contents] were stirred overnight at room temperature. After the reaction solution had been concentrated under reduced pressure, the residue obtained was purified by column chromatography ("Column B;" n-hexane: ethyl acetate = 71:29→50:50), and the title compound (604.4 mg) was obtained.
¹H-NMR (300MHz, CDCl₃); *δ* (ppm) 1.49 (9H, s), 1.70 (9H, s), 3.36-3.74 (4H, m), 4.11 -4.32 (2H, m), 4.90 (1H, brs), 6.68-6.97 (4H, m), 7.53 (1H, d, J=8.7), 7.62 (1H, br s)
LCMS: 565 [M + H]; Retention time: 2.05 min; LCMS conditions: C

### [Reference Example 108]

### (R)-tert-butyl 6-(2-(benzyl(2-(4-chloro-3-nitrophenyl)-2-hydroxyethyl)amino)ethoxy-3-chloroindazole-1-carboxylate

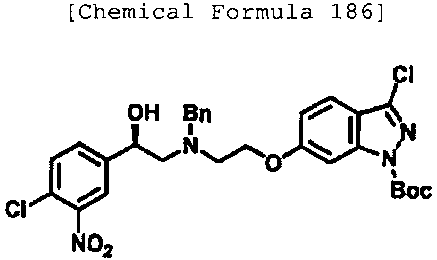

tert-Butyl 6-(2-benzylamino)ethoxy) 3-chloroindazole-1-carboxylate (1.91 g), which can be manufactured by the method described in Reference Example 105 and the like, (R)-2-(4-chloro-3-nitrophenyl)oxirane (971.8 mg), and 2-propanol (8 mL) were added and [the contents] were stirred overnight under reflux. After the reaction solution had been cooled to room temperature, concentrating was performed under reduced pressure. The resulting residue was purified by column chromatography ("Column C;" n-hexane: ethyl acetate = 90:10→75:25), and the title compound (0.9411 g) was obtained.
¹H-NMR (300MHz, CDCl₃); *δ* (ppm) 1. 70 (9H, s), 2. 64 (1H, dd, J=10.2, 13.1), 2.92 (1H , dd, J=3.6, 13.1), 3.01-3.23 (2H, m), 3. 85 (2H, dd, J=13.5, 67.8), 4.06-4.19 (2H , m), 4.70 (1H, dd, J=3.6, 10.2), 6.98 (1H , dd, J=2.1, 8.7), 7.27-7.35 (5H, m), 7.4 2-7.49 (2H, m), 7.56 (1H, d, J=8.7), 7.62 (1H, d, J=2.1), 7.83 (1H, d, J=1.4)
LCMS: 601 [M + H]; Retention time: 5.88 min; LCMS conditions: C

### [Reference Example 109]

### (R)-tert-butyl 6-(2-((2-(3-amino-4-chlorophenyl)-2-hydroxyethyl)(benzyl)amino)ethoxy)-3-chloroindazole-1-carboxylate

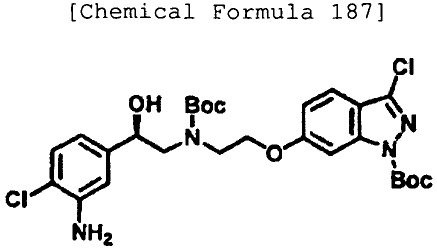

(R)-tert-butyl 6-(2-(benzyl(2-(4-chloro-3-nitrophenyl)-2-hydroxyethyl)amino)ethoxy)-3-chloroindazole-1-carboxylate (931.5 mg), which can be manufactured by the method described in Reference Example 108 and the like, and CM-101 catalyst (2.0962 g; made by N. E. Chemcat Corporation) were suspended in methanol (10 mL) and THF (10 mL). The interior of the reaction system was then purged using hydrogen to create a hydrogen atmosphere, and [the contents] were stirred overnight at room temperature. After the interior of the reaction system had been purged using nitrogen, [the contents were] filtered. After the reaction solution had been concentrated under reduced pressure, CH₂Cl₂ was added to the residue obtained. The organic layer was dried using anhydrous sodium sulfate. The solvent was distilled off under reduced pressure, and the title compound (707 mg) was obtained as a crude product.
LCMS: 571 [M + H]; Retention time: 2.02 min; LCMS conditions: C

### [Reference Example 110]

### (R)-tert-butyl 6-(2-((2-(3-amino-4-chlorophenyl)-2-hydroxyethyl)(tert-butoxycarbonyl)amino)ethoxy)-3-chloroindazole-carboxylate

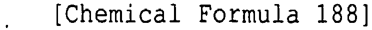

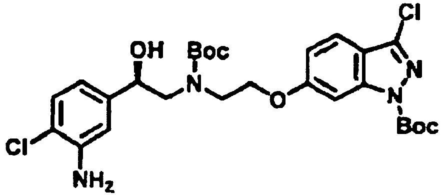

(R)-tert-butyl 6-(2-((2-(3-amino-4-chlorophenyl)-2-hydroxyethyl)(benzyl)amino)ethoxy)-3-chloroindazole-1-carboxylate (707 mg), which can be manufactured by the method described in Reference Example 109 and the like, and 10% palladium carbon (PE-type, containing 50% water) (157.3 mg; made by N. E. Chemcat Corporation) were suspended in 0.1 mol/L hydrochloric acid-ethanol solution (24.6 mL; made by Kanto Chemical Co., Inc.). The interior of the reaction system was then purged using hydrogen to create a hydrogen atmosphere, and [the contents] were stirred for 20 minutes at room temperature. After the interior of the reaction system had been purged using nitrogen, [the contents were] filtered. After adding triethylamine (684 µL; made by Kanto Chemical Co., Inc.) to the filtrate, concentrating was performed under reduced pressure. The resulting residue was dissolved in CH₂Cl₂ (10 mL). Boc₂O (266 µL; made by Wako Pure Chemical Industries Co., Ltd.) was added and [the contents] were stirred for three days at room temperature. After the reaction solution had been concentrated under reduced pressure, the residue obtained was purified by column chromatography ("Column B;" n-hexane: ethyl acetate = 75:25→54:46), and the title compound (406.9 mg) was obtained. ¹H-NMR (300MHz, CDCl₃); *δ* (ppm) 1·48 (9H, s), 1.70 (9H, s) 3.33-3.76 (4H, m), 4.06-4.24 (2H, m), 4.90 (1H, brs), 6.68-6.97 ( 3H, m), 7.20 (1H, d, J=8.0), 7.53 (1H, d, J =8.7), 8.04 (1H, brs)
LCMS: 581 [M + H]; Retention time: 2.14 min; LCMS conditions: C

### [Reference Example 111]

### tert-Butyl 6-(2-(dibenzylamino)ethoxy) 3-(trifluoromethyl)-indazole-1-carboxylate

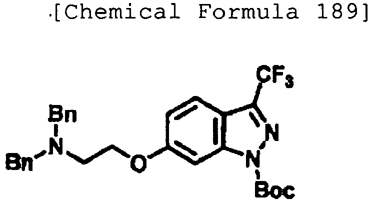

The title compound (9.6091 g) was obtained by the same method as in Reference Example 90 by using tert-butyl 6-hydroxy-3-(trifluoromethyl)-indazole-1-carboxylate (6.056 g), which can be manufactured by the method described in Reference Example 50 and the like, instead of tert-butyl 6-hydroxy-3-methylindazole-1-carboxylate.
¹H-NMR (300MHz, CDCl₃); *δ* (ppm) 1.71 (9H, s), 2.96 (2H, t, J=5.8), 3.73 (4H, s), 4.1 4 (2H, t, J=5.8), 6.97 (1H, dd, J=2.1, 8.7 ), 7.20-7.41(10H, m), 7.59 (1H, d, J=2.1 ), 7.65 (1H, d, J=8.7)
LCMS: 526 [M + H]; Retention time: 2.44 min; LCMS conditions: C

### [Reference Example 112]

### tert-Butyl 6-(2-(benzylamino)ethoxy) 3-(trifluoromethyl)-indazole-1-carboxylate

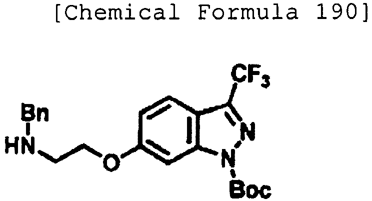

The title compound (6.6701 g) was obtained by the same method as in Reference Example 91 by using tert-butyl 6-(2-(dibenzylamino)ethoxy) 3-(trifluoromethyl)-indazole-1-carboxylate (8.3435 g), which can be manufactured by the method described in Reference Example 111 and the like, instead of tert-butyl 6-(2-(dibenzylamino)ethoxy) 3-methylindazole-1-carboxylate.
¹H-NMR (300MHz, CDCl₃); *δ* (ppm) 1.72 (9H, s), 3.09 (2H, t, J=5.1), 3.89 (2H, s), 4.2 0 (2H, t, J=5.1), 7.03 (1H, dd, J=2.1, 9.1 ), 7.27-7.37 (5H, m), 7.66-7.68 (2H, m)
LCMS: 436 [M + H]; Retention time: 1.37 min; LCMS conditions: C

### [Reference Example 113]

### (R)-tert-butyl 6-(2-(benzyl(2-(4-fluoro-3-nitrophenyl)-2-hydroxyethyl)amino)ethoxy)-3-(trifluoromethyl)-indazole-1-carboxylate

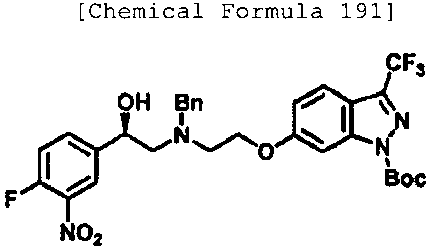

The title compound (828.1 mg) was obtained by the same method as in Reference Example 92 by using tert-butyl 6-(2-(benzylamino)ethoxy) 3-(trifluoromethyl)-indazole-1-carboxylate (1.7941 g), which can be manufactured by the method described in Reference Example 112 and the like, instead of tert-butyl 6-(2-(benzylamino)ethoxy) 3-methylindazole-1-carboxylate.
¹H-NMR (300MHz, CDCl₃) ; *δ* (ppm) 1.72 (9H, s), 2.66 (1H, dd, J=10.2, 12.8), 2.92 (1H , dd, J=3.2, 12.8), 3.03-3.23 (2H, m), 3. 86 (2H, dd, J=13.4, 69.2), 4.12-4.18 (2H , m), 4.71 (1H, dd, J=3.2, 10.2), 7.05 (1H , dd, J=2.1, 8.7), 7.23 (1H, d, J=8.7), 7. 27-7.36 (5H, m), 7.57 (1H, ddd, J=2.1, 4. 0, 8.7), 7.66 (1H, d, J=2.1), 7.69 (1H, d, J=8.7), 8.01 (1H, dd, J=2.1, 6.9)
LCMS: 619 [M + H]; Retention time: 2.23 min; LCMS conditions: C

### [Reference Example 114]

### (R)-tert-butyl 6-(2-((2-(3-amino-4-fluorophenyl)-2-hydroxyethyl)(tert-butoxycarbonyl)amino)ethoxy)-3-(trifluoromethyl)-indazole-1-carboxylate

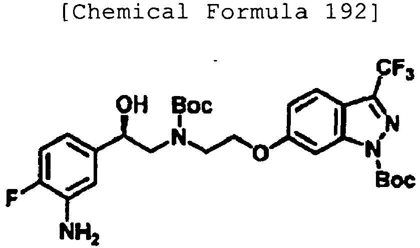

The title compound (681.5 mg) was obtained by the same method as in Reference Example 93 by using (R)-tert-butyl 6-(2-(benzyl(2-(4-fluoro-3-nitrophenyl)-2-hydroxyethyl)amino)ethoxy)-3-(trifluoromethyl)-indazole-1-carboxylate (1.4356 g), which can be manufactured by the method described in Reference Example 113 and the like, instead of (R)-tert-butyl 6-(2-(benzyl(2-(4-fluoro-3-nitrophenyl)-2-hydroxyethyl)amino)ethoxy)-3-methylindazole-1-carboxylate.
LCMS: 599 [M + H]; Retention time: 2.11 min; LCMS conditions: C

### [Reference Example 115]

### (R)-tert-butyl 6-(2-(benzyl(2-(4-chloro-3-nitrophenyl)-2-hydroxyethyl)amino)ethoxy)-3-(trifluoromethyl)-indazole-1-carboxylate

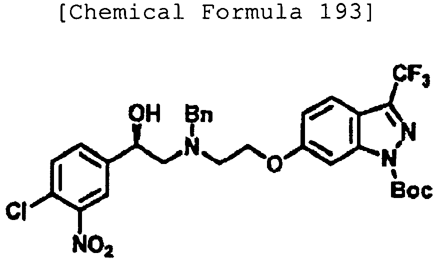

The title compound (1.4533 g) was obtained by the same method as in Reference Example 94 by using tert-butyl 6-(2-(benzylamino)ethoxy) 3-(trifluoromethyl)-indazole-1-carboxylate (3.5926 g), which can be manufactured by the method described in Reference Example 112 and the like, instead of tert-butyl 6-(2-(benzylamino)ethoxy) 3-methylindazole-1-carboxylate.
¹H-NMR (300MHz, CDCl₃); *δ* (ppm) 1.72 (9H, s), 2.65 (1H, dd, J=10.2, 12.8), 2.92 (1H , dd, J=3.6, 12.8), 3.01-3.19 (2H, m), 3. 86 (2H, dd, J=13.5, 68.1), 4.13-4.17 (2H , m), 4.70 (1H, dd, J=3.6, 10.2), 7.04 (1H , d, J=1.8, 8.7), 7.27-7.42 (5H, m), 7.45 -7.46 (2H, m), 7.66 (1H, d, J=1.8), 7.70 ( 1H, d, J=8.7), 7.83 (1H, d, J=1.4)
LCMS: 635 [M + H]; Retention time: 2.32 min; LCMS conditions: C

### [Reference Example 116]

### (R)-tert-butyl 6-(2-((2-(3-amino-4-chlorophenyl)-2-hydroxyethyl)(benzyl)amino)ethoxy)-3-(trifluoromethyl)-indazole-1-carboxylate

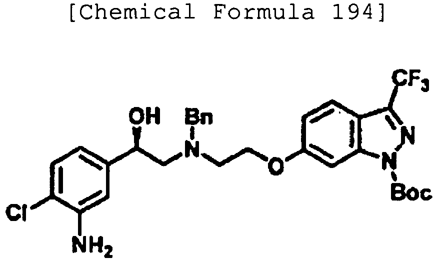

The title compound was obtained as a crude product (1.5143 g) by the same method as in Reference Example 95 by using (R)-tert-butyl 6-(2-(benzyl(2-(4-chloro-3-nitrophenyl)-2-hydroxyethyl)amino)ethoxy)-3-(trifluoromethyl)-indazole-1-carboxylate (2.045 g), which can be manufactured by the method described in Reference Example 115 and the like, instead of (R)-tert-butyl 6-(2-(benzyl(2-(4-chloro-3-nitrophenyl)-2-hydroxyethyl)amino)ethoxy)-3-methylindazole-1-carboxylate.
¹H-NMR (300MHz, CDCl₃) ; *δ* (ppm) 1.71 (9H, s), 2.72-2.75 (1H, m), 2.87 (1H, dd, J=3. 2, 12.8), 3.08-3.19 (2H, m), 3.90 (2H, dd , J=13.9, 73.6), 4.15-4.18 (2H, m), 4.65 (1H, d, J=6.5), 6.59 (1H, dd, J=1.8, 8.4) . 6.78 (1H, d, J=1.8), 7.04 (1H, dd, J=1.8 , 9.1), 7.15 (1H, d, J=8.4), 7.27-7.35 (5 H, m) 7.64 (1H, d, J=1.8), 7.67 (1H, d, J= 9.1)
LCMS: 605 [M + H]; Retention time: 1.88 min; LCMS conditions: C

### [Reference Example 117]

### (R)-tert-butyl 6-(2-((2-(3-amino-4-chlorophenyl)-2-hydroxyethyl)(tert-butoxycarbonyl)amino)ethoxy)-3-(trifluoromethyl)-indazole-carboxylate

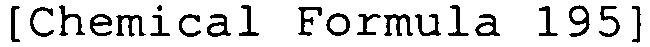

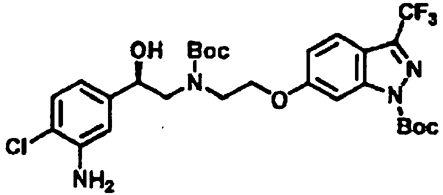

The title compound (0.747 g) was obtained by the same method as in Reference Example 96 by using (R)-tert-butyl 6-(2-((2-(3-amino-4-chlorophenyl)-2-hydroxyethyl)(benzyl)amino)ethoxy)-3-(trifluoromethyl)-indazole-1-carboxylate (1.5033 g), which can be manufactured by the method described in Reference Example 116 and the like, instead of (R)-tert-butyl 6-(2-((2-(3-amino-4-chlorophenyl)-2-hydroxyethyl)(benzyl)amino)ethoxy)-3-methylindazole-1-carboxylate.
¹H-NMR (300MHz, CDCl₃) ; *δ* (ppm) 1.49 (9H, s), 1.71 (9H, s), 3.59-3.66 (4H, m), 4.05 -4.21 (2H, m), 4.90 (1H, brs), 6.68 (1H, b rs), 6.85 (1H, brs), 7.03 (1H, brs), 7.21 (1H, d, J=8.0), 7.67-7.70 (2H, m)
LCMS: 615 [M + H]; Retention time: 2.20 min; LCMS conditions: C

### [Reference Example 118]

### (R)-tert-butyl 6-(2-((2-(3-(3-aminophenylsulfonamide)phenyl)-2-(triethylsilyloxy)ethyl)(tert-butoxycarbonyl)amino)ethoxy)-3-methylindazole-1-carboxylate

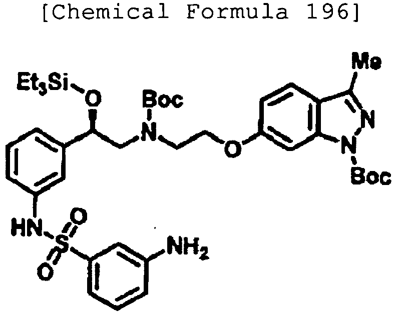

(R)-tert-butyl 6-(2-((2-(3-aminophenyl)-2-(triethylsilyloxy)ethyl)(tert-butoxycarbonyl)amino)ethoxy)-3-methylindazole-1-carboxylate (192.8 mg), which can be manufactured by the method described in Reference Example 1 and the like, was dissolved in dehydrated CH₂Cl₂ (4 mL). Pyridine (40 µL; made by Kanto Chemical Co., Inc.) and 3-nitrobenzene-1-sulfonyl chloride (86.9 mg; made by Wako Pure Chemical Industries Co., Ltd.) were added and [the contents] were stirred overnight at room temperature. The reaction solution was purified by column chromatography ("Column B;" n-hexane: ethyl acetate = 74:26→53:47), and (R)-tert-butyl 6-(2-(tert-butoxycarbonyl(2-(3-(3-nitrophenylsulfonamide)phenyl)-2-(triethylsilyloxy)ethyl)amino)ethoxy)-3-methylindazole-1-carboxylate (229.9 mg) was obtained. 5% Palladium carbon (STD-type, containing 50% water) (89.6 mg; made by N. E. Chemcat Corporation) and methanol (1.5 mL) were then added, the interior of the reaction system was made into a hydrogen atmosphere, and [the contents] were stirred overnight at room temperature. After the reaction solution had been placed under a nitrogen atmosphere and ethyl acetate added (2 mL), [the reaction solution] was filtered. The filtrate was concentrated under reduced pressure, and the title compound was obtained as a crude product (191.3 mg).
LCMS: 796 [M + H]; Retention time: 2.45 min; LCMS conditions: C

### [Reference Example 119]

### Ethyl 6-(2-(((R)-2-(3-aminophenyl)-2-(triethylsilyloxy)ethyl)(tert-butoxycarbonyl)amino)ethoxy)-1-(tetrahydro-2H-pyran-2-yl)indazole-3-carboxylate

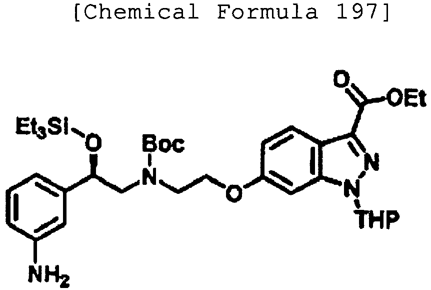

The title compound (2.0364 g) was obtained by the same method as in Reference Example 1 using ethyl 6-hydroxy-1-(tetrahydro-2H-pyran-2-yl)indazole-3-carboxylate (837.8 mg), which can be manufactured by the method described in Reference Example 73 and the like, instead of tert-butyl 6-hydroxy-3-methylindazole-1-carboxylate.
LCMS: 683 [M + H]; Retention time: 1.69 min; LCMS conditions: E

### [Reference Example 120]

### Ethyl 6-(2-(tert-butoxycarbonyl((R)-2-(3-(cyclobutanesulfonamide)phenyl)-2-(triethylsilyloxy)ethyl)amino)ethoxy)-1-(tetrahydro-2H-pyran-2-yl)indazole-3-carboxylate

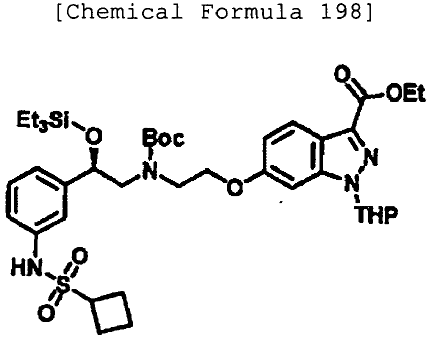

Ethyl 6-(2-(((R)-2-(3-aminophenyl)-2-(triethylsilyloxy)ethyl)(tert-butoxycarbonyl)amino)ethoxy)-1-(tetrahydro-2H-pyran-2-yl)indazole-3-carboxylate (2.0215 g), which can be manufactured by the method described in Reference Example 119 and the like, was dissolved in dehydrated CH₂Cl₂ (20 mL; made by Kanto Chemical Co., Inc.). Dehydrated pyridine (1.21 mL; made by Kanto Chemical Co., Inc.) and cyclobutane sulfonyl chloride (1.428 g; made by Hande Sciences) were added and [the contents] were stirred overnight at room temperature. The reaction solution was washed twice with 1 mol/L aqueous hydrochloric acid and once with brine. After the organic layer was dried using anhydrous sodium sulfate, the solvent was distilled off under reduced pressure. The resulting residue was purified by column chromatography ("Column B;" n-hexane: ethyl acetate = 64:36→43:57), and the title compound (1.3314 g) was obtained.
LCMS: 801 [M + H]; Retention time: 1.78 min; LCMS conditions: E

### [Reference Example 121]

### 6-(2-(tert-Butoxycarbonyl((R)-2-(3-(cyclobutanesulfonamide)phenyl)-2-hydroxyethyl)amino)ethoxy-1-(tetrahydro-2H-pyran-2-yl)indazole-3-carboxylic acid

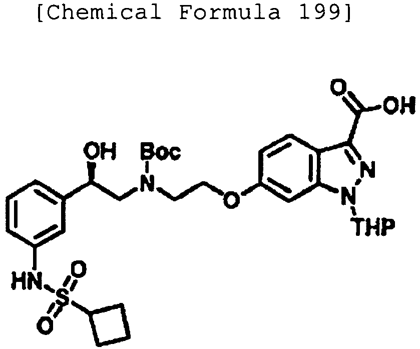

6-(2-(tert-butoxycarbonyl((R)-2-(3-(cyclobutanesulfonamide)phenyl)-2-(triethylsilyloxy)ethyl)amino)ethoxy)-1-(tetrahydro-2H-pyran-2-yl)indazole-3-carboxylate (1.3215 g), which can be manufactured by the method described in Reference Example 120 and the like, was dissolved in methanol (20 mL) and THF (12 mL). 2 mol/L aqueous sodium hydroxide (8.3 mL; made by Kanto Chemical Co., Inc.) was added and [the contents] were stirred overnight at 40°C. The reaction solution was concentrated under reduced pressure. After adding water to the residue obtained, washing was performed twice using diethyl ether. 1 mol/L aqueous hydrochloric acid was added to the water layer, and it was extracted twice with ethyl acetate after making the pH acidic. The organic layer was washed once with brine. After drying had been performed using anhydrous magnesium sulfate, the solvent was distilled off under reduced pressure, and the title compound (868.3 mg) was obtained.
LCMS: 659 [M + H]; Retention time: 1.57 min; LCMS conditions: C

### [Reference Example 122]

### tert-Butyl (R)-2-(3-(cyclobutanesulfonamide)phenyl)-2-hydroxyethyl (2-(3-hydroxymethyl)-1-(tetrahydro-2H-pyran-2-yl)indazol-6-yloxy)ethyl)carbamate

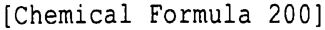

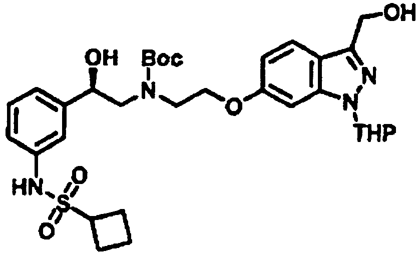

6-(2-(tert-Butoxycarbonyl((R)-2-(3-(cyclobutanesulfonamide)phenyl)-2-hydroxyethyl)amino)ethoxy)-1-(tetrahydro-2H-pyran-2-yl)indazole-3-carboxylic acid (441.9 mg), which can be manufactured by the method described in Reference Example 121 and the like, was dissolved in dehydrated THF (6.7 mL; made by Kanto Chemical Co., Inc.). After cooling to 0°C, 2 mol/L BH₃·SMe₂-toluene solution (2 mL; made by Aldrich Co.) was added and [the contents] were stirred for four hours while warming to room temperature. 2 mol/L BH₃·SMe₂-toluene solution (1 mL; made by Aldrich Co.) was added to the reaction solution and stirring was performed overnight at room temperature. The reaction solution was cooled to 0°C. After adding methanol (1.5 mL) and water (30 mL), the water layer was extracted twice using ethyl acetate. The organic layer was washed once with water and once with brine. After drying had been performed using anhydrous magnesium sulfate, the solvent was distilled off under reduced pressure. The resulting residue was partially purified by column chromatography ("Column B;" n-hexane: ethyl acetate = 54:46→33:67), whereupon the resulting partially purified substance was purified again using column chromatography ("Column B;" n-hexane: ethyl acetate = 36:64→15:85), and the title compound (23.3 mg) was obtained.
LCMS: 645 [M + H]; Retention time: 1.54 min; LCMS conditions: C

### [Working Example 1] "Route A"

### (R)-3-amino-N-(3-(1-hydroxy-2-(2-(3-methoxyindazol-6-yloxy)ethylamino)ethyl)phenyl)benzenesulfonamide

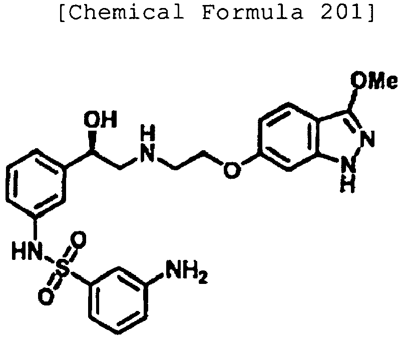

(R)-tert-butyl 6-(2-((2-(3-aminophenyl)-2-(triethylsilyloxy)ethyl)(tert-butoxycarbonyl)amino)ethoxy)-3-methoxyindazole-1-carboxylate (100.5 mg), which can be manufactured by the method described in Reference Example 2 and the like, was dissolved in dehydrated CH₂Cl₂ (1.5 mL). Pyridine (20 µL; made by Kanto Chemical Co., Inc.) and 3-nitrobenzene-1-sulfonyl chloride (38.7 mg; made by Wako Pure Chemical Industries Co., Ltd.) were added and [the contents] were stirred overnight at room temperature. The reaction solution was purified by column chromatography ("Column B;" n-hexane: ethyl acetate = 81:19→60:40), and (R)-tert-butyl 6-(2-(tert-butoxycarbonyl(2-(3-(3-nitrophenylsulfonamide)phenyl)-2-(triethylsilyloxy)ethyl)amino)ethoxy)-3-methoxyindazole-1-carboxylate (128.3 mg) was obtained. 5% Palladium carbon (STD-type, containing 50% water) (60.7 mg; made by N. E. Chemcat Corporation) and methanol (2 mL) were then added, and the interior of the reaction system was placed under a hydrogen atmosphere and stirred overnight at room temperature. The reaction solution was placed under a nitrogen atmosphere and filtered. The filtrate was concentrated under reduced pressure, and the residue obtained was purified by column chromatography ("Column B;" n-hexane: ethyl acetate = 88:12→67:33), and (R)-tert-butyl 6-(2-((2-(3-(3-aminophenylsulfonamide)phenyl)-2-(triethylsilyloxy)ethyl)(tert-butoxycarbonyl)amino)ethoxy)-3-methoxyindazole-1-carboxylate (72 mg) was obtained. It was then dissolved in MTBE (0.2 mL). 4 mol/L hydrochloric acid-1,4-dioxane solution (1.5 mL; made by Kokusan Chemical Co., Ltd.) and 10% hydrochloric acid-methanol solution (0.6 mL; made by Tokyo Chemical Industry Co., Ltd.) were added and shaken (600 min⁻¹) overnight at room temperature. Nitrogen gas was blown into the reaction solution, the solvent was driven off, and the title compound was obtained as a hydrochloride (56.5 mg).
LCMS: 498 [M + H]; Retention time: 0.98 min; LCMS conditions: C

### [Working Example 2] "Route B"

### (R)-N-(3-(1-hydroxy-2-(2-(3-methoxyindazol-6-yloxy)ethylamino)ethyl)phenyl)thiophene-2-sulfonamide

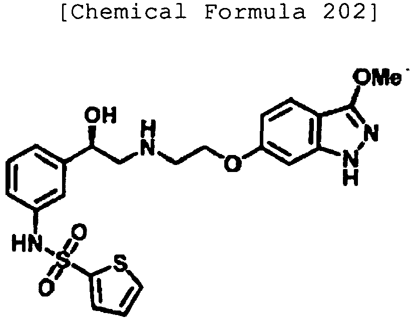

(R)-tert-butyl 6-(2-((2-(3-aminophenyl)-2-(triethylsilyloxy)ethyl)(tert-butoxycarbonyl)amino)ethoxy)-3-methoxyindazole-1-carboxylate (99 mg), which can be manufactured by the method described in Reference Example 2 and the like, was dissolved in dehydrated CH₂Cl₂ (0.5 mL) . Dehydrated pyridine (18 µL; made by Kanto Chemical Co., Inc.), thiophene-2-sulfonyl chloride (68 mg; made by Aldrich Co.), and dehydrated CH₂Cl₂ (0.5 mL) were added and [the contents] were stirred overnight at room temperature. Nitrogen gas was blown into the reaction solution, and the solvent was driven off. The resulting residue was purified by column chromatography ("Column G;" n-hexane: ethyl acetate), and (R)-tert 6-(2-(tert-butoxycarbonyl(2-(3-(thiophene-2-sulfonamide)phenyl)-2-(triethylsilyloxy)ethyl)amino)ethoxy)-3-methoxyindazole-1-carboxylate was obtained. 4 mol/L hydrochloric acid-1,4-dioxane solution (1.5 mL; made by Kokusan Chemical Co., Ltd.) was then added and shaken (600 min⁻¹) overnight at room temperature. Nitrogen gas was blown into the reaction solution, and the solvent was driven off. The title compound was obtained as a hydrochloride (62.0 mg).
LCMS: 489 [M + H]; Retention time: 1.02 min; LCMS conditions: C

### [Working Example 3] "Route C"

### (R)-N-(3-(1-hydroxy-2-(2-(3-methylindazol-6-yloxy)ethylamino)ethyl)phenyl)cyclobutanesulfonamide

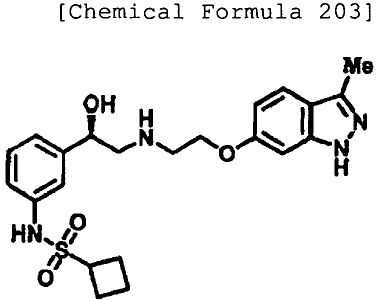

(R)-tert-butyl 6-(2-((2-(3-aminophenyl)-2-(triethylsilyloxy)ethyl)(tert-butoxycarbonyl)amino)ethoxy)-3-methylindazole-1-carboxylate (67.1 mg), which can be manufactured by the method described in Reference Example 1 and the like, was dissolved in dehydrated CH₂Cl₂ (0.5 mL). Pyridine (48 µL; made by Kanto Chemical Co., Inc.) and cyclobutanesulfonyl chloride-CH₂Cl₂ solution [0.5 mL; solution prepared by dissolving cyclobutanesulfonyl chloride (61.3 mg), which can be manufactured by the method described in Reference Example 19 and the like, in dehydrated CH₂Cl₂ (0.5 mL)] were added and shaken (600 min⁻¹) overnight at room temperature. The reaction solution was purified by column chromatography ("Column B;" n-hexane: ethyl acetate = 74:26→53:47), and a crude product of (R)-tert-butyl 6-(2-(tert-butoxycarbonyl(2-(3-(cyclobutanesulfonamide)phenyl)-2-(triethylsilyloxy)ethyl)amino)ethoxy)-3-methylindazole-1-carboxylate was obtained. It was then dissolved in ethyl acetate. After washing with 1 mol/L aqueous hydrochloric acid, the organic layer was dried using anhydrous sodium sulfate. The organic layer was placed under reduced pressure, and the solvent was distilled off, and (R)-tert-butyl 6-(2-(tert-butoxycarbonyl(2-(3-(cyclobutanesulfonamide)phenyl)-2-(triethylsilyloxy)ethyl)amino)ethoxy)-3-methylindazole-1-carboxylate (41.4 mg) was obtained. It was then dissolved in 1,4-dioxane solution (1.5 mL; made by Kokusan Chemical Co., Ltd.) and shaken (600 min⁻¹) overnight at room temperature. Nitrogen gas was blown into the reaction solution, and the solvent was driven off. The title compound was obtained as a hydrochloride (34.5 mg).
¹H-NMR (300MHz, DMSO-d₆) ; *δ* (ppm) 1.81-1 .98 (2H, m), 2.08-2.20 (2H, m), 2.25-2.3 8 (2H, m), 2.45 (3H, s), 3.03-3.07 (1H, m) , 3.23 (1H, brs), 3.40-3.47 (2H, m), 3.89 (1H, qu, J=8.0), 4.35-4.36 (2H, m), 4.99 (1H, d, J=8.0), 6.78 (1H, dd, J=1.8, 8.4) , 6.91 (1H, d, J=1.8), 7.09-7.14 (2H, m), 7.28-7.34 (2H, m), 7.61 (1H, d, J=8.4), 9 .00 (1H, brs), 9.31(1H, brs), 9.78 (1H, s )
LCMS: 445 [M + H]; Retention time: 0.98 min; LCMS conditions: C

### [Working Example 4] "Route D"

### (R)-N-(3-(2-(2-(3-(difluoromethoxy)-indazol-6-yloxy)ethylamino)-1-hydroxyethyl)phenyl)cyclopropanesulfonamide

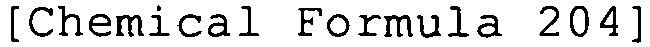

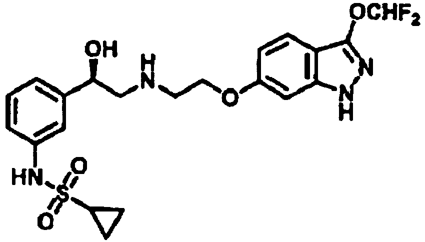

(R)-tert-butyl 6-(2-((2-(3-aminophenyl)-2-(triethylsilyloxy)ethyl)(tert-butoxycarbonyl)amino)ethoxy)-3-(difluoromethoxy)-indazole-1-carboxylate (108.1 mg), which can be manufactured by the method described in Reference Example 3 and the like, was dissolved in dehydrated CH₂Cl₂ (1 µL). Pyridine (83 µL; made by Kanto Chemical Co., Inc.) and cyclopropanesulfonyl chloride-CH₂Cl₂ solution [1 mL; solution prepared by dissolving cyclopropanesulfonyl chloride (107.9 mg; made by Matrix) in dehydrated CH₂Cl₂ (1 mL)] were added and [the contents] were stirred overnight at room temperature. The reaction solution was purified by column chromatography ("Column B;" n-hexane: ethyl acetate = 74:26→53:47), and (R)-tert-butyl 6-(2-(tert-butoxycarbonyl(2-(3-(cyclopropanesulfonamide)phenyl)-2-(triethylsilyloxy)ethyl)amino)ethoxy)-3-(difluoromethoxy)-indazole-1-carboxylate (122.5 mg) was obtained. It was then dissolved in 1,4-dioxane solution (0.2 mL). 4 mol/L hydrochloric acid-1,4-dioxane solution (1.5 mL; made by Kokusan Chemical Co., Ltd.) was added and shaken (600 min⁻¹) overnight at room temperature. Nitrogen gas was blown into the reaction solution, and the solvent was driven off. The title compound was obtained as a hydrochloride (81.8 mg).
¹H-NMR (300MHz, DMSO-d₆) ; *δ* (ppm) 0.91-0 .94 (4H, m), 2.57-2.65 (1H, m), 3.02-3.0 9 (1H, m), 3.23-3.27 (1H, m), 3.47 (2H, br s), 4.36-4.38 (2H, m), 4.99 (1H, d, J=10. 9), 6.24 (1H, d, J=3.6), 6.84 (1H, dd, J=1 .8, 8.7), 6.92 (1H, d, J=1.8), 7.12 (1H, d , J=7.6), 7.17 (1H, d, J=8.7), 7.33 (1H, t, =7.6), 7.47 (1H, t, J=72.9), 7.54 (1H, d, J =8.7), 9.00 (1H, brs), 9.22 (1H, brs), 9. 81 (1H, s), 12.54 (1H, s)
LCMS: 483 [M + H]; Retention time: 1.06 min; LCMS conditions: C

### [Working Example 5] "Route E"

### (R)-2-fluoro-N-(3-(1-hydroxy-2-(2-(3-methoxyindazol-6-yloxy)ethylamino)ethyl)phenyl)benzenesulfonamide

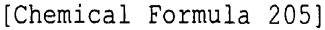

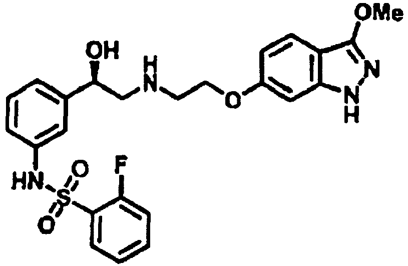

Pyridine (25 µL; made by Kanto Chemical Co., Inc.), 2-fluorobenzene-1-sulfonyl chloride-CH₂Cl₂ solution [0.5 mL; solution prepared by dissolving 2-fluorobenzene-1-sulfonyl chloride (121.4 mg; made by Aldrich Co.) in dehydrated CH₂Cl₂ (2.5 mL)], and dehydrated CH₂Cl₂ (1 mL) were added to (R)-tert-butyl 6-(2-((2-(3-aminophenyl)-2-(triethylsilyloxy)ethyl)(tert-butoxycarbonyl)amino)ethoxy)-3-methoxyindazole-1-carboxylate-CH₂Cl₂ solution [0.5 mL; solution prepared by dissolving (R)-tert-butyl 6-(2-((2-(3-aminophenyl)-2-(triethylsilyloxy)ethyl)(tert-butoxycarbonyl)amino)ethoxy)-3-methoxyindazole-1-carboxylate (2.02 g), which can be manufactured by the method described in Reference Example 2 and the like, in dehydrated CH₂Cl₂ (15 mL)] and shaken overnight at room temperature. PS-Trisamine [200 mg (3.6 mmol/g); made by Argonaut] was added to the reaction solution and shaken for 5.5 hours at room temperature. The reaction solution was filtered. Nitrogen gas was blown into the filtrate, and the solvent was driven off. The resulting residue was purified by column chromatography ("Column I;" methanol). The purified product obtained was dissolved in dehydrated 1,4-dioxane (0.2 mL). 4 mol/L hydrochloric acid-1,4-dioxane solution (1.5 mL; made by Kokusan Chemical Co., Ltd.) was added and shaken overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (47.7 mg).
LCMS: 501 [M + H]; Retention time: 1.03 min; LCMS conditions: C

### [Working Example 6] "Route F"

### (R)-5-amino-2-fluoro-N-(3-(1-hydroxy-2-(2-(3-methoxyindazol-6-yloxy)ethylamino)ethyl)phenyl)benzenesulfonamide

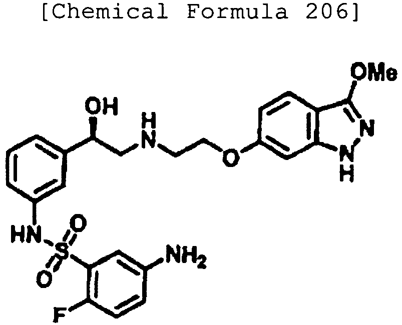

(R)-tert-butyl 6-(2-((2-(3-aminophenyl)-2-(triethylsilyloxy)ethyl)(tert-butoxycarbonyl)amino)ethoxy)-3-methoxyindazole-1-carboxylate (69.1 mg), which can be manufactured by the method described in Reference Example 2 and the like, was dissolved in dehydrated CH₂Cl₂ (1 mL). Pyridine (24 µL; made by Kanto Chemical Co., Inc.) and 2-fluoro-5-nitrobenzene-1-sulfonyl chloride-CH₂Cl₂ solution [0.5 mL; solution prepared by dissolving 2-fluoro-5-nitrobenzene-1-sulfonyl chloride (263.4 mg; made by Chemcollect) in dehydrated CH₂Cl₂ (3.5 mL)] were added and shaken overnight (600 rpm⁻¹) at room temperature. PS-Trisamine [200 mg (4.06 mmol/g); made by Biotage] was added to the reaction solution and shaken for seven hours at room temperature. The reaction solution was filtered. Nitrogen gas was blown into the filtrate, and the solvent was driven off. The resulting residue was purified by column chromatography ("Column I;" methanol). 5% Palladium carbon (STD-type, containing 50% water) (20 mg; made by N. E. Chemcat Corporation) and methanol (2 mL) were added to the purified product (69.1 mg) obtained. The interior of the reaction system was placed under a hydrogen atmosphere and stirred for seven hours at room temperature. The interior of the reaction system was placed under a nitrogen atmosphere and filtered. Nitrogen gas was blown into the filtrate, and the solvent was driven off. The residue (61.5 mg) obtained was dissolved in dehydrated 1,4-dioxane (0.2 mL). 4 mol/L hydrochloric acid-1,4-dioxane solution (1.5 mL; made by Kokusan Chemical Co., Ltd.) and 10% hydrochloric acid-methanol solution (0.2 mL; made by Tokyo Chemical Industry Co., Ltd.) were added and shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (54.9 mg).
LCMS: 516 [M + H]; Retention time: 0.98 min; LCMS conditions: C

### [Working Example 7] "Route G"

### (R)-3-amino-4-chloro-N-(3-(1-hydroxy-2-(2-(3-methylindazol-6-yloxy)ethylamino)ethyl)phenyl)benzenesulfonamide

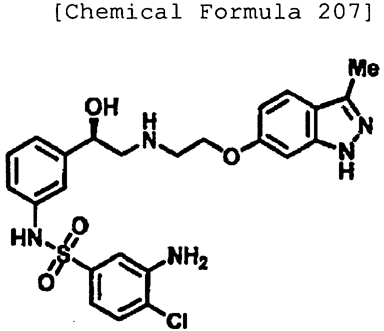

(R)-tert-butyl 6-(2-((2-(3-aminophenyl)-2-(triethylsilyloxy)ethyl)(tert-butoxycarbonyl)amino)ethoxy)-3-methylindazole-1-carboxylate (92.0 mg), which can be manufactured by the method described in Reference Example 1 and the like, was dissolved in dehydrated CH₂Cl₂ (1.5 mL). Pyridine (20 µL; made by Kanto Chemical Co., Inc.) and 4-chloro-3-nitrobenzene-1-sulfonyl chloride (47.2 mg; made by Tokyo Chemical Industry Co., Ltd.) were added and [the contents] were stirred overnight at room temperature. The reaction solution was purified by column chromatography ("Column B;" n-hexane: ethyl acetate = 74:26→53:47), and (R)-tert-butyl 6-(2-(tert-butoxycarbonyl(2-(3-(4-chloro-3-nitrophenylsulfonamide)phenyl)-2-(triethylsilyloxy)ethyl)amino)ethoxy)-3-methylindazole-1-carboxylate (109.7 mg) was obtained. CM-101 catalyst (244.1 mg; made by N. E. Chemcat Corporation), THF (1 mL), and methanol (0.5 mL) were then added. The interior of the reaction system was placed under a hydrogen atmosphere and stirred overnight at room temperature. After placing the interior of the reaction system under a nitrogen atmosphere, [the contents were] filtered. The filtrate was placed under reduced pressure, and the solvent was distilled off. CM-101 catalyst (239.2 mg; made by N. E. Chemcat Corporation), THF (1 mL), and methanol (0.5 mL) were added to the residue obtained. The interior of the reaction system was placed under a hydrogen atmosphere and stirred for four days at room temperature. After placing the interior of the reaction system under a nitrogen atmosphere, CM-101 catalyst (239.2 mg; made by N. E. Chemcat Corporation) was added to the reaction solution and stirred overnight. After placing the interior of the reaction system under a nitrogen atmosphere, [the contents were] filtered. The filtrate was placed under reduced pressure, and the solvent was distilled off. The resulting residue was purified by column chromatography ("Column B;" n-hexane: ethyl acetate = 53:47→32:68), and (R)-tert-butyl 6-(2-((2-(3-amino-4-chlorophenylsulfonamide)phenyl)-2-hydroxyethyl)(tert-butoxycarbonyl)amino)ethoxy)-3-methylindazole-1-carboxylate (21.1 mg) was obtained. It was then dissolved in dehydrated MTBE (50 µL). 4 mol/L hydrochloric acid-1,4-dioxane solution (1 mL; made by Kokusan Chemical Co., Ltd.) and 10% hydrochloric acid-methanol solution (100 µL; made by Tokyo Chemical Industry Co., Ltd.) were added and shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (23.9 mg).
LCMS: 516 [M + H]; Retention time: 1.05 min; LCMS conditions: C

### [Working Example 8] "Route H"

### (R)-N-(2-chloro-5-(1-hydroxy-2-(2-(3-methoxyindazol-6-yloxy)ethylamino)ethyl)-phenyl)thiophene-2-sulfonamide

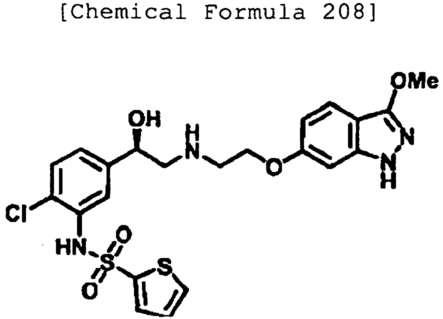

(R)-tert-butyl 6-(2-((2-(3-amino-4-chlorophenyl)-2-(triethylsilyloxy)ethyl)(tert-butoxycarbonyl)amino)ethoxy)-3-methoxyindazole-1-carboxylate-CH₂Cl₂ solution [0.5 mL; solution prepared by dissolving (R)-tert-butyl 6-(2-((2-(3-amino-4-chlorophenyl)-2-(triethylsilyloxy)ethyl)(tert-butoxycarbonyl)amino)ethoxy)-3-methoxyindazole-1-carboxylate (2.101 g), which can be manufactured by the method described in Reference Example 14 and the like, in dehydrated CH₂Cl₂ (15 mL)], dehydrated pyridine (42 µL), thiophene-2-sulfonyl chloride-CH₂Cl₂ solution [0.5 mL; solution prepared by dissolving thiophene-2-sulfonyl chloride (502.3 mg; made by Aldrich Co.) in dehydrated CH₂Cl₂ (4 mL)], and dehydrated CH₂Cl₂ (1 mL) were added and shaken overnight at room temperature. PS-Trisamine [300 mg (3.6 mmol/g); made by Argonaut] was added to the reaction solution and shaken for five hours at room temperature. The reaction solution was filtered. Nitrogen gas was blown into the filtrate, and the solvent was driven off. The resulting residue was purified by column chromatography ("Column I;" methanol). The purified product obtained was dissolved in 1,4-dioxane (0.2 mL). 4 mol/L hydrochloric acid-1,4-dioxane solution (1.5 mL; made by Kokusan Chemical Co., Ltd.) was added and [the contents] were shaken overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (19.8 mg).
LCMS: 523 [M + H]; Retention time: 0.97 min; LCMS conditions: C

### [Working Example 9] "Route I"

### (R)-3-amino-N-(2-chloro-5-(1-hydroxy-2-(2-(3-methoxyindazol-6-yloxy)ethylamino)ethyl)phenyl)benzenesulfonamide

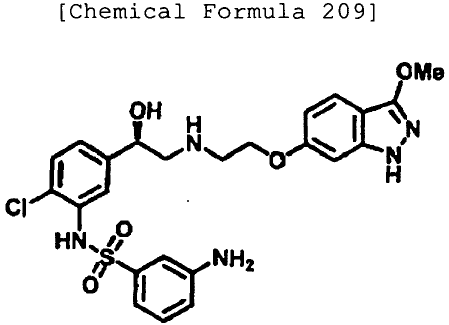

(R)-tert-butyl 6-(2-((2-(3-amino-4-chlorophenyl)-2-(triethylsilyloxy)ethyl)(tert-butoxycarbonyl)amino)ethoxy)-3-methoxyindazole-1-carboxylate-CH₂Cl₂ solution [0.5 mL; solution prepared by dissolving (R)-tert-butyl 6-(2-((2-(3-amino-4-chlorophenyl)-2-(triethylsilyloxy)ethyl)(tert-butoxycarbonyl)amino)ethoxy)-3-methoxyindazole-1-carboxylate (2.101 g), which can be manufactured by the method described in Reference Example 14 and the like, in dehydrated CH₂Cl₂ (15 mL)], dehydrated pyridine (42 µL), 3-nitrobenzenesulfonyl chloride-CH₂Cl₂ solution [0.5 mL; solution prepared by dissolving 3-nitrobenzenesulfonyl chloride (585.1 mg; made by Wako Pure Chemical Industries Co., Ltd.) in dehydrated CH₂Cl₂ (4 mL)], and dehydrated CH₂Cl₂ (1 mL) were added and shaken overnight at room temperature. PS-Trisamine [300 mg (3.6 mmol/g); made by Argonaut] was added to the reaction solution and shaken for five hours at room temperature. The reaction solution was filtered. Nitrogen gas was blown into the filtrate, and the solvent was driven off. The resulting residue was purified by column chromatography ("Column I;" methanol). The purified product obtained was dissolved in THF (2 mL). Rhodium carbon (20 mg; made by Aldrich Co.) and iron(II) acetate (2 mg; made by Wako Pure Chemical Industries Co., Ltd.) were added. The interior of the reaction system was placed under a hydrogen atmosphere and stirred overnight at room temperature. The reaction solution was placed under a nitrogen atmosphere and filtered. Nitrogen gas was blown into the filtrate, and the solvent was driven off. The resulting residue was purified by column chromatography ("Column E;" n-hexane: ethyl acetate = 1:1→0:1). The purified product obtained was then dissolved in 1,4-dioxane (0.2 mL). 4 mol/L hydrochloric acid-dioxane solution (1.5 mL) was added and shaken overnight at room temperature. Nitrogen gas was blown into the reaction solution, and the solvent was driven off. The resulting residue was purified by HPLC. The purified product was then dissolved in EtOH (1 mL). 1 mol/L hydrochloric acid-Et₂O solution (0.4 mL; made by Tokyo Chemical Industry Co., Ltd.) was added. Nitrogen gas was blown into the reaction solution, and the solvent was driven off. The title compound was obtained as a hydrochloride (18.2 mg).
LCMS: 532 [M + H]; Retention time: 0.95 min; LCMS conditions: C

### [Working Example 10] "Route J"

### (R)-3-fluoro-N-(3-(1-hydroxy-2-(2-(3-methylindazol-6-yloxy)ethylamino)ethyl)phenyl)benzenesulfonamide

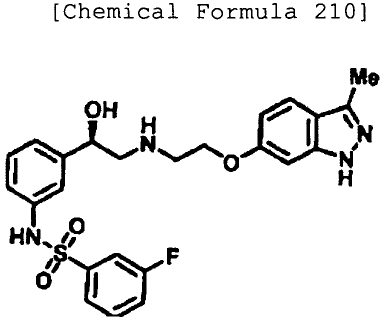

(R)-tert-butyl 6-(2-((2-(3-aminophenyl)-2-(triethylsilyloxy)ethyl)(tert-butoxycarbonyl)amino)ethoxy)-3-methylindazole-1-carboxylate (96 mg), which can be manufactured by the method described in Reference Example 1 and the like, was dissolved in dehydrated CH₂Cl₂ (0.5 mL). Dehydrated pyridine (17 µL; made by Kanto Chemical Co., Inc.), 3-fluorobenzene-1-sulfonyl chloride (36 mg; made by Aldrich Co.), and dehydrated CH₂Cl₂ (0.5 mL) were added and [the contents] were stirred overnight at room temperature. Nitrogen gas was blown into the reaction solution, and the solvent was driven off. The resulting residue was purified by column chromatography ("Column G;" n-hexane: ethyl acetate). The purified product obtained was dissolved in 1,4-dioxane (0.2 mL). 4 mol/L hydrochloric acid-1,4-dioxane solution (1.6 mL; made by Kokusan Chemical Co., Ltd.) was added and shaken overnight at room temperature. Nitrogen gas was blown into the reaction solution, and the solvent was driven off. Dehydrated CH₂Cl₂ (1 mL), triethylamine(25 µL; made by Kokusan Chemical Co., Ltd.), and Boc₂O (27 µL; made by Wako Pure Chemical Industries Co., Ltd.) were added to the residue obtained and shaken (600 rpm⁻¹) for 20 minutes at room temperature. The reaction solution was purified by column chromatography ("Column B;" n-hexane: ethyl acetate = 41:59→20:80). The purified product obtained was dissolved in 1,4-dioxane (0.1 mL), and 4 mol/L hydrochloric acid-1,4-dioxane solution (1 mL; made by Kokusan Chemical Co., Ltd.) was added and shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (62.0 mg).
LCMS: 485 [M + H]; Retention time: 1.02 min; LCMS conditions: C

### [Working Examples 11-234]

The abbreviations in Table 1 mean the following.

"ex" means the working example number. For example, "ex11" means "Working Example 11."

"reagent-1" shows the reagent used. "ref" means the reference example number. For example, "ref-2" represents "Reference Example 2."

"reagent-2" means the reagent used. For example, "SO₂Cl-1" shows that reagent "1" described in Table 2 was used.

"Route" means the production process. For example, one listed as "B" shows that it was produced by the same method as "Route B."

"Route A" shows that it was produced by the same method as in Working Example 1.

"Route B" shows that it was produced by the same method as in Working Example 2.

"Route C" shows that it was produced by the same method as in Working Example 3.

"Route D" shows that it was produced by the same method as in Working Example 4.

"Route E" shows that it was produced by the same method as in Working Example 5.

"Route F" shows that it was produced by the same method as in Working Example 6.

"Route G" shows that it was produced by the same method as in Working Example 7.

"Route H" shows that it was produced by the same method as in Working Example 8.

"Route I" shows that it was produced by the same method as in Working Example 9.

"Route J" shows that it was produced by the same method as in Working Example 10.

However, with regard to Working Example 199, the compound was produced by stirring for 40 minutes at 80°C in a sealed microwave reactor instead of at room temperature in the production process of "Route D."

"LCMS" shows the liquid chromatography-mass spectrum data (m/z). Specifically, these consist of the "method," "R.T.," and "MS" discussed below.

"MS" means the mass spectrum data. "R.T." means the retention time in LCMS. The unit is minutes. "method" means the LCMS conditions discussed above. For example, one listed as "C" shows the conditions of "LCMS-C."

[Table 1]

**Table 1**

| ex | reagent-1 | reagent-2 | Route | Compound | Compound Name | LCMS | | |
|---|---|---|---|---|---|---|---|---|
| | | | | | | MS | R.T. | method |
| 11 | ref-2 | RSO₂Cl-1 | B | 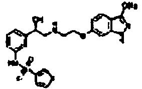 | (R)-N-(3-(1-hydroxy-2-(2-(3-methoxyindazol-6-yloxy)ethylamino)ethyl)phenyl)th iophene-3-sulfonamide | 489 [M+H] | 0.98 | C |
| 12 | ref-2 | RSO₂Cl-2 | B | 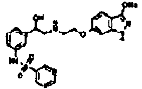 | (R)-N-(3-(1-hydroxy-2-(2-(3-methoxyindazol-6-yloxy)ethylamine(ethyl)phenyl)py ridine-3-sulfunamide | 484 [M+H] | 0.91 | C |
| 13 | ref-2 | RSO₂Cl-3 | B | 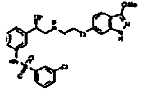 | (R)-3-chloro-N-(3-(1-hydroxy-2-(2-(8-methoxyindazol-6-yloxy)ethylamino)ethyl)phenyl)be nzenesulfonamido | 517 [M+H] | 1.12 | C |
| 14 | ref-2 | RSO₂Cl-4 | B | 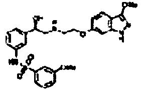 | (R)-N-(3-(1-hydroxy-2-(2-(3-methoxyindazol-6-yloxy)ethylamino)ethyl)phenyl)-8-methoxybezonesulfonamido | 513 [M+H] | 1.07 | C |
| 15 | ref-2 | RSO₂Cl-5 | B | 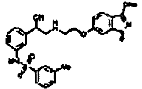 | (R)-N-(3-(1-hydroxy-2-(2-(3-methoxyindazol-6-yloxy)ethylamino)ethyl)phenyl)-3-methylbenzenesulfonamide | 497 [M+H] | 1.10 | C |
| 16 | ref-2 | RSO₂Cl-6 | D | 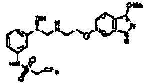 | (R)-2,2,2-trifluoro-N-(3-(1-hydroxy-2-(2-(3-methoxyindazol-6-yloxy)ethylamino)ethyl)phenyl)et hanesulfonamido | 489 [M+H] | 0.97 | C |
| 17 | ref-2 | RSO₂Cl-7 | D | 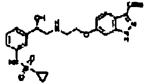 | (R)-N-(3-(1-hydroxy-2-(2-(3-methoxyindazol-6-yloxy)ethylamino)ethyl)phenyl)cy clopropanesulfonamide | 447 [M+H] | 0.90 | C |
| 18 | ref-2 | RSO₂Cl-8 | E | 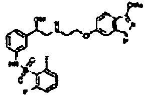 | (R)-2,6-difluoro-N-(3-(1-hydroxy-2-(2-(3-methoxyindazol-6-yloxy)ethylamino)ethyl)phenyl)be nzanesulfonamide | 519 [M+H] | 1.04 | C |
| 19 | ref-2 | RSO₂Cl-11 | E | 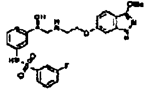 | (R)-3-fluoro-N-(3-(1-hydroxy-2-(2-(3-methoxyindazol-6-yloxy)ethylamino)ethyl)phenyl)be nzensulfonamide | 501 [M+H] | 1.04 | C |
| 20 | ref-2 | RSO₂Cl-13 | E | 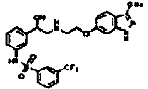 | (R)-N-(3-(1-hydroxy-2-(2-(3-methoxyndazol-6-yloxy)ethylamino)ethyl)phenyl)-8-(trifluoromethyl)-benzensulfonamide | 551 [M+H] | 1.2 | C |
| 21 | ref-2 | RSO₂Cl-14 | E | 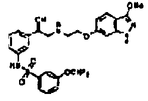 | (R)-3-(difluoromethoxy)-N-(3-(1-hydroxy-2-(2-(3-methoxyindazol-6-yloxy)ethylamino)ethyl)phenyl)be nzenesulfonamide | 549 [M+H] | 1.12 | C |
| 22 | ref-2 | RSO₂Cl-15 | E | 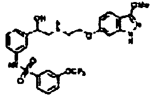 | (R)-N-(3-(1-hydroxy-2-(2-(3-methoxyindazole-6-yloxy)ethylamino)ethyl)phenyl)-3-(trifluoromethoxy)-benzenesulfonamide | 567 [M+H] | 1.22 | C |
| 23 | ref-2 | RSO₂Cl-16 | B | 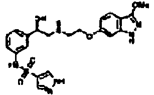 | (R)-N-(3-(1-hydroxy-2-(2-(3-methoxyindazol-6-yloxy)ethylamino)ethyl)phenyl)py razole-4-sulfonamide | 473 [M+H] | 0.88 | C |
| 24 | ref-2 | RSO₂Cl-17 | B | 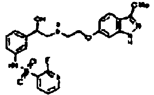 | (R)-2-fluoro-N-(3-(1-hydroxy-2-(2-(3-methoxyindazol-6-yloxy)ethylamino)ethyl)phenyl)py ridine-3-sulfonamide | 502 [M+H] | 1.01 | C |
| 25 | ref-13 | RSO₂Cl-18 | H | 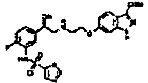 | (R)-N-(2-fluoro-5-(1-hydroxy-2-(2-(3-methoxyindazol-6-yloxy)ethylamino)ethyl)phenyl)th iophene-2-sulfonamide | 507 [M+H] | 0.94 | C |
| 26 | ref-13 | RSO₂Cl-1 | H | 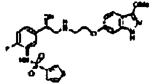 | (R)-N-(2-fluoro-5-(1-hydroxy-2-(2-(3-methoxyindazol-6-yloxy)ethylamino)ethyl)phenyl)th iophene-3-sulfonamide | 507 [M+H] | 0.95 | C |
| 27 | ref-13 | RSO₂Cl-10 | H | 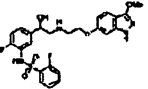 | (R)-2-fluoro-N-(2-fluoro-5-(1-hydroxy-2-(2-(3-methoxyindazol-6-yloxy)ethylamino)-ethyl)phenyl)benzene-sulfonamide | 519 [M+H] | 0.94 | C |
| 28 | ref-13 | RSO₂Cl-19 | I | 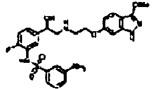 | (R)-3-amino-N-(2-fluoro-5-(1-hydroxy-2-(2-(3-methoxyindazol-6-yloxy)ethylamino)ethyl)phenyl)be benzenesulfonamide | 516 [M+H] | 0.90 | C |
| 29 | ref-14 | RSO₂Cl-1 | H | 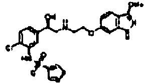 | (R)-N-(2-chloro-5-(1-hydroxy-2-(2-(3-methoxyindazol-6-yloxy)ethylamino)ethyl)phenyl)th iophene-3-sulfonamide | 523 [M+H] | 0.97 | C |
| 30 | ref-14 | RSO₂Cl-10 | H | 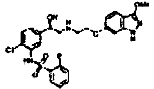 | (R)-N-(2-choro-5-(1-hydroxy-2-(2-(R)-N-(2-choro-5-(1-hydroxy-2-(2-(3-methoxyindazol-6-yloxy)ethylamino)ethyl)phenyl-2-fluorobenzenesulfonamide | 535 [M+H] | 1.03 | C |
| 31 | ref-14 | RSO₂Cl-9 | I | 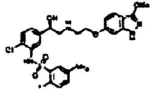 | (R)-5-amino-N-(2-chloro-3-(1-hydroxy-2-(2-(3-methoxyindazol-6-yloxy)ethylamino)ethyl)phenyl)-2-fluorobenzenesulfonamide | 550 [M+H] | 1.01 | C |
| 32 | ref-2 | RSO₂Cl-20 | E | 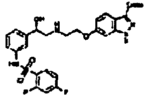 | (R)-2,4-difluoro-N-(3-(1-hydroxy-2-(2-(3-methoxyindazol-6-yloxy)ethylamino)ethyl)phenyl)be nzenesulfonamide | 519 [M+H] | 0.99 | C |
| 33 | ref-2 | RSO₂Cl-21 | E | 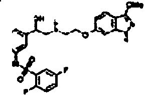 | (R)-2,5-difluoro-N-(3-(1-hydroxy-2-(2-(3-methoxyindazol-6-yloxy)ethylamino)ethyl)phenyl)be nzenesulfonamido | 519 [M+H] | 1.07 | C |
| 34 | ref-2 | RSO₂Cl-22 | E | 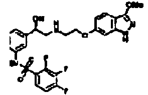 | (R)-2,3,4-trifluoro-N-(3-(1-hydroxy-2-(2-(3-methoxyindazol-6-yloxy)ethylamino)ethyl)phenyl)be nzenesulfonamide | 537 [M+H] | 1.04 | C |
| 35 | ref-2 | RSO₂Cl-23 | E | 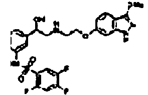 | (R)-2,4,5-trifluoro-N-(3-(1-hydroxy-2-(2-(3-methoxyindazol-6-yloxy)ethylamino)ethyl)phenyl)be nzenesulfonamide | 537 [M+H] | 1.09 | C |
| 36 | ref-2 | RSO₂Cl-12 | E | 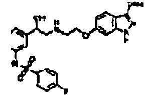 | (R)-4-fluoro-N-(3-(1-hydroxy-2-(2-(3-methoxyindazol-6-yloxy)ethylamino)ethyl)phenyl)be nzenesulfonamide | 501 [M+H] | 0.97 | C |
| 37 | ref-2 | RSO₂Cl-24 | B | 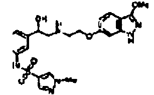 | (R)-N-(3-(1-hydroxy-2-(2-(3-methoxyindazol-6-yloxy)-ethylamino)ethyl)phenyl)-1-methylpyrazole-4-sulfonamide | 487 [M+H] | 0.73 | C |
| 38 | ref-2 | RSO₂Cl-25 | C | 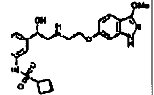 | (R)-N-(3-(1-hydroxy-2-(2-(3-methoxyindazol-6-yloxy)ethylamino)ethyl)phenyl)ey clobutanesulfonamide | 461 [M+H] | 0.88 | C |
| 39 | ref-2 | RSO₂Cl-26 | B | 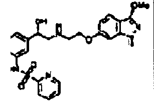 | (R)-N-(3-(1-hydroxy-2-(2-(3-methoxyindazol-6-yloxy)ethylamino)ethyl)phenyl)py ridine-2-sulfonamide | 484 [M+H] | 0.87 | C |
| 40 | ref-1 | RSO₂Cl-19 | B | 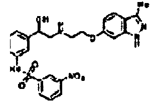 | (R)-N-(3-(1-hydroxy-2-(2-3-methylindazol-6-yloxy)ethylamino)ethyl)phenyl)-3-nitrobenzenesulfonamide | 512 [M+H] | 0.99 | C |
| 41 | ref-1 | RSO₂Cl-19 | A | 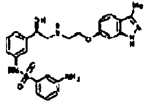 | (R)-3-amino-N-(3-(1-hydroxy-2-(2-(3-methylindazol-6-yloxy)ethylamino)phenyl)be nzenesulfonamide | 482 [M+H] | 0.91 | C |
| 42 | ref-1 | RSO₂Cl-3 | B | 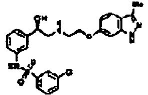 | (R)-3-chloro-N-(3-(1-hydroxy-2-(2-(3-methylindazol-6-yloxy)ethylamino)ethyl)phenyl)be nzenesulfonamide | 501 [M+H] | 1.02 | C |
| 43 | ref-1 | RSO₂Cl-4 | B | 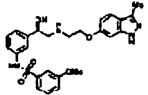 | (R)-N-(3-(1-hydroxy-2-(2-(3-methylindazol-6-yloxy)ethylamino)ethyl)phenyl)-3-methoxybenzenesulfonamide | 497 [M+H] | 0.99 | C |
| 44 | ref-1 | RSO₂Cl-2 | B | 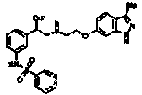 | (R)-N-(3-(1-hydroxy-2-(2-(3-methylindazol-6-yloxy)ethylamino)ethyl)phenyl)py ridine-3-sulfonamide | 468 [M+H] | 0.87 | C |
| 45 | ref-1 | RSO₂Cl-18 | B | 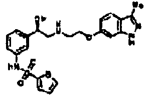 | (R)-N-(3-(1-hydroxy-2-(2-(3-methylindazol-6-yloxy)ethylamino)ethyl)phenyl)th iophene-2-sulfonamide | 473 [M+H] | 0.99 | C |
| 46 | ref-1 | RSO₂Cl-1 | B | 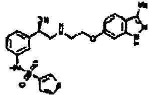 | (R)-N-(3-(1-hydroxy-2-(2-(3-methylindazol-6-yloxy)ethylamino)ethyl)phenyl)th iophene-3-sulfonamide | 473 [M+H] | 0.91 | C |
| 47 | ref-1 | RSO₂Cl-7 | D | 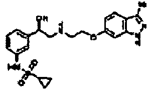 | (R)-N-(3-(1-hydroxy-2-(2-(3-methylindazol-6-yloxy)ethylamino)ethyl)phenyl)cy clopropanesulfonamide | 431 [M+H] | 0.83 | C |
| 48 | ref-1 | RSO₂Cl-6 | D | 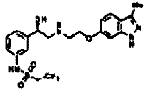 | (R)-2,2,2-trifluoro-N-(3-(1-hydroxy-2-(2-(3-methylindazol-6-yloxy)ethylamino)ethyl)phenyl)et hanesulfonamide | 473 [M+H] | 0.95 | C |
| 49 | ref-1 | RSO₂Cl-5 | B | 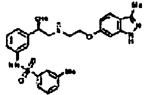 | (R)-N-(3-(1-hydroxy-2-(2-(3-methylindazol-6-yloxy)ethylamino)ethyl)phenyl)-3-mehtylbenzenesulfonamide | 481 [M+H] | 1.03 | C |
| 50 | ref-1 | RSO₂Cl-27 | B | 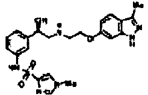 | (R)-N-(3-(1-hydroxy-2-(2-(3-methylindazol-6-yloxy)ethylamino)ethyl)phenyl)-1-methylimidazole-4-sulfonamide | 471 [M+H] | 0.82 | C |
| 51 | ref-1 | RSO₂Cl-28 | B | 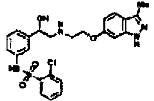 | (R)-2-chloro-N-(3-(1-hydoxy-2-(2-(3-methylindazol-6-yloxy)ethylamino)ethyl)phenyl)be nzenesulfonamide | 501 [M+H] | 1.04 | C |
| 52 | ref-1 | RSO₂Cl-29 | B | 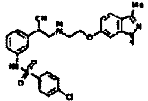 | (R)-4-chloro-N-(3-(1-hydroxy-2-(2-(3-methylindazol-6-yloxy)ethylamino)ethyl)phenyl)be nzenesulfonamide | 501 [M+H] | 1.12 | C |
| 53 | ref-1 | RSO₂Cl-30 | A | 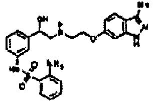 | (R)-2-amino-N-(3-(1-hydroxy-2-(2-(3-methylindazol-6-yloxy)ethylamino)ethyl)phenyl)be nzenesulfonamide | 482 [M+H] | 0.99 | C |
| 54 | ref-1 | RSO₂Cl-31 | A | 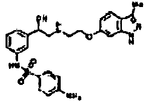 | (R)-4-amino-N-(3-(1-hydroxy-2-(2-(3-methylindazol-6-yloxy)ethylamino)ethyl)phenyl)be nzenesulfonamide | 482 [M+H] | 0.92 | C |
| 55 | ref-1 | RSO₂Cl-32 | B | 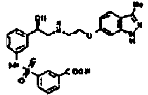 | (R)-3-(N-(3-(1-hydroxy-2-(2-(3-methylindazol-6-yloxy)ethylamino)ethyl)phenyl)su lfamoyl)benzoic acid | 511 [M+H] | 0.95 | C |
| 56 | ref-1 | RSO₂Cl-33 | A | 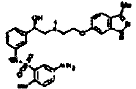 | (R)-5-amino-N-(3-(1-hydroxy-2-(2-(3-methylindazol-6-yloxy)ethylamino)ethyl)phenyl)-2-methylbenzenesulfonamide | 496 [M+H] | 0.96 | C |
| 57 | ref-1 | RSO₂Cl-34 | A | 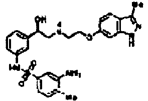 | (R)-3-amino-N-(3-(1-hydroxy-2-(2-(3-methylindazol-6-yloxy)ethylamino)ethyl)phenyl)-4-methylbenzenesulfonamide | 498 [M+H] | 0.96 | C |
| 58 | ref-1 | RSO₂Cl-35 | A | 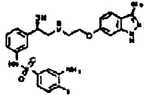 | (R)-3-amino-4-fluoro-N-(3-(1-hydroxy-2-(2-(3-methylindazol-6-yloxy)ethylamino)ethyl)phenyl)be nzenesulfonamide | 500 [M+H] | 0.96 | C |
| 59 | ref-1 | RSO₂Cl-36 | B | 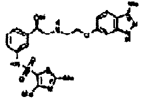 | (R)-N-(3-(1-hydroxy-2-(2-(3-methylindazol-6-yloxy)ethylamino)ethyl)phenyl)-2,4-dimethylthiazole-5-sulfonamide | 502 [M+H] | 0.98 | C |
| 60 | ref-1 | RSO₂Cl-37 | B | 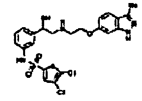 | (R)-4,5-dichloro-N-(3-(1-hydroxy-2-(2-(3-methylindazol-6-yloxy)ethylamino)ethyl)phenyl)th iophene-2-sulfonamide | 541 [M+H] | 1.26 | C |
| 61 | ref-1 | RSO₂Cl-9 | A | 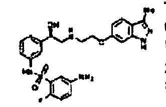 | (R)-5-amino-2-fluoro-N-(3-(1-hydroxy-2-(2-(3-methylindazol-6-yloxy)ethylamino)ethyl)phenyl)be nzenesulfonamide | 500 [M+H] | 0.90 | C |
| 62 | ref-1 | RSO₂Cl-12 | J | 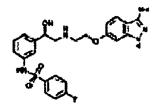 | (R)-4-fluoro-N-(3-(1-hydroxy-2-(2-(3-methylindazol-5-yloxy)ethylamino)ethyl)phenyl)be nzenesulfonamide | 485 [M+H] | 0.98 | C |
| 63 | ref-1 | RSO₂Cl-38 | J | 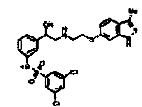 | (R)-3,5-dichloro-N-(3-(1-hydroxy-2-(2-(3-methylindazol-6-yloxy)ethylamino)ethyl)phenyl)be nzenesulfonamide | 535 [M+H] | 1.15 | C |
| 64 | ref-1 | RSO₂Cl-26 | B | 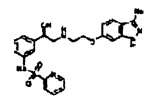 | (R)-N-(3-(1-hydroxy-2-(2-(3-methylindazol-6-yloxy)ethylamino)ethyl)phenyl)py ridine-2-sulfonamide | 408 [M+H] | 0.87 | C |
| 65 | ref-1 | RSO₂Cl-13 | J | 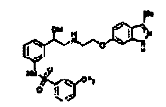 | (R)-N-(3-(1-hydroxy-2-(2-(3-methylindazol-6-yloxy)ethylamino)ethyl)phenyl)-3-(trifluoromethyl)-benzenesulfonamide | 535 [M+H] | 1.16 | C |
| 66 | ref-1 | RSO₂Cl-14 | J | 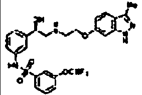 | (R)-3-(difluoromethoxy)-N-(3-(1-hydroxy-2-(2-(3-methylindazol-6-yloxy)ethylamino)ethyl)phenyl)be nzenesulfonamide | 533 [M+H] | 1.09 | C |
| 67 | ref-1 | RSO₂Cl-15 | J | 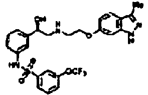 | (R)-N-(3-(1-hydroxy-2-(2-(3-methylindazol-6-yloxy)ethylamino)ethyl)phenyl)-3-(trifluoromethoxy)-benzenesulfonamide | 551 [M+H] | 1.19 | C |
| 68 | ref-1 | RSO₂Cl-39 | B | 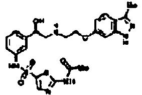 | (R)-N-(5-(N-(3-(1-hydroxy-2-(2-(3-methylindazol-6-yloxy)ethylamino)ethyl)phenyl)su lfamoyl)thiazol-2-yl)acetamide | 531 [M+H] | 0.89 | C |
| 69 | ref-1 | RSO₂Cl-8 | B | 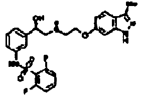 | (R)-2,6-difluoro-N-(3-(1-hydroxy-2-(2-(3-methylindazol-6-yloxy)ethylamino)ethyl)phenyl)be nzenosulfonamide | 503 [M+H] | 1.00 | C |
| 70 | ref-1 | RSO₂Cl-40 | J | 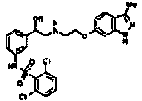 | (R)-2,6-dichloro-N-(3-(1-hydroxy-2-(2-(3-methylindazol-6-yloxy)ethylamino)ethyl)phenyl)be nzenesulfonamide | 535 [M+H] 1.10 | 1.10 | C |
| 71 | ref-1 | RSO₂Cl-20 | J | 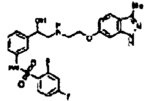 | (R)-2,3-difluoro-N-(3-(1-hydroxy-2-(2-(3-methylindazol-6-yloxy)ethylamino)ethyl)phenyl)be nzenesulfonamide | 503 [M-H] | 1.03 | C |
| 72 | ref-1 | RSO₂Cl-21 | J | 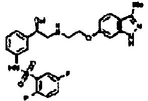 | (R)-2,5-difluoro-N-(3-(1-hydroxy-3-(2-(3-methylindazol-6-yloxy)ethylamino)ethyl)phenyl)be nzenesulfonamide | 503 [M+H] | 1.04 | C |
| 73 | ref-1 | RSO₂Cl-41 | J | 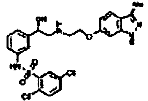 | (R)-2,5-dichloro-N-(3-(1-hydroxy-2-(2-(3-methylindazol)-6-yloxy)ethylamino)ethyl)phenyl)be nzenesulfonamide | 535 [M+H] | 1.16 | C |
| 74 | ref-1 | RSO₂Cl-42 | B | 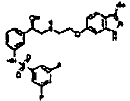 | (R)-3,5-difluoro-N-(3-(1-hydroxy-2-(2-(3-methylindazol-6-yloxy)ethylamino)ethyl)phenyl)be nzenesulfonamide | 503 [M-H] | 1.04 | C |
| 75 | ref-1 | RSO₂Cl-43 | J | 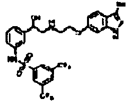 | (R)-N-(3-(1-hydroxy-2-(2-(3-methylindazol-6-yloxy)ethylamino)ethyl)phenyl)-3,5-bis(trifluoromethyl)-benzenesulfonamide | 603 [M+H] | 1.29 | C |
| 76 | ref-1 | RSO₂Cl-44 | B | 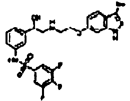 | (R)-3,4,5-trifluoro-N-(3-(1-hydroxy-2-(2-(3-methylindazol-6-yloxy)ethylamino)ethyl)-phenyl)benzenesulfonamide | 521 [M+H] | 1.12 | C |
| 77 | ref-1 | KSO₂Cl-22 | B | 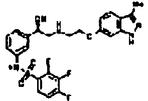 | (R)-2,3,4-trifluoro-N-(9-(1-hydroxy-2-(2-(3-methylindazol-6-yloxy)ethylamino)ethyl)phenyl)be nzensulfonamide | 521 [M+H] | 1.06 | C |
| 78 | ref-1 | RSO₂Cl-45 | B | 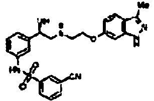 | (R)-3-cyano-N-(1-hydroxy-2-(2-(8-methylindazol-6-yloxy)ethylamino)ethyl)phenyl)be nzensulfonamide | 492 [M+H] | 1.04 | C |
| 79 | ref-1 | RSO₂Cl-24 | B | 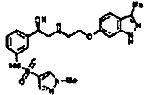 | (R)-N-(3-(1-hydroxy-2-(2-(8-methylindazol-8-yloxy)ethylamino)ethyl)phenyl)-1-methylpyrazole-4-sulfonamide | 471 [M+H] | 0.87 | C |
| 80 | ref-1 | RSO₂Cl-46 RSO₂Cl-46 | B | 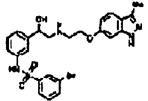 | (R)-8-bromo-N-(8-1-hydroxy-2-(2-(3-methylindazol-6-yloxy)ethylamino)ethyl)phenyl)be nzensulfonamide | 545 [M+H] | 1.10 | C |
| 81 | ref-1 | RSO₂Cl-47 | B | 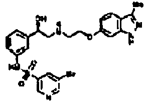 | (R)-5-bromo-N-(3-(1-hydroxy-2-(2-(8-methylindazol-6-yloxy)ethylamino)ethyl)phenyl)py ridine-8-sulfonamide | 546 [M+H] | 0.99 | C |
| 82 | ref-1 | RSO₂Cl-16 | B | 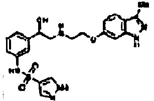 | (R)-N-(3-(1-hydroxy-2-(2-(3-methylindazol-6-yloxy)ethylamino)ethyl)phenyl)py razole-4-sulfonamide | 457 [M+H] | 0.34 | C |
| 83 | ref-1 | RSO₂Cl-48 | B | 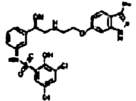 | (R)-3,5-dichloro-2-hydroxy-N-(3-(1-hydroxy-2-(2-(3-methylindazol-6-yloxy)ethylamino)ethyl)phenyl)be nzensulfonamide | 551 [M+H] | 1.15 | C |
| 84 | ref-1 | RSO₂Cl-49 | E | 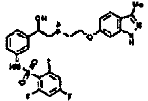 | (R)-2,4,6-trifluoro-N-(3-(1-hydroxy-2-(2-(3-methylindazol-6-yloxy)ethylamino)ethyl)phenyl)be nzensulfonamide | 521 [M+H] | 1.08 | C |
| 85 | rer-1 | RSO₂Cl-23 | E | 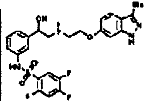 | (R)-2,4,5-trifluoro-N-(3-(1-hydroxy-2-(2-(3-methylindazol-6-yloxy)ethylamino)ethyl)phenyl)be nzensulfonamide | 521 [M+H] | 1.12 | C |
| 86 | ref-1 | RSO₂Cl-17 | B | 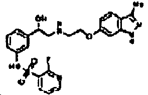 | (R)-2-fluoro-N-(3-(1-hydroxy-2-(2-(8-methylindazol-6-yloxy)ethylamino)ethyl)phenyl)py ridine-3-sulfonamide | 486 [M+H] | 1.01 | C |
| 87 | ref-11 | RSO₂Cl-18 | H | 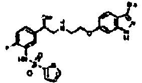 | (R)-N-(2-fluoro-5-(1-hydroxy-2-(2-(3-methylindazol-6-yloxy)ethylamino)ethyl)phenyl)th iopheno-2-sulfonamide | 491 [M+H] | 1.01 | C |
| 88 | ref-11 | RSO₂Cl-1 | H | 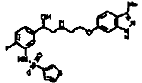 | (R)-N-(2-fluoro-5-(1-hydroxy-2-(2-(3-methylindazol-6-yloxy)ethylamino)ethyl)phenyl)th iophene-3-sulfonamide | 491 [M+H] | 0.98 | C |
| 89 | ref-11 | RSO₂Cl-10 | H | 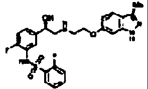 | (R)-2-fluoro-N-(2-fluoro-5-(1-hydroxy-2-(2-(3-methylindazol-6-yloxy)ethylamino)ethyl)-phenyl)benzensulfonamide | 503 [M+H] | 1.03 | C |
| 90 | ref-11 | RSO₂Cl-19 | I | 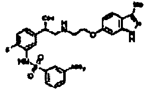 | (R)-3-amino-N-(2-fluoro-5-(1-hydroxy-2-(2-(3-methylindazol-6-yloxy)ethylamino)ethyl)phenyl)be nzenesulfonamide | 500 [M+H] | 0.87 | C |
| 91 | ref-11 | RSO₂Cl-9 | I | 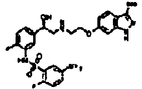 | (R)-5-amino-2-fluoro-N-(2-fluoro-5-(1-hydroxy-2-(2-(3-methylindazol-6-yloxy)ethylamino)ethyl)phenyl)be nzanesulfonamide | 518 [M+H] | 0.91 | C |
| 92 | ref-12 | RSO₂Cl-18 | H | 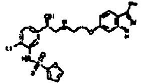 | (R)-N-(2-chloro-5-(1-hydroxy-2-(2-(3-methylindazol-6-yloxy)ethylamino)ethyl)phenyl)th iophene-2-sulfonamide | 507 [M+H] | 1.01 | C |
| 93 | ref-12 | RSO₂Cl-1 | H | 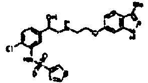 | (R)-N-(2-chloro-5-(1-hydroxy-2-(2-(3-methylindazol-6-yloxy)ethylamino)ethyl)phenyl)th iophene-3-sulfonamide | 507 [M+H] | 0.97 | C |
| 94 | ref-12 | RSO₂Cl-10 | H | 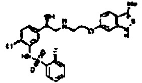 | (R)-N-(2-chloro-5-(1-hydroxy-2-(2-(3-methylindazol-6-yloxy)ethylamino)ethyl)phenyl). 2-fluorobenzenesulfonamido | 519 [M+H] | 1.04 | C |
| 95 | ref-12 | RSO₂Cl-19 | I | 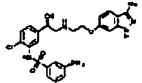 | (R)-3-amino-N-(2-chloro-5-(1-hydroxy-2-(2-(3-methylindazol-6-yloxy)ethylamino)ethyl)phenyl)be nzenesulfonamide | 516 [M+H] | 0.93 | C |
| 96 | ref-12 | RSO₂Cl-9 | I | 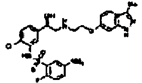 | (R)-5-amino-N-(2-ethanol-5-(1-hydroxy-2-(2-(3-methylindazol-6-yloxy)ethylamino)ethyl)phenyl)-2-fluorobenzensulfonamido | 534 [M+H] | 0.97 | C |
| 97 | ref-8 | RSO₂Cl-18 | B | 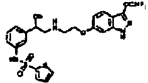 | (R)-N-(3-(2-(2-(3-(difluoromethoxy)-indazol-6-yloxy)ethylamino)-1-hydroxyethyl)phenyl)thiophene-2-sulfonamide | 525 [M+H] | 1.12 | C |
| 98 | ref-3 | RSO₂Cl-1 | B | 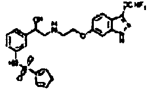 | (R)-N-(3-(2-(2-(3-(difluoromethoxy)-indazol-6-yloxy)ethylamino)-1-hydroxyethyl)phenyl)thiophene-3-sulfonamide | 525 [M+H] | 1.13 | C |
| 99 | ref-3 | RSO₂Cl-2 | B | 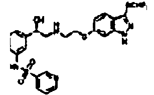 | (R)-N-(3-(2-(2-(3-(difluoromethoxy)-indazol-6-yloxy)ethylamino)-1-hydroxyethyl)phenyl)pyridine-3-sulfonamide | 520 [M+H] | 1.04 | C |
| 100 | ref-3 | RSO₂Cl-3 | B | 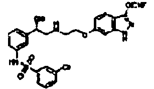 | (R)-3-chloro-N-(3-(2-(2-(3-(difluoromethoxy)-indazol-6-yloxy)ethylamino)-1-hydroxyethy)phenyl)benzenesulf onamide | 553 [M+H] | 1.26 | C |
| 101 | ref-3 | RSO₂Cl-4 | B | 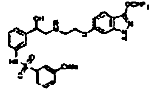 | (R)-N-(3-(2-(2-(3-(difluoromethoxy)-indazol-6-yloxy)ethylamino)-1-hydroxyethyl)phenyl)-3-methoxybenzenesulfonamide | 549 [M+H] | 1.18 | C |
| 102 | ref-3 | RSO₂Cl-3 | B | 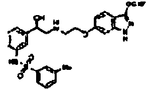 | (R)-N-(3-(2-(2-(3-(difluoromethoxy)-indazol-6-yloxy)ethylamino)-1-hydroxyethyl)phenyl)-3-methylbenzenesulfonamide | 533 [M+H] | 1.23 | C |
| 103 | ref-3 | RSO₂Cl-6 | D | 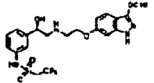 | (R)-N-(3-(2-(2-(3-(difluoromethoxy)-indazol-6-yloxy)ethylamino)-1-hydroxyethyl)phenyl)-2,2,2-trifluoroethanesulfonamide | 525 [M+H] | 1.11 | C |
| 104 | ref-3 | RSO₂Cl-19 | A | 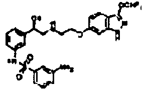 | (R)-3-amino-N-(3-(2-(2-(3-(difluoromethoxy)-indazol-6-yloxy)ethylamino)-1-hydroxyethyl)phenyl)-benzenesulfonamide | 534 [M+H] | 0.97 | C |
| 105 | ref-3 | RSO₂Cl-8 | E | 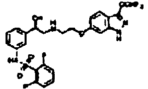 | (R)-N-(3-(2-(2-(3-(difluoromethoxy)-indazol-6-yloxy)ethylamino)-1-hydroxyethyl)phenyl)-2,6-difluorobenzenesulfonamide | 555 [M+H] | 1.14 | C |
| 106 | ref-3 | RSO₂Cl-9 | F | 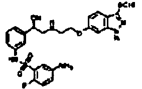 | (R)-5-amino-N-(3-(2-(2-3-(difluoromethoxy)-indazol-6-yloxy)ethylamino)-1-hydroxyethyl)phenyl)-2-fluorobenzenesulfonamide | 552 [M+H] | 1.06 | C |
| 107 | ref-3 | RSO₂Cl-10 | E | 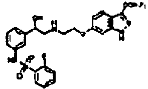 | (R)-N-(3-(2-(2-(3-(difluoromethoxy)-indazol-6-yloxy)ethylamino)-1-hydroxyethyl)phenyl)-2-fluorobenzenesulfonamide | 537 [M+H] | 1.16 | C |
| 108 | ref-3 | RSO₂Cl-11 | E | 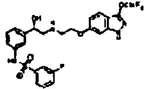 | (R)-N-(3-(2-(2-(3-(difluoromethoxy)-indazol-6-yloxy)ethylamino)-1-hydroxyethyl)phenyl)-3-fluorobenzenesulfonamide | 537 [M+H] | 1.19 | C |
| 109 | ref-3 | RSO₂Cl-12 | E | 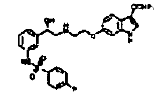 | (R)-N-(3-(2-(2-(3-(difluoromethoxy)-indazol-6-yloxy)ethylamino)-1-hydroxyethyl)phenyl)-4-fluorobenzenesulfonamide | 537 [M+H] | 1.14 | C |
| 110 | ref-3 | RSO₂Cl-13 | E | 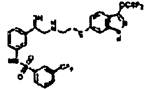 | (R)-N-(3-(2(-2-(3-(difluoromethoxy)-indazol-6-yloxy)ethylamino)-1-hydroxyethyl)phenyl)-3-(trifluoromethyl)-benzenesulfonamide | 587 [M+H] | 1.25 | C |
| 111 | ref-3 | RSO₂Cl-14 | E | 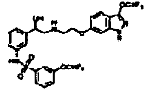 | (R)-2-(difluoromethoxy)-N-(3-(2-(2-(3-(difluoromethoxy)-indazol-6-yloxy)ethylamino)-1-hydroxyethyl)phenyl) benzenesulfonamide | 585 [M+H] | 1.26 | C |
| 112 | ref-3 | RSO₂Cl-15 | E | 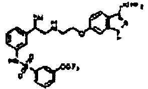 | (R)-N-(3-(2-(2-(3-(difluoromethoxy)-indazol-6-yloxy)ethylamino)-1-hydroxyethyl)phenyl)-3-(trifluoromethoxy)-benzenesulfonamide | 603 [M+H] | 1.33 | C |
| 113 | ref-3 | RSO₂Cl-16 | B | 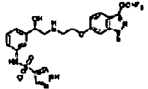 | (R)-N-(3-(2-(2-(3-(difluoromethoxy)-indazol-6-yloxy)ethylamino)-1-hydroxyethyl)phenyl)pyrazole-4-sulfonamide | 509 [M+H] | 1.00 | C |
| 114 | ref-3 | RSO₂Cl-24 | B | 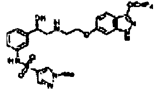 | (R)-N-(3-(2-(2-(3-(difluoromethoxy)-indazol-6-yloxy)ethylamino)-1-hydroxyethyl)phenyl)-1-methylpyrazolo-4-sulfonamide | 523 [M+H] | 0.87 | C |
| 115 | ref-3 | RSO₂Cl-25 | C | 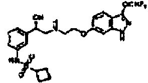 | (R)-N-(3-(2-(2-(3-(difluoromethoxy)-indazol-3-yloxy)ethylamino)-1-hydroxyethyl)phenyl)-cyclobutanesulfonamide | 497 [M+H] | 0.99 | C |
| 116 | ref-3 | RSO₂Cl-26 | B | 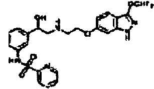 | (R)-N-(3-(2-(2-(3-(difluoromethoxy)-indazol-6-yloxy)ethylamino)-1-hydroxyethyl)phenyl)pyridine-2-sulfonamide | 520 [M+H] | 0.97 | C |
| 117 | ref-4 | RSO₂Cl-19 | B | 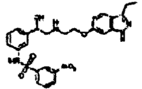 | (R)-N-(3-(2-(2-(3-ethylindazol-6-yloxy)ethylamino)-1-hydroxyethyl)phenyl)-3-nitrobenzenesulfonamide | 526 [M+H] | 1.09 | C |
| 118 | ref-4 | RSO₂Cl-19 | A | 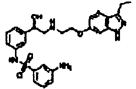 | (R)-3-amino-N-(3-(2-(2-(3-ethylindazol-6-yloxy)-ethylamino)-1-hydroxyethyl)-phenyl)benzenesulfonamide | 496 [M+H] | 0.98 | C |
| 119 | ref-4 | RSO₂Cl-3 | B | 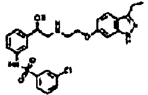 | (R)-3-chloro-N-(3-(2-(2-(3-ethylindazol-6-yloxy)-ethylaminol)-1-hydroxyethyl)-phenyl)benzenesulfonamide | 515 [M+H] | 1.08 | C |
| 120 | ref-4 | RSO₂Cl-4 | B | 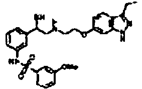 | (R)-N-(3-(2-(2-(3-ethylindazol-6-yloxy)ethylamino)-1-hydroxyethyl)phenyl)-3-methoxybenzenesulfonamide | 511 [M+H] | 1.07 | C |
| 121 | ref-4 | RSO₂Cl-2 | B | 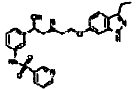 | (R)-N-(3-(2-(2-(3-ethylindazol-6-yloxy)ethylamino)-1-hydroxyethyl)phenyl)pyridine-3-sulfonamide | 482 [M+H] | 0.94 | C |
| 122 | rer-4 | RSO₂Cl-18 | B | 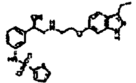 | (R)-N-(3-(2-(2-(3-ethylindazol-6-yloxy)ethylamino)-1-hydroxyethyl)phenyl)thiophenen 3-sulfonamide | 487 [M+H] | 0.98 | C |
| 123 | ref-4 | RSO₂Cl-1 | B | 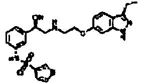 | (R)-N-(3-(2-(2-(3-ethylindazol-6-yloxy)ethylamino)-1-hydroxyethyl)phenyl)thiophene-3-sulfonamide | 487 [M+H] | 0.99 | C |
| 124 | ref-4 | RSO₂Cl-7 | D | 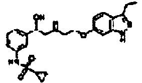 | (R)-N-(3-(2-(2-(3-ethylindazol-6-yloxy)ethylamino)-1-hydroxyethyl)phenyl)-cyclopropanesulfonamide | 445 [M+H] | 0.91 | C |
| 125 | ref-4 | RSO₂Cl-6 | D | 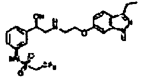 | (R)-N-(3-(2-(2-(3-ethylindazol-6-yloxy)ethylamino)-1-hydroxyethyl)phenyl)-2,2,2-trifluoroethanosulfonamide | 487 [M+H] | 0.99 | C |
| 126 | ref-4 | RSO₂Cl-5 | B | 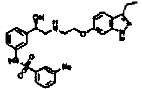 | (R)-N-(3-(2-(2-(3-ethylindazol-6-yloxy)ethylamino)-1-hydroxyethyl)phenyl)-3-methylbenzenesulfonamide | 495 [M+H] | 1.08 | C |
| 127 | ref-4 | RSO₂Cl-27 | B | 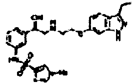 | (R)-N-(3-(2-(2-(3-ethylindazol-6-yloxy)ethylamino)-1-hydroxyethyl)phenyl)-1-methylimidazole-4-sulfonamide | 485 [M+H] | 0.88 | C |
| 128 | ref-4 | RSO₂Cl-10 | B | 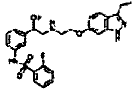 | (R)-N-(3-(2-(2-(3-ethylindazol-6-yloxy)ethylamino)-1-hydroxyethyl)phenyl)-2-fluorobenzenesulfonamide | 499 [M+H] | 1.08 | C |
| 129 | ref-4 | RSO₂Cl-11 | B | 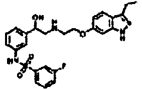 | (R)-N-(3-(2-(2-(3-ethylindazol-6-yloxy)ethylamino)-1-hydroxyethyl)phenyl)-3-fluorobenenesulfonamide | 499 [M+H] | 2.08 | C |
| 130 | ref-4 | RSO₂Cl-12 | B | 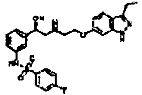 | (R)-N-(3-(2-(2-(3-ethylindazol-6-yloxy)ethylamino)-1-hydroxyethyl)phenyl)-4-fluorobenzenesulfonamide | 499 [M+H] | 1.04 | C |
| 131 | ref-4 | RSO₂Cl-13 | B | 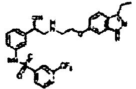 | (R)-N-(3-(2-(2-(3-ethylindazol-6-yloxy)ethylamino)-1-hydroxyethyl)phenyl)-3-(trifluoromethyl)benzenesulfonamide | 549 [M+H] | 1.19 | C |
| 132 | ref-4 | RSO₂Cl-14 | B | 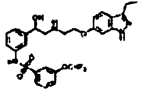 | (R)-3-(difluoromethoxy)-N-(3-(2-(2-(3-ethylindazol-6-yloxy)ethylamino)-1-hydroxyethyl)phenyl)-benzenesulfonamide | 547 [M+H] | 1.13 | C |
| 133 | ref-1 | RSO₂Cl-15 | B | 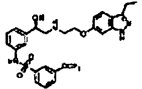 | (R)-N-(8-(2-(2-(3-ethylindazol-6-yloxy)ethylamino)-1-hydroxyethyl)phenyl)-3-(trifluoromethoxy)-benzenesulfonamide | 565 [M+H] | 1.23 | C |
| 134 | ref-4 | RSO₂Cl-50 | B | 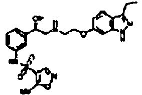 | (R)-N-(3-(2-(2-(3-ethylindazol-6-yloxy)ethylamino)-1-hydroexyethyl)phenyl)-5-methylisoxazole-4-sulfonamide | 486 [M+H] | 1.02 | C |
| 135 | ref-4 | RSO₂Cl-24 | B | 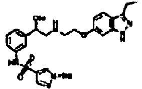 | (R)-N-(3-(2-(2-(3-ethylindazol-6-yloxy)ethylamino)-1-hydroxyethyl)phenyl)-1-methylpyrazole-4-sulfonamide | 4M [M+H] | 0.92 | C |
| 136 | ref-4 | RSO₂Cl-8 | B | 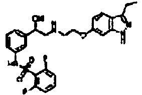 | (R)-N-(3-(2-(2-(3-othylindazol-6-yloxy)ethylamino)-1-hydroxyethyl)phenyl)-2,6-difluorobenzenesulfonamido | 517 [M+H] | 1.06 | C |
| 137 | ref-4 | RSO₂Cl-20 | B | 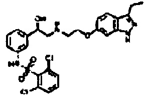 | (R)-2,6-dichloro-N-(3-2-(2-(3-ethylindazol-6-yloxy)-ethylamino)-1-hydroxyethyl)-phenyl)benzenesulfonamide | 549 [M+H] | 1.14 | C |
| 138 | ref-4 | RSO₂Cl-20 | B | 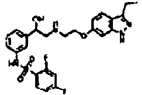 | (H)-N-(3-(2-(2-(3-ethylindazol-6-yloxy)ethylamino)-1-hydroxyethyl)phenyl)-2,4-difluorobenzenesulfonamido | 517 [M+H] | 1.06 | C |
| 139 | ref-4 | RSO₂Cl-21 | B | 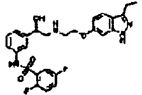 | (R)-N-(3-(2-(2-(3-ethylindazol-6-yloxy)ethylamino)-1-hydroxyethyl)phenyl)-2,5-difluorobenzenesulfonamide | 517 [M+H] | 1.09 | C |
| 140 | ref-4 | RSO₂Cl-41 | B | 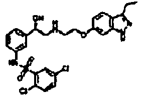 | (R)-2,5-dichloro-N-(3-(2-(2-(3-ethylindazol-6-yloxy)-ethylamino)-1-hydroxyethyl)-phenyl)benzenesulfonamide | 540 [M+H] | 1.19 | C |
| 141 | ref-4 | RSO₂Cl-42 | B | 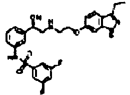 | (R)-N-(3-(2-(2-(3-ethylindazol-6-yloxy)ethylamino)-1-hydroxyethyl)phenyl)-3,5-difluorobezonesulfonamide | 517 [M+H] | 1.11 | C |
| 142 | ref-4 | RSO₂Cl-38 | B | 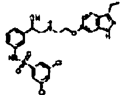 | (R)-3,5-dichloro-N-(3-(2-(2-(3-ethylindazol-6-yloxy)-ethylamino)-1-hydroxyethyl)-phenyl)benzenesulfonamide | 549 [M+H] | 1.2 | C |
| 143 | ref-4 | RSO₂Cl-43 | B | 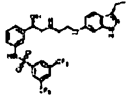 | (R)-N-(3-(2-(2-(3-ethylindazol-6-yloxyethylamino)-1-hydroxyethyl)phenyl)-3,5-bis(trifluoromethyl)benzenesulfonamide | 617 [M+H] | 1.36 | C |
| 144 | ref-4 | RSO₂Cl-44 | B | 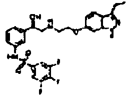 | (R)-N-(3-(2-(2-(3-ethylindazol-6-yloxy)ethylamino)-1-hydroxyethyl)phenyl)-3,4,5-trifluorobenzenesulfonamide | 535 [M+M] | 1.17 | C |
| 145 | ref-4 | RSO₂Cl-22 | B | 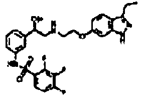 | (R)-N-(3-(2-(2-(3-ethylindazol-6-yloxy)ethylamino)-1-hydroxyethyl)phenyl)-2,3,4-trifluorobenzenesulfonamide | 535 [M+H] | 1.13 | C |
| 146 | ref-4 | RSO₂Cl-45 | B | 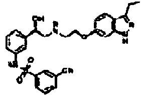 | (R)-3-cyano-N-(3-(2-(2-(3-ethylindazol-6-yloxy)-ethylamino)-1-hydroxyethyl)-phenyl)benzenesulfonamide | 506 [M+H] | 1.00 | C |
| 147 | ref-4 | RSO₂Cl-39 | B | 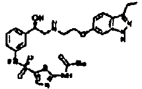 | (R)-N-(5-N-(3-(2-(2-(3-ethylindazol-6-yloxy)ethylamino)-1-hydroxyethyl)-phenyl)sulfamoyl)thiazol-2-yl)acetamide | 545 [M+H] | 1.02 | C |
| 148 | ref-4 | RSO₂Cl-9 | F | 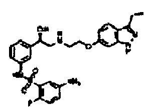 | (R)-5-amino-N-(3-(2-(2-(3-ethylindazol-6-yloxy)-ethylamino)-1-hydroxethyl)phenyl)-2-fluorobenzenesulfonamide | 514 [M+H] | 1.00 | C |
| 149 | ref-4 | RSO₂Cl-16 | B | 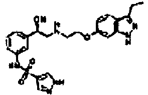 | (R)-N-(3-(2-(2-(3-ethylindazol-6-yloxy)ethylamino)-1-hydroxyethyl)phenyl)pyrazole-4-sulfonamide | 471[M+H] | 0.94 | C |
| 100 | ref-4 | RSO₂Cl-25 | C | 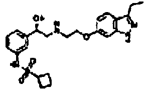 | (R)-N-(3-(2-(2-(3-ethylindazol-6-yloxy)ethylamino)-1-hydroxyethyl)phenyl)cyclobutane sulfonamide | 459 [M+H] | 0.91 | C |
| 151 | ref-4 | RSO₂Cl-26 | B | 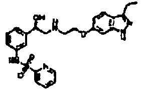 | (R)-N-(3-(2-(2-(3-ethylindazol-6-yloxy)ethylamino)-1-hydroxyethyl)phenyl)pyridine-2-sulfonamide | 482 [M+H] | 0.91 | C |
| 162 | ref-5 | RSO₂Cl-7 | D | 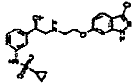 | (R)-N-(3-(2-(2-(3-chloroindazol-6-yloxy)ethylamino)-1-hydroxyethyl)phenyl-cyclopropanesulfonamide | 451 [M+H] | 0.95 | C |
| 153 | ref-5 | RSO₂Cl-19 | G | 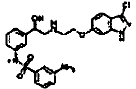 | (R)-3-amino-N-(3-(2-(2-(3-chloroindazol-6-yloxy)-ethylamino)-1-hydroxyethyl)-phenyl)benzenesulfonamide | 502 [M+H] | 0.94 | C |
| 154 | ref-5 | RSO₂C1-8 | E | 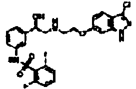 | (R)-N-(3-(2-(2-(3-chloroindazol-6-yloxy)ethylamino)-1-hydroxyethyl)phenyl)-2,6-difluorobenzenesulfonamide | 523 [M+H] | 1. 09 | C |
| 155 | ref-5 | RSO₂Cl-2 | B | 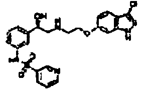 | (R)-N-(S-(2-(2-chloroindazol-6-ylory)ethylemino)-1-hydroxythyl)phenyl)pyridine-3-sulfonamido | 488 [M+H] | 1.04 | C |
| 156 | ref-5 | RSO₂Cl-18 | E | 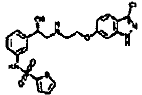 | (R)-N-(3-(2-(2-(3-chloroindazol-6-yloxy)ethylamino)-1-hydroxyethyl)phenyl)thiophene-2-sulfonamide | 493 [M+H] | 1.02 | C |
| 167 | ref-5 | RSO₂Cl-1 | E | 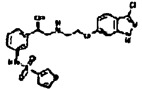 | (R)-N-(3-(2-(2-(3-chloroindazol-6-yloxy)ethylamino)-1-hydroxyethyl)phenyl)-biophene-3-sulfonamide | 493 [M+H] | 0.99 | C |
| 168 | ref-5 | RSO₂Cl-10 | E | 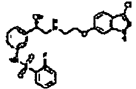 | (R)-N-(3-(2-(2-(3-chloroindazol-6-yloxy)ethylamino)-1-hydroxyethyl)phenyl)-2-fluorobenesulfonamide | 505[M+H] | 1.03 | C |
| 159 | ref-5 | RSO₂Cl-11 | E | 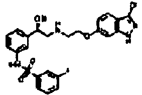 | (R)-N-(3-(2-(2-(3-chloroindazol-6-yloxy)ethylamino)-1-hydroxyethyl)phenyl)-3-fluorobenzenesulfonamide | 505 [M+H] | 1.12 | C |
| 160 | ref-5 | RSO₂Cl-12 | E | 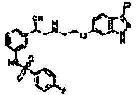 | (R)-N-(3-(2-(2-(3-chloroindazol-6-yloxy)ethylamino)-1-hydroxyethyl)phenyl)-4-fluorobenzenesulfonamido | 505 [M+H] | 1.10 | C |
| 161 | ref-5 | RSO₂Cl-4 | E | 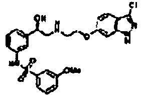 | (R)-N-(3-(2-(2-(3-chloroindazol-6-yloxy)ethylamino)-1-hydroxyethyl)phenyl)-3-methoxybenzenesulfonamide | 512 [M+H] | 1.09 | C |
| 162 | ref-5 | RSO₂Cl-5 | E | 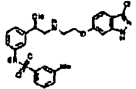 | (R)-N-(3-(2-(2-(3-chloroindazol-6-yloxy)ethylamino)-1-hydroxyethyl)phenyl)-3-methylbenzonesulfonamide | 501 [M+H] | 1.13 | C |
| 163 | ref-5 | RSO₂Cl-3 | E | 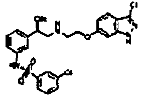 | (R)-3-chloro-N-(3-(2-(2-(3-chloroindazol-6-yloxy) ethylamino)-1-hydroxyethyl)-phenyl)benzenesulfonamide | 521 [M+H] | 1.31 | C |
| 164 | ref-5 | RSO₂Cl-13 | E | 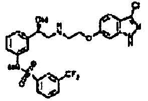 | (R)-N-(3-(2-(2-(3-chloroindazol-6-yloxy)ethylamino-1-hydroxyethyl)phenyl-3-trifluoromethyl)benzenesulfonamide | 555 [M+H] | 1.18 | C |
| 165 | ref-5 | RSO₂Cl-14 | E | 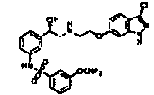 | (R)-N-(3-(2-(2-(3-chloroindazol-6-yloxy)ethylamino)-1-hydroxyethyl)phenyl)-3-(difluoromethoxy)benzenesulfonamide | 553[M+H] | 1.13 | C |
| 166 | ref-5 | RSO₂Cl-15 | E | 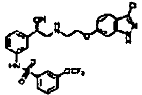 | (R)-N-(3-(2-(2-(3-chloroindazol-6-yloxy)ethylamino)-1-hydroxyethyl)phenyl)-3-(trifluoromethoxy)benzenesulfonamide | 571 [M+H] | 1.26 | C |
| 167 | ref-5 | RSO₂Cl-16 | B | 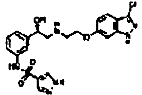 | (R)-N-(3-(2-(2-(3-chloroindazol)-6-yloxy)ethylamino)-1-hydroxyethyl)phenyl)pyrazole-4-sulfonamide | 477 [M+H] | 0.98 | C |
| 168 | ref-15 | RSO₂Cl-18 | H | 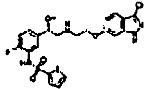 | (R)-N-(5-(2-(2-(3-chloroindazol-6-yloxy)ethylamino)-1-hydroxyethyl)-2-fluorophenyl)-thiophene-2-sulfonamide | 511 [M+H] | 1.06 | C |
| 169 | ref-15 | RSO₂Cl-1 | H | 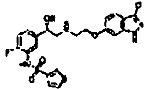 | (R)-N-(5-(2-(2-(3-chloroindazol-6-yloxy)ethylamino)-1-hydroxyethyl)-2-fluorophenyl)-thiophene-9-sulfonamido | 511 [M+H] | 1.01 | C |
| 170 | rer-16 | RSO₂Cl-10 | H | 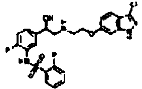 | (R)-N-(5-(2-(3-(3-chloroindazol-6-yloxy)ethylamino)-1-hydroxyethyl)-2-fluorophenyl)-2-fluorobenzenesulfonamide | 523 [M+H] | 1.05 | C |
| 171 | ref-15 | RSO₂Cl-19 | I | 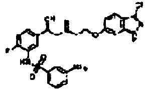 | (R)-3-amino-N-(5-(2-(2-(3-chloroindazol-6-yloxy)-ethylamino)-1-hydroxyethyl)-2-fluorophenyl)benzene sulfonamide | 520 [M+H] | 0.96 | C |
| 172 | ref-15 | RSO₂Cl-9 | I | 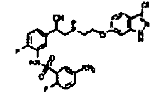 | (R)-5-amino-N-(5-(2-(2-(3-chloroindazol-6-yloxy)ethylamino)-1-hydroxyethyl)-2-fluorophenyl)-2-fluorobenzenesulfonamide | 538 [M+H] | 1.01 | C |
| 173 | ref-16 | RSO₂Cl-18 | H | 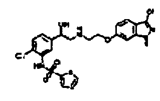 | (R)-N-(2-chloro-5-(2-(2-(3-chloroindazol-6-yloxy)-ethylamino)-1-hydroxyethyl)-phenyl)thiophone-2-sulfonamide | 527 [M+H] | 1.12 | C |
| 174 | ref-16 | RSO₂Cl-1 | H | 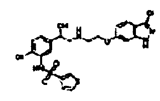 | (R)-N-(2-chloro-5-(2-(2-(3-chloroindazol-6-yloxy)-ethylamino)-1-hydroxyethyl)-phenyl)thiophene-3-sulfonamide | 5Z7 [M+H] | 1.07 | C |
| 175 | ref-16 | RSO₂Cl-10 | H | 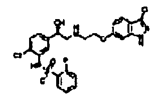 | (R)-N-(2-chloro-5-(2-(2-(3-chloroindazol-6-yloxy)-ethylamino)-1-hydroxyethyl)-phenyl)-2-fluorobenzenesulfonamide | 539 [M+H] | 1.11 | C |
| 176 | ref-16 | RSO₂Cl-19 | I | 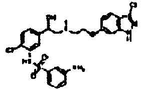 | (R)-3-amino-N-(2-chloro-5-(2-(2-(3-chloroindazol-6-yloxy)ehylamino)-1-hydroxyethyl)phenyl)benzenesulfonamide | 536 [M+H] | 1.02 | C |
| 177 | ref-16 | RSO₂Cl-9 | I | 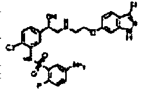 | (R)-5-amino-N-(2-chloro-5-(2-(-2-(3-chloroindazol-6-yloxy)ethylamino)-1-hydroxyethyl)phenyl)-2-fluorobenzonesulfonamide | 554 [M+H] | 1.07 | C |
| 178 | ref-5 | RSO₂Cl-24 | B | 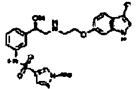 | (R)-N-(3-(2-(2-(3-chloroindazol-6-yloxyl)ethylamino)-1-hydroxyethyl)phenyl)-1-methylpyrazole-5-sulfonamide | 491 [M+H] 0.87 | 0.81 | C |
| 179 | ref-5 | RSO₂Cl-25 | C | 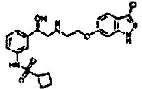 | (R)-N-(3-(2-(2-(3-chloroindazol-6-yloxy)ethylamino)-1-hydroxyethyl)phenyl)-cyclobutanosulfonamide | 465 [M+H] | 0.90 | C |
| 180 | ref-5 | RSO₂Cl-26 | B | 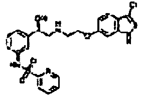 | (R)-N-(3-(2-(2-(3-chloroindazol-6-yloxy)ethylamino)-1-hydroxyethyl)phenyl)pyridino-2-sulfonamide | 488 [M+H] | 0.94 | C |
| 181 | ref-6 | RSO₂Cl-7 | D | 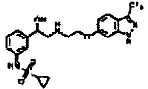 | (R)-N-(3-(1-hydroxy-2-(2-(3-(trifluoromethyl)indazol-6-yloxy)ethylamino)ethyl)phenyl)cy clopropanesulfonamide | 485 [M+H] | 0.95 | C |
| 182 | ref-6 | RSO₂Cl-19 | A | 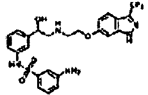 | (R)-3-amino-N-(3-(1-hydroxy-2-(2-(3-(trifluoromethyl)-indazol-6-yloxy)ethylamino)-ethyl)phenyl)benzenesulfonamide | 536 [M+H] | 1.02 | C |
| 183 | ref-18 | RSO₂Cl-19 | G | 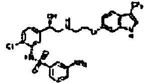 | (R)-8-amino-N-(2-chloro-5-(1-hydroxy-2-(2-(3-trifluoromethyl)-indazol-6-yloxy)ethylamino)ethyl)phenyl)be nzenesulfonamide | 570 | 1.16 | C |
| 184 | ref-6 | RSO₂Cl-8 | E | 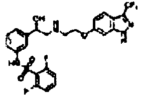 | (R)-2,6-difluoro-N-(3-(1-hydroxy-2-(2-(3-(trifluoro-methyl)-indazol-6-yloxy)ethylamino)ethyl)phenyl)be nzenesulfonamide | 557 | 1.18 | C |
| 185 | ref-6 | RSO₂Cl-9 | F | 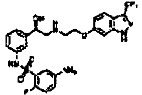 | (R)-5-amino-2-fluoro-N-(3-(1-hydroxy-2-(2-(3-(trifluoromethyl)-indazol-6-yloxy)ethylamino)ethyl)phenyl)be nzenesulfonamide | 554 [M+H] | 1.12 | C |
| 186 | ref-6 | RSO₂Cl-2 | B | 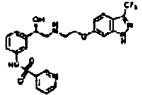 | (R)-N-(3-(1-hydroxy-2-(2-(3-(trifluoromethyl)-indazol-6-yloxy)ethylamino)ethyl)phenyl)py ridine-3-sulfonamide | 522 [M+H] | 1.12 | C |
| 187 | ref-6 | RSO₂Cl-16 | E | 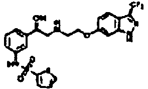 | (R)-N-(3-(1-hydroxy-2-(2-(3-(trifluoromethyl)-indazol-6-yloxy)ethylamino)ethyl)phenyl)th iophene-2-sulfonamide | 627 [M+H] | 1.12 | C |
| 188 | ref-6 | RSO₂Cl-1 | E | 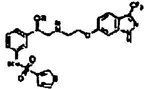 | (R)-N-(3-(1-hydroxy-2-(2-(3-(trifluoromethyl)-indazol-6-yloxy)ethylamino)ethyl)phenyl)th iophene-3-sulfonamido | 527 [M+H] | 1.15 | C |
| 189 | ref-6 | HSO₂Cl-10 | E | 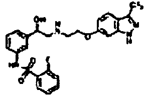 | (R)-2-fluoro-N-(3-(1-hydroxy-2-(2-(3-(trifluoromethyl)-indazol-6-yloxy)ethylamino)-ethy)phenyl)benzenesulfonamide | 530 [M+H] | 1.17 | C |
| 190 | ref-6 | RO₂Cl-11 | E | 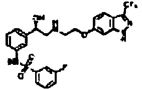 | (R)-3-fluoro-N-(3-(1-hydroxy-2-(2-(3-(trifloromethyl}-indazol-6-yloxy)ethylamino)-ethyl)phenyl)benzenesulfonamide | 539 [M+H] | 1.17 | C |
| 192 | ref-6 | RSO₂Cl-12 | E | 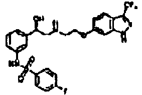 | (R)-4-fluoro-N-(3-(1-hydroxy-2-(2-(3-(trifluoromethyl)-indazol-6-yloxy)ethylamino)-ethyl)phenyl)benzenesulfonamide | 539 [M+H] | 1.22 | C |
| 192 | ref-6 | RSO₂Cl-4 | E | 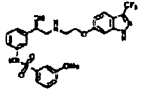 | (R)-N-(3-(1-hydroxy-2-(2-(3-(trifluoromethyl)-indazol-6-yloxy)ethylamino)ethyl)phenyl)-3-methoxybenzenesulfonamide | 531 [M+H] | 1.21 | C |
| 193 | ref-6 | RSO₂Cl-5 | E | 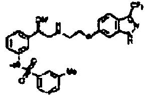 | (R)-N-(3-(1-hydroxy-2-(2-(3-(trifluoromethyl)-indazol-6-yloxy)ethylamino)ethyl)phenyl)-3-methylbenzenesulfonamide | 535 [M+H] | 1.26 | C |
| 194 | ref-6 | RSO₂Cl-3 | E | 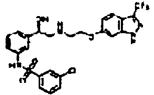 | (R)-3-chloro-N-(3-(1-hydroxy-2-(2-(3-(trifluoromethyl)-indazol-6-yloxy)ethylamino)-ethyl)phenyl)benzenesulfonamide | 555 [M+H] | 1.26 | C |
| 195 | ref-6 | RSO₂Cl-13 | E | 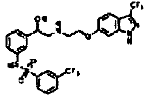 | (R)-N-(3-(1-hydroxy-2-(2-(3-(trifluoromethyl)indazol-6-yloxy)ethylamino)ethyl)phenyl)-3-(trifluoromethyl)benzenesulfonamide | 589 [M+H] | 1.34 | C |
| 196 | ref-6 | RSO₂Cl-14 | E | 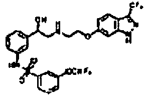 | (R)-3-(difluoromethoxy)-N-(3-(1-hydroxy-2-(2-(3-(trifluoromethyl)-indazol-6-yloxy)ethylamino)ethyl)phenyl)be nzenesulfonamide | 687 [M+H] | 1.27 | C |
| 197 | ref-6 | RSO₂Cl-15 | E | 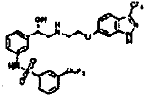 | (R)-N(3-(1-hydroxy-2-(2-(3-(trifluoromethyl)-indazol-6-yloxy)ethylamino)ethyl)phenyl)-3-(trifluoromethoxy)benzenesulfonamide | 605 [M+H] | 1.35 | C |
| 198 | ref-6 | RSO₂Cl-16 | B | 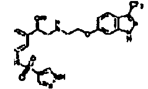 | (R)-N-(3-(1-hydroxy-2-(2-(3-(trifluoromethyl)indazol-6-yloxy)ethylamino)ethyl)phenyl)-pyrazole-4-sulfonamide | 511 [M+H] | 1.05 | C |
| 199 | ref-18 | RSO₂Cl-7 | | 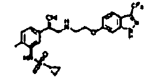 | (R)-N-(2-chloro-5-(1-hydroxy-2-(2-(3-(trifluoromethyl)-indazol-6-yloxy)ethylamino)-ethyl)phenyl)-cyclopropanesulfonamide | 519 [M+H] | 1.26 | C |
| 200 | ref-17 | RSO₂Cl-18 | H | 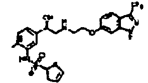 | (R)-N-(2-fluoro-5-(1-hydroxy-2-(2-(3-(trifluoromethyl)-indazol-6-yloxy)ethylamino)-ethyl)phenyl)thiophene-2-sulfonamide | 545 [M+M] | 1.20 | C |
| 201 | ref-17 | RSO₂Cl-1 | H | 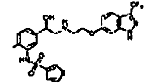 | (R)-N-(2-fluoro-5-(1-hydroxy-2-(2-(3-(trifluoromethyl)-indazol-6-yloxy)ethylamino)-ethyl)phenyl)thiophene-3-sulfonamide | 545 [M+H] | 1.14 | C |
| 02 | ref-17 | RSO₂Cl-10 | H | 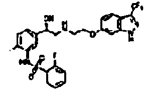 | (R)-2-fluoro-N-(2-fluoro-5-(1-hydroxy-2-(2-(3-(trifluoromethyl)-indazol-6-yloxy)ethylamino)ethyl)phenyl)be nzenesulfonamide | 557 [M+H] | 1.20 | C |
| 203 | ref 17 | RSO₂Cl-19 | I | 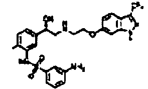 | (R)-3-amino-N-(2-fluoro-5-(1-hydroxy-2-(2-(3-(trifluoromethyl)-indazol-5-yloxy)ethylamino)ethyl)phenyl)be nzenesulfonamide | 554 [M+H] | 1.07 | C |
| 204 | ref-17 | RSO₂Cl-9 | I | 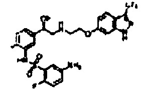 | (R)-5-amino-2-fluoro-N-(2-fluoro-5-(1-hydroxy-2-(2-(3-(trifluoromethyl)-indazol-6-yloxy)ethylamino)ethyl)phenyl)be nzenesulfonamide | 572 [M+H] | 1.09 | C |
| 205 | ref-18 | RSO₂Cl-18 | H | 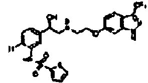 | (R)-N-(2-chloro-5-(1-hydroxy-2-(2-(3-(trifluoromethyl)-indazol-6-yloxy)ethylamino)-ethyl)phenyl)thiophene-2-sulfonamide | 561 [M+H] | 1.24 | C |
| 6 | ref- 18 | RSO₂Cl-1 | H | 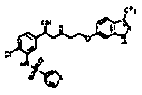 | (R)-N-(2-chloro-6-(1-hydroxy-2-(2-(3-(trifluoromethyl)-indazol-6-yloxy)ethylamino)-ethyl)phenyl)thiophene-3-sulfonamide | 561 [M+H] | 1.24 | C |
| 207 | ref-18 | RSO₂Cl-10 | H | 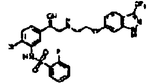 | (R)-N-(2-chloro-5-(1-hydroxy-2-(2-(3-(trifluoromethyl)-indazol-6-yloxy)ethylamino)-ethyl)phenyl)-2-fluorobenzenesulfonamide | 573 [M+H] | 1.16 | C |
| 208 | ref-18 | RSO₂Cl-9 | I | 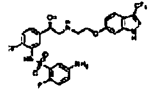 | (R)-5-amino-N-(2-chloro-5-(1-hydroxy-2-(2-(3-(trifluoromethyl)-indazol-6-yloxy)ethylamino)ethyl)phenyl)-2-fluorobenzenesulfonamide | 588 [M+H] | 1.15 | C |
| 209 | ref-6 | RSO₂Cl-24 | B | 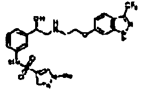 | (R)-N-(3-(1-hydroxy-2-(2-(3-(trifluoromeyhyl)-indazol-6-yloxy)ethylamino)ethyl)phenyl)-1-methyl-pyrazole-4-sulfonamide | 525 [M+H] | 0.92 | C |
| 210 | ref-6 | RSO₂Cl-25 | C | 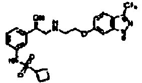 | (R)-N-(3-(1-hydroxy-2(2-(3-(trifluoromethyl)-indazol-6-yloxy)ethylamino)ethyl)phenyl)cy clobutanesulfonamide | 499 [M+H] | 1.03 | C |
| 211 | ref-6 | RSO₂Cl-26 | B | 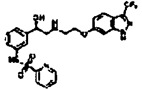 | (R)-N-(3-(1-hydroxy-2-(2-3-(trifluoromethyl)-indazol-6-yloxy)ethylamino)ethyl)phenyl)py ridine-2-sulfonamide | 522 [M+H] | 1.01 | C |
| 212 | ref-7 | RSO₂Cl-7 | D | 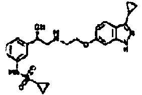 | (R)-N-(3-(2-(2-(3-cyclopropylindazol-6-yloxy)ethylamino)-1-hydroxyethyl)phenyl)-cyclopropanosulfonamide | 457 [M+H] | 0.89 | C |
| 213 | ref-7 | RSO₂Cl-19 | A | 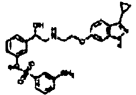 | (R)-3-amino-N-(3-2-(2-(3-cyclopropylindazol-6-yloxy)ethylamino)-1-hydroxyethyl)phenyl)benzenesulfonamide | 508 [M+H] | 0.92 | C |
| 214 | ref-7 | RSO₂Cl-6 | E | 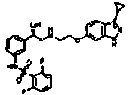 | (R)-N-(3-(2-(2-3-cyclopropylindazol-6-yloxy)ethylamino)-1-hydroxyethyl)phenyl)-2,6-difluorobenzenesulfonamide | 529 [M+H] | 1.09 | C |
| 216 | ref-7 | RSO₂Cl-9 | F | 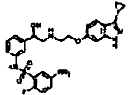 | (R)-5-amino-N-(3-(2(2-(3 cyclopropylindazol-6-yloxy)ethylamino)-1-hydroxyethyl)phenyl)-2-fluorobenzenesulfonamide | 526 [M+H] | 1.03 | C |
| 216 | ref-7 | RSO₂Cl-16 | E | 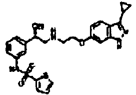 | (R)-N-(3-(2-(2-(3-cyclopropylindazol-6-yloxy)ethylamino)-1-hydroxyethyl)phenyl)-thiophene-2-sulfonamide | 499 [M+H] | 1.04 | C |
| 217 | ref-7 | RSO₂Cl-1 | E | 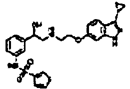 | (R)-N-(3-(2-(2-(3-cyclopropylindazol-6-yloxy)ethylamino)-1-hydroxyethyl)phenyl)-thiophene-3-sulfonamide | 499 [M+H] | 1.03 | C |
| 218 | ref-7 | RSO₂Cl-11 | E | 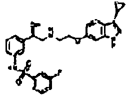 | (R)-N-(3-(2-(2-(3-cyclopropylindazol-6-yloxy)ethylamino)-1-hydroxyethyl)phenyl)-3-fluorobenzenesulfonamide | 511 [M+H] | 1.12 | C |
| 219 | ref-7 | RSO₂Cl-12 | E | 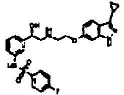 | (R)-N-(3-(2-(2-(3-cyclopropylindazol-6-yloxy)ethylamino)-1-hydroxyethyl)phenyl)-4-fluorobenzenesulfonamide | 511 [M+H] | 1.11 | C |
| 220 | ref-7 | RSO₂Cl-4 | E | 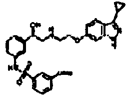 | (R)-N-(3-(2-(2-(3-cyclopropylindazol-6-yloxy)ethylamino)-1-hydroxyethyl)phenyl)-3-methoxybenzenesulfonamide | 523 [M+H] | 1.11 | C |
| 221 | ref-7 | RSO₂Cl-5 | E | 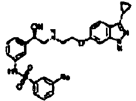 | (R)-N-(3-(2-(2-(3-cyclopropylindazol-6-yloxy)ethylamino)-1-hydroxyethyl)phenyl)-3-methylbenzanosulfonamide | 507 [M+H] | 1.11 | C |
| 222 | ref-7 | RSO₂Cl-13 | E | 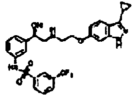 | (R)-N-(3-(2-(2-(3-cyclopropylindazol-6-yloxy)ethylamino)-1-hydroxyethyl)phenyl)-3-(trifluoromethyl)benzenesulfonamide | 501 [M+H] | 1.25 | C |
| 223 | ref-7 | RSO₂Cl-14 | E | 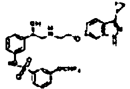 | (R)-N-(3-(2-(2-(3-cyclopropylindazol-6-yloxy)ethylamino)-1-hydroxyethyl)phenyl)-3-(difluoromethoxy)benzenesulfonamide | 559 [M+H] | 1.18 | C |
| 224 | ref-7 | RSO₂Cl-16 | E | 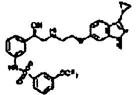 | (R)-N-(3-(2-(2-(3-cyclopropylindazol-6-yloxy)ethylamino)-1-hydroxyethyl)phenyl)-3-(trifluoromethoxy)benzenesulfonamide | 577 [M+H] | 1.28 | C |
| 225 | ref-7 | RSO₂Cl-24 | B | 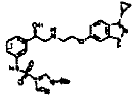 | (R)-N-(3-(2-(2-(3-cyclopropylindazol-6-yloxy)ethylamino)-1-hydroxyethyl)phenyl)-1-methylpyrazole-4-sulfonamide | 497 [M+H] | 0.86 | C |
| 226 | ref-7 | RSO₂Cl-25 | C | 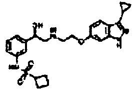 | (R)-N-(3-(2-(2-(3-cyclopropylindazol-6-yloxy)ethylamino)-1-hydroxyethyl)phenyl)-cyclobutanesulfonamide | 471 [M+H] | 0.92 | C |
| 227 | ref-8 | RSO₂Cl-7 | D | 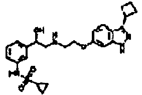 | (R)-N-(3-(2-(2-(3-cyclobutylindazol-6-yloxy)ethylamino)-1-hydroxyethyl)phenyl)-cyclopropanesulfonamide | 471 [M+H] | 0.98 | C |
| 228 | ref-9 | RSO₂Cl-7 | D | 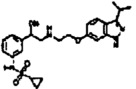 | (R)-N-(3-(1-hydroxy-2-(2-(3-iso-propylindazol-6-yloxy)ethylamino)ethyl)phenyl)cy clopropanesulfonamide | 409 [M+H] | 0.96 | c |
| 229 | ref-10 | RSO₂Cl-7 | D | 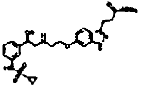 | (R)-3-(6-(2-(2-(3-(cyclopropanesulfonamide)-phenyl)-2-hydroxyethylamino)-ethoxy)indazol-3-yl)-N,N-dimethyl-propanamide | 516 [M+H] | 0.79 | C |
| 230 | ref-8 | RSO₂Cl-19 | A | 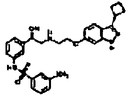 | (R)-3-amino-N-(3-(2-(2-(3-cyclobutylindazol-6-yloxy)ethylamino)-1-hydroxyethyl)phenyl)-benzenesulfonamide | 522 [M+H] | 1.00 | C |
| 231 | ref-9 | RSO₂Cl-19 | A | 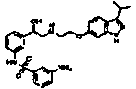 | (R)-3-amino-N-(3-(1-hydroxy-2-(2-(3-iso-propylindazol-6-yloxy)ethylamino)ethyl)phenyl)be nzenesulfonamide | 510 [M+H] | 0.96 | C |
| 32 | ref-10 | RSO₂Cl-19 | A | 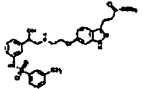 | (R)-3-(6-(2-(2-(3-(3-amino-phenylsulfonamido)phenyl)-2-hydroxyethylamino)ethoxy)indaz ol-3-yl)-N,N-dimethyl-propanamide | 567 [M+H] | 0.78 | C |
| 233 | ref-8 | RSO₂Cl-25 | C | 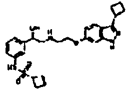 | (R)-N-(3-(2-(2(-3-cyclobutylindazol-6-yloxy)ethylamino)-1-hydroxyethyl)phenyl)cyclobutane sulfonamide | 435 [M+H] | 0.96 | C |
| 234 | ref-9 | RSO₂Cl-25 | C | 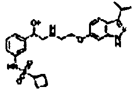 | (R)-N-(a-(1-hydroxy-2-(2-(3-iso-propylindazol-6-yloxy)ethylamino)ethyl)phenyl)cy clobutanesulfonamide | 473 [M+H] | 0.97 | C |

### [Working Example 235]

### (R)-2-fluoro-N-(3-(1-hydroxy-2-(2-(3-methylindazol-6-yloxy)ethylamino)ethyl)phenyl)benzenesulfonamide

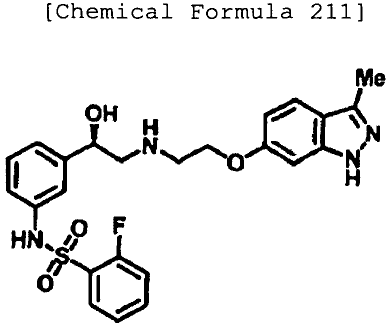

(R)-tert-butyl 6-(2-((2-(3-aminophenyl)-2-(triethylsilyloxy)ethyl)(tert-butoxycarbonyl)amino)ethoxy)-3-methylindazole-1-carboxylate (67.2 mg), which can be manufactured by the method described in Reference Example 1 and the like, was dissolved in dehydrated CH₂Cl₂ (1.5 mL). Pyridine (16 µL; made by Kanto Chemical Co., Inc.) and 2-fluorobenzene-1-sulfonyl chloride (25.4 mg; made by Aldrich Co.) were added and [the contents] were stirred overnight at room temperature. Pyridine (10 µL; made by Kanto Chemical Co., Inc.) and 2-fluorobenzene-1-sulfonyl chloride (13 mg; made by Aldrich Co.) were added to the reaction solution and [the contents] were stirred for three hours at room temperature. The reaction solution was purified by column chromatography ("Column B;" n-hexane: ethyl acetate = 75:25→54:46), and (R)-tert-butyl 6-(2-(tert-butoxycarbonyl(2-(3-(2-fluorophenylsulfonamide)phenyl)-2-(triethylsilyloxy)ethyl)amino)ethoxy-3-methylindazole-1-carboyxlate (73.9 mg) was obtained. It was then dissolved in dehydrated MTBE (0.1 mL). 4 mol/L hydrochloric acid-1,4-dioxane solution (1.5 mL; made by Kokusan Chemical Co., Ltd.) was added and shaken (600 min⁻¹) overnight at room temperature. Nitrogen gas was blown into the reaction solution, and the solvent was driven off. The title compound was obtained as a hydrochloride (58.4 mg).
LCMS: 485 [M + H]; Retention time: 0.98 min; LCMS conditions: C

### [Working Example 236]

### (R)-3-hydroxy-N-(3-(1-hydroxy-2-(2-(3-methylindazol-6-yloxy)ethylamino)ethyl)phenyl)benzenesulfonamide

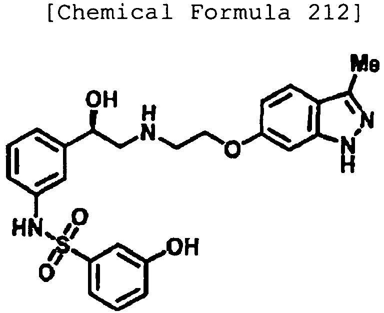

(R)-tert-butyl 6-(2-((2-(3-aminophenyl)-2-(triethylsilyloxy)ethyl)(tert-butoxycarbonyl)amino)ethoxy)-3-methylindazole-1-carboxylate (101.2 mg), which can be manufactured by the method described in Reference Example 1 and the like, was dissolved in dehydrated CH₂Cl₂ (1.5 mL). Pyridine (92 µL; made by Kanto Chemical Co., Inc.) and 3-(benzyloxy)benzene-1-sulfonyl chloride-CH₂Cl₂ solution [0.5 mL; solution prepared by dissolving 3-(benzyloxy)benzene-1-sulfonyl chloride (160.7 mg), which can be manufactured by the method described in Reference Example 21 and the like, in dehydrated CH₂Cl₂ (0.5 mL)] were added and [the contents] were stirred for seven hours at room temperature. Pyridine (137 µL; made by Kanto Chemical Co., Inc.) and 3-(benzyloxy)benzene-1-sulfonyl chloride-CH₂Cl₂ solution [0.5 mL; solution prepared by dissolving 3-(benzyloxy)benzene-1-sulfonyl chloride (242 mg), which can be manufactured by the method described in Reference Example 21 and the like, in dehydrated CH₂Cl₂ (0.5 mL)] were added and [the contents] were stirred for 2.5 days at room temperature. The reaction solution was crudely purified by column chromatography ("Column B;" n-hexane: ethyl acetate = 77:23→56:44). After dissolving the crudely purified product obtained in ethyl acetate, it was washed twice with 0.5 mol/L aqueous hydrochloric acid. The organic layer was washed with brine. After drying had been performed using anhydrous sodium sulfate, the solvent was distilled off under reduced pressure. 5% Palladium carbon (STD-type, containing 50% water) (64.1 mg; made by N. E. Chemcat Corporation) and THF (2 mL) were added to the residue (109.4 mg) obtained and stirred overnight at room temperature in a hydrogen atmosphere. The reaction solution was placed under a nitrogen atmosphere and filtered. The filtrate was placed under reduced pressure, and the solvent was distilled off. The resulting residue was crudely purified by column chromatography ("Column B;" n-hexane: ethyl acetate = 64:36→43:57). The crudely purified product obtained was dissolved in dehydrated MTBE (0.1 mL). 4 mol/L hydrochloric acid-1,4-dioxane solution (1.5 mL; made by Kokusan Chemical Co., Ltd.) was added and shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (80.9 mg).
LCMS: 483 [M + H]; Retention time: 0.96 min; LCMS conditions: C

### [Working Example 237]

### (R)-N-(3-(1-hydroxy-2-(2-(3-methylindazol-6-yloxy)ethylamino)ethyl)phenyl)cyclopentanesulfonamide

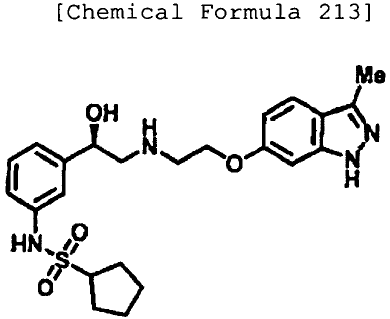

(R)-tert-butyl 6-(2-((2-(3-aminophenyl)-2-(triethylsilyloxy)ethyl)(tert-butoxycarbonyl)amino)ethoxy)-3-methylindazole-1-carboxylate (94.6 mg), which can be manufactured by the method described in Reference Example 1 and the like, was dissolved in dehydrated CH₂Cl₂ (1 mL). DBU (134 µL; made by Tokyo Chemical Industry Co., Ltd.) and cyclopentanesulfonyl chloride (1 mL; solution prepared by dissolving cyclopentanesulfonyl chloride (111.3 mg; made by Aldrich Co.) in dehydrated CH₂Cl₂ (1 mL)) were added and [the contents] were stirred for 1.5 days at room temperature. Nitrogen gas was blown into the reaction solution, and the solvent was driven off. The resulting residue was dissolved in methanol (2 mL). 5 mol/L sodium hydroxide aqueous solution (0.2 mL; made by Kanto Chemical Co., Inc.) was added and [the contents] were stirred for 2.5 days at room temperature. The reaction solution was poured into saturated ammonium chloride solution and extracted twice using ethyl acetate. The organic layer was washed twice with 2 mol/L hydrochloric acid and once with brine. It was dried using anhydrous sodium sulfate, and the solvent was distilled off under reduced pressure. The resulting residue was crudely purified by column chromatography ("Column B;" n-hexane: ethyl acetate = 47:53→20:80). The crudely purified product obtained was purified by PTLC (made by Merck (1.05715.0009); CHCl₃: MeOH = 10:1; Rf = 0.27-0.45), and (R)-tert-butyl 2-(3-(cyclopropanesulfonamide)phenyl)-2-hydroxyethyl(2-(3-methylindazol-6-yloxy)ethyl)carbamate (34.8 mg) was obtained. 4 mol/L hydrochloric acid-1,4-dioxane solution (1.5 mL; made by Kokusan Chemical Co., Ltd.) was then added and [the contents] were shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (34.3 mg).
LCMS: 459 [M + H]; Retention time: 0.95 min; LCMS conditions: C

### [Working Example 238]

### (R)-3-(dimethylamino)-N-(3-(1-hydroxy-2-(2-(3-methylindazol-6-yloxy)ethylamino)ethyl)phenyl)benzenesulfonamide

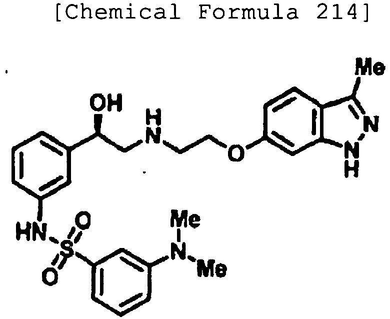

(R)-tert-butyl 6-(2-((2-(3-(3-aminophenylsulfonamide)phenyl)-2-(triethylsilyloxy)ethyl)(tert-butoxycarbonyl)amino)ethoxy)-3-methylindazole-1-carboxylate (70.1 mg), which can be manufactured by the method described in Reference Example 118 and the like, was dissolved in dehydrated CH₂Cl₂ (1.5 mL). 35% Formalin aqueous solution (232 µL; made by Aldrich Co.), acetic acid (33 mL; made by Wako Pure Chemical Industries Co., Ltd.), and sodium triacetoxyborohydride (46.6 mg; made by Wako Pure Chemical Industries Co., Ltd.) were added and [the contents] were stirred for five hours at room temperature. Sodium triacetoxyborohydride (153.2 mg; made by Wako Pure Chemical Industries Co., Ltd.) was added to the reaction solution and stirred overnight at room temperature. Sodium triacetoxyborohydride (159 mg; made by Wako Pure Chemical Industries Co., Ltd.) was added to the reaction solution and stirred for seven hours at room temperature. Sodium triacetoxyborohydride (627.3 mg; made by Wako Pure Chemical Industries Co., Ltd.) was added to the reaction solution and stirred for three days at room temperature. Saturated sodium bicarbonate aqueous solution was added to the reaction solution, and it was extracted once using ethyl acetate. The organic layer was washed once with brine. After drying had been performed using anhydrous sodium sulfate, the solvent was distilled off under reduced pressure. The resulting residue was dissolved in dehydrated THF (2 mL). 1 mol/L TBAF-THF solution (300 µL; made by Tokyo Chemical Industry Co., Ltd.) was added and [the contents] were stirred for 2.5 hours at room temperature. Ethyl acetate was added to the reaction solution. The organic layer was washed once with brine, once with water, and once with brine. After drying had been performed using anhydrous sodium sulfate, the solvent was distilled off under reduced pressure. The resulting residue was purified by column chromatography ("Column B;" n-hexane: ethyl acetate = 57:43→36:64), and (R)-tert-butyl 6-(2-(tert-butoxycarbonyl(2-(3-(3-(dimethylamino)phenylsulfonamide)phenyl)-2-hydroxy)amino)ethoxy)-3-methylindazole-1-carboxylate (27.9 mg) was obtained. MTBE (100 µL), 4 mol/L hydrochloric acid-1,4-dioxane solution (1 mL; made by Kokusan Chemical Co., Ltd.), and 10% hydrochloric acid-methanol (100 µL; made by Tokyo Chemical Industry Co., Ltd.) were then added and [the contents] were shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (30.6 mg).
LCMS: 510 [M + H]; Retention time: 1.12 min; LCMS conditions: C

### [Working Example 239]

### (R)-N-(3-(1-hydroxy-2-(2-(3-methylindazol-6-yloxy)ethylamino)ethyl)phenyl)-3-(methylamino)benzenesulfonamide

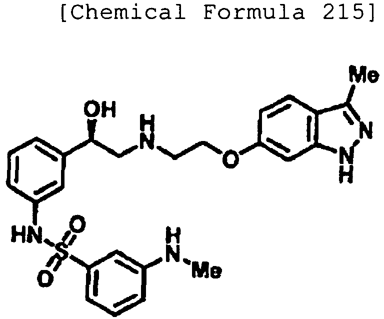

(R)-tert-butyl 6-(2-((2-(3-(3-aminophenylsulfonamide)phenyl)-2-(triethylsilyloxy)ethyl)(tert-butoxycarbonyl)amino)ethoxy)-3-methylindazole-1-carboxylate (70.3 mg), which can be manufactured by the method described in Reference Example 118 and the like, was dissolved in ethanol (1.5 mL). After heating to reflux, (1H-benzo[d][1,2,3]triazol-1-yl)methanol (19.9 mg; made by Tokyo Chemical Industry Co., Ltd.) was added and [the contents] were stirred for three minutes under reflux. The reaction solution was cooled to room temperature. Sodium borohydride (15.2 mg; made by Kanto Chemical Co., Inc.) was added and [the contents] were stirred for six days at room temperature. Ethyl acetate was added to the reaction solution. The organic layer was washed three times using water. After drying had been performed using anhydrous sodium sulfate, the solvent was distilled off under reduced pressure. Ethanol (1.5 mL) was added to the residue obtained. (1H-benzo[d][1,2,3]triazol-1-yl)methanol (20.2 mg; made by Tokyo Chemical Industry Co., Ltd.) was added and [the contents] were stirred for 12 hours under reflux. The reaction solution was cooled to room temperature. Sodium borohydride (13 mg; made by Kanto Chemical Co., Inc.) was added and [the contents] were stirred for two hours at room temperature. Ethyl acetate was added to the reaction solution. The organic layer was washed three times using water. After drying had been performed using anhydrous sodium sulfate, the solvent was distilled off under reduced pressure. The resulting residue was dissolved in dehydrated THF (2 mL). 1 mol/L TBAF-THF solution (300 µL; made by Tokyo Chemical Industry Co., Ltd.) was added and [the contents] were stirred for three hours at room temperature. Ethyl acetate was added to the reaction solution. The organic layer was washed once with brine, once with water, and once with brine. After drying had been performed using anhydrous sodium sulfate, the solvent was distilled off under reduced pressure. The resulting residue was crudely purified by column chromatography ("Column B;" n-hexane: ethyl acetate = 57:43→36:64). The crudely purified product obtained was purified by PTLC [made by Merck (1.05715.0009); Et₂O; Rf = 0.3-0.4], and (R)-tert-butyl 6-(2-(tert-butoxycarbonyl(2-hydroxy-2-(3-(3-(methylamino)phenylsulfonamide)phenyl)ethyl)amino)ethoxy)-3-methylindazole-1-carboxylate (8.3 mg) was obtained. MTBE (50 µL) and 4 mol/L hydrochloric acid-1,4-dioxane solution (1 mL; made by Kokusan Chemical Co., Ltd.) were then added and shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (7.0 mg)
LCMS: 496 [M + H]; Retention time: 1.03 min; LCMS conditions: C

### [Working Example 240]

### (R)-N-(3-(N-(3-(1-hydroxy-2-(2-(3-methylindazol-6-yloxy)ethylamino)ethyl)phenyl)sulfamoyl)phenyl)acetamide

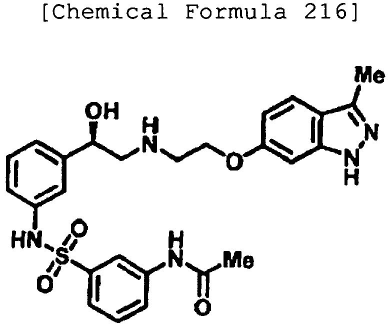

(R)-tert-butyl 6-(2-((2-(3-(3-aminophenylsulfonamide)phenyl)-2-(triethylsilyloxy)ethyl)(tert-butoxycarbonyl)amino)ethoxy)-3-methylindazole-1-carboxylate (70.3 mg), which can be manufactured by the method described in Reference Example 118 and the like, was dissolved in CH₂Cl₂ (1 mL). Acetic anhydride (10 µL; made by Wako Pure Chemical Industries Co., Ltd.) was added and [the contents] were stirred overnight at room temperature. Nitrogen gas was blown into the reaction solution, and the solvent was driven off. Anhydrous 1,4-dioxane (0.1 mL) and 4 mol/L hydrochloric acid-1,4-dioxane solution (1.5 mL; made by Kokusan Chemical Co., Ltd.) were added to the residue obtained and shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (19.1 mg).
LCMS: 524 [M + H]; Retention time: 0.93 min; LCMS conditions: C

### [Working Example 241]

### (R)-2-hydroxy-N-(3-(1-hydroxy-2-(2-(3-methylindazol-6-yloxy)ethylamino)ethyl)phenyl)benzenesulfonamide

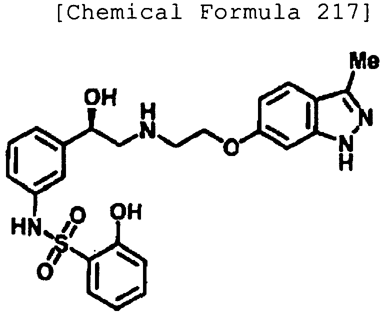

(R)-tert-butyl 6-(2-((2-(3-aminophenyl)-2-(triethylsilyloxy)ethyl)(tert-butoxycarbonyl)amino)ethoxy)-3-methylindazole-1-carboxylate (191 mg), which can be manufactured by the method described in Reference Example 1 and the like, was dissolved in dehydrated CH₂Cl₂ (1.5 mL). Pyridine (49 µL; made by Kanto Chemical Co., Inc.) and 3,5-dichloro-2-hydroxybenzene-1-sulfonyl chloride (116.4 mg; made by Aldrich Co.) were added and [the contents] were stirred overnight at room temperature. The reaction solution was purified by column chromatography ("Column B;" n-hexane: ethyl acetate = 60:40→39:61), and (R)-tert-butyl 6-(2-(tert-butoxycarbonyl(2-(3-(3,5-dichloro-2-hydroxyphenylsulfonamide)phenyl)-2-(triethylsilyloxy)ethyl)amino)ethoxy)-3-methylindazole-1-carboxylate (145.1 mg) was obtained. 10% Palladium carbon (PE-type, containing 50% water) (103.8 mg; made by N. E. Chemcat Corporation), methanol (1.5 mL), and triethylamine (42 µL; made by Kanto Chemical Co., Inc.) were added to (R)-tert-butyl 6-(2-(tert-butoxycarbonyl(2-(3-(3,5-dichloro-2-hydroxyphenylsulfonamide)phenyl)-2-(triethylsilyloxy)ethyl)amino)ethoxy)-3-methylindazole-1-carboxylate (90.9 mg). The interior of the reaction system was placed under a hydrogen atmosphere and stirred overnight at room temperature. The reaction solution was warmed to 50°C and stirred overnight at 50°C. The reaction solution was placed under a nitrogen atmosphere and filtered. The filtrate was placed under reduced pressure, and the solvent was distilled off. After dissolving the residue obtained in ethyl acetate, the organic layer was washed once with 1 mol/L aqueous hydrochloric acid and once using brine. After drying had been performed using anhydrous sodium sulfate, the solvent was distilled off under reduced pressure. The resulting residue was dissolved in MTBE (0.2 mL). 4 mol/L hydrochloric acid-1,4-dioxane solution (1.5 mL; made by Kokusan Chemical Co., Ltd.) was added and shaken (600 rpm⁻¹) overnight at room temperature. Nitrogen gas was blown into the reaction solution, and the solvent was driven off. MTBE was added to the resulting residue to make a suspension. Nitrogen gas was blown into the suspension, and the solvent was driven off. The title compound was obtained as a hydrochloride (57 mg).
LCMS: 483 [M + H]; Retention time: 0.98 min; LCMS conditions: C

### [Working Example 242]

### (R)-5-amino-N-(3-(2-(2-(3-chloroindazole-6-yloxy)ethylamino)-1-hydroxyethyl)phenyl)-2-fluorobenzenesulfonamide

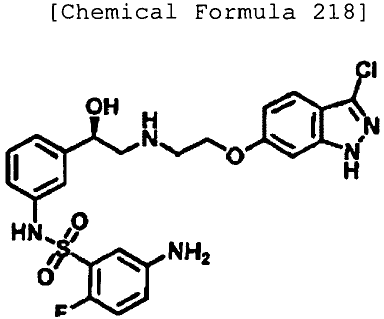

(R)-tert-Butyl 6-(2-((2-(3-aminophenyl)-2-(triethylsilyloxy)ethyl)(tert-butoxycarbonyl)amino)ethoxy)-3-chloroindazole-1-carboxylate (68.6 mg), which can be manufactured by the method described in Reference Example 5 and the like, was dissolved in dehydrated CH₂Cl₂ (1 mL). Pyridine (24 µL; made by Kanto Chemical Co., Inc.) and 2-fluoro-5-nitrobenzene-1-sulfonyl chloride-CH₂Cl₂ solution [0.5 mL; solution prepared by dissolving 2-fluoro-5-nitrobenzene-1-sulfonyl chloride (263.4 mg; made by Chemcollect) in dehydrated CH₂Cl₂ (3.5 mL)] were added and shaken (600 rpm⁻¹) overnight at room temperature. PS-Trisamine (200 mg (4.06 mmol/g); made by Biotage) was added to the reaction solution and shaken for seven hours at room temperature. The reaction solution was filtered. Nitrogen gas was blown into the filtrate, and the solvent was driven off. The resulting residue was purified by column chromatography ("Column I;" methanol). CM-101 catalyst (120 mg; made by N. E. Chemcat Corporation), THF (1 mL), and methanol (1 mL) were added to the purified product (57.0 mg) obtained. The interior of the reaction system was placed under a hydrogen atmosphere and stirred for 30 hours at room temperature. The interior of the reaction system was placed under a nitrogen atmosphere and filtered. Nitrogen gas was blown into the filtrate, and the solvent was driven off. The obtained residue (57.0 mg) was dissolved in dehydrated 1,4-dioxane (0.2 mL). 4 mol/L hydrochloric acid-1,4-dioxane solution (1.5 mL; made by Kokusan Chemical Co., Ltd.) and 10% hydrochloric acid-methanol solution (0.2 mL; made by Tokyo Chemical Industry Co., Ltd.) were added and [the contents] were shaken (600 rpm⁻¹) overnight at room temperature. Nitrogen gas was blown into the reaction solution, and the solvent was driven off. MTBE was added to the resulting residue to make a suspension Nitrogen gas was blown into the suspension, and the solvent was driven off. The title compound was obtained as a hydrochloride (38 mg)
LCMS: 520 [M + H]; Retention time: 1.01 min; LCMS conditions: C

### [Working Example 243]

### (R)-N-(3-(1-hydroxy-2-(2-(3-(hydroxymethyl)indazole-6-yloxy)ethylamino)ethyl)phenyl)cyclobutanesulfonamide

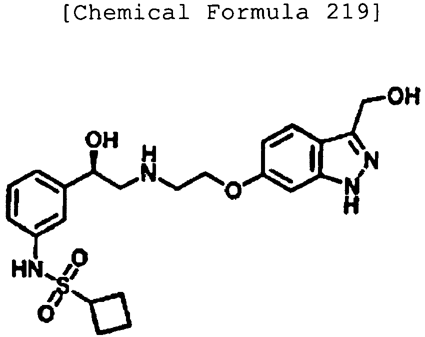

tert-Butyl (R)-2-(3-(cyclobutanesulfonamide)phenyl)-2-hydroxyethyl(2-(3-hydroxymethyl)-1-(tetrahydro-1H-pyran-2-yl)indazole-6-yloxy)ethyl)carbamate (23.3 mg), which can be manufactured by the method described in Reference Example 122 and the like, was dissolved in ethanol (0.1 mL; made by Wako Pure Chemical Industries Co., Ltd.). 4 mol/L hydrochloric acid-1,4-dioxane solution (1 mL; made by Kokusan Chemical Co., Ltd.) was added and [the contents] were shaken (600 rpm⁻¹) overnight at room temperature. Nitrogen gas was blown into the reaction solution, and the solvent was driven off. The title compound was obtained as a hydrochloride (7.1 mg). This hydrochloride (0.4349 g) was dissolved in DMSO (250 µL) and pure water (4750 µL). Chloride ion (9 ppm) was detected as a result of measuring anions by ion chromatography.
¹H-NMR (300MHz, DMSO-d₆); *δ* (ppm) 1.84-1 .89 (2H, m), 2.12-2.17 (2H, m), 2.26-2.3 4 (2H, m), 3. 04-3. 07 (1H, m), 3. 24 (brs, 1 H), 3. 8 8 (1H, qu, J=8. 0), 4.34 (2H, brs), 4.72 (2H, s), 4. 94 (1H, d, J=8. 0), 6.78 (1 H, dd, J=2.1, 8.7), 6.91 (1 H, d, J=1. 8), 7 .08-7.1 3 (2 H, m), 7. 28-7.34 (2 H, m), 7. 7 3 (1 H, d, J=8. 7), 8.88 (1H, brs), 9. 02 (1 H brs), 9.7 7 (1 H. s)
LCMS: 461 [M + H]; Retention time: 0.75 min; LCMS conditions: C

### [Working Example 244]

### (R)-N-(3-(2-(2-(3-cyclopropylindazol-6-yloxy)ethylamino)-1-hydroxyethyl)phenyl)-2-fluorobenzenesulfonamide

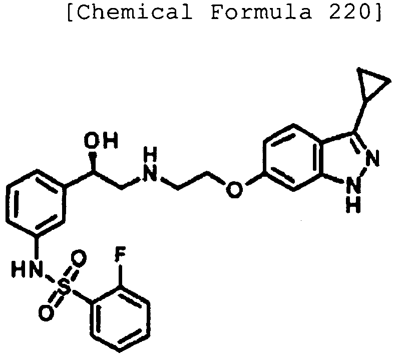

The title compound (36.1 mg) was obtained by the same method as in Working Example 5 using (R)-tert-butyl 6-(2-((2-(3-aminophenyl)-2-(triethylsilyloxy)ethyl)(tert-butoxycarbonyl)amino)ethoxy)-3-cyclopropylindazole-1-carboxylate (41.5 mg), which can be manufactured by the method described in Reference Example 7 and the like, instead of (R)-tert-butyl 6-(2-((2-(3-aminophenyl)-2-(triethylsilyloxy)ethyl)(tert-butoxycarbonyl)amino)ethoxy)-3-methoxyindazole-1-carboxylate.
LCMS: 511 [M + H]; Retention time: 1.04 min; LCMS conditions: C
The details of the reagents in Table 1 are shown in Table 2.

The meanings of the abbreviations in Table 2 are as follows.

"RSO₂Cl" is an abbreviation that corresponds to the reagent used in the "Reagent 2" column of table 1. For example, "1" means "RSO₂Cl-1." "Structure" means the structure of the reagent. "Name" means the name of the reagent. "Supplier" means the supplier of the reagent used.

The abbreviations used for the suppliers of the reagents used are as follows.

Tokyo Chemical Industry Co., Ltd.: "TCI;" Aldrich Co.: "Ald;" Apollo: "Apollo;" Kanto Chemical Co., Inc.: "KANTO;" Wako Pure Chemical Industries Co., Ltd.: "WAKO;" Maybridge: "MAYB;" Acros: "Acros;" Alfa Aesar: "AlfaA;" CombiBlock: "Comb;" FluoroChem: "Fchem;" Matrix: "Matrix;" Enamine: "Enam;" Hande Sciences: "Hande;" ASINEX: "ASIN;" Chemcollect: "Chemco."

As a source of the reagents used, "ref-20" means a compound that can be manufactured by Reference Example 20 and the like.

[Table 2]

**Table 2**

| RSO₂Cl | Structure | Name | Supplier | | RSO₂Cl | Structure | Name | Supplier |
|---|---|---|---|---|---|---|---|---|
| 1 | 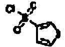 | thiophene-3-sulfonyl chloride | MAYB | | 18 | 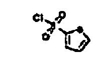 | thiophene-2-sulfonyl chloride | Ald |
| 2 | 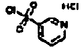 | pyridine-3-sulfonyl chloride hydrogen | chloride Comb | | 19 | 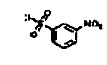 | 9-nitrobenzene-1-sulfonyl chloride | WAKO |
| 3 | 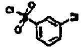 | 3-chlorobenzene-1-sulfonyl chloride | Ald | | 20 | 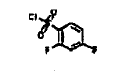 | 2,4-difluorobenzene-1-sulfonyl chloride | TCI |
| 4 | 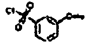 | 3-methoxy-benzene-1-sulfonyl chloride | Ald | | 21 | 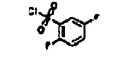 | 2,5-difluorobenzene-1-sulfonyl chloride | Ald |
| 5 | 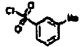 | 3-methylbenzene-1-sulfonyl chloride | Ald | | 22 | 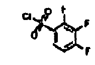 | 2,3,4-trifluorobenzene-1-sulfonyl chloride | Ald |
| 6 | 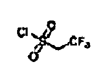 | 2,2,2-trifluoroethanesulfonyl chloride | Acros | | 23 | 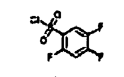 | 2,4,5-trifluorobenzene-1-sulfonyl chloride | Matrix |
| 7 | 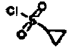 | cyclopropanesulfonyl chloride | Matrix | | 24 | 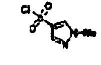 | 1-methylpyrazole-4-sulfonyl chloride | Enam |
| 8 | 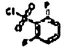 | 2,6-difluoro-benzene-1-sulfonyl chloride | TCI | | 25 | 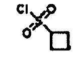 | cyclobutanesulfonyl chloride | Hande |
| 9 | 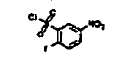 | 2-fluoro-5-nitrobenzene-1-sulfonyl chloride | Chemco | | 26 | 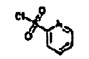 | pyridine-2-sulfonyl chloride | Comb |
| 10 | 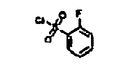 | 2-fluorobenzene-1-sulfonyl chloride | Ald | | 27 | 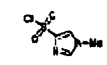 | 1-methylimidazole-4-sulfonyl chloride | Fchern |
| 11 | 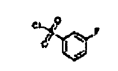 | 3-fluorobenzene-1-sulfonyl chloride | Ald | | 28 | 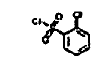 | 2-chlorobenzene-1-sulfonyl chloride | Ald |
| 12 | 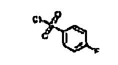 | 4-fluorobenzene-1-sulfonyl chloride | Ald | | 29 | 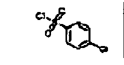 | 4-chlorobenzene-1-sulfonyl chloride | Ald |
| 13 | 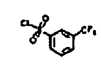 | 3-(trifluoromethyl)-benzene-1-sulfonyl chloride | Ald | | 30 | 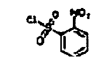 | 2-nitrobenzene-1-sulfonyl chloride | Ald |
| 14 | 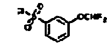 | 3-(difluoromethoxy)-benzene-1-sulfonyl chloride | Ald | | 31 | 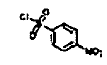 | 4-nitrobenzene-1-sulfonyl chloride | Ald |
| 15 | 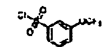 | 3-(trifluoromethoxy)-benzene-1-sulfonyl chloride | Ald | | 32 | 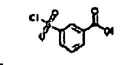 | 3-(chlorosulfonyl)-benzoic acid | Ald |
| 16 | 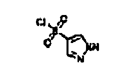 | pyrazole-4-sulfonyl chloride | Matrix | | 33 | 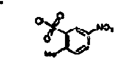 | 5-amino-2-methylbenzene-1-sulfonyl chloride | Ald |
| 17 | 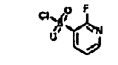 | 2-fluoropyridine-3-sulfonyl chloride | ref-20 | | 34 | 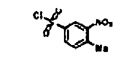 | 4-methyl-3-nitrobenzene-1-sulfonyl chloride | TCI |
| 35 | 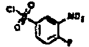 | 4-fluoro-3-nitrobenzene-1-sulfonyl chloride | Apollo | | 43 | 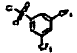 | 3,5-bis(trifluoromethyl)benzene-1-sulfonyl chloride | WAKO |
| 36 | 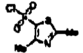 | 2,4-dimethylthiazole-5-sulfonyl chloride | MAYB | | 44 | 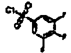 | 3,4,5-trifluorobenzene-1-sulfonyl chloride | Ald |
| 37 | 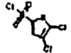 | 4,6-dichlorothiophene-2-sulfonyl chloride | Ald | | 45 | 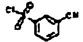 | 3-cyanobenzene-1-sulfonyl chloride | Ald |
| 38 | 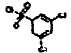 | 3,5-dichlorobenzene-1-sulfonyl chloride | TCI | | 46 | 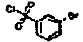 | 3-bromobenzene-1-sulfonyl chloride | AlfaA |
| 39 | 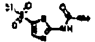 | 2-acetamidothiazole-5-sulfonyl chloride | ASIN | | 47 | 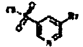 | 5-bromopyridine-3-sulfonyl chloride | MAYB |
| 40 | 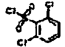 | 2,6-dichlorobenzene-1-sulfonyl chloride | Ald | | 48 | 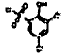 | 3,5-dichloro-2-hydroxybenzene-1-sulfonyl chloride | Ald |
| 41 | 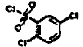 | 2,5-dichlorobenzene-1-sulfonyl chloride | KANTO | | 49 | 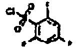 | 2,4,6-trifluorobenzene-1-sulfonyl chloride | Matrix |
| 42 | 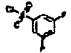 | 3,6-difluorobenzene-1-sulfonyl chloride | Ald | | 50 | 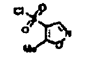 | 5-methyliscxazole-4-sulfonyl chloride | MAYB |

### [Test Case 1-A]

### Measurement of human β3-adrenergic receptor agonist activity

The human β3-adrenergic receptor agonist activity is measured using CHO (Chinese hamster ovary) cells transfected by a human β3 gene inserted in pcDNA3 (Invitrogen). For the human β3 gene, a human β3 fragment is first obtained by PCR using human adipose tissue cDNA (made by Clontech) by β3 primer (Krief et al., J. Clin. Invest., Vol. 91, pp. 344-349 (1993)). A full-length human β3 gene is obtained from a human genomic library (made by Clontech) using this as the probe. These cells are cultured in Ham's F-12 medium containing 10% fetal calf serum and 400 µg/mL genectin (Invitrogen). These cells are sown in 24-well plates to make 1 × 10⁵ cells/well. After culturing for approximately 20 hours, they are allowed to stand for two hours in serum-free Ham's F-12 medium. After first dissolving the test compound by DMSO, it is serially diluted by Ham's F-12 containing 20 mmol/L HEPES, 1 mmol/L isobutylmethylxanthine, and 1 mmol/L ascorbic acid and added to the cells. After culturing for 30 minutes, the medium is removed, and 0.1 mL of 1 N NaOH is added and allowed to stand for 20 minutes. A quantity of 0.1 mL of 1 N acetic acid is added. After stirring, centrifugation is performed, and cAMP is assayed using a cAMP-EIA kit (made by Cayman). Taking the maximum response of isoproterenol, the positive control, as 100%, the ratio of the maximum response of each test compound is calculated as the intrinsic activity [I.A. (%)]. The chemical concentration that gives a response rate of 50% (EC50) is also calculated.

### [Test Case 1-B]

### Measurement of human β3-adrenergic receptor agonist activity

The human β3-adrenergic receptor agonist activity is measured using CHO (Chinese hamster ovary) cells transfected by a human β3 gene inserted in pcDNA3 (Invitrogen). For the human β3 gene, a human β3 fragment is first obtained by PCR using human adipose tissue cDNA (made by Clontech) by β3 primer (Krief et al., J. Clin. Invest., Vol. 91, pp. 344-349 (1993)). A full-length human β3 gene is obtained from a human genomic library (made by Clontech) using this as the probe. These cells are cultured in Ham's F-12 medium containing 10% fetal calf serum and 400 µg/mL genectin (Invitrogen). These cells are sown in 96-well plates to make 2 × 10⁴ cells/well. After culturing for approximately 20 hours, they are allowed to stand for 15 minutes in 80 µL of serum-free Ham's F-12 medium. After first dissolving the test compound by DMSO, it is serially diluted by Ham's F-12 containing 100 mmol/L HEPES and 1 mmol/L isobutylmethylxanthine, and 20 µL is added to the cells. After culturing for 30 minutes, the medium is removed, and 0.1 mL of the assay/lysis buffer contained in the cAMP-Screen kit (made by Applied Bioscience) is added and incubated for 30 minutes at 37°C. The cAMP in this cell lysate is assayed by the above cAMP-Screen kit. Taking the maximum response of isoproterenol, the positive control, as 100%, the ratio of the maximum response of each test compound is calculated as the intrinsic activity [I.A. (%)]. The chemical concentration that gives a response rate of 50% (EC50) is also calculated.

### [Test Case 2-A]

### Measurement of human β1-adrenergic receptor agonist activity

The human β1-adrenergic receptor agonist activity is measured by the same method as in Test Case 1-A using CHO (Chinese hamster ovary) cells transfected by a human β1 gene inserted in pcDNA3 (Invitrogen). Taking the maximum response of isoproterenol, the positive control, as 100%, the ratio of the maximum response of each test compound is calculated as the intrinsic activity [I.A. (%)]. The chemical concentration that gives a response rate of 50% (EC50) is also calculated.

### [Test Case 2-B]

### Measurement of human β1-adrenergic receptor agonist activity

The human β1-adrenergic receptor agonist activity is measured by the same method as in Test Case 1-B using CHO (Chinese hamster ovary) cells transfected by a human β1 gene inserted in pcDNA3 (Invitrogen). Taking the maximum response of isoproterenol, the positive control, as 100%, the ratio of the maximum response of each test compound is calculated as the intrinsic activity [I.A. (%)]. The chemical concentration that gives a response rate of 50% (EC50) is also calculated.

### [Test Case 2-C]

### Measurement of human β1-adrenergic receptor agonist activity

[The human β1-adrenergic receptor agonist activity] can be measured by CELLULAR Functional GPCR·Gs, beta 1 (Ref. 758-28a) listed on p. 54 of the Cerep 2008 CATALOG IN VITRO PHARMACOLOGY & ADME-TOX.

### [Test Case 3-A]

### Measurement of human β2-adrenergic receptor agonist activity

The human β2-adrenergic receptor agonist activity is measured by the same method as in Test Case 1-A using CHO (Chinese hamster ovary) cells transfected by a human β2 gene inserted in pcDNA3 (Invitrogen). Taking the maximum response of isoproterenol, the positive control, as 100%, the ratio of the maximum response of each test compound is calculated as the intrinsic activity [I.A. (%)]. The chemical concentration that gives a response rate of 50% (EC50) is also calculated.

### [Test Case 3-B]

### Measurement of human β2-adrenergic receptor agonist activity

The human β2-adrenergic receptor agonist activity is measured by the same method as in Test Case 1-B using CHO (Chinese hamster ovary) cells transfected by a human β2 gene inserted in pcDNA3 (Invitrogen). Taking the maximum response of isoproterenol, the positive control, as 100%, the ratio of the maximum response of each test compound is calculated as the intrinsic activity [I.A. (%)]. The chemical concentration that gives a response rate of 50% (EC50) is also calculated.

### [Test Case 3-C]

### Measurement of human β2-adrenergic receptor agonist activity

[The human β2-adrenergic receptor agonist activity] can be measured by CELLULAR Functional GPCR·Gs, beta 2 (Ref. 758-55a) listed on p. 55 of the Cerep 2008 CATALOG IN VITRO PHARMACOLOGY & ADME-TOX.

### [Test Case 4-A]

### Measurement of human α1A-adrenergic receptor agonist activity

The human α1A-adrenergic receptor agonist activity is measured using HEK293 cells transfected by a human α1A gene inserted in pcDNA3.1 (Invitrogen). After culturing these cells in DMEM medium containing 10% fetal calf serum, 400 µg/mL of hygromycin B (Gibco BRL), 100 U/mL of penicillin, and 100 µg/mL of streptomycin, they are prepared to make 5 × 106 cells/mL by assay buffer (20 mmol/L HEPES-HOH (pH 7.4), 115 mmol/L NaCl, 5.4 mmol/L KCl, 0.8 mmol/L MgCl₂, 1.8 mmol/L CaCl₂, 13.8 mmol/L D-glucose, 0.1% bovine serum albumin) containing 0.2% Pluronic F-127 (Invitrogen) and 20 µmol/L Fura-2AM (made by Wako Pure Chemical Industries Co., Ltd.). After loading for 30 minutes in a CO₂ incubator, the excess Fura-2AM is removed by washing twice with assay buffer. After preparing the centrifuged cells to make 5 × 10⁶ cells/mL by assay buffer, 80 µL/well is dispensed into 96-well UV plates (made by Corning) to make cell plates. Sample plates with the test compound diluted 10-fold by assay buffer from 10⁻⁵ to 10⁻¹² M and cell plates are set in an FDSS4000 (made by Hamamatsu Photonics). After preincubating for 180 seconds, measurement of fluorescence intensity (excitation wavelength 340 nm, 380 nm, measurement wavelength 500 nm) is begun at 2-second intervals. After approximately 30 seconds of measurement, 20 µL of test sample from a sample plate is added to the cell plate, and measurement is continued for another 270 seconds. The Ca flux caused by the test compound is calculated taking the difference in the maximum value of the fluorescence intensity ratio at 340 nm and 380 nm after adding the test compound and the fluorescence intensity ratio before adding the test compound as the peak height. Taking the maximum response of norepinephrine, the positive control, as 100%, the ratio of the maximum response of each test compound is calculated as the intrinsic activity [I.A. (%)]. The chemical concentration that gives a response rate of 50% (EC50) is also calculated.

### [Test Case 4-B]

### Measurement of human α1A-adrenergic receptor agonist activity

The human α1A-adrenergic receptor agonist activity] can be measured by CELLULAR Functional GPCR**·**Gs, alpha 1A (Ref. 722-36a) listed on p. 52 of the Cerep 2008 CATALOG IN VITRO PHARMACOLOGY & ADME-TOX.

### [Test Case 5]

### Measurement of human α1B-adrenergic receptor agonist activity

For the human α1B-adrenergic receptor agonist activity, a human α1B gene inserted into pcDNA3.1 (Invitrogen) and a p-SRE-Luc plasmid (Strata gene) of a luciferase gene expression vector are transiently transfected into HEK293 cells. 40,000 cells/well are sown in 96-well plates and cultured overnight at 37°C under 5% CO₂ in DMEM medium containing 2% fetal calf serum. A solution prepared by dissolving the test compound in DMSO and subsequently diluting it with medium is added to the cells and reacted for a number of hours. The medium is suctioned off, and 30 µL/well of PicaGene LT2.0 (Toyo Ink) is added. The luminescence value is measured 30 minutes later. Taking the maximum response of phenylephrine, the positive control, as 100%, the ratio of the maximum response of each test compound is calculated as the intrinsic activity [I.A. (%)]. The chemical concentration that gives a response rate of 50% (EC50) is also calculated.

### [Test Case 6]

### Measurement of human α1D-adrenergic receptor agonist activity

For the human α1D-adrenergic receptor agonist activity, a human α1D gene inserted into pcDNA3.1 (Invitrogen) and a p-SRE-Luc plasmid (Strata gene) of a luciferase gene expression vector are temporarily transfected into HEK293 cells. 40,000 cells/well are sown in 96-well plates and cultured overnight at 37°C under 5% CO₂ in DMEM medium containing 2% fetal calf serum. A solution prepared by dissolving the test compound in DMSO and subsequently diluting it with medium is added to the cells and reacted for a number of hours. The medium is suctioned off, and 30 µL/well of PicaGene LT2.0 (Toyo Ink) is added. The luminescence value is measured 30 minutes later. Taking the maximum response of phenylephrine, the positive control, as 100%, the ratio of the maximum response of each test compound is calculated as the intrinsic activity [I.A. (%)]. The chemical concentration that gives a response rate of 50% (EC50) is also calculated.

The results of Test Case 1-A, Test Case 2-A, Test Case 3-A, and Test Case 4-A are shown in Table 3.

The abbreviations in Table 3 are defined as follows.

β3receptor represents human β3-adrenergic receptor agonist activity. β1receptor represents human β1-adrenergic receptor agonist activity. β2receptor represents human β2-adrenergic receptor agonist activity. α1Areceptor represents human α1A-adrenergic receptor agonist activity.

EC50 and IA have the same meanings as described in the above Test Case 1-A, Test Case 2-A, Test Case 3-A, and Test Case 4-A.

N in Table 3 is the number of runs. Specifically, A; n = 1, duplicate, B; n = 1, triplicate, C; n = 2, duplicate, D; n = 2, triplicate, E; n = 3, duplicate, F; n = 3, triplicate.

The term "compound" means the test compound, while "ex" means a working example. For example, "ex1" means Working Example 1. "Z" means a comparative example. For example, Z1 means Comparative Example 1. Comparative examples are compounds described in the International Publication No. WO03/035620 pamphlet. Comparative Example 1 is Working Example 86, Comparative Example 2 is Working Example 88, and Comparative Example 3 is Working Example 90 of this international publication.

It was judged based on the results of Test Case 5 that Z1 does not have α1B agonist activity. Furthermore, it was judged based on the results of Test Case 6 that Z1 also does not have α1D agonist activity.

[Table 3]

**Table 3**

| compound | *β*3 receptor | | | *β*1 receptor | | | *β*2 receptor | | | α1A receptor | | |
|---|---|---|---|---|---|---|---|---|---|---|---|---|
| | N | EC50 [nM] | I.A. [%] | N | EC50 [nM] | I.A. [%] | N | EC50 [nM] | I.A. [%] | N | EC50 [nM] | I.A. [%] |
| Z1 | D | 26 | 64 | D | a) | 7.2 | D | 150 | 23 | A | 252 | 60 |
| Z2 | D | 4.7 | 52 | D | 140 | 22 | D | 18 | 15 | C | 39 | 59 |
| Z3 | D | 14 | 72 | D | 220 | 26 | D | 53 | 20 | C | 402 | 83 |
| isoproterenol | F | 54 | 100 | F | 1.3 | 100 | F | 5.8 | 100 | c) | | |
| Norepinephrine | c) | | | c) | | | c) | | | F | 6.5 | 100 |

| | | | | | | | | | | | | |
|---|---|---|---|---|---|---|---|---|---|---|---|---|
| a);much weaker activities b);Not active c);Not tested | | | | | | | | | | | | |

The results of Test Case 1-B, Test Case 2-B, Test Case 3-B, and Test Case 4-A are shown in Table 4.

The abbreviations in Table 4 are defined as follows.

β3receptors represents human β3-adrenergic receptor agonist activity. β1receptor represents human β1-adrenergic receptor agonist activity. β2receptor represents human β2-adrenergic receptor agonist activity. α1Areceptor represents human α1A-adrenergic receptor agonist activity.

EC50 and IA have the same meanings as described in the above Test Case 1-A, Test Case 2-A, Test Case 3-A, and Test Case 4-A.

N in Table 4 is the number of runs. Specifically, A; n = 1, duplicate, B; n = 1, triplicate, C; n = 2, duplicate, D; n = 2, triplicate, E; n = 3, duplicate, F; n = 3, triplicate.

The term "compound" means the test compound, while "ex" means a working example. For example, "ex1" means Working Example 1. "Z" means a comparative example. For example, Z1 means Comparative Example 1. Comparative examples are compounds described in the International Publication No. WO03/035620 pamphlet. Comparative Example 1 is Working Example 86, Comparative Example 2 is Working Example 88, and Comparative Example 3 is Working Example 90 of this international publication.

It was judged based on the results of Test Case 5 that Z1 does not have α1B agonist activity. Furthermore, it was judged based on the results of Test Case 6 that Z1 also does not have α1D agonist activity.

[Table 4]

**Table 4**

| compound | *β*3 receptor | | | *β*1 receptor | | | *β*2 receptor | | | α1A receptor | | |
|---|---|---|---|---|---|---|---|---|---|---|---|---|
| | N | EC50 [nM] | I.A. [%] | N | EC50 [nM] | I.A. [%] | N | EC50 | I.A. [nM] [%] | N | EC50 [nM] | I.A. [%] |
| Z1 | D | 40 | 67 | D | a) | 2.4 | c) | | | A | 252 | 60 |
| Z2 | c) | | | c) | | | c) | | | C | 39 | 59 |
| Z3 | c) | | | c) | | | c) | | | C | 402 | 83 |
| ex1 | B | 0.52. | 110 | B | a) | 6 | B | a) | 1 | A | b) | 0 |
| ex2 | B | 3.5 | 109 | B | a) | 6 | B | a) | 3 | A | b) | 0 |
| ex3 | B | 17 | 105 | B | a) | 4 | B | a) | 1 | A | b) | 0 |
| ex4 | B | 38 | 77 | B | a) | 2 | B | a) | 1 | A | b) | 0 |
| ex5 | B | 90 | 90 | B | a) | 5 | B | b) | 0 | A | b) | 0 |
| ex6 | B | 0.70 | 84 | B | a) | 1 | B | a) | 7 | A | b) | 0 |
| ex11 | B | 32 | 115 | B | a) | 3 | B | a) | 3 | A | b) | 0 |
| ex12 | B | 31 | 52 | B | a) | 5 | B | a) | 5 | A | b) | 0 |
| ex13 | B | 9.4 | 82 | B | a) | 4 | B | a) | -4 | A | b) | 0 |
| ex14 | c) | | | c) | | | c) | | | A | b) | 0 |
| ex15 | c) | | | c) | | | c) | | | A | b) | C |
| ex17 | B | 39 | 70 | B | a) | 1 | B | a) | 12 | A | a) | 13 |
| ex24 | B | d) | 41 | c) | | | c) | | | o) | | |
| ex40 | B | d) | 5C | c) | | | o) | | | c) | | |
| ex41 | B | 0.21 | 76 | B | a) | 12 | B | a) | 5 | C | b) | 0 |
| ex42 | D | 3.8. | 83 | B | a) | 4 | B | b) | 0 | C | b) | 0 |
| ex43 | D | 15 | 65 | B | a) | -2 | B | a) | -5 | C | b) | 0 |
| ex44 | D | 13 | 58 | B | a) | 9 | B | a) | 5 | C | b) | 0 |
| ex45 | D | 2.4 | 80 | B | a) | 9 | B | a) | 2 | C | b) | 0 |
| ex46 | D | 2.4 | 78 | B | a) | 5 | B | a) | -2 | C | b) | 0 |
| ex47 | B | 41 | 72 | B | a) | 3 | B | a) | 16 | C | 1224 | 28 |
| ex48 | B | 27 | 48 | c) | | | o) | | | C | a) | 29 |
| ex49 | B | 2.5 | 98 | B | a) | 10 | B | a) | -3 | C | b) | 0 |
| ex50 | B | d) | 18 | c) | | | c) | | | A | b) | 0 |
| ex51 | B | 148 | 64 | a) | | | c) | | | A | b) | 0 |
| ex52 | B | 248 | 62 | c) | | | c) | | | A | b) | 0 |
| ex53 | B | 4.1 | 98 | B | a) | 14 | B | a) | 2 | A | b) | 0 |
| ex54 | B | 125 | 48 | c) | | | c) | | | A | b) | 0 |
| ex61 | B | 0.2 | 64 | B | a) | 17 | B | a) | -3 | A | b) | 0 |
| ex64 | B | 208 | 66 | c) | | | c) | | | c) | | |
| ex65 | B | 579 | 65 | c) | | | c) | | | c) | | |
| ex66 | B | 291 | 86 | c) | | | c) | | | c) | | |
| ex69 | B | 594 | 72 | c) | | | c) | | | c) | | |
| ex77 | B | d) | 59 | c) | | | c) | | | c) | | |
| ex79 | B | 54 | 84 | B | a) | 7 | 8 | a) | 7 | A | b) | 0 |
| ex82 | B | 16 | 71 | B | a) | 7 | 8 | a) | 2 | A | a) | 13 |
| ex86 | B | 401 | 60 | c) | | | c) | | | c) | | |
| ex104 | B | 0.42 | 110 | B | a) | 6 | B | a) | 4 | A | b) | 0 |
| ex113 | B | 22 | 108 | c) | | | B | a) | 3 | c) | | |
| ex117 | B | d) | 54 | c) | | | c) | | | C | b) | 0 |
| ex118 | D | 0.25 | 77 | B | a) | 13 | B | a) | -1 | C | b) | 0 |
| ex119 | B | 23 | 47 | c) | | | c) | | | C | b) | 0 |
| ex120 | B | 131 | 32 | c) | | | c) | | | C | b) | |
| ex121 | D | 2.8 | 53 | c) | | | c) | | | C | b) | 0 |
| ex122 | D | 0.91 | 59 | c) | | | c) | | | C | b) | |
| ex123 | B | 0.67 | 50 | c) | | | c) | | | C | b) | 0 |
| ex124 | B | 13 | 52 | B | a) | -6 | B | a) | -1 | C | b) | 0 |
| ex125 | B | 14 | 34 | c) | | | c) | | | C | a) | 15 |
| ex126 | B | 14 | 86 | B | a) | 8 | | B a) | 1 | A | b) | 0 |
| ex135 | B | 28 | 105 | c) | | | B | a) | 7 | c) | | |
| ex149 | B | 12 | 100 | c) | | | B | b) | 0 | c) | | |
| ex152 | B | 22 | 104 | B a) | | 3 | B | a) | 15 | A | a) | 8 |
| ex153 | B | 0.22 | 95 | B | a) | 12 | B | a) | 13 | A | b) | 0 |
| ex181 | B | 18 | 80 | B | a) | 1 | B | a) | 6 | A | a) | 4 |
| ex183 | B | d) | 72 | c) | | | c) | | | | c) | |
| ex212 | B | 29 | 95 | B | a) | 6 | B | a) | 15 | A | b) | 0 |
| ex231 | B | 0.20 | 124 | B | a) | 18 | B | a) | 3 | A | b) | 0 |
| ex232 | B | 0.20 | 104 | B | a) | 19 | B | a) | 6 | A | b) | 0 |
| ex235 | B | 28 | 113 | B | a) | 19 | B | a) | 2 | A | b) | 0 |
| ex236 | B | 1.0 | 58 | B | a) | 11 | B | a) | 5 | A | b) | 0 |
| ex237 | B | 293 | 58 | c) | | | c) | | | A | b) | 0 |
| ex239 | B | 16 | 108 | B | a) | 15 | B | a) | 2 | A | b) | 0 |
| ex241 | B | 43 | 94 | B | a) | 9 | B | a) | 8 | A | b) | 0 |
| ex150 | B | 5.4 | 85 | c) | | | c) | | | B | 1280 | 61 |
| ex38 | B | 31 | 89 | c) | | | c) | | | B | 376 | 75 |
| ex115 | B | 16 | 86 | c) | | | c) | | | B | 1100 | 35 |
| ex210 | B | 17 | 88 | c) | | | c) | | | B | 1700 | 54 |
| ex179 | B | 17 | 93 | o) | | | c) | | | B | 436 | 28 |
| ex234 | B | 7.1 | 78 | c) | | | c) | | | B | 966 | 37 |
| ex228 | B | 5.7 | 91 | c) | | | c) | | | B | 981 | 41 |
| ex233 | B | 10 | 73 | c) | | | c) | | | B | a) | 9 |
| ex243 | B | 72 | 72 | c) | | | c) | | | B | 44 | 89 |
| ex228 | B | 14 | 47 | c) | | | c) | | | B | b) | 0 |
| ex227 | B | 31 | 65 | c) | | | c) | | | B | a) | 1 |
| ex107 | B | 101 | 75 | c) | | | c) | | | B | a) | 1 |
| ex158 | B | 82 | 90 | c) | | | c) | | | B | b) | 0 |
| ex189 | B | 65 | 91 | c) | | | c) | | | B | a) | 1 |
| ex71 | B | 152 | 98 | c) | | | c) | | | B | a) | 2 |
| ex72 | B | 8.3 | 92 | c) | | | c) | | | B | a) | 1 |
| isoproterenol | n=52 triplicate | 98 | 100 | n=16 triplicate | 1.4 | 100 | n=17 triplicate | 11.1 | 100 | c) | | |
| Norepinephrine | | c) | | c) | | | c) | | | F | 6.5 | 100 |

| | | | | | | | | | | | | |
|---|---|---|---|---|---|---|---|---|---|---|---|---|
| a);much weaker activities b):Not active c);Not tested d);Not calculate | | | | | | | | | | | | |

The results of Test Case 2-C are shown in Table 5.

The abbreviations in Table 5 are defined as follows.

The terms "compound," "Z," and "ex" are as defined above.

The term "concentration" means the concentration of the compound while "n" means the number of runs.

The % of control agonist response is the ratio of each test compound calculated taking the maximum response of isoproterenol, the positive control, as 100%.

[Table 5]

**Table 5 β 1 receptor**

| compound | concentration | n | % of Control Agonist Response |
|---|---|---|---|
| Z1 | 10 *µ* M | 2 | 13 |
| Z2 | 10 *µ* M | 2 | 44 |
| ex23 | 10 *µ* M | 2 | 2.1 |
| ex24 | 10 *µ* M | 2 | 6.1 |
| ex7 | 10 *µ* M | 2 | 15 |
| ex58 | 10 *µ* M | 2 | 18 |
| ex71 | 10 *µ* M | 2 | 32 |
| ex72 | 10 *µ* M | 2 | 19 |
| ex85 | 10 *µ* M | 2 | 30 |
| ex86 | 10 *µ* M | 2 | 8.8 |
| ex106 | 10 *µ* M | 2 | 11 |
| ex107 | 10 *µ* M | 2 | 8.7 |
| ex113 | 10 *µ* M | 2 | 3.7 |
| ex128 | 10 *µ* M | 2 | 10 |
| ex134 | 10 *µ* M | 2 | 7.0 |
| ex135 | 10 *µ* M | 2 | 3.7 |
| ex148 | 10 *µ* M | 2 | 15 |
| ex149 | 10 *µ* M | 2 | 5.2 |
| ex155 | 10 *µ* M | 2 | 12 |
| ex156 | 10 *µ* M | 2 | 12 |
| ex157 | 10 *µ* M | 2 | 7.0 |
| ex158 | 10 *µ* M | 2 | 17 |
| ex167 | 10 *µ* M | 2 | 6.4 |
| ex185 | 10 *µ* M | 2 | 13 |
| ex186 | 10 *µ* M | 2 | 7.2 |
| ex198 | 10 *µ* M | 2 | 3.5 |
| ex199 | 10 *µ* M | 2 | 8.0 |
| ex215 | 10 *µ* M | 2 | 23 |
| ex242 | 10 *µ* M | 2 | 9.4 |
| isoproterenol | 10 *µ* M | 2 | 100 |

The results of Test Case 3-C are shown in Table 6.

The terms "compound," "Z," and "ex" are as defined above.

The % of control agonist response is the ratio of each test compound calculated taking the maximum response of isoproterenol, the positive control, as 100%.

[Table 6]

**Table 6 β 2 receptor**

| compound | concentration | n | % of Control Agonist Response |
|---|---|---|---|
| Z1 | 10 *µ* M | 2 | 8.2 |
| Z2 | 10 *µ* M | 2 | 17 |
| ex23 | 10 *µ* M | 2 | 0.5 |
| ex24 | 10 *µ* M | 2 | 1.2 |
| ex7 | 10 *µ* M | 2 | 3.7 |
| ex58 | 10 *µ* M | 2 | 1.8 |
| ex71 | 10 *µ* M | 2 | 1.5 |
| ex72 | 10 *µ* M | 2 | 1.8 |
| ex85 | 10 *µ* M | 2 | 1.0 |
| ex86 | 10 *µ* M | 2 | -0.7 |
| ex106 | 10 *µ* M | 2 | 2.1 |
| ex107 | 10 *µ* M | 2 | -0.6 |
| ex113 | 10 *µ* M | 2 | 0.2 |
| ex128 | 10 *µ* M | 2 | 0.3 |
| ex134 | 10 *µ* M | 2 | 2.0 |
| ex135 | 10 *µ* M | 2 | 1.3 |
| ex148 | 10 *µ* M | 2 | 0.8 |
| ex149 | 10 *µ* M | 2 | 0.2 |
| ex155 | 10 *µ* M | 2 | 2.2 |
| ex156 | 10 *µ* M | 2 | 3.5 |
| ex157 | 10 *µ* M | 2 | 3.2 |
| ex158 | 10 *µ* M | 2 | 1.6 |
| ex167 | 10 *µ* M | 2 | 0.3 |
| ex185 | 10 *µ* M | 2 | 0.3 |
| ex186 | 10 *µ* M | 2 | 1.9 |
| ex198 | 10 *µ* M | 2 | -0.5 |
| ex199 | 10 *µ* M | 2 | 8.8 |
| ex215 | 10 *µ* M | 2 | 0.5 |
| ex242 | 10 *µ* M | 2 | -0.2 |
| isoproterenol | 10 *µ* M | 2 | 100 |

The results of Test Case 4-B are shown in Table 7.

The abbreviations in Table 7 are defined as follows.

The terms "compound," "concentration," "n," "Z," and "ex" are as defined above.

The % of control agonist response is the ratio of each test compound calculated taking the maximum response of epinephrine or norepinephrine, the positive control, as 100%.

[Table 7]

**Table 7 α 1A receptor**

| compound | concentration | n | % of Control Agonist Response | |
|---|---|---|---|---|
| | | | Control; Epinephrine | Control; Norepinephrine |
| Z1 | 10 *µ* M | 2 | 62 | 54 |
| Z2 | 10 *µ* M | 2 | 59 | 51 |
| ex23 | 10 *µ* M | 2 | 32 | 27 |
| ex24 | 10 *µ* M | 2 | 5.6 | 4.8 |
| ex7 | 10 *µ* M | 2 | 25 | 21 |
| ex58 | 10 *µ* M | 2 | 0.6 | 0.5 |
| ex71 | 10 *µ* M | 2 | 4.3 | 3.7 |
| ex72 | 10 *µ* M | 2 | 6.8 | 5.9 |
| ex85 | 10 *µ* M | 2 | 0.1 I | 0.1 |
| ex88 | 10 *µ* M | 2 | -0.4 | -0.3 |
| ex106 | 10 *µ* M | 2 | -0.2 | -0.2 |
| ex107 | 10 *µ* M | 2 | 17 | 14 |
| ex113 | 10 *µ* M | 2 | 6.9 | 6.0 |
| ex128 | 10 *µ* M | 2 | 16 | 14 |
| ex134 | 10 *µ* M | 2 | 7.0 | 6.0 |
| ex135 | 10 *µ* M | 2 | 0 | 0 |
| ex148 | 10 *µ* M | 2 | -0.2 | -0.2 |
| ex149 | 10 *µ* M | 2 | 8.5 | 7.3 |
| ex155 | 10 *µ* M | 2 | 0 | 0 |
| ex156 | 10 *µ* M | 2 | 3.9 | 3.4 |
| ex157 | 10 *µ* M | 2 | 8.1 | 7.0 |
| ex158 | 10 *µ* M | 2 | 7.6 | 6.7 |
| ex167 | 10 *µ* M | 2 | 26 | 22 |
| ex185 | 10 *µ* M | 2 | 6.4 | 6 |
| ex186 | 10 *µ* M | 2 | 0.6 | 0.5 |
| ex198 | 10 *µ* M | 2 | 19 | 17 |
| ex199 | 10 *µ* M | 2 | 17 | 15 |
| ex215 | 10 *µ* M | 2 | 0.1 | 0.1 |
| ex242 | 10 *µ* M | 2 | 0.7 | 0.6 |
| Norepinephrine | 10 *µ* M | 2 | 116 | 100 |
| Epinephrine | 10 *µ* M | 2 | 100 | - |

### [Test Case 7]

Common marmoset isolated bladder smooth muscle relaxation test
The study is conducted using the British Journal of Pharmacology, 1997, No. 122, pp. 1720-1724 as a reference. The common marmoset isolated bladder smooth muscle-relaxing effect of the test compounds can be verified.
After sacrificing common marmosets (Japan CLEA) by exsanguination, they are laparotomized and the urinary bladder is removed. Smooth muscle specimens are prepared from the isolated bladder and suspended in an organ bath filled with 10 mL of Krebs-Henseleit solution ventilated by a mixed gas of 95% O₂ and 5% CO₂. The specimens are loaded with one gram of resting tension and stabilized for 30 or more minutes. After the resting tension of the specimen has stabilized, a final concentration of 40 mmol/L of KCl is added repeatedly, and it is confirmed that the contraction in response to KCl has basically become constant. After the specimen has been contracted by a final concentration of 40 mmol/L of KCl and the developed tension has stabilized, the test compound is added cumulatively in a 10-fold ratio (at 20-minute intervals), and the relaxation response is observed. The final concentrations are to be 10⁻⁹, 10⁻⁸, 10⁻⁷, 10⁻⁶, 10⁻⁵, and 10⁻⁴ mol/L. After the relaxation response to the maximum concentration of test compound has ended, the maximum relaxation response of each specimen is determined by adding a final concentration of 10⁻⁴ mol/L of papaverine. Taking that relaxation response as 100%, the relaxation rate (%) at test compound concentrations of 10⁻⁵ and 10⁻⁴ mol/L is calculated.

The results of Test Case 7 are shown in Table 8.

The abbreviations in Table 8 are defined as follows.

The term "n" means the number of runs, and "relaxant activity (%)" means the relaxation rate (%). The terms "compound" and "ex" are as defined above.

[Table 8]

**Table 8**

| compound | relaxant activity (%) | | |
|---|---|---|---|
| | n | 10⁻⁵ M | 10⁻⁴ M |
| ex3 | 2 | 36.6 | 63.2 |
| ex4 | 2 | 30.1 | 53.7 |
| ex235 | 2 | 24.3 | 55.3 |
| isoproterenol | 2 | 50.6 | 54.4 |

### [Test Case 8]

### Human isolated bladder smooth muscle relaxation test

The study is conducted using the Journal of Urology, 2003, No. 170, pp. 649-653 as a reference. The human isolated bladder smooth muscle-relaxing effect of the test compounds can be verified. Specifically, smooth muscle specimens from isolated human bladder are suspended in an organ bath filled with Krebs-Henseleit solution ventilated by a mixed gas of 95% O₂ and 5% CO₂. The specimens are loaded with one gram of resting tension and stabilized for 30 or more minutes. After the resting tension of the specimen has stabilized, a final concentration of 0.1 µmol/L of carbachol is added repeatedly, and it is confirmed that the contraction in response to carbachol has basically become constant. After the specimen has been contracted by a final concentration of 0.1 µmol/L of carbachol and the developed tension has stabilized, the test compound is added cumulatively in a 10-fold ratio at 10-minute intervals, and the relaxation response is observed. The final concentrations are to be 10⁻⁹, 10⁻⁸, 10⁻⁷, 10⁻⁶, 10⁻⁵, and 10⁻⁴ mol/L. After the relaxation response to the maximum concentration of the test compound has ended, the maximum relaxation response of each specimen is determined by adding a final concentration of 10⁻⁴ mol/L of papaverine. Taking that relaxation response as 100%, the relaxation rate (%) is calculated.

### [Test Case 9]

### Effects on the blood pressure and heart rate in pentobarbital-anesthetized rats

The blood pressure and heart rate of rats under pentobarbital anesthesia are measured, and the effects of rapid intravenous administration of the test compound on the blood pressure and heart rate can be studied. After anesthetizing male SD rats (Japan SLC) by intraperitoneal administration of 50 mg/kg of pentobarbital sodium (Tokyo Chemical Industry Co., Ltd.), 25 mg/kg of pentobarbital sodium is administered subcutaneously as maintenance anesthesia. The left femoral vein is exposed and stripped, and a SP10 polyethylene tube filled with saline (connected to a three-way stopcock via a ¼ venous needle) is inserted and left in place in the vein.
The inside of the left thigh is cut, and the femoral artery is exposed and stripped. A polyethylene tube filled with heparinized physiological saline solution (SP31, connected to a three-way stopcock via a Terumo injection needle 22G) is inserted and connected to a pressure transducer. The blood pressure is measured from the pressure transducer via a strain pressure amplifier (AP-641G, Nihon Kohden). The heart rate is measured with the pulse wave of the blood pressure as a trigger via a heart rate counter (AT-601G, Nihon Kohden). The blood pressure, mean blood pressure, and heart rate are output to a recorder and recorded. Furthermore, the mean blood pressure is recorded via the strain pressure amplifier (AP-641G) using the formula {diastolic blood pressure = (systolic blood pressure - diastolic blood pressure)/3}.

After beginning measurement of the blood pressure and heart rate and confirming that the respective values have basically become constant, 3 mg/kg of test compound is administered over 30 seconds from the left femoral vein. Specifically, 3 mg/mL of test compound is administered rapidly at a volume of 1 mL/kg. The relative value (%) of the values at each point in time versus the mean blood pressure and heart rate before beginning administration are determined for each individual, and the mean±standard deviation of the relative value (%) at the time of the maximum change in each parameter is determined.

The results of Test Case 9 are shown in Table 9.

The abbreviations in Table 9 are defined as follows.

The term "n" is the number of runs, while "compound," "ex," and "Z" are as defined above. "MBP (mean blood pressure)" means the mean blood pressure.

[Table 9]

**Table 9**

| compound | n | increase in MBP (%) |
|---|---|---|
| Z1 | 3 | 12.8±4.4 |
| Z2 | 3 | 12.6±4.1 |
| ex2 | 3 | 4.9±0.9 |
| ex3 | 3 | 2.0±1.6 |
| ex4 | 3 | 2.4±2.2 |
| ex5 | 3 | 5.1±2.4 |
| ex 11 | 3 | 6.1±3.7 |
| ex41 | 3 | 5.7±0.8 |
| ex44 | 3 | 5.7±3.0 |
| ex45 | 3 | 3.9±2.1 |
| ex46 | 3 | 5.6±1.1 |
| ex61 | 3 | 4.7±1.2 |
| ex79 | 3 | 4.2±1.8 |
| ex82 | 3 | 5.7±0.8 |
| ex113 | 3 | 3.3±1.7 |
| ex135 | 3 | 5.6±1.2 |
| ex149 | 3 | 2.1±1.9 |
| ex212 | 3 | 2.1±1.7 |
| ex235 | 3 | 3.4±1.0 |

### [Test Case 10]

### Mouse piloerection test

Male C57BL/6 Cr mice (Japan SLC) are used. The dissolved compound is administered rapidly via a caudal vein. The animal is placed on the observation stand 5, 15, 30, and 60 minutes after administration, and the state of piloerection is observed. The state of piloerection is evaluated by the following 7-level scoring system according to the examples by the method of Irwin (Psychopharmacologia 1968, 13; 222-257).

Scoring system: "Score 0": No piloerection at all; "Score 1": Slight piloerection (piloerection on part of the body such as the back or neck); "Score 2": Moderate piloerection (piloerection over the entire body); "Score 3": Marked piloerection (piloerection over the entire body, conspicuous to the extent that the skin can be seen).

"Score 0.5" is between "Score 0" and "Score 1" "Score 1.5" is between "Score 2" and "Score 3". "Score 2.5" is between "Score 2" and "Score 3."

The results of Test Case 10 are shown in Table 10 as the number of experimental animals matching each score at that time.

The abbreviations in Table 10 are defined as follows.

The term "n" means the number of runs, while "compound" and "ex" are as defined above.

The term "Dose" means the dose, "Score" means the scoring system as defined above, and "Observation time" means the observation time; the unit is minutes. - means not applicable.

[Table 10]

**Table 10**

| Compound | Dose (mg/kg, i.v.) | n | Score | Observation time (min) | | | |
|---|---|---|---|---|---|---|---|
| | | | | 5 | 15 | 30 | 60 |
| Control | - | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex3 | 10 | 4 | 0 | 4 | 3 | 3 | 4 |
| | | | 0.5 | - | 1 | 1 | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex4 | 10 | 4 | 0 | 4 | 4 | 3 | 3 |
| | | | 0.5 | - | - | 1 | 1 |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex5 | 10 | 4 | 0 | 2 | 2 | 2 | 2 |
| | | | 0.5 | 2 | 2 | 2 | 2 |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex6 | 10 | 4 | 0 | 1 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex11 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex44 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex45 | 10 | 4 | 0 | 1 | - | 1 | 1 |
| | | | 0.5 | 1 | 3 | 2 | 2 |
| | | | 1 | 1 | 2 | 1 | 1 |
| | | | 1.5 | - | - | - | - |
| | | | 2 | - | - | - | - |
| | | | 2.5 | - | - | - | - |
| | | | 3 | - | - | - | - |
| ex46 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex61 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex79 | 10 | 4 | 0 | 3 | 3 | 3 | 4 |
| | | | 0.5 | 1 | 1 | 1 | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex113 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex124 | 10 | 4 | 0 | 2 | - | 2 | 2 |
| | | | 0.5 | 1 | 3 | 2 | 2 |
| | | | 1 | 1 | 1 | - | - |
| | | | 1.5 | - | - | - | - |
| | | | 2 | - | - | - | - |
| | | | 2.5 | - | - | - | - |
| | | | 3 | - | - | - | - |
| ex135 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex149 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex212 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |
| ex235 | 10 | 4 | 0 | 4 | 4 | 4 | 4 |
| | | | 0.5 | - | - | - | - |
| | | | 1 | - | - | - | - |
| | | | 1.5 | - | - | - | - |
| | | | 2 | - | - | - | - |
| | | | 2.5 | - | - | - | - |
| | | | 3 | - | - | - | - |

| | | | | | | | |
|---|---|---|---|---|---|---|---|
| **Criteria of scoring** **0: None** **1: Produced slight piloerection** **2: Produced mild piloerection** **3: Produced severe piloerection** | | | | | | | |

### [Test Case 11]

### Saturation solubility in pure water

The test compound is prepared to reach a state of saturation in pure water. The solution is shaken for one hour at room temperature. After shaking in a filter tube, the entire amount of solution is transferred and centrifugally filtered at room temperature. The filtrate is analyzed by HPLC, and the saturation solubility of the test compound is determined from the peak area using a calibration curve.

The standard solution is prepared by measuring out each test compound exactly and preparing a solution that dissolves adequately in pure water. The calibration curve is produced by plotting the concentration of the standard solution on the horizontal axis and the HPLC area value at that concentration on the vertical axis.
A YMC-Pack C18 (4.6 mm × 150 mm) (made by YMC) is used as the separatory column. Detection is carried out by UV-254 nm. The temperature inside the column is 40°C. Elution is performed at a flow rate of 1 mL/min, using solution A = water [containing 0.1% (v/v) acetic acid] and solution B = acetonitrile as solvents. After running a 5-98% (v/v) linear gradient of solution B from 0 to 20 minutes, elution is performed by 98% solution B up to 25 minutes and by 5% solution B from 25.01 to 35 minutes.

### [Test Case 12]

### Solubility test in pH 1.2 hydrochloric acid buffer

Exactly 500 µg of test compound is measured out and added to pH 1.2 hydrochloric acid buffer to make 1000 µg/mL. This solution is shaken for one hour at 37°C. After shaking in a filter tube, the entire amount of solution is transferred and centrifugally filtered at room temperature. The filtrate is analyzed by HPLC, the peak area of the filtrate is divided by the peak area of the standard solution, and the solubility of the test compound is determined.

The standard solution is prepared by measuring out exactly 500 µg of test compound and dissolving it in DMSO solution to make 1 mg/mL.

A YMC-Pack C18 (4.6 mm × 150 mm) (made by YMC) is used as the separatory column. Detection is carried out by UV-254 nm. The temperature inside the column is 40°C. Elution is performed at a flow rate of 1 mL/min, using solution A = water [containing 0.1% (v/v) acetic acid] and solution B = acetonitrile as solvents. After running a 5-98% (v/v) linear gradient of solution B from 0 to 20 minutes, elution is performed by 98% solution B up to 25 minutes and by 5% solution B from 25.01 to 35 minutes.

### [Test Case 13]

### Solubility test in physiological saline solution

The test is conducted in the same way as in Test Case 12 using physiological saline solution instead of pH 1.2 hydrochloric acid buffer, and the solubility of the test compound is determined.

### [Test Case 14]

### Stability test in pure water

The test compound is prepared to reach a state of saturation in pure water. This solution is shaken for one hour at room temperature. After shaking in a filter tube, the entire amount of solution is transferred and centrifugally filtered at room temperature. The filtrate is analyzed by HPLC immediately, after 24 hours, and after 48 hours, and the stability of the test compound is determined from the peak area values using a calibration curve.

The standard solution is prepared by measuring out each test compound exactly and preparing a solution that dissolves adequately in pure water. The calibration curve is produced by plotting the concentration of the standard solution on the horizontal axis and the HPLC area value at that concentration on the vertical axis.
A YMC-Pack C18 (4.6 mm × 150 mm) (made by YMC) is used as the separatory column. Detection is carried out by UV-254 nm. The temperature inside the column is 40°C. Elution is performed at a flow rate of 1 mL/min, using solution A = water [containing 0.1% (v/v) acetic acid] and solution B = acetonitrile as solvents. After running a 5-98% (v/v) linear gradient of solution B from 0 to 20 minutes, elution is performed by 98% solution B up to 25 minutes and by 5% solution B from 25.01 to 35 minutes.

### [Test Case 15]

### Stability test in pH 6.8 phosphate buffer

The test is conducted in the same way as in Test Case 14 using pH 6.8 phosphate buffer instead of pure water, and the stability of the test compound is determined.

### [Test Case 16]

### Cytotoxicity test using frozen hepatocytes

A cytotoxicity test is carried out using bound cryopreserved human hepatocytes, and the hepatocytotoxicity can be studied. A vial of cryopreserved human hepatocytes is thawed in a 37°C thermostatic bath and quickly added to prewarmed CHRM medium (made by Invitrogen). After centrifuging (100 × g) for 10 minutes at room temperature, the supernatant is removed and the cells are suspended in medium for sowing (William's medium E (made by GIBCO), 5% fetal calf serum (made by GIBCO), 10⁻⁹M insulin (made by Sigma), 10⁻⁹M dexamethasone (made by Wako Pure Chemical Industries Co., Ltd.)). After calculating the cell count and survival rate using 0.4% trypan blue stain (made by GIBCO), [the culture is] diluted to 5 × 10⁵ cells/mL by medium for sowing and sown in a quantity of 0.5 mL/well (2.5 × 10⁵ cells/well) in 24-well plates coated by Matrigel (made by Japan BD). After sowing, [the plates] are allowed to stand for two hours in a CO₂ incubator. After two hours, the medium is exchanged (500 µL) with serum-free medium for culture (William's medium E, 10⁻⁹M insulin, 10⁻⁹M dexamethasone, 5 KIU aprotinin (made by Wako Pure Chemical Industries Co., Ltd.)) and culture is carried out for 12 or more hours. A test compound solution 100 times the concentration to be added is prepared and added to the serum-free medium to make a 1% concentration. Serum-free medium is also prepared with 1% concentrations of the solvent used and Triton X-100 added. After culturing for 12 or more hours, the medium is exchanged (500 µL) by each of the above serum-free media, and culture is carried out for 24 hours after exchanging the medium. After culturing for 24 hours, the medium is recovered in a microtube. After recovering the medium, the wells are washed once with D-PBS (made by Wako Pure Chemical Industries Co., Ltd.). 100 µL/well of 1 N NaOH solution is added, and the cells are dissolved. The cell lysate is similarly recovered in a microtube. The recovered medium is centrifuged for 15 minutes at 4°C at approximately 450 × g, and the supernatant is recovered. The cell lysate is centrifuged for 10 minutes at 4°C at approximately 5000 × g, and the supernatant is recovered.

### [Measurement of cytotoxicity (LDH release)]

The hepatocytotoxicity of treatment by the test compound is measured by the LDH release rate in the medium. The centrifuged supernatant of the recovered medium is diluted as is appropriate by D-PBS, and 100 µL aliquots of the diluted solution are dispensed into 96-well plates. 100 µL of the reagent of an LDH Cytotoxicity Detection Kit (made by Takara Bio Inc.) are added to each, and [the plates] are incubated for 30 minutes at 37°C shielded from light. After 30 minutes, the OD₄₉₂ is measured using a SpectraMax 250 (made by Molecular Device). Taking the cytotoxicity of the solvent control as 0% and the cytotoxicity of 1% Triton X-100 as 100%, the cytotoxicity due to treatment by the test compound is calculated.

### (Measurement of liver escape enzymes)

The AST (aspartic aminotransferase) and ALT (alanine aminotransferase) activity that has escaped into the medium is measured, and the hepatocytotoxicity of the test compound is evaluated. The liver escape enzyme activity is measured using an autoanalyzer TBA-120FR (made by Toshiba Medical Systems Corp.). R1 and R2 of L-Type Wako GOT·J2 F (made by Wako Pure Chemical Industries Co., Ltd.) are used as measurement reagents for the AST activity, and R1 and R2 of L-Type Wako GPT·J2 F (made by Wako Pure Chemical Industries Co., Ltd.) are used as measurement reagents for the ALT activity. Enzyme calibrator (made by Wako Pure Chemical Industries Co., Ltd.) is used as the enzyme standard solution.

### [Measurement of albumin synthesis level]

The albumin level secreted in the medium is measured by ELISA, and the changes in hepatocyte function due to treatment by the test compound are evaluated. 100 µL/well of unlabeled anti-human albumin antibody (made by Cappel) is dispensed into immunoplates (made by NUNC) and immobilized by being allowed to stand for approximately 12 hours at 4°C. After approximately 12 hours, the wells are washed four times by assay buffer (0.22% gelatin, 50mM phosphate buffer, 150mM NaCl, 0.05% Proclin 300 (made by SUPELCO)), and 100 µL of the recovered medium properly diluted by assay buffer is then dispensed. After incubating for four hours at 4°C, the wells are washed four times with assay buffer, and 100 µL of horseradish peroxidase-labeled anti-human antibody (made by Cappel) diluted 5000-fold by assay buffer is dispensed. After standing for three hours at 4°C, the wells are washed four times by assay buffer. After washing, 100 µL/well of coloring solution (40 mL of 50mM citrate-phosphate buffer, 10 mg of o-phenylenediamine, 7 µL of 30% aqueous hydrogen peroxide) is dispensed, and [the plates are] incubated for 30 minutes while shaking at 37°C shielded from light. After 30 minutes, the coloring reaction is stopped by adding 100 µL of 1 N H₂SO₄ solution, and the OD₄₉₂ is measured using a SpectraMax 250. The albumin level in the medium is calculated by human albumin (made by Rockland) standard solution.

### [Measurement of ATP level]

The ATP level of the cell lysate is measured, and the cytotoxicity due to treatment by the test compound is evaluated. The cell lysate is mixed with the reagent of a Cell Titer-Glo Luminescent Cell Viability Assay (made by Promega) and dispensed into white plates. The luminescence is measured using Fluoriskan Ascent FL (made by Thermo Scientific), and the ATP level of the cell lysate is calculated by ATP made by Wako Pure Chemical Industries Co., Ltd.) standard solution.

### [Measurement of cell protein level]

The protein level of the cell lysate is measured to correct each measured value by the cell protein level. A Micro BCA Protein Assay Kit (made by Thermo Scientific) is used in measurement. A quantity of 150 µL of cell lysate that has been properly diluted by D-PBS is dispensed into 96-well plates. It is mixed with an equal amount of reagent of the Micro BCA Protein Assay Kit and allowed to stand for two hours at 37°C. After two hours, the OD₅₆₂ is measured using a SpectraMax 250. The protein level of the cell lysate is calculated by BSA (bovine serum albumin) standard solution.

### INDUSTRIAL APPLICABILITY

The compounds of the present invention shown by the general formula (I), possible stereoisomers and racemates thereof, pharmaceutically acceptable salts of these, hydrates and/or solvates of these, and crystals of these are β3-adrenergic receptor agonists and are therefore useful in the prevention and treatment of diabetes, obesity, hyperlipidemia, depression, cholelithiasis, diseases caused by biliary hyperkinesia, diseases caused by hyperfunction of the gastrointestinal tract, diseases associated with decreased lacrimation, interstitial cystitis, overactive bladder and urinary incontinence, and the like and can be utilized in the pharmaceutical industry.

## Claims

1. A compound shown by the following general formula (I) 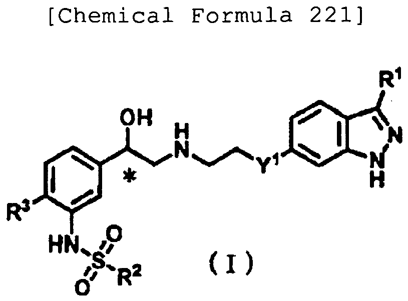 (in general formula (I), R¹ is a lower alkyl group, optionally substituted cyclic lower alkyl group, halogen atom, perfluoroalkyl group, -OR⁴-, -CH(R⁵)OR⁶, or - (CH₂)ₙCONR⁷⁻¹R⁷⁻²; R² is an optionally substituted cyclic lower alkyl group, lower alkyl group substituted by one, two, or more halogen atoms, optionally substituted heterocycle, or substituted phenyl group; R³ is a hydrogen atom or halogen atom; Y¹ is an oxygen atom, -NR⁸-, or methylene group; R⁴ is a lower alkyl group, optionally substituted cyclic lower alkyl group, difluoromethyl group, or trifluoromethyl group; R⁵ is a hydrogen atom, lower alkyl group, or optionally substituted cyclic lower alkyl group; R⁶ is a hydrogen atom, lower alkyl group, optionally substituted cyclic lower alkyl group, difluoromethyl group, or trifluoromethyl group; R⁷⁻¹ and R⁷⁻² may be the same or different and are each independently a hydrogen atom, lower alkyl group, or optionally substituted cyclic lower alkyl group; R⁸ is a hydrogen atom, lower alkyl group, or optionally substituted cyclic lower alkyl group; n is 0, 1, or 2; and the "*" symbol denotes an asymmetric carbon); or a salt thereof.

2. The compound or salt thereof of Claim 1 in which R¹ is a lower alkyl group, optionally substituted cyclic lower alkyl group, halogen atom, trifluoromethyl group, -OR⁴-, -CH₂OR⁶, or -(CH₂)₂CONR⁷⁻¹R⁷⁻²; R² is an optionally substituted cyclic lower alkyl group, optionally substituted heterocycle, or substituted phenyl group; R³ is a hydrogen atom, fluorine atom, or chlorine atom; Y¹ is an oxygen atom, -NH-, or methylene group; R⁴ is a lower alkyl group, optionally substituted cyclic lower alkyl group, difluoromethyl group, or trifluoromethyl group; R⁶ is a hydrogen atom, lower alkyl group, or optionally substituted cyclic lower alkyl group; and R⁷⁻¹ and R⁷⁻² may be the same or different and are each independently a hydrogen atom, lower alkyl group, or optionally substituted cyclic lower alkyl group.

3. The compounds or salt thereof of Claim 1 in which R¹ is a lower alkyl group, optionally substituted cyclic lower alkyl group, halogen atom, trifluoromethyl group, -OR⁴-, -CH₂OH, or -(CH₂)₂CONR⁷⁻¹R⁷⁻²; R² is an optionally substituted cyclic lower alkyl group, optionally substituted heterocycle, or substituted phenyl group; R³ is a hydrogen atom, fluorine atom, or chlorine atom; Y¹ is an oxygen atom; R⁴ is a lower alkyl group, optionally substituted cyclic lower alkyl group, difluoromethyl group, or trifluoromethyl group; and R⁷⁻¹ and R⁷⁻² may be the same or different and are each independently a hydrogen atom, lower alkyl group, or optionally substituted cyclic lower alkyl group.

4. The compound or salt thereof of any of Claims 1-3 in which R¹ is a methyl group, ethyl group, isopropyl group, cyclopropyl group, cyclobutyl group, chlorine atom, trifluoromethyl group, -OMe, or -OCHF₂.

5. The compound or salt thereof of any of Claims 1-4 in which R² is an optionally substituted cyclic lower alkyl group, group shown by any of the following formulas (V-I)-(V-V) 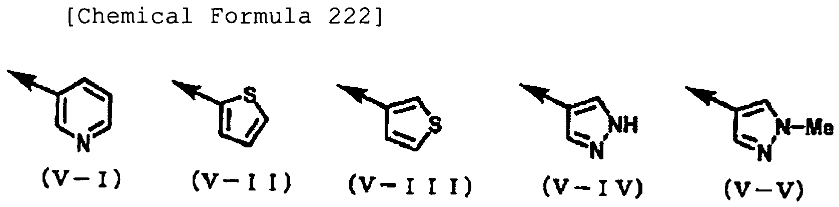 or group shown by any of the following formulas (VII-I)-(VII-XI)

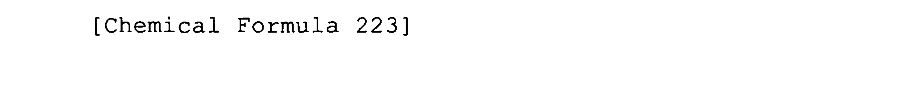

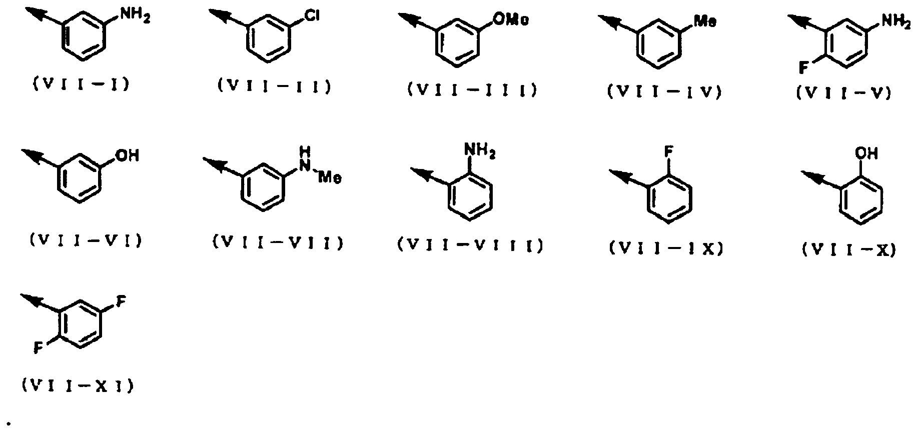

6. The compound or salt thereof of any of Claims 1-5 in which R² is an optionally substituted cyclic lower alkyl group.

7. The compound or salt thereof of any of Claims 1-5 in which R² is a group shown by any of the following formulas (V-I)-(V-V) 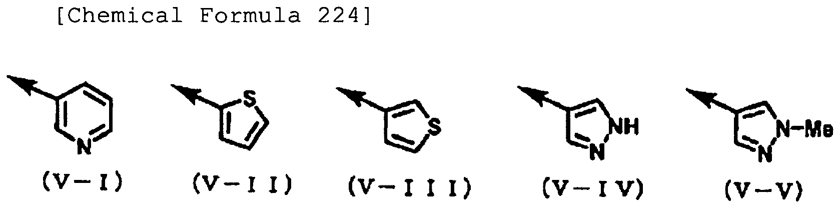 or a group shown by any of the following formulas (VII-I)-(VII-V) and (VII-IX)

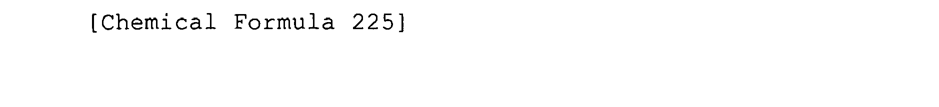

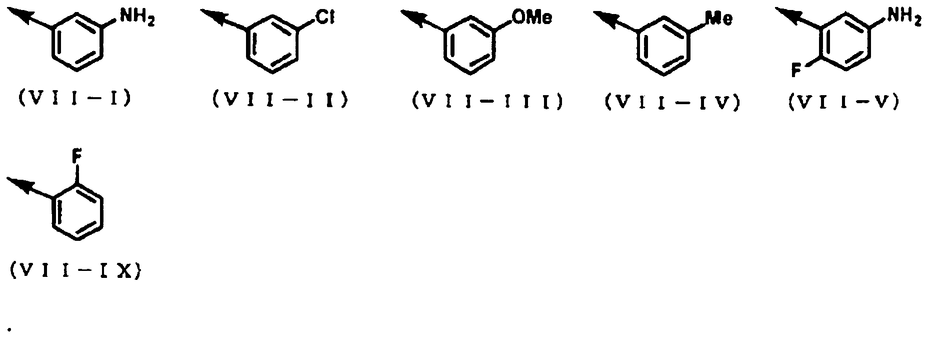

8. The compound or salt thereof of any of Claims 1-6 in which, when R² is a cyclobutyl group or cyclopentyl group, R¹ is a methyl group, isopropyl group, cyclopropyl group, cyclobutyl group, chlorine atom, or -OCHF₂.

9. The compound or salt thereof of any of Claims 1-6 in which, when R² is a cyclopropyl group, R¹ is a methyl group, ethyl group, isopropyl group, cyclopropyl group, cyclobutyl group, chlorine atom, trifluoromethyl group, -OMe, or -OCHF₂.

10. The compound or salt thereof of any of Claims 1, 2, or 4-9 in which Y¹ is an oxygen atom.

11. The compound or salt thereof of any of Claims 1-10 in which R³ is a hydrogen atom.

12. The compound or salt thereof of any of Claims 1-11 in which R¹ is a methyl group.

13. The compound or salt thereof of any of Claims 1-11 in which R¹ is -OCHF₂.

14. The compound or salt thereof of Claim 1 which R¹ is a lower alkyl group, optionally substituted cyclic lower alkyl group, chlorine atom, trifluoromethyl group, -OR₄, -CH₂OH, or -(CH₂)₂CONMe₂; R² is an optionally substituted cyclic lower alkyl group, optionally substituted heterocycle, or substituted phenyl group; R³ is a hydrogen atom, fluorine atom, or chlorine atom; Y¹ is an oxygen atom; and R⁴ is a lower alkyl group, optionally substituted cyclic lower alkyl group, difluoromethyl group, or trifluoromethyl group.

15. The compound or salt thereof of Claim 1 in which R¹ is a lower alkyl group, optionally substituted cyclic lower alkyl group, chlorine atom, trifluoromethyl group, -OR⁴, or - (CH₂)₂CONMe₂, R² is a cyclopropyl group, cyclobutyl group, optionally substituted heterocycle, or substituted phenyl group; R³ is a hydrogen atom, fluorine atom, or chlorine atom; Y¹ is an oxygen atom; and R⁴ is a lower alkyl group, cyclic lower alkyl group, or difluoromethyl group.

16. The compound or salt thereof of Claim 1 in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is an optionally substituted cyclic lower alkyl group, optionally substituted heterocycle, or substituted phenyl group; R³ is a hydrogen atom, fluorine atom, or chlorine atom; and Y¹ is an oxygen atom.

17. The compound or salt thereof of Claim 1 in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group, cyclobutyl group, optionally substituted heterocycle, or substituted phenyl group; R³ is a hydrogen atom, fluorine atom, or chlorine atom; and Y¹ is an oxygen atom.

18. The compound or salt thereof of Claim 1 in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group, cyclobutyl group, optionally substituted heterocycle, or substituted phenyl group; R³ is a hydrogen atom; and Y¹ is an oxygen atom.

19. The compound or salt thereof of Claim 1 in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group, cyclobutyl group, group shown by any of the following formulas (V-I)-(V-V) 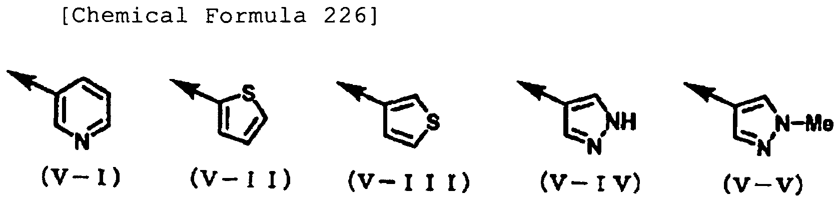 or group shown by any of the following formulas (VII-I)-(VII-X) 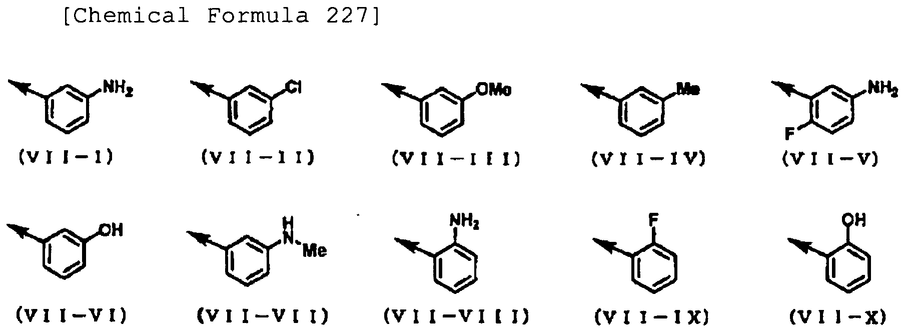 ; R³ is a hydrogen atom; and Y¹ is an oxygen atom.

20. The compound or salt thereof of Claim 1 in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a cyclopropyl group or cyclobutyl group; R³ is a hydrogen atom; and Y¹ is an oxygen atom.

21. The compound or salt thereof of Claim 1 in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a group shown by any of the following formulas (V-I)-(V-V) 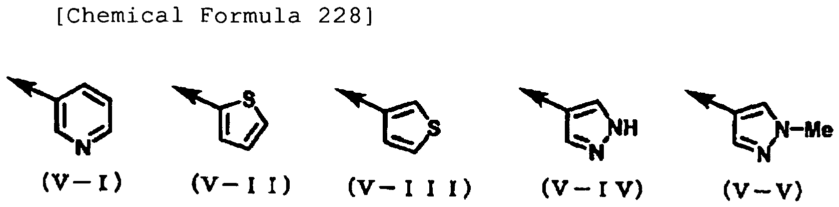 ; R³ is a hydrogen atom; and Y¹ is an oxygen atom.

22. The compound or salt thereof of Claim 1 in which R¹ is a methyl group, ethyl group, isopropyl group, cyclopropyl group, cyclobutyl group, chlorine atom, trifluoromethyl group, -OMe, -OCHF₂, or -(CH₂)₂CONMe₂; R² is a group shown by any of the following formulas (VII-I)-(VII-X) 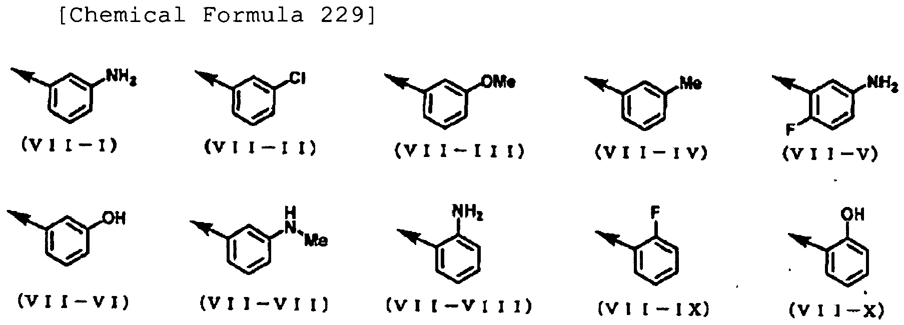 ; R³ is a hydrogen atom; and Y¹ is an oxygen atom.

23. The compound or salt thereof of Claim 1 in which R¹ is a methyl group or -OCHF₂; R² is a cyclopropyl group, cyclobutyl group, or 2-fluorophenyl group; R³ is a hydrogen atom; and Y¹ is an oxygen atom.

24. The compound or salt thereof of Claim 1 in which R¹ is a methyl group or -OCHF₂; R² is a cyclopropyl group or cyclobutyl group; R³ is a hydrogen atom; and Y¹ is an oxygen atom.

25. The compound or salt thereof of Claim 1 in which R¹ is a methyl group, chlorine atom, trifluoromethyl group, or -OMe; R² is a group shown by the following formulas (V-II), (V-III), (VII-I), or (VII-IX) 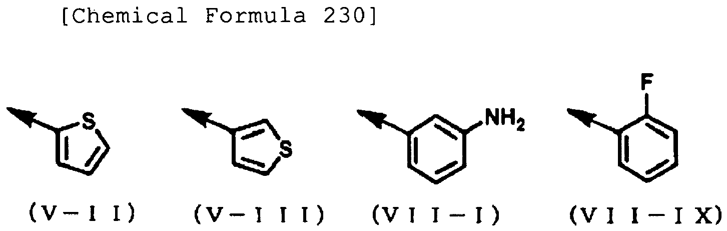 ; R³ is a hydrogen atom, fluorine atom, or chlorine atom; and Y¹ is an oxygen atom.

26. The compound or salt thereof of Claim 1 in which the configuration of the asymmetric carbon shown by the "*" symbol is (R).

27. A compound selected from the group consisting of
(R)-3-amino-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide;
(R)-2-fluoro-N-(3-(1-hydroxy-2-(2-(3-methoxyindazol-6-yloxy)ethylamino)ethyl)phenyl)benzenesulfonamide;
(R)-5-amino-2-fluoro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-3-sulfonamide;
(R)-N-(3-(1-hydroxy-2-(2-(3-methoxyindazol-6-yloxy)ethylamino)ethyl)phenyl)pyridine-3-sulfonamide;
(R)-3-chloro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)cyclopropane sulfonamide;
(R)-3-amino-N-(3-(1-hydroxy-2-(2-(3-methylindazol-6-yloxy)ethylamino)ethyl)phenyl)benzenesulfonamide;
(R)-3-chloro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-methoxybenzenesulfonamide;
(R)-N-(3-(1-hydroxy-2-(2-(3-methylindazol-6-yloxy)ethylamino)ethyl)phenyl)pyridine-3-sulfonamide;
(R)-N-(3-(1-hydroxy-2-(2-(3-methylindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)thiophene-3-sulfonamide;
(R)-N-(3-(1-hydroxy-2-(2-(3-methylindazol-6-yloxy)ethylamino)ethyl)phenyl)cyclopropane sulfonamide;
(R)-N-(3-(1-hydroxy-2-(2-(3-methylindazol-6-yloxy)ethylamino)ethyl)phenyl)-3-methylbenzenesulfonamide;
(R)-2-amino-N-(3-(1-hydroxy-2-(2-(3-methylindazol-6-yloxy)ethylamino)ethyl)phenyl)benzenesulfonamide;
(R)-5-amino-2-fluoro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-1-methylpyrazole-4-sulfonamide;
(R)-N-(3-(1-hydroxy-2-(2-(3-methylindazol-6-yloxy)ethylamino)ethyl)phenyl)pyrazole-4-sulfonamide;
(R)-3-amino-N-(3-(2-(2-(3-(difluoromethoxy)-indazol-6-yloxy)ethylamino)-1-hydroxyethyl)phenyl)benzenesulfonamide;
(R)-N-(3-(2-(2-(3-(difluoromethoxy)-indazol-6-yloxy)ethylamino)-1-hydroxyethyl)phenyl)pyrazole-4-sulfonamide;
(R)-3-amino-N-(3-(2-(2-(3-ethylindazol-6-yloxy)ethylamino)-1-hydroxyethyl)phenyl)benzenesulfonamide;
(R)-N-(3-(2-(2-(3-ethylindazol-6-yloxy)ethylamino)-1-hydroxyethyl)phenyl)cyclopropane sulfonamide;
(R)-N-(3-(2-(2-(3-ethylindazol-6-yloxy)ethylamino)-1-hydroxyethyl)phenyl)-3-methylbenzenesulfonamide;
(R)-N-(3-(2-(2-(3-ethylindazol-6-yloxy)ethylamino)-1-hydroxyethyl)phenyl)-1-methylpyrazole-4-sulfonamide;
(R)-N-(3-(2-(2-(3-ethylindazol-6-yloxy)ethylamino)-1-hydroxyethyl)phenyl)pyrazole-4-sulfonamide;
(R)-N-(3-(2-(2-(3-chloroindazol-6-yloxy)ethylamino)-1-hydroxyethyl)phenyl)cyclopropane sulfonamide;
(R)-3-amino-N-(3-(2-(2-(3-chloroindazol-6-yloxy)ethylamino)-1-hydroxyethyl)phenyl)benzenesulfonamide;
(R)-N-(3-(1-hydroxy-2-(2-(3-(trifluoromethyl)-indazol-6-yloxy)ethylamino)ethyl)phenyl)cyclopropane sulfonamide;
(R)-N-(3-(2-(2-(3-cyclopropylindazol-6-yloxy)ethylamino)-1-hydroxyethyl)phenyl)cyclopropane sulfonamide;
(R)-3-amino-N-(3-(1-hydroxy-2-(2-(3-isopropylindazol-6-yloxy)ethylamino)ethyl)phenyl)benzenesulfonamide;
(R)-3-(6-(2-(2-(3-(3-amino-phenylsulfonamide)phenyl)-2-hydroxyethylamino)ethoxy)indazol-3-yl)-N,N-dimethylpropanamide;
(R)-2-fluoro-N-(3-(1-hydroxy-2-(2-(3-methylindazol-6-yloxy)ethylamino)ethyl)phenyl)benzenesulfonamide;
(R)-3-hydroxy-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-(methylamino)-benzenesulfonamide;
(R)-2-hydroxy-N-(3-(1-hydroxy-2-(2-(3-methylindazol-6-yloxy)ethylamino)ethyl)phenyl)benzenesulfonamide;
(R)-N-(3-(2-(2-(3-cyclobutylindazol-6-yloxy)ethylamino)-1-hydroxyethyl)phenyl)cyclobutane sulfonamide;
(R)-N-(3-(2-(2-(3-cyclobutylindazol-6-yloxy)ethylamino)-1-hydroxyethyl)phenyl)cyclopropane sulfonamide;
(R)-N-(3-(2-(2-(3-chloroindazol-6-yloxy)ethylamino)-1-hydroxyethyl)phenyl)-2-fluorobenzenesulfonamide;
(R)-2-fluoro-N-(3-(1-hydroxy-2-(2-(3-(trifluoromethyl)-indazol-6-yloxy)ethylamino)ethyl)phenyl)benzenesulfonamide; and
(R)-2,5-difluoro-N-(3-(1-hydroxy-2-(2-(3-methylindazol-6-yloxy)ethylamino)ethyl)phenyl)benzenesulfonamide;
or a salt thereof.

28. A compound selected from the group consisting of
(R)-3-amino-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide;
(R)-2-fluoro-N-(3-(1-hydroxy-2-(2-(3-methoxyindazol-6-yloxy)ethylamino)ethyl)phenyl)benzenesulfonamide;
(R)-5-amino-2-fluoro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)thiophene-3-sulfonamide;
(R)-N-(3-(1-hydroxy-2-(2-(3-methoxyindazol-6-yloxy)ethylamino)ethyl)phenyl)pyridine-3-sulfonamide;
(R)-3-chloro-N-(3-(1-hydroxy-2-(2-(3-methoxyindazol-6-yloxy)ethylamino)ethyl)phenyl)benzenesulfonamide;
(R)-N-(3-(1-hydroxy-2-(2-(3-methoxyindazol-6-yloxy)ethylamino)ethyl)phenyl)cyclopropane sulfonamide;
(R)-3-amino-N-(3-(1-hydroxy-2-(2-(3-methylindazol-6-yloxy)ethylamino)ethyl)phenyl)benzenesulfonamide;
(R)-3-chloro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-methoxybenzenesulfonamide;
(R)-N-(3-(1-hydroxy-2-(2-(3-methylindazol-6-yloxy)ethylamino)ethyl)phenyl)pyridine-3-sulfonamide;
(R)-N-(3-(1-hydroxy-2-(2-(3-methylindazol-6-yloxy)ethylamino)ethyl)phenyl)thiophene-2-sulfonamide;
(R)-N-(3-(1-hydroxy-2-(2-(3-methylindazol-6-yloxy)ethylamino)ethyl)phenyl)thiophene-3-sulfonamide;
(R)-N-(3-(1-hydroxy-2-(2-(3-methylindazol-6-yloxy)ethylamino)ethyl)phenyl)cyclopropane sulfonamide;
(R)-N-(3-(1-hydroxy-2-(2-(3-methylindazol-6-yloxy)ethylamino)ethyl)phenyl)-3-methylbenzenesulfonamide;
(R)-2-amino-N-(3-(1-hydroxy-2-(2-(3-methylindazol-6-yloxy)ethylamino)ethyl)phenyl)benzenesulfonamide;
(R)-5-amino-2-fluoro-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-1-methylpyrazole-4-sulfonamide;
(R)-N-(3-(1-hydroxy-2-(2-(3-methylindazol-6-yloxy)ethylamino)ethyl)phenyl)pyrazole-4-sulfonamide;
(R)-3-amino-N-(3-(2-(2-(3-(difluoromethoxy)-indazol-6-yloxy)ethylamino)-1-hydroxyethyl)phenyl)benzenesulfonamide;
(R)-N-(3-(2-(2-(3-(difluoromethoxy)-indazol-6-yloxy)ethylamino)-1-hydroxyethyl)phenyl)pyrazole-4-sulfonamide;
(R)-3-amino-N-(3-(2-(2-(3-ethylindazol-6-yloxy)ethylamino)-1-hydroxyethyl)phenyl)benzenesulfonamide;
(R)-N-(3-(2-(2-(3-ethylindazol-6-yloxy)ethylamino)-1-hydroxyethyl)phenyl)cyclopropane sulfonamide;
(R)-N-(3-(2-(2-(3-ethylindazol-6-yloxy)ethylamino)-1-hydroxyethyl)phenyl)-3-methylbenzenesulfonamide;
(R)-N-(3-(2-(2-(3-ethylindazol-6-yloxy)ethylamino)-1-hydroxyethyl)phenyl)-1-methylpyrazole-4-sulfonamide;
(R)-N-(3-(2-(2-(3-ethylindazol-6-yloxy)ethylamino)-1-hydroxyethyl)phenyl)pyrazole-4-sulfonamide;
(R)-N-(3-(2-(2-(3-chloroindazol-6-yloxy)ethylamino)-1-hydroxyethyl)phenyl)cyclopropane sulfonamide;
(R)-3-amino-N-(3-(2-(2-(3-chloroindazol-6-yloxy)ethylamino)-1-hydroxyethyl)phenyl)benzenesulfonamide;
(R)-N-(3-(1-hydroxy-2-(2-(3-(trifluoromethyl)-indazol-6-yloxy)ethylamino)ethyl)phenyl)cyclopropane sulfonamide;
(R)-N-(3-(2-(2-(3-cyclopropylindazol-6-yloxy)ethylamino)-1-hydroxyethyl)phenyl)cyclopropane sulfonamide;
(R)-3-amino-N-(3-(1-hydroxy-2-(2-(3-isopropylindazol-6-yloxy)ethylamino)ethyl)phenyl)benzenesulfonamide;
(R)-3-(6-(2-(2-(3-(3-amino-phenylsulfonamide)phenyl)-2-hydroxyethylamino)ethoxy)indazol-3-yl)-N,N-dimethylpropanamide;
(R)-2-fluoro-N-(3-(1-hydroxy-2-(2-(3-methylindazol-6-yloxy)ethylamino)ethyl)phenyl)benzenesulfonamide;
(R)-3-hydroxy-N-(3-(1-hydroxy-2-(2-(3-methylindazol-6-yloxy)ethylamino)ethyl)phenyl)benzenesulfonamide;
(R)-N-(3-(1-hydroxy-2-(2-(3-methylindazol-6-yloxy)ethylamino)ethyl)phenyl)-3-(methylamino)-benzenesulfonamide; and
(R)-2-hydroxy-N-(3-(1-hydroxy-2-(2-(3-methylindazol-6-yloxy)ethylamino)ethyl)phenyl)benzenesulfonamide; or a salt thereof.

29. A compound selected from the group consisting of
(R)-N-(3-(1-hydroxy-2-(2-(3-methylindazol-6-yloxy)ethylamino)ethyl)phenyl)cyclobutane sulfonamide;
(R)-N-(3-(2-(2-(3-(difluoromethoxy)-indazol-6-yloxy)ethylamino)-1-hydroxyethyl)phenyl)cyclopropane sulfonamide; and
(R)-2-fluoro-N-(3-(1-hydroxy-2-(2-(3-methylindazol-6-yloxy)ethylamino)ethyl)phenyl)benzenesulfonamide; or a salt thereof.

30. A compound selected from the group consisting of
(R)-N-(3-(1-hydroxy-2-(2-(3-methylindazol-6-yloxy)ethylamino)ethyl)phenyl)cyclobutane sulfonamide; and
(R)-N-(3-(2-(2-(3-(difluoromethoxy)-indazol-6-yloxy)ethylamino)-1-hydroxyethyl)phenyl)cyclopropane sulfonamide; or a salt thereof.

31. A compound selected from the group consisting of
(R)-N-(3-(2-(2-(3-(difluoromethoxy)-indazol-6-yloxy)ethylamino)-1-hydroxyethyl)phenyl)cyclopropane sulfonamide and
(R)-2-fluoro-N-(3-(1-hydroxy-2-(2-(3-methylindazol-6-yloxy)ethylamino)ethyl)phenyl)benzenesulfonamide; or a salt thereof.

32. A compound selected from the group consisting of
(R)-N-(3-(1-hydroxy-2-(2-(3-methylindazol-6-yloxy)ethylamino)ethyl)phenyl)cyclobutane sulfonamide; and
(R)-2-fluoro-N-(3-(1-hydroxy-2-(2-(3-methylindazol-6-yloxy)ethylamino)ethyl)phenyl)benzenesulfonamide; or a salt thereof.

33. A β3-adrenergic receptor agonist containing the compound described in any one of Claims 1-32 or a salt thereof as the active ingredient.

34. A drug containing the compound described in any one of Claims 1-32 or a salt thereof as the active ingredient.

35. The drug of Claim 34 that is a drug to prevent and/or treat overactive bladder and urinary incontinence.

36. A method for acting on a β3-adrenergic receptor in the body of a patient **characterized in that** the compound described in any one of Claims 1-32 or a salt thereof is administered to a patient who requires prevention and/or treatment of overactive bladder and urinary incontinence.

37. A method for preventing and/or treating overactive bladder and urinary incontinence **characterized in that** an effective dose of the compound described in any one of Claims 1-32, or a salt thereof, is administered to a patient.
